# EUROPEAN PATENT APPLICATION

(11) **EP 2 194 045 A1**
(43) Date of publication of application: **09.06.2010**
(21) Application number: 08828418.7
(22) Date of filing: 27.08.2008
(51) Int. Cl.: C07D 231/12, A61K 31/415, A61K 31/4162, A61K 31/4178, A61K 31/4192, A61K 31/4196, A61K 31/42, A61K 31/422, A61K 31/427, A61K 31/433, A61K 31/4439, A61K 31/4545, A61K 31/4709, A61K 31/497, A61K 31/498, A61K 31/506, A61K 31/5377, A61P 5/28, A61P 13/08, A61P 35/00, A61P 43/00

(54) **SUBSTITUTED PYRAZOLE DERIVATIVE**

(30) Priority: 30.08.2007 JP 2007224910
(71) Applicant: Takeda Pharmaceutical Company Limited, Osaka-shi, Osaka 541-0045 (JP)
(72) Inventor: ITO, Mitsuhiro, Tsukuba-shi Ibaraki 300-4293 (JP); SUZAKI, Tomohiko, Osaka-shi Osaka 532-8686 (JP); YAMAMOTO, Satoshi, Osaka-shi Osaka 532-8686 (JP)
(74) Representative: Wright, Robert Gordon McRae
(86) International application number: PCT/JP2008/065286
(87) International publication number: WO 2009/028543

(57) **Abstract**

The present invention provides a novel pyrazole derivative and an androgen receptor antagonist containing the derivative. The present invention provides a compound represented by the formula (I') 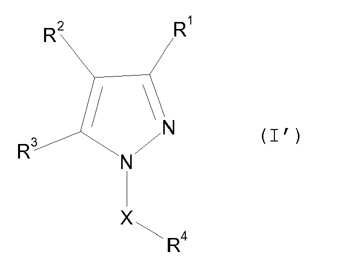 wherein R¹ is a hydrogen atom, a group via a carbon atom, a group via a nitrogen atom, a group via an oxygen atom, or a group via a sulfur atom; R² is a phenyl group having cyano (the phenyl group may further have substituent(s) other than cyano); R³ is a hydrogen atom, a group via a carbon atom, a group via a nitrogen atom, a group via an oxygen atom, or a group via a sulfur atom; R⁴ is a cyclic group optionally having substituent(s); and X is methylene optionally having substituent(s), or CO, or a salt thereof.

## Description

### Technical Field

The present invention relates to a novel pyrazole derivative and an androgen receptor antagonist containing the derivative, more particularly, the invention relates to a novel pyrazole derivative having a prophylactic.therapeutic effect on diseases dependent on androgen, which effect resulting from the inhibition of the receptor of androgen (AR), an androgenic hormone, and showing an androgen receptor antagonistic action free of an influence of mutation and the like, and an androgen receptor antagonist comprising the derivative.

### Background of the Invention

Patent references 1 to 4 by the Applicant of the present invention disclose pyrrole derivatives having an androgen receptor binding inhibitory action.
In addition, patent references 5 to 14 and non-patent references 1 to 4 describe substituted pyrazole derivatives.
patent reference 1: WO 03/57669
patent reference 2: WO 2006/064944
patent reference 3: JP 2006-163636
patent reference 4: PCT/JP2007/062194
patent reference 5: JP-A-4-189183
patent reference 6: JP-A-2000-305295
patent reference 7: National Publication of International
Patent Application No. 2003-502329
patent reference 8: WO01/07413
patent reference 9: National Publication of International
Patent Application No. 2004-502772
patent reference 10: National Publication of International
Patent Application No. 2005-516934
patent reference 11: National Publication of International
Patent Application No. 2005-532291
patent reference 12: US-A-2005/277647
patent reference 13: WO2007/027842
patent reference 14: WO2007/052943
non-patent reference 1: Journal of Chemical Research, Synopses (1998), (11), 690-691, 2946-2957
non-patent reference 2: Bioorganic & Medicinal Chemistry Letters 14 (2004), 4949-4953
non-patent reference 3: J. Med. Chem. 2004, 47, 4645-4648
non-patent reference 4: Archiv der Pharmazie (Weinheim Germany) (2006), 339(6), 305-312

### Disclosure of the Invention

### Problems to be Solved by the Invention

The main object of the present invention is to provide a novel pyrazole derivative exhibiting a superior androgen receptor antagonistic action.

### Means of Solving the Problems

The present inventors have conducted various studies of pyrazole derivatives having an androgen receptor antagonistic action and found that the following novel pyrazole derivative unexpectedly shows a superior androgen receptor antagonistic action, superior pharmacokinetics and the like, or decreased toxicity, which resulted in the completion of the present invention.

Accordingly, the present invention provides
[1] a compound represented by the formula (I')

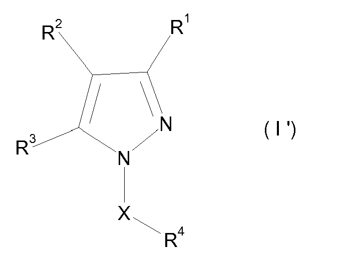

wherein
R¹ is a hydrogen atom, a group via a carbon atom, a group via a nitrogen atom, a group via an oxygen atom, or a group via a sulfur atom;
R² is a phenyl group having cyano (the phenyl group optionally further has substituent(s) other than cyano);
R³ is a hydrogen atom, a group via a carbon atom, a group via a nitrogen atom, a group via an oxygen atom, or a group via a sulfur atom;
R⁴ is a cyclic group optionally having substituent(s); and
X is CO or methylene optionally having substituent(s)
(hereinafter sometimes to be abbreviated as compound (I')),
or a salt thereof;
[2] the compound of the above-mentioned [1], wherein the cyclic group optionally having substituent(s) for R⁴ is phenyl, 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrimidyl, 5-imidazolyl, 3-pyrazolyl, 4-pyrazolyl, 5-pyrazolyl, 4-isoxazolyl, 5-isoxazolyl, 4-oxazolyl, 1,2,3-triazol-4-yl, 5-thiadiazolyl, 3-indolyl, 6-quinolyl, 5-benzotriazolyl, 2-benzothienyl, 3-benzothienyl, thienopyrazol-5-yl, 2-tetrahydrofuryl, 2-tetrahydropyranyl, piperidin-3-yl, 4-dihydrobenzofuranyl, 7-dihydrobenzofuranyl, 1,3-benzodioxol-5-yl, 1,4-benzodioxan-5-yl, 1,4-benzodioxan-6-yl, 1,5-benzodioxepin-6-yl, 1,5-benzodioxepin-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl or 3,4-dihydro-2H-1,5-benzodioxepin-7-yl;
[3] a compound represented by the formula (IA)

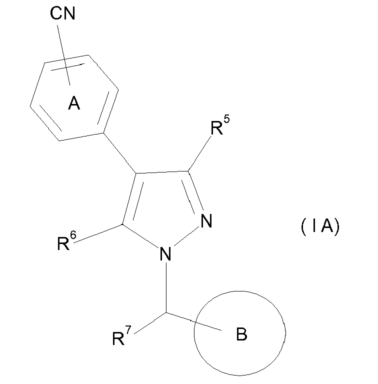

wherein
ring A is a benzene ring optionally further having substituent(s) other than cyano;
ring B is an aromatic hydrocarbon ring optionally having substituent(s) or heterocycle optionally having substituent(s);
R⁵ and R⁶ are the same or different and each is a hydrogen atom, a cyano group, a C₁₋₆ alkyl group optionally having substituent(s), -COORₐ, -CONRₐR_{b} or -NRₐCOR_{b};
Rₐ and R_{b} are the same or different and each is a hydrogen atom, a C₁₋₆ alkyl group optionally having substituent(s), a C₃₋₇ cycloalkyl group or a phenyl group optionally having substituent(s); and
R⁷ is a hydrogen atom, a C₁₋₆ alkyl group, a C₃₋₇ cycloalkyl group, or a phenyl group optionally having substituent(s)
(hereinafter sometimes to be abbreviated as compound (IA)),
or a salt thereof;
[4] the compound of the above-mentioned [3], wherein ring A is a benzene ring optionally further having substituent(s) other than cyano, which is/are selected from a halogen atom, a C₁₋₆ alkyl group, a halogeno-C₁₋₆ alkyl group, and -OR_{c} wherein R_{c} is a hydrogen atom, a C₁₋₆ alkyl group optionally having substituent(s) or a C₃₋₇ cycloalkyl group;
[5] the compound of the above-mentioned [3], wherein ring B is a benzene ring optionally having substituent(s), a pyridine ring optionally having substituent(s), a thiazole ring optionally having substituent(s) or a pyrazole ring optionally having substituent(s);
[6] the compound of the above-mentioned [3], wherein ring B is a benzene ring, a pyridine ring, a thiazole ring or a pyrazole ring, which optionally has substituent(s) selected from a halogen atom, a C₁₋₆ alkyl group optionally having substituent(s), a C₂₋₆ alkenyl group optionally having substituent(s), a C₂₋₆ alkynyl group optionally having substituent(s), a nitro group, a phenyl group optionally having substituent(s), a heterocyclic group optionally having substituent(s), a cyano group, -CONR_{d}Rₑ, -CO₂R_{d}, -NR_{d}CORₑ, - SO₂NR_{d}Rₑ, -NR_{d}SO₂Rₑ, -C(OH)R_{d}Rₑ, -NR_{d}Rₑ, -OR_{d} and -SR_{d} wherein R_{d} and Rₑ are the same or different and each is a hydrogen atom, a C₁₋₆ alkyl group optionally having substituent(s), a C₃₋₇ cycloalkyl group or a phenyl group optionally having substituent (s) ;
[7] the compound of the above-mentioned [3], wherein R⁵ and R⁶ are the same or different and each is a hydrogen atom, a cyano group, a C₁₋₆ alkyl group, a halogeno-C₁₋₆ alkyl group, -CONRₐR_{b} or -NRₐCOR_{b};
[8] 3-{[4-(3-chloro-4-cyanophenyl)-3,5-dimethyl-1H-pyrazol-1-yl]methyl}benzamide, or a salt thereof;
[9] 4-{[4-(3-chloro-4-cyanophenyl)-3,5-dimethyl-1H-pyrazol-1-yl]methyl}benzamide, or a salt thereof;
[10] 5-{[4-(3-chloro-4-cyanophenyl)-3,5-dimethyl-1H-pyrazol-1-yl]methyl}-2-fluorobenzamide, or a salt thereof;
[11] 5-{[4-(3-chloro-4-cyanophenyl)-3,5-dimethyl-1H-pyrazol-1-yl]methyl}pyridine-2-carboxamide, or a salt thereof;
[12] 5-{[4-(3-chloro-4-cyanophenyl)-3,5-dimethyl-1H-pyrazol-1-yl]methyl}-N-methylpyridine-2-carboxamide, or a salt thereof;
[13] 4-{[4-(3-chloro-4-cyanophenyl)-3,5-dimethyl-1H-pyrazol-1-yl]methyl}-N-(2-hydroxy-2-methylpropyl)benzamide, or a salt thereof;
[14] 2-chloro-4-f3,5-dimethyl-l-[4-(5-methyl-1,3,4-oxadiazol-2-yl)benzyl]-1H-pyrazol-4-yl}benzonitrile, or a salt thereof;
[15] 4-{[4-(3-chloro-4-cyanophenyl)-3,5-dimethyl-1H-pyrazol-1-yl]methyl}-N-cyclopropylbenzamide, or a salt thereof;
[16] 5-{[4-(3-chloro-4-cyanophenyl)-3,5-dimethyl-1H-pyrazol-1-yl]methyl}-N-cyclopropylpyridine-2-carboxamide, or a salt thereof;
[17] 5-({4-[4-cyano-3-(trifluoromethyl)phenyl]-3,5-dimethyl-1H-pyrazol-1-yl}methyl)pyridine-2-carboxamide, or a salt thereof;
[18] 4-{[4-(3-chloro-4-cyanophenyl)-3,5-dimethyl-1H-pyrazol-1-yl]methyl}benzenesulfonamide, or a salt thereof;
[19] 4-{[4-(3-chloro-4-cyano-2-fluorophenyl)-3,5-dimethyl-1H-pyrazol-1-yl]methyl}benzamide, or a salt thereof;
[20] 2-chloro-4-(3,5-dimethyl-1-{[6-(5-methyl-1,3,4-oxadiazol-2-yl)pyridin-3-yl]methyl}-1H-pyrazol-4-yl)benzonitrile, or a salt thereof;
[21] a prodrug of the compound of the above-mentioned [1] or [3];
[22] a pharmaceutical agent comprising the compound of the above-mentioned [1] or [3], or a prodrug thereof;
[23] the pharmaceutical agent of the above-mentioned [22], which is an androgen receptor antagonist;
[24] the pharmaceutical agent of the above-mentioned [22], wherein the androgen receptor is a normal androgen receptor and/or a mutant androgen receptor;
[25] the pharmaceutical agent of the above-mentioned [22], which is an agent for the prophylaxis or treatment of hormone sensitive cancer in the androgen-dependent phase and/or the androgen-independent phase;
[26] the pharmaceutical agent of the above-mentioned [22], which is an agent for the prophylaxis or treatment of prostate cancer;
[27] a method of antagonizing an androgen receptor, comprising administering an effective amount of the compound of the above-mentioned [1] or [3], or a prodrug thereof to a mammal;
[28] a method of preventing · treating a hormone sensitive cancer in the androgen-dependent phase and/or the androgen-independent phase, comprising administering an effective amount of the compound of the above-mentioned [1] or [3], or a prodrug thereof to a mammal;
[29] a method of preventing · treating prostate cancer, comprising administering an effective amount of the compound of the above-mentioned [1] or [3], or a prodrug thereof to a mammal;
[30] use of the compound of the above-mentioned [1] or [3], or a prodrug thereof for the production of an androgen receptor antagonist;
[31] use of the compound of the above-mentioned [1] or [3], or a prodrug thereof for the production of an agent for the prophylaxis·treatment of a hormone sensitive cancer in the androgen-dependent phase and/or the androgen-independent phase; and
[32] use of the compound of the above-mentioned [1] or [3], or a prodrug thereof for the production of an agent for the prophylaxis·treatment of prostate cancer.

The present invention also provides
[1] a compound represented by the formula

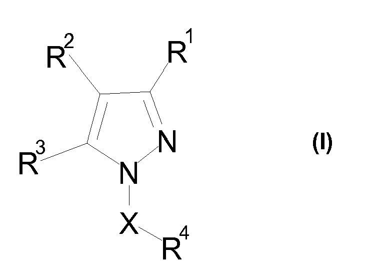

wherein
R¹ is a hydrogen atom, a group via a carbon atom, a group via a nitrogen atom, a group via an oxygen atom, or a group via a sulfur atom;
R² is a cyclic group having cyano (the cyclic group optionally further has substituent(s) other than cyano);
R³ is a hydrogen atom, a group via a carbon atom, a group via a nitrogen atom, a group via an oxygen atom, or a group via a sulfur atom;
R⁴ is a cyclic group optionally having substituent(s); and X is CO, SO₂ or methylene optionally having substituent(s)
(hereinafter sometimes to be abbreviated as compound (I)),
or a salt thereof;
[2] a prodrug of the compound of the above-mentioned [1];
[3] a pharmaceutical agent comprising the compound of the above-mentioned [1], or a salt thereof or a prodrug thereof;
[4] the pharmaceutical agent of the above-mentioned [3], which is an androgen receptor antagonist;
[5] the pharmaceutical agent of the above-mentioned [4], wherein the androgen receptor is a normal androgen receptor and/or a mutant androgen receptor;
[6] the pharmaceutical agent of the above-mentioned [3], which is an agent for the prophylaxis or treatment of hormone sensitive cancer in the androgen-dependent phase and/or the androgen-independent phase;
[7] the pharmaceutical agent of the above-mentioned [3], which is an agent for the prophylaxis or treatment of prostate cancer;
[8] a method of antagonizing an androgen receptor, comprising administering an effective amount of the compound of the above-mentioned [1], or a salt thereof, or a prodrug thereof to a mammal;
[9] a method of preventing · treating a hormone sensitive cancer in the androgen-dependent phase and/or the androgen-independent phase, comprising administering an effective amount of the compound of the above-mentioned [1] or a salt thereof or a prodrug thereof to a mammal;
[10] a method of preventing · treating prostate cancer, comprising administering an effective amount of the compound of the above-mentioned [1] or a salt thereof, or a prodrug thereof to a mammal;
[11] use of the compound of the above-mentioned [1], or a salt thereof, or a prodrug thereof for the production of an androgen receptor antagonist;
[12] use of the compound of the above-mentioned [1], or a salt thereof, or a prodrug thereof for the production of an agent for the prophylaxis·treatment of a hormone sensitive cancer in the androgen-dependent phase and/or the androgen-independent phase; and
[13] use of the compound of the above-mentioned [1], or a salt thereof, or a prodrug thereof for the production of an agent for the prophylaxis·treatment of prostate cancer.

When compound (I), compound (I') and compound (IA), or salts thereof contain an asymmetric carbon in the structure, all of optically active forms and racemates are encompassed within the scope of the present invention, and these compounds and salts thereof may be either hydrates or anhydrides.

### Effect of the Invention

Compound (I), compound (I') and compound (IA), and salts thereof of the present invention show not only strong antagonistic activity against natural androgen receptors, but also high antagonistic action on mutant androgen receptors. Moreover, these compounds are useful as, for example, pharmaceutical agents effective on prostate cancer in the hormone-independent phase, which can be administered orally, show extremely low toxicity, and have an androgen receptor antagonistic action.

### Detailed Description of the Invention

In the present specification, examples of the "group via a carbon atom" include
(1) a cyano group,
(2) alkyl (C₁₋₆ alkyl) optionally having substituent(s),
(3) alkenyl (C₂₋₆ alkenyl) optionally having substituent(s),
(4) alkynyl (C₂₋₆ alkynyl) optionally having substituent(s),
(5) cycloalkyl (C₃₋₈ cycloalkyl) optionally having substituent(s),
(6) cycloalkenyl (C₃₋₈ cycloalkenyl) optionally having substituent(s),
(7) aryl (C₆₋₁₀ aryl) optionally having substituent(s),
(8) acyl,
(9) a heterocyclic group optionally having substituent(s)
(which has a bond at carbon atom),
and the like.

Examples of the "C₁₋₆ alkyl" of the above-mentioned "C₁₋₆ alkyl optionally having substituent(s)" include methyl, ethyl, propyl, isopropyl, butyl, isobutyl, sec-butyl, tert-butyl, pentyl, hexyl and the like.
Examples of the "substituent" of the above-mentioned "C₁₋₆ alkyl optionally having substituent(s)" include substituent selected from the following substituent group A. While the number of substituents is not particularly limited as long as the substituents in the designated number are substitutable, it is preferably 1 to 5, more preferably 1 to 3.
Substituent group A: substituent group consisting of
(1) a halogen atom (e.g., fluorine atom, chlorine atom, bromine atom, iodine atom etc.);
(2) cyano;
(3) nitro;
(4) hydroxy;
(5) C₃₋₈ cycloalkyl (e.g., cyclopropyl, cyclobutyl, cyclopentyl, cyclohexyl, cycloheptyl, cyclooctyl etc.) optionally having 1 to 3 halogen atoms;
(6) C₆₋₁₀ aryl (e.g., phenyl, 1-naphthyl, 2-naphthyl etc.) optionally having 1 to 3 substituents selected from a halogen atom and cyano;
(7) C₁₋₆ alkoxy (e.g., methoxy, ethoxy, propoxy, isopropoxy, butoxy, isobutoxy, tert-butoxy etc.) optionally having 1 to 3 substituents selected from a halogen atom, N-methyl-N-(2-pyrazyl)amino and 2-quinolyl;
(8) C₂₋₆ alkenyloxy (e.g., ethenyloxy, propenyloxy, butenyloxy, pentenyloxy, hexenyloxy etc.) optionally having 1 to 3 halogen atoms;
(9) C₂₋₆ alkynyloxy (e.g., ethynyloxy, propynyloxy, butynyloxy, pentynyloxy, hexynyloxy etc.) optionally having 1 to 3 halogen atoms;
(10) C₃₋₈ cycloalkyl-oxy (e.g., cyclopropyloxy, cyclobutyloxy, cyclopentyloxy, cyclohexyloxy etc.) optionally having 1 to 3 halogen atoms;
(11) C₃₋₈ cycloalkenyloxy (e.g., cyclopropenyloxy, cyclobutenyloxy, cyclopentenyloxy, cyclohexenyloxy etc.) optionally having 1 to 3 halogen atoms;
(12) C₆₋₁₀ aryloxy (e.g., phenyloxy, 1-naphthyloxy, 2-naphthyloxy etc.) optionally having 1 to 3 halogen atoms;
(13) C₃₋₈ cycloalkyl-C₁₋₆ alkoxy (e.g., cyclopropylmethyloxy, cyclopropylethyloxy, cyclobutylmethyloxy, cyclopentylmethyloxy, cyclohexylmethyloxy, cyclohexylethyloxy etc.) optionally having 1 to 3 halogen atoms;
(14) C₃₋₈ cycloalkenyl-C₁₋₆ alkoxy (e.g., cyclopentenylmethyloxy, cyclohexenylmethyloxy, cyclohexenylethyloxy, cyclohexenylpropyloxy etc.) optionally having 1 to 3 halogen atoms;
(15) C₆₋₁₀ aryl-C₁₋₆ alkoxy (e.g., phenylmethyloxy, phenylethyloxy etc.) optionally having 1 to 3 halogen atoms;
(16) C₁₋₆ alkyl-aminosulfonyl (e.g., methylaminosulfonyl, ethylaminosulfonyl, propylaminosulfonyl, isobutylaminosulfonyl, tert-butylaminosulfonyl etc.) optionally having hydroxy;
(17) di-C₁₋₆ alkyl-aminosulfonyl (e.g., dimethylaminosulfonyl, diethylaminosulfonyl, dipropylaminosulfonyl etc.);
(18) carbamoyl;
(19) C₁₋₆ alkyl-aminocarbonyl (e.g., methylaminocarbonyl, ethylaminocarbonyl, propylaminocarbonyl, isobutylaminocarbonyl, tert-butylaminocarbonyl, hexylaminocarbonyl etc.) optionally having substituent(s) selected from (i) hydroxy, (ii) C₃₋₈ cycloalkyl, (iii) cyano, (iv)carboxy, (v) C₁₋₆ alkoxy-carbonyl, (vi) carbamoyl, (vii) C₁₋₆ alkyl-aminocarbonyl, (viii) di-C₁₋₆ alkyl-aminocarbonyl, and (ix) 5- or 6-membered monocyclic aromatic heterocycle (the heterocycle may be substituted by cyano or oxo);
(20) di-C₁₋₆ alkyl-aminocarbonyl (e.g., dimethylaminocarbonyl, diethylaminocarbonyl, dipropylaminocarbonyl etc.);
(21) formyl;
(22) C₁₋₆ alkyl-carbonyl (e.g., acetyl, ethylcarbonyl, propylcarbonyl, isopropylcarbonyl etc.);
(23) C₂₋₆ alkenyl-carbonyl (e.g., ethenylcarbonyl, propenylcarbonyl, butenylcarbonyl, pentenylcarbonyl, hexenylcarbonyl etc.);
(24) C₂₋₆ alkynyl-carbonyl (e.g., ethynylcarbonyl, propynylcarbonyl, butynylcarbonyl, pentynylcarbonyl, hexynylcarbonyl etc.);
(25) C₃₋₈ cycloalkyl-carbonyl (e.g., cyclopropylcarbonyl, cyclobutylcarbonyl, cyclopentylcarbonyl, cyclohexylcarbonyl etc.);
(26) C₃₋₈ cycloalkenyl-carbonyl (e.g., cyclopropenylcarbonyl, cyclobutenylcarbonyl, cyclopentenylcarbonyl, cyclohexenylcarbonyl etc.);
(27) C₆₋₁₀ aryl-carbonyl (e.g., benzoyl, 1-naphthylcarbonyl, 2-naphthylcarbonyl etc.);
(28) C₃₋₈ cycloalkyl-C₁₋₆ alkyl-carbonyl (e.g., cyclopropylmethylcarbonyl, cyclopropylethylcarbonyl, cyclobutylmethylcarbonyl, cyclopentylmethylcarbonyl, cyclohexylmethylcarbonyl, cyclohexylethylcarbonyl etc.);
(29) C₃₋₈ cycloalkenyl-C₁₋₆ alkyl-carbonyl (e.g., cyclopentenylmethylcarbonyl, cyclohexenylmethylcarbonyl, cyclohexenylethylcarbonyl, cyclohexenylpropylcarbonyl etc.);
(30) C₆₋₁₀ aryl-C₁₋₆ alkyl-carbonyl (e.g., benzylcarbonyl, phenylethylcarbonyl etc.);
(31) 5- or 6-membered monocyclic aromatic heterocyclyl-carbonyl (e.g., furylcarbonyl, thienylcarbonyl, pyrrolylcarbonyl, oxazolylcarbonyl, isooxazolylcarbonyl, thiazolylcarbonyl, isothiazolylcarbonyl, imidazolylcarbonyl, pyridylcarbonyl, pyrazolylcarbonyl etc.);
(32) 8- to 12-membered condensed aromatic heterocyclyl-carbonyl (e.g., benzofurylcarbonyl, isobenzofurylcarbonyl, benzothienylcarbonyl, isobenzothienylcarbonyl, indolylcarbonyl, isoindolylcarbonyl, 1H-indazolylcarbonyl, benzimidazolylcarbonyl, benzoxazolylcarbonyl etc.);
(33) 4- to 6-membered non-aromatic heterocyclyl-carbonyl (e.g., oxiranylcarbonyl, azetidinylcarbonyl, oxetanylcarbonyl, thietanylcarbonyl, pyrrolidinylcarbonyl, tetrahydrofurylcarbonyl, thiolanylcarbonyl, piperidinylcarbonyl, morpholinylcarbonyl etc.);

(34) C₁₋₆ alkylsulfonyl (e.g., methylsulfonyl, ethylsulfonyl etc.);
(35) C₂₋₆ alkenylsulfonyl (e.g., ethenylsulfonyl, propenylsulfonyl etc.);
(36) C₂₋₆ alkynylsulfonyl (e.g., ethynylsulfonyl, propynylsulfonyl, butynylsulfonyl, pentynylsulfonyl, hexynylsulfonyl etc.);
(37) C₃₋₈ cycloalkylsulfonyl (e.g., cyclopropylsulfonyl, cyclobutylsulfonyl etc.);
(38) C₃₋₈ cycloalkenylsulfonyl (e.g., cyclopropenylsulfonyl, cyclobutenylsulfonyl etc.);
(39) C₆₋₁₀ arylsulfonyl (e.g., phenylsulfonyl etc.);
(40) C₃₋₈ cycloalkyl-C₁₋₆ alkyl-sulfonyl (e.g., cyclopropylmethylsulfonyl etc.);
(41) C₃₋₈ cycloalkenyl-C₁₋₆ alkyl-sulfonyl (e.g., cyclopentenylmethylsulfonyl etc.);
(42) C₆₋₁₀ aryl-C₁₋₆ alkyl-sulfonyl (e.g., benzylsulfonyl etc.);
(43) 5- or 6-membered monocyclic aromatic heterocyclyl-sulfonyl (e.g., furylsulfonyl, thienylsulfonyl, pyridylsulfonyl etc.);
(44) 8- to 12-membered condensed aromatic heterocyclyl-sulfonyl (e.g., benzofurylsulfonyl, isobenzofurylsulfonyl etc.);
(45) 4- to 6-membered non-aromatic heterocyclyl-sulfonyl (e.g., oxiranylsulfonyl, azetidinylsulfonyl, pyrrolidinylsulfonyl, morpholinosulfonyl etc.) optionally having hydroxy;
(46) amino;
(47) mono-C₁₋₆ alkylamino (e.g., methylamino, ethylamino, propylamino, isopropylamino, butylamino, isobutylamino, tert-butylamino etc.);
(48) di-C₁₋₆ alkylamino (e.g., dimethylamino, diethylamino, dipropylamino, diisopropylamino, dibutylamino, diisobutylamino, di-tert-butylamino etc.);
(49) C₁₋₆ alkoxy-carbonylamino (e.g., tert-butoxycarbonylamino etc.);
(50) mono-(C₁₋₆ alkyl-carbonyl)amino (e.g., acetylamino, ethylcarbonylamino, propylcarbonylamino, tert-butylcarbonylamino etc.) optionally having 1 to 3 halogen atoms;
(51) mono-(C₃₋₈ cycloalkyl-carbonyl)amino (e.g., cyclopropylcarbonylamino, cyclobutylcarbonylamino, cyclopentylcarbonylamino, cyclohexylcarbonylamino etc.);
(52) mono-(C₆₋₁₀ aryl-carbonyl) amino (e.g., benzoylamino etc.) optionally having 1 to 3 halogen atoms;
(53) mono-(5- or 6-membered monocyclic aromatic heterocyclyl-carbonyl)amino (e.g., furylcarbonylamino, thienylcarbonylamino, pyrrolylcarbonylamino, oxazolylcarbonylamino, isooxazolylcarbonylamino, thiazolylcarbonylamino, isothiazolylcarbonylamino, imidazolylcarbonylamino, pyridylcarbonylamino, pyrazolylcarbonylamino etc.);
(54) mono-(8- to 12-membered condensed aromatic heterocyclyl-carbonyl)amino (e.g., benzofurylcarbonylamino, isobenzofurylcarbonylamino, benzothienylcarbonylamino, isobenzothienylcarbonylamino etc.);
(55) mono-(5- or 6-membered non-aromatic heterocyclyl-carbonyl)amino (e.g., oxiranylcarbonylamino, azetidinylcarbonylamino, oxetanylcarbonylamino etc.);
(56) thiol;
(57) C₁₋₆ alkylsulfanyl (e.g., methylsulfanyl, ethylsulfanyl etc.);
(58) C₂₋₆ alkenylsulfanyl (e.g., ethenylsulfanyl, propenylsulfanyl etc.);
(59) C₂₋₆ alkynylsulfanyl (e.g., ethynylsulfanyl, propynylsulfanyl, butynylsulfanyl, pentynylsulfanyl, hexynylsulfanyl etc.);
(60) C₃₋₈ cycloalkylsulfanyl (e.g., cyclopropylsulfanyl, cyclobutylsulfanyl etc.);
(61) C₃₋₈ cycloalkenylsulfanyl (e.g., cyclopropenylsulfanyl, cyclobutenylsulfanyl etc.);
(62) C₆₋₁₀ arylsulfanyl (e.g., phenylsulfanyl etc.);
(63) C₃₋₈ cycloalkyl-C₁₋₆ alkyl-sulfanyl (e.g., cyclopropylmethylsulfanyl etc.);
(64) C₃₋₈ cycloalkenyl-C₁₋₆ alkyl-sulfanyl (e.g., cyclopentenylmethylsulfanyl etc.);

(65) a 5- or 6-membered monocyclic aromatic heterocyclic group (e.g., furyl, thienyl, pyrrolyl, oxazolyl, isoxazolyl, thiazolyl, isothiazolyl, imidazolyl, pyridyl, pyrazolyl, triazolyl, oxadiazolyl etc.) optionally having (i) C₁₋₆ alkyl optionally having 1 to 3 halogen atoms or hydroxy, (ii) carboxy, (iii) carbamoyl, or (iv) C₁₋₆ alkoxy-carbonyl;
(66) a 8- to 12-membered condensed aromatic heterocyclic group (e.g., benzofuryl, isobenzofuryl, benzothienyl, isobenzothienyl, indolyl, isoindolyl, 1H-indazolyl, benzimidazolyl, benzoxazolyl etc.);
(67) a 5- or 6-membered nonaromatic heterocyclic group (e.g., oxiranyl, azetidinyl, oxetanyl, thietanyl, pyrrolidinyl, tetrahydrofuryl, thiolanyl, piperidinyl, morpholinyl, isothiazolidinyl etc.) optionally having 1 or 2 oxo;
(68) 5- or 6-membered monocyclic aromatic heterocyclyl-oxy (e.g., furyloxy, thienyloxy, pyrrolyloxy, oxazolyloxy, isoxazolyloxy, thiazolyloxy, isothiazolyloxy, imidazolyloxy, pyridyloxy, pyrazolyloxy etc.);
(69) 8- to 12-membered condensed aromatic heterocyclyl-oxy (e.g., benzofuryloxy, isobenzofuryloxy, benzothienyloxy, isobenzothienyloxy, indolyloxy, isoindolyloxy, 1H-indazolyloxy, benzimidazolyloxy, benzoxazolyloxy etc.);
(70) 5- or 6-membered non-aromatic heterocyclyl-oxy (e.g., oxiranyloxy, azetidinyloxy, oxetanyloxy, thietanyloxy, pyrrolidinyloxy, tetrahydrofuryloxy, thiolanyloxy, piperidinyloxy etc.);
(71) oxo;
(72) C₁₋₆ alkylsulfinyl (e.g., methylsulfinyl, ethylsulfinyl etc.);
(73) C₂₋₆ alkenylsulfinyl (e.g., ethenylsulfinyl, propenylsulfinyl etc.);
(74) C₂₋₆ alkynylsulfinyl (e.g., ethynylsulfinyl, propynylsulfinyl, butynylsulfinyl, pentynylsulfinyl, hexynylsulfinyl etc.);
(75) C₃₋₈ cycloalkylsulfinyl (e.g., cyclopropylsulfinyl, cyclobutylsulfinyl etc.);
(76) C₃₋₈ cycloalkenylsulfinyl (e.g., cyclopropenylsulfinyl, cyclobutenylsulfinyl etc.);
(77) C₆₋₁₀ arylsulfinyl (e.g., phenylsulfinyl etc.);
(78) C₃₋₈ cycloalkyl-C₁₋₆ alkyl-sulfinyl (e.g., cyclopropylmethylsulfinyl etc.);
(79) C₃₋₈ cycloalkenyl-C₁₋₆ alkyl-sulfinyl (e.g., cyclopentenylmethylsulfinyl etc.);
(80) C₁₋₆ alkyl-aminothiocarbonyl (e.g., methylaminothiocarbonyl, ethylaminothiocarbonyl, propylaminothiocarbonyl etc.);
(81) di-C₁₋₆ alkyl-aminothiocarbonyl (e.g., dimethylaminothiocarbonyl, diethylaminothiocarbonyl, dipropylaminothiocarbonyl etc.);
(82) carboxy;
(83) C₁₋₆ alkoxy-carbonyl (e.g., methoxycarbonyl, ethoxycarbonyl, propoxycarbonyl, isopropoxycarbonyl, butoxycarbonyl, isobutoxycarbonyl, tert-butoxycarbonyl etc.);
(84) C₂₋₆ alkenyloxy-carbonyl (e.g., ethenyloxycarbonyl, propenyloxycarbonyl, butenyloxycarbonyl, pentenyloxycarbonyl, hexenyloxycarbonyl etc.);
(85) C₂₋₆ alkynyloxy-carbonyl (e.g., ethynyloxycarbonyl, propynyloxycarbonyl, butynyloxycarbonyl, pentynyloxycarbonyl, hexynyloxycarbonyl etc.);
(86) C₃₋₈ cycloalkyl-oxy-carbonyl (e.g., cyclopropyloxycarbonyl, cyclobutyloxycarbonyl, cyclopentyloxycarbonyl, cyclohexyloxycarbonyl etc.);
(87) C₃₋₈ cycloalkenyloxy-carbonyl (e.g., cyclopropenyloxycarbonyl, cyclobutenyloxycarbonyl, cyclopentenyloxycarbonyl, cyclohexenyloxycarbonyl etc.);
(88) C₆₋₁₀ aryloxy-carbonyl (e.g., phenyloxycarbonyl, 1-naphthyloxycarbonyl, 2-naphthyloxycarbonyl etc.);
(89) C₃₋₈ cycloalkyl-C₁₋₆ alkoxy-carbonyl (e.g., cyclopropylmethyloxycarbonyl, cyclopropylethyloxycarbonyl, cyclobutylmethyloxycarbonyl, cyclopentylmethyloxycarbonyl, cyclohexylmethyloxycarbonyl, cyclohexylethyloxycarbonyl etc.);
(90) C₃₋₈ cycloalkenyl-C₁₋₆ alkoxy-carbonyl (e.g., cyclopentenylmethyloxycarbonyl, cyclohexenylmethyloxycarbonyl, cyclohexenylethyloxycarbonyl, cyclohexenylpropyloxycarbonyl etc.);
(91) C₆₋₁₀ aryl-C₁₋₆ alkoxy-carbonyl (e.g., phenylmethyloxycarbonyl, phenylethyloxycarbonyl etc.);
(92) C₃₋₈ cycloalkyl-aminocarbonyl (e.g., cyclopropylaminocarbonyl, cyclobutylaminocarbonyl etc.);
(93) aminosulfonyl;
(94) sulfo;
(95) halogenated sulfonyl (e.g., chlorosulfonyl etc.);
(96) C₆₋₁₀ aryloxy-sulfonyl (e.g., phenyloxysulfonyl etc.);
(97) C₃₋₆ cycloalkyl-aminosulfonyl (e.g., cyclopropylaminosulfonyl etc.);
(98) C₁₋₆ alkyl (C₁₋₆ alkoxycarbonyl)aminosulfonyl (e.g., methyl(tert-butoxycarbonyl)aminosulfonyl etc.);
(99) C₁₋₆ alkylsulfonylamino (e.g., methylsulfonylamino, propylsulfonylamino, isopropylsulfonylamino etc.) optionally having 1 to 3 halogen atoms;
(100) C₆₋₁₀ arylsulfonylamino (e.g., phenylsulfonylamino etc.) optionally having 1 to 3 halogen atoms;
(101) C₁₋₆ alkyl (C₁₋₆ alkylsulfonyl)amino (e.g., methyl(isopropylsulfonyl)amino etc.);
(102) thiocarbamoyl; and
(103) hydrazinocarbonyl

In the present specification, the "halogeno-C₁₋₆ alkyl" is C₁₋₆ alkyl substituted by 1 to 5 (preferably 1 to 3) halogen atoms (e.g., fluorine atom, chlorine atom, bromine atom, iodine atom), from among the above-mentioned "C₁₋₆ alkyl optionally having substituent(s)".

Examples of the "C₂₋₆ alkenyl" of the above-mentioned "C₂₋₆ alkenyl optionally having substituent(s)" include ethenyl, propenyl, butenyl, pentenyl, hexenyl and the like.
Examples of the "substituent" of the above-mentioned "C₂₋₆ alkenyl optionally having substituent(s)" include substituent selected from substituent group A. While the number of substituents is not particularly limited as long as the substituents in the designated number are substitutable, it is preferably 1 to 5, more preferably 1 to 3.

Examples of the "C₂₋₆ alkynyl" of the above-mentioned "C₂₋₆ alkynyl optionally having substituent(s)" include ethynyl, propynyl, butynyl, pentynyl, hexynyl and the like.
Examples of the "substituent" of the above-mentioned "C₂₋₆ alkynyl optionally having substituent(s)" include substituents selected from substituent group A. While the number of substituents is not particularly limited as long as the substituents in the designated number are substitutable, it is preferably 1 to 5, more preferably 1 to 3.

Examples of the "C₃₋₈ cycloalkyl" of the above-mentioned "C₃₋₈ cycloalkyl optionally having substituent(s)" include cyclopropyl, cyclobutyl, cyclopentyl, cyclohexyl, cycloheptyl, cyclooctyl and the like. In the present specification, the "C₃₋₇ cycloalkyl" is cycloalkyl having a carbon number of 3 to 7, from among the above-mentioned "C₃₋₈ cycloalkyl".
Examples of the "substituent" of the above-mentioned "C₃₋₈ cycloalkyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₃₋₈ cycloalkenyl" of the above-mentioned "C₃₋₈ cycloalkenyl optionally having substituent(s)" include cyclopropenyl, cyclobutenyl, cyclopentenyl, cyclohexenyl, cycloheptenyl, cyclooctenyl and the like.
Examples of the "substituent" of the above-mentioned "C₃₋₈ cycloalkenyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₆₋₁₀ aryl" of the above-mentioned "C₆₋₁₀ aryl optionally having substituent(s)" include phenyl, 1-naphthyl, 2-naphthyl and the like.
Examples of the "substituent" of the above-mentioned "C₆₋₁₀ aryl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.
In the present specification, the "acyl" includes
(1) formyl,
(2) alkyl-carbonyl (C₁₋₆ alkyl-carbonyl) optionally having substituent(s),
(3) alkenyl-carbonyl (C₂₋₆ alkenyl-carbonyl) optionally having substituent(s),
(4) alkynyl-carbonyl (C₂₋₆ alkynyl-carbonyl) optionally having substituent(s),
(5) cycloalkyl-carbonyl (C₃₋₈ cycloalkyl-carbonyl) optionally having substituent(s),
(6) cycloalkenyl-carbonyl (C₃₋₈ cycloalkenyl-carbonyl) optionally having substituent(s),
(7) aryl-carbonyl (C₆₋₁₀ aryl-carbonyl) optionally having substituent(s),
(8) heterocyclyl-carbonyl optionally having substituent(s),
(9) carboxyl,
(10) alkoxy-carbonyl (C₁₋₆ alkoxy-carbonyl) optionally having substituent(s),
(11) alkenyloxy-carbonyl (C₂₋₆ alkenyloxy-carbonyl) optionally having substituent(s),
(12) alkynyloxy-carbonyl (C₂₋₆ alkynyloxy-carbonyl) optionally having substituent(s),
(13) cycloalkyloxy-carbonyl (C₃₋₈ cycloalkyloxy-carbonyl) optionally having substituent(s),
(14) cycloalkenyloxy-carbonyl (C₃₋₈ cycloalkenyloxy-carbonyl) optionally having substituent(s),
(15) cycloalkenyloxy-carbonyl (C₃₋₈cycloalkynyloxy-carbonyl) optionally having substituent(s),
(16) aryloxy-carbonyl (C₆₋₁₀ aryloxy-carbonyl) optionally having substituent(s),
(17) heterocyclyl-oxy-carbonyl optionally having substituent(s),
(18) carbamoyl optionally having substituent(s),
and the like.

Examples of the "C₁₋₆ alkyl-carbonyl" of the above-mentioned "C₁₋₆ alkyl-carbonyl optionally having substituent(s)" include acetyl, ethylcarbonyl, propylcarbonyl, isopropylcarbonyl, butylcarbonyl, isobutylcarbonyl, sec-butylcarbonyl, tert-butylcarbonyl, pentylcarbonyl, hexylcarbonyl and the like.
Examples of the "substituent" of the above-mentioned "C₁₋₆ alkyl-carbonyl optionally having substituent(s)" include substituents selected from substituent group A. While the number of substituents is not particularly limited as long as the substituents in the designated number are substitutable, it is preferably 1 to 5, more preferably 1 to 3.

Examples of the "C₂₋₆ alkenyl-carbonyl" of the above-mentioned "C₂₋₆ alkenyl-carbonyl optionally having substituent(s)" include ethenylcarbonyl, propenylcarbonyl, butenylcarbonyl, pentenylcarbonyl, hexenylcarbonyl and the like.
Examples of the "substituent" of the above-mentioned "C₂₋₆ alkenyl-carbonyl optionally having substituent(s)" include substituents selected from substituent group A. While the number of substituents is not particularly limited as long as the substituents in the designated number are substitutable, it is preferably 1 to 5, more preferably 1 to 3.

Examples of the "C₂₋₆ alkynyl-carbonyl" of the above-mentioned "C₂₋₆ alkynyl-carbonyl optionally having substituent(s)" include ethynylcarbonyl, propynylcarbonyl, butynylcarbonyl, pentynylcarbonyl, hexynylcarbonyl and the like.
Examples of the "substituent" of the above-mentioned "C₂₋₆ alkynyl-carbonyl optionally having substituent(s)" include substituents selected from substituent group A. While the number of substituents is not particularly limited as long as the substituents in the designated number are substitutable, it is preferably 1 to 5, more preferably 1 to 3.

Examples of the "C₃₋₈ cycloalkyl-carbonyl" of the above-mentioned "C₃₋₈ cycloalkyl-carbonyl optionally having substituent(s)" include cyclopropylcarbonyl, cyclobutylcarbonyl, cyclopentylcarbonyl, cyclohexylcarbonyl, cycloheptylcarbonyl, cyclooctylcarbonyl and the like.
Examples of the "substituent" of the above-mentioned "C₃₋₈ cycloalkyl-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₃₋₈ cycloalkenyl-carbonyl" of the above-mentioned "C₃₋₈ cycloalkenyl-carbonyl optionally having substituent(s)" include cyclopropenylcarbonyl, cyclobutenylcarbonyl, cyclopentenylcarbonyl, cyclohexenylcarbonyl, cycloheptenylcarbonyl, cyclooctenylcarbonyl and the like.
Examples of the "substituent" of the above-mentioned "C₃₋₈ cycloalkenyl-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₆₋₁₀ aryl-carbonyl" of the above-mentioned "C₆₋₁₀ aryl-carbonyl optionally having substituent(s)" include benzoyl, 1-naphthoyl, 2-naphthoyl and the like.
Examples of the "substituent" of the above-mentioned "C₆₋₁₀ aryl-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "heterocycle" of the above-mentioned "heterocyclyl-carbonyl optionally having substituent(s)" include (1) 5- or 6-membered monocyclic aromatic heterocycle (e.g., furan, thiophene, pyrrole, oxazole, isoxazole, thiazole, isothiazole, imidazole, pyridine, pyrazole etc.), (2) 8- to 12-membered condensed aromatic heterocycle (e.g., benzofuran, isobenzofuran, benzothiophene, isobenzothiophene, indole, isoindole, 1H-indazole, benzimidazole, benzoxazole etc.), (3) 5- or 6-membered non-aromatic heterocycle (e.g., oxirane, azetidine, oxetane, pyrrolidine, tetrahydrofuran, thioran, piperidine etc.) and the like.
Examples of the "substituent" of the above-mentioned "heterocyclyl-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₁₋₆ alkoxy-carbonyl" of the above-mentioned "C₁₋₆ alkoxy-carbonyl optionally having substituent(s)" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl and the like.
Examples of the "substituent" of the above-mentioned "C₁₋₆ alkoxy-carbonyl optionally having substituent(s)" include substituents selected from substituent group A. While the number of substituents is not particularly limited as long as the substituents in the designated number are substitutable, it is preferably 1 to 5, more preferably 1 to 3.

Examples of the "C₂₋₆ alkenyloxy-carbonyl" of the above-mentioned "C₂₋₆ alkenyloxy-carbonyl optionally having substituent(s)" include ethenyloxycarbonyl, propenyloxycarbonyl, butenyloxycarbonyl, pentenyloxycarbonyl, hexenyloxycarbonyl and the like.
Examples of the "substituent" of the above-mentioned "C₂₋₆ alkenyloxy-carbonyl optionally having substituent(s)" include substituents selected from substituent group A. While the number of substituents is not particularly limited as long as the substituents in the designated number are substitutable, it is preferably 1 to 5, more preferably 1 to 3.

Examples of the "C₂₋₆ alkynyloxy-carbonyl" of the above-mentioned "C₂₋₆ alkynyloxy-carbonyl optionally having substituent(s)" include ethynyloxycarbonyl, propynyloxycarbonyl, butynyloxycarbonyl, pentynyloxycarbonyl, hexynyloxycarbonyl and the like.
Examples of the "substituent" of the above-mentioned "C₂₋₆ alkynyloxy-carbonyl optionally having substituent(s)" include substituents selected from substituent group A. While the number of substituents is not particularly limited as long as the substituents in the designated number are substitutable, it is preferably 1 to 5, more preferably 1 to 3.

Examples of the "C₃₋₈ cycloalkyloxy-carbonyl" of the above-mentioned "C₃₋₈ cycloalkyloxy-carbonyl optionally having substituent(s)" include cyclopropyloxycarbonyl, cyclobutyloxycarbonyl, cyclopentyloxycarbonyl, cyclohexyloxycarbonyl, cycloheptyloxycarbonyl, cyclooctyloxycarbonyl and the like.
Examples of the "substituent" of the above-mentioned "C₃₋₈ cycloalkyloxy-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₃₋₈ cycloalkenyloxy-carbonyl" of the above-mentioned "C₃₋₈ cycloalkenyloxy-carbonyl optionally having substituent(s)" include cyclopropenyloxycarbonyl, cyclobutenyloxycarbonyl, cyclopentenyloxycarbonyl, cyclohexenyloxycarbonyl, cycloheptenyloxycarbonyl, cyclooctenyloxycarbonyl and the like.
Examples of the "substituent" of the above-mentioned "C₃₋₈ cycloalkenyloxy-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₃₋₈ cycloalkynyloxy-carbonyl" of the above-mentioned "C₃₋₈ cycloalkynyloxy-carbonyl optionally having substituent(s)" include cyclopropynyloxycarbonyl, cyclobutynyloxycarbonyl, cyclopentynyloxycarbonyl, cyclohexynyloxycarbonyl, cycloheptynyloxycarbonyl, cyclooctynyloxycarbonyl and the like.
Examples of the "substituent" of the above-mentioned "C₃₋₈ cycloalkynyloxy-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "C₆₋₁₀ aryloxy-carbonyl" of the above-mentioned "C₆₋₁₀ aryloxy-carbonyl optionally having substituent(s)" include phenoxycarbonyl, 1-naphthyloxycarbonyl, 2-naphthyloxycarbonyl and the like.
Examples of the "substituent" of the above-mentioned "C₆₋₁₀ aryloxy-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

Examples of the "heterocycle" of the above-mentioned "heterocyclyl-oxy-carbonyl optionally having substituent(s)" include (1) 5- or 6-membered monocyclic aromatic heterocycle (e.g., furan, thiophene, pyrrole, oxazole, isoxazole, thiazole, isothiazole, imidazole, pyridine, pyrazole etc.), (2) 8- to 12-membered condensed aromatic heterocycle (e.g., benzofuran, isobenzofuran, benzothiophene, isobenzothiophene, indole, isoindole, 1H-indazole, benzimidazole, benzoxazole etc.), (3) 5- or 6-membered non-aromatic heterocycle (e.g., oxirane, azetidine, oxetane, pyrrolidine, tetrahydrofuran, thioran, piperidine etc.) and the like.
Examples of the "substituent" of the above-mentioned "heterocyclyl-oxy-carbonyl optionally having substituent(s)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

The above-mentioned "carbamoyl optionally having substituent(s)" is carbamoyl optionally having 1 or 2 substituents selected from the aforementioned alkyl (C₁₋₆ alkyl) optionally having substituent(s), alkenyl (C₂₋₆ alkenyl) optionally having substituent(s), alkynyl (C₂₋₆ alkynyl) optionally having substituent(s), cycloalkyl (C₃₋₈ cycloalkyl) optionally having substituent(s), cycloalkenyl (C₃₋₈ cycloalkenyl) optionally having substituent(s) and aryl (C₆₋₁₀ aryl) optionally having substituent(s).

Examples of the above-mentioned "heterocyclic group optionally having substituent(s) (which has a bond at carbon atom)" include an aromatic heterocyclic group (e.g., monocyclic aromatic heterocyclic group, condensed aromatic heterocyclic group), a nonaromatic heterocyclic group and the like.

Examples of the monocyclic aromatic heterocyclic group include a 5- to 7-membered monocyclic aromatic heterocyclic group containing, as a ring-constituting atom besides carbon atom, 1 to 4 hetero atoms selected from an oxygen atom, a sulfur atom and a nitrogen atom, and the like.
Specific examples of the monocyclic aromatic heterocyclic group include furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 2-imidazolyl, 4-imidazolyl, 5-imidazolyl), pyrazolyl (e.g., 3-pyrazolyl, 4-pyrazolyl), thiazolyl (e.g., 2-thiazolyl, 4-thiazolyl, 5-thiazolyl), isothiazolyl, oxazolyl (e.g., 2-oxazolyl, 4-oxazolyl, 5-oxazolyl), isoxazolyl, oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-4-yl), tetrazolyl (e.g., tetrazol-5-yl), triazinyl and the like.

Examples of the condensed aromatic heterocyclic group include a condensed group of a 5- to 7-membered monocyclic aromatic heterocyclic group containing, as a ring-constituting atom besides carbon atom, 1 to 4 hetero atoms selected from an oxygen atom, a sulfur atom and a nitrogen atom, and the like, and C₆₋₁₀ aryl and the like; condensed group of the above-mentioned 5- to 7-membered monocyclic aromatic heterocyclic groups and the like.
Specific examples of the condensed aromatic heterocyclic group include quinolyl (e.g., 2-quinolyl, 3-quinolyl, 4-quinolyl, isoquinolyl), quinazolyl (e.g., 2-quinazolyl, 4-quinazolyl), quinoxalyl (e.g., 2-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzothiazolyl (e.g., 2-benzothiazolyl, 5-benzothiazolyl, 6-benzothiazolyl), benzimidazolyl (e.g., benzimidazol-2-yl, benzimidazol-5-yl), indolyl (e.g., indol-3-yl, indol-4-yl, indol-5-yl, indol-6-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridyl (e.g., 1H-imidazo[4,5-b]pyridin-2-yl, 1H-imidazo[4,5-c]pyridin-2-yl), imidazopyrazinyl (e.g., 1H-imidazo[4,5-b]pyrazin-2-yl), benzisoxazolyl, benzotriazolyl, pyrazolopyridyl, pyrazolothienyl, pyrazolotriazinyl and the like.

Examples of the nonaromatic heterocyclic group include a 3- to 8-membered (preferably 5- or 6-membered) saturated or unsaturated (preferably saturated) nonaromatic heterocyclic group and the like.
Specific examples of the nonaromatic heterocyclic group include oxiranyl, azetidinyl, oxetanyl, thietanyl, pyrrolidinyl, tetrahydrofuryl, thiolanyl, piperidinyl, tetrahydropyranyl, thianyl, morpholinyl, thiomorpholinyl, piperazinyl, azepanyl, oxepanyl, thiepanyl, oxazepanyl, thiazepanyl, azocanyl, oxocanyl, thiocanyl, oxazocanyl, thiazocanyl, dioxinyl and the like.

Examples of the "substituent" of the above-mentioned "heterocyclic group optionally having substituent(s) (which has a bond at carbon atom)" include (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo). While the number of substituents is not particularly limited as long as the substituents in the designated number are substitutable, it is preferably 1 to 5, more preferably 1 to 3.

In the present specification, examples of the "group via a nitrogen atom" include (1) nitro, and (2) amino optionally having 1 or 2 of the above-mentioned "group via a carbon atom".

In the present specification, examples of the "group via an oxygen atom" include hydroxyl optionally having one "group via a carbon atom" mentioned above.

In the present specification, examples of the "group via a sulfur atom" include thiol optionally having one "group via a carbon atom" mentioned above or "group via a nitrogen atom" mentioned above, and the group may be oxidized.

In the present specification, examples of the "cyclic group" of the "cyclic group having cyano (the cyclic group optionally further has substituent(s) other than cyano group)" and "cyclic group optionally having substituent(s)" include an aromatic hydrocarbon group, an aromatic heterocyclic group (e.g., monocyclic aromatic heterocyclic group, condensed aromatic heterocyclic group), a nonaromatic cyclic hydrocarbon group, a nonaromatic heterocyclic group, a fused ring group thereof and the like.
Examples of the aromatic hydrocarbon group include C₆₋₁₀ aryl and the like. Specifically, phenyl, 1-naphthyl, 2-naphthyl and the like can be mentioned.

Examples of the monocyclic aromatic heterocyclic group include a 5- to 7-membered monocyclic aromatic heterocyclic group containing, as a ring-constituting atom besides carbon atom, 1 to 4 hetero atoms selected from an oxygen atom, a sulfur atom and a nitrogen atom, and the like.
Specific examples of the monocyclic aromatic heterocyclic group include furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl, 5-pyrazolyl), thiazolyl (e.g., 2-thiazolyl, 4-thiazolyl, 5-thiazolyl), isothiazolyl, oxazolyl (e.g., 2-oxazolyl, 4-oxazolyl, 5-oxazolyl), isoxazolyl (e.g., 4-isoxazolyl, 5-isoxazolyl), oxadiazolyl (e.g., 1,2,4-oxadiazol-5-yl, 1,3,4-oxadiazol-2-yl), thiadiazolyl (e.g., 5-thiadiazolyl such as 1,3,4-thiadiazol-2-yl and the like), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl and the like.

Examples of the condensed aromatic heterocyclic group include a condensed group of a 5- to 7-membered monocyclic aromatic heterocyclic group containing, as a ring-constituting atom besides carbon atom, 1 to 4 hetero atoms selected from an oxygen atom, a sulfur atom and a nitrogen atom, and the like, and C₆₋₁₀ aryl and the like; a condensed group of the above-mentioned 5- to 7-membered monocyclic aromatic heterocyclic groups and the like.
Specific examples of the condensed aromatic heterocyclic group include quinolyl (e.g., 2-quinolyl, 3-quinolyl, 4-quinolyl, 6-quinolyl, isoquinolyl), quinazolyl (e.g., 2-quinazolyl, 4-quinazolyl), quinoxalyl (e.g., 2-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzothiazolyl (e.g., 2-benzothiazolyl, 5-benzothiazolyl, 6-benzothiazolyl), benzimidazolyl (e.g., benzimidazol-1-yl, benzimidazol-2-yl, benzimidazol-5-yl), indolyl (e.g., indol-1-yl, indol-3-yl, indol-4-yl, indol-5-yl, indol-6-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridyl (e.g., 1H-imidazo[4,5-b]pyridin-2-yl, 1H-imidazo[4,5-c]pyridin-2-yl), imidazopyrazinyl (e.g., 1H-imidazo[4,5-b]pyrazin-2-yl), benzisoxazolyl, benzotriazolyl (e.g., 5-benzotriazolyl), pyrazolopyridyl, pyrazolothienyl (e.g., thienopyrazol-5-yl), pyrazolotriazinyl and the like.

Examples of the nonaromatic cyclic hydrocarbon group include cycloalkyl, cycloalkenyl, cycloalkadienyl and the like, each of which may be condensed with a benzene ring.
Specific examples of the nonaromatic cyclic hydrocarbon group include C₃₋₈ cycloalkyl (e.g., cyclopropyl, cyclobutyl, cyclopentyl, cyclohexyl, cycloheptyl, cyclooctyl), C₃₋₈ cycloalkenyl (e.g., cyclopropenyl, cyclobutenyl, cyclopentenyl, cyclohexenyl, cycloheptenyl, cyclooctenyl), C₄₋₁₀ cycloalkadienyl (e.g., cyclobutadienyl, cyclopentadienyl, cyclohexadienyl, cycloheptadienyl, cyclooctadienyl, cyclononadienyl, cyclodecadienyl), fused ring group wherein these groups are condensed with a benzene ring (e.g., indanyl (e.g., 1-indanyl), tetrahydronaphthyl (e.g., 1,2,3,4-tetrahydronaphthalen-1-yl), fluorenyl (e.g., 9-fluorenyl) etc.) and the like.

Examples of the nonaromatic heterocyclic group include a 3- to 8-membered (preferably 5- or 6-membered) saturated or unsaturated (preferably saturated) nonaromatic heterocyclic group and the like.
Specific examples of the nonaromatic heterocyclic group include oxiranyl, azetidinyl, oxetanyl, thietanyl, pyrrolidinyl, tetrahydrofuryl (e.g., 2-tetrahydrofuryl), thiolanyl, piperidinyl (e.g., piperidin-3-yl), tetrahydropyranyl (e.g., 2-tetrahydropyranyl), thianyl, morpholinyl, thiomorpholinyl, piperazinyl, azepanyl, oxepanyl, thiepanyl, oxazepanyl, thiazepanyl, azocanyl, oxocanyl, thiocanyl, oxazocanyl, thiazocanyl, dioxinyl, dihydrobenzofuranyl (e.g., 4-dihydrobenzofuranyl, 7-dihydrobenzofuranyl), benzodioxolyl (e.g., 1,3-benzodioxol-5-yl), benzodioxanyl (e.g., 1,4-benzodioxan-5-yl, 1,4-benzodioxan-6-yl), benzodioxepinyl (e.g., 1,5-benzodioxepin-6-yl, 1,5-benzodioxepin-7-yl), dihydroisoindolyl (e.g., dihydroisoindol-5-yl), tetrahydrophthalazinyl (e.g., 1,2,3,4-tetrahydrophthalazin-6-yl), dihydrobenzodioxinyl (e.g., 2,3-dihydro-1,4-benzodioxin-5-yl, 2,3-dihydro-1,4-benzodioxin-6-yl), dihydrobenzodioxepinyl (e.g., 3,4-dihydro-2H-1,5-benzodioxepin-6-yl, 3,4-dihydro-2H-1,5-benzodioxepin-7-yl) and the like.

In the present specification, examples of the "heterocyclic group optionally having substituent(s)" include those exemplified as the above-mentioned "heterocyclic group optionally having substituent(s) (which has a bond at carbon atom)". However, the position of the bond is not limited to the carbon atom and may be a hetero atom constituting the ring.

In the present specification, examples of the "aromatic hydrocarbon ring" of the "aromatic hydrocarbon ring optionally having substituent(s)" include C₆₋₁₀ arene rings such as a benzene ring, a naphthalene ring etc., and the like.

In the present specification, examples of the "heterocycle" of the "heterocycle optionally having substituent(s)" include (1) 5- or 6-membered monocyclic aromatic heterocycle (e.g., furan, thiophene, pyrrole, oxazole, isoxazole, thiazole, isothiazole, imidazole, pyridine, pyrazole etc.), (2) 8- to 12-membered condensed aromatic heterocycle (e.g., benzofuran, isobenzofuran, benzothiophene, isobenzothiophene, indole, isoindole, 1H-indazole, benzimidazole, benzoxazole etc.), (3) 5- or 6-membered non-aromatic heterocycle (e.g., oxirane, azetidine, oxetane, pyrrolidine, tetrahydrofuran, thioran, piperidine etc.) and the like.

In the present specification, examples of the "substituent" of the "cyclic group having cyano (the cyclic group optionally further has substituent(s) other than a cyano group)", "phenyl group having cyano (the phenyl group optionally further has substituent(s) other than cyano)", "benzene ring optionally further having substituent(s) other than cyano", "benzene ring optionally having substituent(s)", "pyridine ring optionally having substituent(s)", "thiazole ring optionally having substituent(s)", "pyrazole ring optionally having substituent(s)", "aromatic hydrocarbon ring optionally having substituent(s)", "heterocycle optionally having substituent(s)" and "cyclic group optionally having substituent(s)" include (1) C₁₋₆ alkyl (e.g., methyl, ethyl, propyl, isopropyl, butyl, isobutyl, tert-butyl etc.) optionally having 1 to 3 substituents selected from hydroxy, a halogen atom (e.g., fluorine atom) and di-C₁₋₆ alkylamino (e.g., dimethylamino), (2) C₂₋₆ alkynyl (e.g., ethynyl etc.), (3) C₆₋₁₀ aryl-C₁₋₆ alkyl (e.g., benzyl), (4) C₂₋₆ alkenyl (e.g., ethenyl) optionally having C₆₋₁₀ aryl (e.g., phenyl) optionally having C₁₋₆ alkyl (e.g., methyl) optionally having 1 to 3 halogen atoms (e.g., fluorine atom) and (5) a substituent selected from substituent group A. While the number of substituents is not particularly limited as long as the substituents in the designated number are substitutable, it is preferably 1 to 5, more preferably 1 to 3.

In the present specification, examples of the "methylene optionally having substituent(s)" include methylene optionally having 1 or 2 substituents selected from (1) C₁₋₆ alkyl (e.g., methyl, ethyl, propyl, isopropyl, butyl, isobutyl, tert-butyl etc.) optionally having 1 to 3 substituents selected from a halogen atom and cyano, and (2) a substituent selected from substituent group A (excluding oxo).

Compound (I) is explained in detail in the following.

In compound (I), R¹ is a hydrogen atom, a group via a carbon atom, a group via a nitrogen atom, a group via an oxygen atom or a group via a sulfur atom.
Preferred as R¹ is a hydrogen atom or a group via a carbon atom, particularly preferably a hydrogen atom or C₁₋₆ alkyl optionally having substituent(s). Of these, a hydrogen atom or C₁₋₆ alkyl (particularly methyl) is preferable.

In compound (I), R² is a cyclic group having cyano.
Preferred as R² is an aromatic hydrocarbon group having cyano (the aromatic hydrocarbon group optionally further has substituent(s), a nonaromatic heterocyclic group having cyano (the nonaromatic heterocyclic group optionally further has substituent(s)), particularly preferably C₆₋₁₀ aryl having cyano (the C₆₋₁₀ aryl optionally further have 1 or 2 substituents selected from (1) a halogen atom and (2) C₁₋₆ alkyl optionally having 1 to 3 halogen atoms), a 6-membered nonaromatic heterocyclic group having cyano (the 6-membered nonaromatic heterocyclic group may have C₁₋₆ alkyl and oxo).
As R², 4-cyanophenyl, 3-chloro-4-cyanophenyl, 3-cyano-4-chlorophenyl, 4-cyano-3-fluorophenyl, 4-cyano-3-(trifluoromethyl)phenyl, 5-chloro-4-cyano-2-fluorophenyl, 3-cyano-1-methyl-2-oxo-1,2-dihydropyridin-5-yl and the like are specifically preferable.

In compound (I), R³ is a hydrogen atom, a group via a carbon atom, a group via a nitrogen atom, a group via an oxygen atom or a group via a sulfur atom.
Preferred as R³ is a hydrogen atom or a group via a carbon atom, particularly preferably a hydrogen atom or C₁₋₆ alkyl optionally having substituent(s). Of these, a hydrogen atom or C₁₋₆ alkyl (particularly methyl) is preferable.

In compound (I), R⁴ is a cyclic group optionally having substituent(s). Preferred as R⁴ is C₆₋₁₀ aryl optionally having substituent(s), a 5- or 6-membered aromatic heterocyclic group optionally having substituent(s), a condensed aromatic heterocyclic group optionally having substituent(s), a 5- or 6-membered nonaromatic heterocyclic group optionally having substituent(s) and a nonaromatic fused heterocycle group optionally having substituent(s). Particularly,
(A) C₆₋₁₀ aryl (e.g., phenyl) optionally substituted by 1 to 3 substituents selected from
   (1) C₁₋₆ alkyl (e.g., methyl, isopropyl, tert-butyl, specifically, methyl, trifluoromethyl, 1-hydroxy-1-methylethyl, tert-butyl) optionally having 1 to 3 substituents selected from hydroxy and a halogen atom (e.g., fluorine atom),
   (2) C₂₋₆ alkynyl (e.g., ethynyl)
   (3) a halogen atom (e.g., fluorine atom, chlorine atom, bromine atom),
   (4) cyano,
   (5) nitro,
   (6) C₆₋₁₀ aryl (e.g., phenyl),
   (7) C₁₋₆ alkoxy (e.g., methoxy, ethoxy, specifically, methoxy, trifluoromethoxy, 2-(N-methyl-N-(2-pyrazyl)amino)ethoxy, quinolin-2-ylmethoxy) optionally having 1 to 3 substituents selected from a halogen atom (e.g., fluorine atom), N-methyl-N-(2-pyrazyl)amino and 2-quinolyl,
   (8) C₆₋₁₀ aryloxy (e.g., phenoxy),
   (9) C₆₋₁₀ aryl-C₁₋₆ alkoxy (e.g., benzyloxy),
   (10) carbamoyl,
   (11) C₁₋₆ alkyl-aminocarbonyl (e.g., methylcarbamoyl, ethylcarbamoyl, specifically, methylcarbamoyl, 2-hydroxyethylcarbamoyl) optionally having hydroxy,
   (12) di-C₁₋₆ alkyl-aminocarbonyl (e.g., dimethylcarbamoyl),
   (13) 5- or 6-membered non-aromatic heterocyclyl-carbonyl (e.g., pyrrolidin-1-ylcarbonyl, piperidin-1-ylcarbonyl, morpholin-1-ylcarbonyl),
   (14) amino,
   (15) C₁₋₆ alkoxy-carbonylamino (e.g., tert-butoxycarbonylamino),
   (16) C₁₋₆ alkyl-carbonylamino (e.g., acetylamino, tert-butylcarbonylamino),
   (17) C₃₋₆ cycloalkyl-carbonylamino (e.g., cyclopropylcarbonylamino),
   (18) C₁₋₆ alkylsulfanyl (e.g., methylsulfanyl),
   (19) a 5-membered monocyclic aromatic heterocyclic group (e.g., imidazolyl, pyrazolyl, triazolyl),
   (20) a 6-membered nonaromatic heterocyclic group (e.g., 1-morpholinyl),
   (21) carboxy and
   (22) C₁₋₆ alkoxycarbonyl (e.g., methoxycarbonyl),
(B) a 5- or 6-membered aromatic heterocyclic group (e.g., 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrazyl, 5-imidazolyl, 3-pyrazolyl, 4-pyrazolyl, 5-pyrazolyl, 4-isoxazolyl, 5-isoxazolyl, 4-oxazolyl, 4-triazolyl, 5-thiadiazolyl) optionally substituted by 1 to 3 substituents selected from
   (1) C₁₋₆ alkyl (e.g., methyl, propyl, isopropyl, tert-butyl, specifically, methyl, hydroxymethyl, trifluoromethyl, dimethylaminomethyl, propyl, 1-hydroxy-1-methylethyl, tert-butyl) optionally having 1 to 3 substituents selected from hydroxy, a halogen atom (e.g., fluorine atom) and di-C₁₋₆ alkylamino (e.g., dimethylamino),
   (2) C₆₋₁₀ aryl-C₁₋₆ alkyl (e.g., benzyl),
   (3) C₂₋₆ alkenyl (e.g., ethenyl, specifically, 2-(4-(trifluoromethyl)phenyl)ethenyl) optionally having C₆₋₁₀ aryl (e.g., phenyl) optionally having C₁₋₆ alkyl (e.g., methyl) optionally having 1 to 3 halogen atoms (e.g., fluorine atom),
   (4) a halogen atom (e.g., fluorine atom, chlorine atom, bromine atom),
   (5) cyano,
   (6) nitro,
   (7) C₆₋₁₀ aryl (e.g., phenyl, specifically, phenyl, 4-fluorophenyl, 4-nitrophenyl) optionally having 1 to 3 substituents selected from a halogen atom (e.g., fluorine atom) and nitro,
   (8) C₁₋₆ alkoxy (e.g., ethoxy, specifically, 2,2,2-trifluoroethoxy) optionally having 1 to 3 halogen atoms (e.g., fluorine atom),
   (9) C₆₋₁₀ aryloxy (e.g., phenoxy),
   (10) carbamoyl,
   (11) C₆₋₁₀ aryl-C₁₋₆ alkoxy (e.g., benzyloxy),
   (12) C₁₋₆ alkyl-aminocarbonyl (e.g., methylcarbamoyl),
   (13) di-C₁₋₆ alkyl-aminocarbonyl (e.g., dimethylcarbamoyl),
   (14) 5- or 6-membered non-aromatic heterocyclyl-carbonyl (e.g., pyrrolidin-1-ylcarbonyl, morpholin-1-ylcarbonyl),
   (15) amino,
   (16) a 5- or 6-membered aromatic heterocyclic group (e.g., 2-thienyl, 2-pyridyl, 4-thiazolyl, specifically, 2-thienyl, 2-pyridyl, 5-trifluoromethyl-2-pyridyl, 2-methylthiazol-4-yl) optionally having C₁₋₆ alkyl (e.g., methyl) optionally having 1 to 3 halogen atoms (e.g., fluorine atom),
   (17) a 5- or 6-membered nonaromatic heterocyclic group (e.g., 1-pyrrolidinyl, 1-piperidinyl, 1-morpholinyl),
   (18) C₁₋₆ alkoxycarbonyl (e.g., methoxycarbonyl, ethoxycarbonyl) and
   (19) carboxy,
(C) a condensed aromatic heterocyclic group (e.g., 3-indolyl, 6-quinolyl, 5-benzotriazolyl, 2-benzothienyl, 3-benzothienyl, thienopyrazol-5-yl) optionally substituted by 1 to 3 substituents selected from
   (1) C₁₋₆ alkyl (e.g., methyl) and
   (2) a halogen atom (e.g., chlorine atom),
(D) a 5- or 6-membered nonaromatic heterocyclic group (e.g., 2-tetrahydrofuryl, 2-tetrahydropyranyl, piperidin-3-yl) optionally substituted by 1 to 3 C₁₋₆ alkyl (e.g., methyl) ,
(E) a nonaromatic fused heterocycle group (e.g., 4-dihydrobenzofuranyl, 7-dihydrobenzofuranyl, 1,3-benzodioxol-5-yl, 1,4-benzodioxan-5-yl, 1,5-benzodioxen-6-yl, 1,5-benzodioxen-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl, 3,4-dihydro-2H-1,5-benzodioxepin-7-yl) optionally substituted by 1 to 3 substituents selected from
   (1) C₁₋₆ alkyl (e.g., methyl),
   (2) C₁₋₆ alkoxy (e.g., methoxy) and
   (3) oxo
are preferable.

In compound (I), X is CO, SO₂ or methylene optionally having substituent(s). Preferred as X is methylene optionally having substituent(s). Particularly, preferred as X is methylene optionally having one substituent selected from (1) C₁₋₆ alkyl (e.g., methyl), (2) C₃₋₈ cycloalkyl (e.g., cyclopropyl) and (3) C₆₋₁₀ aryl (e.g., phenyl, 4-chlorophenyl etc.) optionally having 1 to 3 halogen atoms (e.g., chlorine), particularly methylene.

Preferred as compound (I) is a compound wherein
R¹ is a hydrogen atom or C₁₋₆ alkyl optionally having substituent (s) ;
R² is an aromatic hydrocarbon group having cyano (the aromatic hydrocarbon group optionally further has substituent(s)), a nonaromatic heterocyclic group having cyano (the nonaromatic heterocyclic group optionally further has substituent(s)); R³ is a hydrogen atom or C₁₋₆ alkyl optionally having substituent(s);
R⁴ is C₆₋₁₀ aryl optionally having substituent(s), a 5- or 6-membered aromatic heterocyclic group optionally having substituent(s), a condensed aromatic heterocyclic group optionally having substituent(s), a 5- or 6-membered nonaromatic heterocyclic group optionally having substituent(s), a nonaromatic fused heterocycle group optionally having substituent(s); and
X is methylene optionally having substituent(s).

Furthermore, preferred as compound (I) is a compound wherein
R¹ is a hydrogen atom or methyl;
R² is C₆₋₁₀ aryl having cyano (the C₆₋₁₀ aryl may further have 1 or 2 substituents selected from (1) a halogen atom and (2) C₁₋₆ alkyl optionally having 1 to 3 halogen atoms), a 6-membered nonaromatic heterocyclic group having cyano (the 6-membered nonaromatic heterocyclic group may have C₁₋₆ alkyl and oxo);
R³ is a hydrogen atom or methyl;
R⁴ is

(A) C₆₋₁₀ aryl (e.g., phenyl) optionally substituted by 1 to 3 substituents selected from
   (1) C₁₋₆ alkyl (e.g., methyl, isopropyl, tert-butyl, specifically, methyl, trifluoromethyl, 1-hydroxy-1-methylethyl, tert-butyl) optionally having 1 to 3 substituents selected from hydroxy and a halogen atom (e.g., fluorine atom),
   (2) C₂₋₆ alkynyl (e.g., ethynyl),
   (3) a halogen atom (e.g., fluorine atom, chlorine atom, bromine atom),
   (4) cyano,
   (5) nitro,
   (6) C₆₋₁₀ aryl (e.g., phenyl),
   (7) C₁₋₆ alkoxy (e.g., methoxy, ethoxy, specifically, methoxy, trifluoromethoxy, 2-(N-methyl-N-(2-pyrazyl)amino)ethoxy, quinolin-2-ylmethoxy) optionally having 1 to 3 substituents selected from a halogen atom (e.g., fluorine atom), N-methyl-N-(2-pyrazyl)amino and 2-quinolyl,
   (8) C₆₋₁₀ aryloxy (e.g., phenoxy),
   (9) C₆₋₁₀ aryl-C₁₋₆ alkoxy (e.g., benzyloxy),
   (10) carbamoyl,
   (11) C₁₋₆ alkyl-aminocarbonyl (e.g., methylcarbamoyl, ethylcarbamoyl, specifically methylcarbamoyl, 2-hydroxyethylcarbamoyl) optionally having hydroxy,
   (12) di-C₁₋₆ alkyl-aminocarbonyl (e.g., dimethylcarbamoyl),
   (13) 5- or 6-membered non-aromatic heterocyclyl-carbonyl (e.g., pyrrolidin-1-ylcarbonyl, piperidin-1-ylcarbonyl, morpholin-1-ylcarbonyl),
   (14) amino,
   (15) C₁₋₆ alkoxy-carbonylamino (e.g., tert-butoxycarbonylamino),
   (16) C₁₋₆ alkyl-carbonylamino (e.g., acetylamino, tert-butylcarbonylamino),
   (17) C₃₋₆ cycloalkyl-carbonylamino (e.g., cyclopropylcarbonylamino),
   (18) C₁₋₆ alkylsulfanyl (e.g., methylsulfanyl),
   (19) a 5-membered monocyclic aromatic heterocyclic group (e.g., imidazolyl, pyrazolyl, triazolyl),
   (20) a 6-membered nonaromatic heterocyclic group (e.g., 1-morpholinyl),
   (21) carboxy, and
   (22) C₁₋₆ alkoxycarbonyl (e.g., methoxycarbonyl),
(B) a 5- or 6-membered aromatic heterocyclic group (e.g., 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrazyl, 5-imidazolyl, 3-pyrazolyl, 4-pyrazolyl, 5-pyrazolyl, 4-isoxazolyl, 5-isoxazolyl, 4-oxazolyl, 4-triazolyl, 5-thiadiazolyl) optionally substituted by 1 to 3 substituents selected from
   (1) C₁₋₆ alkyl (e.g., methyl, propyl, isopropyl, tert-butyl, specifically, methyl, hydroxymethyl, trifluoromethyl, dimethylaminomethyl, propyl, 1-hydroxy-1-methylethyl, tert-butyl) optionally having 1 to 3 substituents selected from hydroxy, a halogen atom (e.g., fluorine atom) and di-C₁₋₆ alkylamino (e.g., dimethylamino),
   (2) C₆₋₁₀ aryl-C₁₋₆ alkyl (e.g., benzyl),
   (3) C₂₋₆ alkenyl (e.g., ethenyl, specifically, 2-(4-(trifluoromethyl)phenyl)ethenyl) optionally having C₆₋₁₀ aryl (e.g., phenyl) optionally having C₁₋₆ alkyl (e.g., methyl) optionally having 1 to 3 halogen atoms (e.g., fluorine atom),
   (4) a halogen atom (e.g., fluorine atom, chlorine atom, bromine atom),
   (5) cyano,
   (6) nitro,
   (7) C₆₋₁₀ aryl (e.g., phenyl, specifically, phenyl, 4-fluorophenyl, 4-nitrophenyl) optionally having 1 to 3 substituents selected from a halogen atom (e.g., fluorine atom) and nitro,
   (8) C₁₋₆ alkoxy (e.g., ethoxy, specifically, 2,2,2-trifluoroethoxy) optionally having 1 to 3 halogen atoms (e.g., fluorine atom),
   (9) C₆₋₁₀ aryloxy (e.g., phenoxy),
   (10) carbamoyl,
   (11) C₆₋₁₀ aryl-C₁₋₆ alkoxy (e.g., benzyloxy),
   (12) C₁₋₆ alkyl-aminocarbonyl (e.g., methylcarbamoyl),
   (13) di-C₁₋₆ alkyl-aminocarbonyl (e.g., dimethylcarbamoyl),
   (14) 5- or 6-membered non-aromatic heterocyclyl-carbonyl (e.g., pyrrolidin-1-ylcarbonyl, morpholin-1-ylcarbonyl),
   (15) amino,
   (16) a 5- or 6-membered aromatic heterocyclic group (e.g., 2-thienyl, 2-pyridyl, 4-thiazolyl, specifically, 2-thienyl, 2-pyridyl, 5-trifluoromethyl-2-pyridyl, 2-methylthiazol-4-yl) optionally having C₁₋₆ alkyl (e.g., methyl) optionally having 1 to 3 halogen atoms (e.g., fluorine atom),
   (17) a 5- or 6-membered nonaromatic heterocyclic group (e.g., 1-pyrrolidinyl, 1-piperidinyl, 1-morpholinyl),
   (18) C₁₋₆ alkoxycarbonyl (e.g., methoxycarbonyl, ethoxycarbonyl) and
   (19) carboxy,
(C) a condensed aromatic heterocyclic group (e.g., 3-indolyl, 6-quinolyl, 5-benzotriazolyl, 2-benzothienyl, 3-benzothienyl, thienopyrazol-5-yl) optionally substituted by 1 to 3 substituents selected from
   (1) C₁₋₆ alkyl (e.g., methyl), and
   (2) a halogen atom (e.g., chlorine atom),
(D) a 5- or 6-membered nonaromatic heterocyclic group (e.g., 2-tetrahydrofuryl, 2-tetrahydropyranyl, piperidin-3-yl) optionally substituted by 1 to 3 C₁₋₆ alkyl (e.g., methyl),
(E) a nonaromatic fused heterocyclic group (e.g., 4-dihydrobenzofuranyl, 7-dihydrobenzofuranyl, 1,3-benzodioxol-5-yl, 1,4-benzodioxan-5-yl, 1,5-benzodioxen-6-yl, 1,5-benzodioxen-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl, 3,4-dihydro-2H-1,5-benzodioxepin-7-yl) optionally substituted by 1 to 3 substituents selected from
   (1) C₁₋₆ alkyl (e.g., methyl),
   (2) C₁₋₆ alkoxy (e.g., methoxy) and
   (3) oxo; and
      X is methylene optionally having one substituent selected from (1) C₁₋₆ alkyl (e.g., methyl), (2) C₃₋₈ cycloalkyl (e.g., cyclopropyl) and (3) C₆₋₁₀ aryl (e.g., phenyl, 4-chlorophenyl etc.) optionally having 1 to 3 halogen atoms (e.g., chlorine).

More specifically, the following compounds and salts thereof are preferable.
(1) 2-chloro-5-{[4-(3-chloro-4-cyanophenyl)-3,5-dimethyl-1H-pyrazol-1-yl]methyl}benzamide (Example 71)
(2) 2-{[4-(3-chloro-4-cyanophenyl)-3,5-dimethyl-1H-pyrazol-1-yl]methyl}benzamide (Example 72)
(3) 5-{[4-(3-chloro-4-cyanophenyl)-3,5-dimethyl-1H-pyrazol-1-yl]methyl}-2-fluorobenzamide (Example 77)
(4) 5-{[4-(3-chloro-4-cyanophenyl)-3,5-dimethyl-1H-pyrazol-1-yl]methyl}-N-methylpyridine-2-carboxamide (Example 106)
(5) 5-{[4-(3-chloro-4-cyanophenyl)-3,5-dimethyl-1H-pyrazol-1-yl]methyl}nicotinonitrile (Example 118)
(6) 4-({4-[4-cyano-3-(trifluoromethyl)phenyl]-3,5-dimethyl-1H-pyrazol-1-yl}methyl)benzamide (Example 35)
(7) 2-chloro-4-{3,5-dimethyl-1-[4-(1H-pyrazol-1-yl)benzyl]-1H-pyrazol-4-yl}benzonitrile (Example 42)
(8) 4-{[4-(3-chloro-4-cyanophenyl)-3,5-dimethyl-1H-pyrazol-1-yl]methyl}-1H-pyrazole-5-carboxamide (Example 66)
(9) 4-{[4-(3-chloro-4-cyanophenyl)-3,5-dimethyl-1H-pyrazol-1-yl]methyl}benzamide (Example 69)
(10) 5-{[4-(3-chloro-4-cyanophenyl)-3,5-dimethyl-1H-pyrazol-1-yl]methyl}-2-furamide (Example 73)
(11) 2-{[4-(3-chloro-4-cyanophenyl)-3,5-dimethyl-1H-pyrazol-1-yl]methyl}-N,N-dimethyl-1,3-thiazole-4-carboxamide (Example 101)
(12) 2-chloro-4-(1-{[6-chloro-5-(1-hydroxy-1-methylethyl)pyridin-3-yl]methyl}-3,5-dimethyl-1H-pyrazol-4-yl)benzonitrile (Example 124)
(13) 4-{[4-(4-chloro-3-cyanophenyl)-3,5-dimethyl-1H-pyrazol-1-yl]methyl}benzamide (Example 268)

Compound (I') is now explained in detail in the following.

In compound (I'), R¹ is a hydrogen atom, a group via a carbon atom, a group via a nitrogen atom, a group via an oxygen atom or a group via a sulfur atom.
Preferred as R¹ is a hydrogen atom or a group via a carbon atom, more preferably a hydrogen atom, a cyano group, C₁₋₆ alkyl optionally having substituent(s) or acyl (particularly, carboxyl, C₁₋₆ alkoxy-carbonyl optionally having substituent(s), carbamoyl optionally having substituent(s)). Of these, a hydrogen atom, a cyano group, C₁₋₆ alkyl (particularly methyl), carboxyl, C₁₋₆ alkoxy-carbonyl (particularly ethoxycarbonyl) and carbamoyl are preferable.
Specific examples of R¹ include cyano, methyl, carboxyl, ethoxycarbonyl, carbamoyl and the like.

In compound (I'), R² is a phenyl group having cyano (the phenyl group optionally further has substituent(s) other than cyano).
Preferred as R² is a phenyl group having cyano (the phenyl group optionally further has 1 or 2 substituents other than cyano, which is/are selected from (1) a halogen atom, (2) C₁₋₆ alkyl optionally having 1 to 3 halogen atoms and (3) C₁₋₆ alkoxy (e.g., methoxy, ethoxy, propoxy, butoxy, pentoxy, hexoxy etc.)), and more preferred is a phenyl group having cyano (the phenyl group optionally further has 1 or 2 substituents other than cyano, which is/are selected from (1) a halogen atom, (2) C₁₋₆ alkyl having 1 to 3 halogen atoms and (3) C₁₋₆ alkoxy (e.g., methoxy, ethoxy, propoxy, butoxy, pentoxy, hexoxy etc.)). Of these, a phenyl group having cyano (the phenyl group optionally further has 1 or 2 substituents other than cyano, which is/are selected from a fluorine atom, a chlorine atom, trifluoromethyl and methoxy) is preferable.
Specific examples of R² include 4-cyanophenyl, 3-chloro-4-cyanophenyl, 3-cyano-4-chlorophenyl, 4-cyano-3-fluorophenyl, 4-cyano-3-(trifluoromethyl)phenyl, 5-chloro-4-cyano-2-fluorophenyl, 4-cyano-3-chloro-2-fluorophenyl, 3-methoxy-4-cyanophenyl and the like.

In compound (I'), R³ is a hydrogen atom, a group via a carbon atom, a group via a nitrogen atom, a group via an oxygen atom or a group via a sulfur atom.
Preferred as R³ is a hydrogen atom or a group via a carbon atom, and more preferred is a hydrogen atom or C₁₋₆ alkyl optionally having substituent(s). Of these, a hydrogen atom and C₁₋₆ alkyl (particularly methyl) are preferable.

In compound (I'), R⁴ is a cyclic group optionally having substituent(s).
Preferred as R⁴ is C₆₋₁₀ aryl optionally having substituent(s), a 5- or 6-membered aromatic heterocyclic group optionally having substituent(s), a condensed aromatic heterocyclic group optionally having substituent(s), a 5- or 6-membered nonaromatic heterocyclic group optionally having substituent(s), a nonaromatic fused heterocyclic group optionally having substituent(s), and more preferred are phenyl, 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrimidyl, 5-imidazolyl, 3-pyrazolyl, 4-pyrazolyl, 5-pyrazolyl, 4-isoxazolyl, 5-isoxazolyl, 4-oxazolyl, 1,2,3-triazol-4-yl, 5-thiadiazolyl, 3-indolyl, 6-quinolyl, 5-benzotriazolyl, 2-benzothienyl, 3-benzothienyl, thienopyrazol-5-yl, 2-tetrahydrofuryl, 2-tetrahydropyranyl, piperidin-3-yl, 4-dihydrobenzofuranyl, 7-dihydrobenzofuranyl, 1,3-benzodioxol-5-yl, 1,4-benzodioxan-5-yl, 1,4-benzodioxan-6-yl, 1,5-benzodioxepin-6-yl, 1,5-benzodioxepin-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl and 3,4-dihydro-2H-1,5-benzodioxepin-7-yl, each of which optionally has substituent(s).
Specifically preferred as R⁴ is
(A) C₆₋₁₀ aryl (e.g., phenyl) optionally substituted by 1 to 3 substituents selected from
   (1) C₁₋₆ alkyl (e.g., methyl, isopropyl, tert-butyl, specifically, methyl, trifluoromethyl, 1-hydroxy-1-methylethyl, tert-butyl) optionally having 1 to 3 substituents selected from hydroxy and a halogen atom (e.g., fluorine atom),
   (2) C₂₋₆ alkynyl (e.g., ethynyl)
   (3) a halogen atom (e.g., fluorine atom, chlorine atom, bromine atom),
   (4) cyano,
   (5) nitro,
   (6) C₆₋₁₀ aryl (e.g., phenyl),
   (7) C₁₋₆ alkoxy (e.g., methoxy, ethoxy, specifically, methoxy, trifluoromethoxy, 2-(N-methyl-N-(2-pyrazyl)amino)ethoxy, quinolin-2-ylmethoxy) optionally having 1 to 3 substituents selected from a halogen atom (e.g., fluorine atom), N-methyl-N-(2-pyrazyl)amino and 2-quinolyl,
   (8) C₆₋₁₀ aryloxy (e.g., phenoxy),
   (9) C₆₋₁₀ aryl-C₁₋₆ alkoxy (e.g., benzyloxy),
   (10) carbamoyl,
   (11) C₁₋₆ alkyl-aminocarbonyl (e.g., methylcarbamoyl, ethylcarbamoyl, propylcarbamoyl, isobutylcarbamoyl, tert-butylcarbamoyl, 2-ethylbutylcarbamoyl, specifically, methylcarbamoyl, ethylcarbamoyl, propylcarbamoyl, 2-hydroxy-2-methylpropylcarbamoyl, tert-butylcarbamoyl, 2-ethylbutylcarbamoyl, 2-hydroxyethylcarbamoyl, 1-hydroxycyclopropylmethylcarbamoyl, methoxycarbonylmethylcarbamoyl, 2-hydroxy-2-ethylbutylcarbamoyl, carboxymethylcarbamoyl, carbamoylmethylcarbamoyl, methylcarbamoylmethylcarbamoyl, dimethylcarbamoylmethylcarbamoyl, cyanomethylcarbamoyl, 2-pyridylmethylcarbamoyl, 3-pyridylmethylcarbamoyl, 4-pyridylmethylcarbamoyl, 2-thienylmethylcarbamoyl, (1-oxidopyridin-2-yl)methylcarbamoyl, (6-cyanopyridin-2-yl)methylcarbamoyl) optionally having 1 to 3 substituents selected from cyano, hydroxy, C₃₋₈ cycloalkyl (e.g., cyclopropyl), carboxyl, C₁₋₆ alkoxycarbonyl (e.g., methoxycarbonyl), carbamoyl, C₁₋₆ alkyl-aminocarbonyl (e.g., methylcarbamoyl), di-C₁₋₆ alkyl-aminocarbonyl (e.g., dimethylcarbamoyl), and a 5- or 6-membered aromatic heterocyclic group (e.g., 2-pyridyl, 3-pyridyl, 4-pyridyl, 2-thienyl; the aromatic heterocyclic group may be substituted by cyano or oxo),
   (12) di-C₁₋₆ alkyl-aminocarbonyl (e.g., dimethylcarbamoyl),
   (13) C₃₋₈ cycloalkyl-aminocarbonyl (e.g., cyclopropylcarbamoyl, cyclobutylcarbamoyl),
   (14) 4- to 6-membered non-aromatic heterocyclyl-carbonyl (e.g., azetidin-1-ylcarbonyl, pyrrolidin-1-ylcarbonyl, piperidin-1-ylcarbonyl, morpholin-1-ylcarbonyl),
   (15) amino,
   (16) C₁₋₆ alkoxy-carbonylamino (e.g., tert-butoxycarbonylamino),
   (17) C₁₋₆ alkyl-carbonylamino (e.g., acetylamino, tert-butylcarbonylamino),
   (18) C₃₋₆ cycloalkyl-carbonylamino (e.g., cyclopropylcarbonylamino),
   (19) C₁₋₆ alkylsulfanyl (e.g., methylsulfanyl),
   (20) a 5-membered monocyclic aromatic heterocyclic group (e.g., imidazolyl, pyrazolyl, thiazolyl, triazolyl, 1,3,4-oxadiazolyl, specifically, 4-carboxy-1,3-thiazol-2-yl, 4-ethoxycarbonyl-1,3-thiazol-2-yl, 4-(1-hydroxy-1-methylethyl)-1,3-thiazol-2-yl, 4-carbamoyl-1,3-thiazol-2-yl, 4,5-dimethyl-1,3-thiazol-2-yl, 1,3,4-oxadiazol-2-yl, 5-methyl-1,3,4-oxadiazol-2-yl) optionally having substituent(s) selected from C₁₋₆ alkyl (e.g., methyl, isopropyl) optionally having hydroxy, carboxyl, C₁₋₆ alkoxycarbonyl (e.g., ethoxycarbonyl) and carbamoyl,
   (21) a 5- or 6-membered nonaromatic heterocyclic group (e.g., 1,1-dioxidoisothiazolidin-2-yl, 1-morpholinyl) optionally having 1 or 2 oxo,
   (22) carboxy,
   (23) C₁₋₆ alkoxycarbonyl (e.g., methoxycarbonyl, tert-butoxycarbonyl),
   (24) C₁₋₆ alkylsulfonyl (e.g., methylsulfonyl),
   (25) aminosulfonyl,
   (26) C₁₋₆ alkyl-aminosulfonyl (e.g., methylaminosulfonyl, ethylaminosulfonyl, isobutylaminosulfonyl, tert-butylaminosulfonyl, specifically, 2-hydroxyethylaminosulfonyl, 2-hydroxy-2-methylpropylaminosulfonyl) optionally having hydroxy,
   (27) C₃₋₆ cycloalkyl-aminosulfonyl (e.g., cyclopropylaminosulfonyl),
   (28) 4- to 6-membered non-aromatic heterocyclyl-sulfonyl (e.g., azetidin-1-ylsulfonyl, pyrrolidin-1-ylsulfonyl, morpholin-4-ylsulfonyl, specifically, 3-hydroxyazetidin-1-ylsulfonyl) optionally having hydroxy,
   (29) C₁₋₆ alkyl (C₁₋₆ alkoxycarbonyl)aminosulfonyl (e.g., methyl(tert-butoxycarbonyl)aminosulfonyl),
   (30) sulfo,
   (31) C₆₋₁₀ aryloxy-sulfonyl (e.g., phenyloxysulfonyl),
   (32) halogenated sulfonyl (e.g., chlorosulfonyl),
   (33) C₁₋₆ alkylsulfonylamino (e.g., methylsulfonylamino, propylsulfonylamino, isopropylsulfonylamino, specifically, 3-chloropropylsulfonylamino) optionally having 1 to 3 halogen atoms (e.g., chlorine atom),
   (34) C₆₋₁₀ arylsulfonylamino (e.g., phenylsulfonylamino, specifically, 4-fluorophenylsulfonylamino) optionally having 1 to 3 halogen atoms (e.g., fluorine atom),
   (35) C₁₋₆ alkyl (C₁₋₆ alkylsulfonyl)amino (e.g., methyl(isopropylsulfonyl)amino), and
   (36) thiocarbamoyl,
(B) a 5- or 6-membered aromatic heterocyclic group (e.g., 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrimidyl, 5-imidazolyl, 3-pyrazolyl, 4-pyrazolyl, 5-pyrazolyl, 4-isoxazolyl, 5-isoxazolyl, 4-oxazolyl, 1,2,3-triazol-4-yl, 5-thiadiazolyl) optionally substituted by 1 to 3 substituents selected from
   (1) C₁₋₆ alkyl (e.g., methyl, propyl, isopropyl, tert-butyl, specifically, methyl, hydroxymethyl, trifluoromethyl, dimethylaminomethyl, propyl, 1-hydroxy-1-methylethyl, tert-butyl) optionally having 1 to 3 substituents selected from hydroxy, a halogen atom (e.g., fluorine atom) and di-C₁₋₆ alkylamino (e.g., dimethylamino),
   (2) C₆₋₁₀ aryl-C₁₋₆ alkyl (e.g., benzyl),
   (3) C₂₋₆ alkenyl (e.g., ethenyl, specifically, 2-(4-(trifluoromethyl)phenyl)ethenyl) optionally having C₆₋₁₀ aryl (e.g., phenyl) optionally having C₁₋₆ alkyl (e.g., methyl) optionally having 1 to 3 halogen atoms (e.g., fluorine atom),
   (4) a halogen atom (e.g., fluorine atom, chlorine atom, bromine atom),
   (5) cyano,
   (6) nitro,
   (7) C₆₋₁₀ aryl (e.g., phenyl, specifically, phenyl, 4-fluorophenyl, 4-nitrophenyl) optionally having 1 to 3 substituents selected from a halogen atom (e.g., fluorine atom) and nitro,
   (8) C₁₋₆ alkoxy (e.g., ethoxy, specifically, 2,2,2-trifluoroethoxy) optionally having 1 to 3 halogen atoms (e.g., fluorine atom),
   (9) C₆₋₁₀ aryloxy (e.g., phenoxy),
   (10) carbamoyl,
   (11) C₆₋₁₀ aryl-C₁₋₆ alkoxy (e.g., benzyloxy),
   (12) C₁₋₆ alkyl-aminocarbonyl (e.g., methylcarbamoyl, ethylcarbamoyl, propylcarbamoyl, isobutylcarbamoyl, specifically, 2-hydroxy-2-methylpropylcarbamoyl) optionally having hydroxy,
   (13) di-C₁₋₆ alkyl-aminocarbonyl (e.g., dimethylcarbamoyl),
   (14) C₃₋₈ cycloalkyl-aminocarbonyl (e.g., cyclopropylcarbamoyl, cyclobutylcarbamoyl),
   (15) 5- or 6-membered non-aromatic heterocyclyl-carbonyl (e.g., pyrrolidin-1-ylcarbonyl, morpholin-1-ylcarbonyl),
   (16) amino,
   (17) a 5- or 6-membered aromatic heterocyclic group (e.g., 2-thienyl, 2-pyridyl, 4-thiazolyl, 1,3,4-oxadiazol-2-yl, specifically, 2-thienyl, 2-pyridyl, 5-trifluoromethyl-2-pyridyl, 2-methylthiazol-4-yl, 5-methyl-1,3,4-oxadiazol-2-yl) optionally having C₁₋₆ alkyl (e.g., methyl) optionally having 1 to 3 halogen atoms (e.g., fluorine atom),
   (18) a 5- or 6-membered nonaromatic heterocyclic group (e.g., 1-pyrrolidinyl, 1-piperidinyl, 1-morpholinyl),
   (19) C₁₋₆ alkoxycarbonyl (e.g., methoxycarbonyl, ethoxycarbonyl),
   (20) carboxy, and
   (21) hydrazinocarbonyl,
(C) a condensed aromatic heterocyclic group (e.g., 3-indolyl, 6-quinolyl, 5-benzotriazolyl, 2-benzothienyl, 3-benzothienyl, thienopyrazol-5-yl) optionally substituted by 1 to 3 substituents selected from
   (1) C₁₋₆ alkyl (e.g., methyl), and
   (2) a halogen atom (e.g., chlorine atom),
(D) a 5- or 6-membered nonaromatic heterocyclic group (e.g., 2-tetrahydrofuryl, 2-tetrahydropyranyl, piperidin-3-yl) optionally substituted by 1 to 3 C₁₋₆ alkyl (e.g., methyl), or
(E) a nonaromatic fused heterocycle group (e.g., 4-dihydrobenzofuranyl, 7-dihydrobenzofuranyl, 1,3-benzodioxol-5-yl, 1,4-benzodioxan-5-yl, 1,4-benzodioxan-6-yl, 1,5-benzodioxepin-6-yl, 1,5-benzodioxepin-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl, 3,4-dihydro-2H-1,5-benzodioxepin-7-yl) optionally substituted by 1 to 3 substituents selected from
   (1) C₁₋₆ alkyl (e.g., methyl),
   (2) C₁₋₆ alkoxy (e.g., methoxy), and
   (3) oxo.

In compound (I'), X is CO or methylene optionally having substituent(s).
Preferred as X is methylene optionally having substituent(s). Specifically, preferred as X is methylene optionally having one substituent(s) selected from (1) C₁₋₆ alkyl (e.g., methyl), (2) C₃₋₈ cycloalkyl (e.g., cyclopropyl) and (3) C₆₋₁₀ aryl (e.g., phenyl, 4-chlorophenyl etc.) optionally having 1 to 3 hetero atoms (e.g., chlorine), and particularly preferred is methylene.

Preferred as compound (I') is a compound wherein
R¹ is a hydrogen atom or a group via a carbon atom;
R² is a phenyl group having cyano (the phenyl group optionally further has 1 or 2 substituents other than cyano, which is/are selected from (1) a halogen atom, (2) C₁₋₆ alkyl optionally having 1 to 3 halogen atoms, and (3) C₁₋₆ alkoxy (e.g., methoxy, ethoxy, propoxy, butoxy, pentoxy, hexoxy etc.));
R³ is a hydrogen atom or a group via a carbon atom;
R⁴ is C₆₋₁₀ aryl optionally having substituent(s), a 5- or 6-membered aromatic heterocyclic group optionally having substituent(s), a condensed aromatic heterocyclic group optionally having substituent(s), a 5- or 6-membered nonaromatic heterocyclic group optionally having substituent(s) or a nonaromatic fused heterocyclic group optionally having substituent(s); and
X is methylene optionally having substituent(s).

Furthermore, preferred as compound (I') is a compound wherein
R¹ is a hydrogen atom, a cyano group, C₁₋₆ alkyl optionally having substituent(s), or acyl (particularly, carboxyl, C₁₋₆ alkoxy-carbonyl optionally having substituent(s), carbamoyl optionally having substituent(s));
R² is a phenyl group having cyano (the phenyl group optionally further has 1 or 2 substituents other than cyano, which is/are selected from (1) a halogen atom, (2) C₁₋₆ alkyl having 1 to 3 halogen atoms and (3) C₁₋₆ alkoxy);
R³ is a hydrogen atom or C₁₋₆ alkyl optionally having substituent (s) ;
R⁴ is phenyl, 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrimidyl, 5-imidazolyl, 3-pyrazolyl, 4-pyrazolyl, 5-pyrazolyl, 4-isoxazolyl, 5-isoxazolyl, 4-oxazolyl, 1,2,3-triazol-4-yl, 5-thiadiazolyl, 3-indolyl, 6-quinolyl, 5-benzotriazolyl, 2-benzothienyl, 3-benzothienyl, thienopyrazol-5-yl, 2-tetrahydrofuryl, 2-tetrahydropyranyl, piperidin-3-yl, 4-dihydrobenzofuranyl, 7-dihydrobenzofuranyl, 1,3-benzodioxol-5-yl, 1,4-benzodioxan-5-yl, 1,4-benzodioxan-6-yl, 1,5-benzodioxepin-6-yl, 1,5-benzodioxepin-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl or 3,4-dihydro-2H-1,5-benzodioxepin-7-yl, each of which optionally has substituent(s); and
X is methylene optionally having one substituent selected from (1) C₁₋₆ alkyl (e.g., methyl), (2) C₃₋₈ cycloalkyl (e.g., cyclopropyl) and (3) C₆₋₁₀ aryl (e.g., phenyl, 4-chlorophenyl etc.) optionally having 1 to 3 halogen atoms (e.g., chlorine).

Particularly preferred as compound (I') is a compound wherein
R¹ is a hydrogen atom, a cyano group, C₁₋₆ alkyl (particularly methyl), carboxyl, C₁₋₆ alkoxy-carbonyl (particularly ethoxycarbonyl) or carbamoyl;
R² is a phenyl group having cyano (the phenyl group optionally further has 1 or 2 substituents other than cyano, which is/are selected from a fluorine atom, a chlorine atom, trifluoromethyl and methoxy);
R³ is a hydrogen atom or methyl;
R⁴ is

(A) C₆₋₁₀ aryl (e.g., phenyl) optionally substituted by 1 to 3 substituents selected from
   (1) C₁₋₆ alkyl (e.g., methyl, isopropyl, tert-butyl, specifically, methyl, trifluoromethyl, 1-hydroxy-1-methylethyl, tert-butyl) optionally having 1 to 3 substituents selected from hydroxy and a halogen atom (e.g., fluorine atom),
   (2) C₂₋₆ alkynyl (e.g., ethynyl)
   (3) a halogen atom (e.g., fluorine atom, chlorine atom, bromine atom),
   (4) cyano,
   (5) nitro,
   (6) C₆₋₁₀ aryl (e.g., phenyl),
   (7) C₁₋₆ alkoxy (e.g., methoxy, ethoxy, specifically, methoxy, trifluoromethoxy, 2-(N-methyl-N-(2-pyrazyl)amino)ethoxy, quinolin-2-ylmethoxy) optionally having 1 to 3 substituents selected from a halogen atom (e.g., fluorine atom), N-methyl-N-(2-pyrazyl)amino, and 2-quinolyl,
   (8) C₆₋₁₀ aryloxy (e.g., phenoxy),
   (9) C₆₋₁₀ aryl-C₁₋₆ alkoxy (e.g., benzyloxy),
   (10) carbamoyl,
   (11) C₁₋₆ alkyl-aminocarbonyl (e.g., methylcarbamoyl, ethylcarbamoyl, propylcarbamoyl, isobutylcarbamoyl, tert-butylcarbamoyl, 2-ethylbutylcarbamoyl, specifically, methylcarbamoyl, ethylcarbamoyl, propylcarbamoyl, 2-hydroxy-2-methylpropylcarbamoyl, tert-butylcarbamoyl, 2-ethylbutylcarbamoyl, 2-hydroxyethylcarbamoyl, 1-hydroxycyclopropylmethylcarbamoyl, methoxycarbonylmethylcarbamoyl, 2-hydroxy-2-ethylbutylcarbamoyl, carboxymethylcarbamoyl, carbamoylmethylcarbamoyl, methylcarbamoylmethylcarbamoyl, dimethylcarbamoylmethylcarbamoyl, cyanomethylcarbamoyl, 2-pyridylmethylcarbamoyl, 3-pyridylmethylcarbamoyl, 4-pyridylmethylcarbamoyl, 2-thienylmethylcarbamoyl, (1-oxidopyridin-2-yl)methylcarbamoyl, (6-cyanopyridin-2-yl)methylcarbamoyl) optionally having 1 to 3 substituents selected from cyano, hydroxy, C₃₋₈ cycloalkyl (e.g., cyclopropyl), carboxyl, C₁₋₆ alkoxycarbonyl (e.g., methoxycarbonyl), carbamoyl, C₁₋₆ alkyl-aminocarbonyl (e.g., methylcarbamoyl), di-C₁₋₆ alkyl-aminocarbonyl (e.g., dimethylcarbamoyl), and a 5- or 6-membered aromatic heterocyclic group (e.g., 2-pyridyl, 3-pyridyl, 4-pyridyl, 2-thienyl; the aromatic heterocyclic group may be substituted by cyano or oxo),
   (12) di-C₁₋₆ alkyl-aminocarbonyl (e.g., dimethylcarbamoyl),
   (13) C₃₋₈ cycloalkyl-aminocarbonyl (e.g., cyclopropylcarbamoyl, cyclobutylcarbamoyl),
   (14) 4- to 6-membered non-aromatic heterocyclyl-carbonyl (e.g., azetidin-1-ylcarbonyl, pyrrolidin-1-ylcarbonyl, piperidin-1-ylcarbonyl, morpholin-1-ylcarbonyl),
   (15) amino,
   (16) C₁₋₆ alkoxy-carbonylamino (e.g., tert-butoxycarbonylamino),
   (17) C₁₋₆ alkyl-carbonylamino (e.g., acetylamino, tert-butylcarbonylamino),
   (18) C₃₋₆ cycloalkyl-carbonylamino (e.g., cyclopropylcarbonylamino),
   (19) C₁₋₆ alkylsulfanyl (e.g., methylsulfanyl),
   (20) a 5-membered monocyclic aromatic heterocyclic group (e.g., imidazolyl, pyrazolyl, thiazolyl, triazolyl, 1,3,4-oxadiazolyl, specifically, 4-carboxy-1,3-thiazol-2-yl, 4-ethoxycarbonyl-1,3-thiazol-2-yl, 4-(l-hydroxy-l-methylethyl)-1,3-thiazol-2-yl, 4-carbamoyl-1,3-thiazol-2-yl, 4,5-dimethyl-1,3-thiazol-2-yl, 1,3,4-oxadiazol-2-yl, 5-methyl-1,3,4-oxadiazol-2-yl) optionally having substituent(s) selected from C₁₋₆ alkyl (e.g., methyl, isopropyl) optionally having hydroxy, carboxyl, C₁₋₆ alkoxycarbonyl (e.g., ethoxycarbonyl) and carbamoyl,
   (21) a 5- or 6-membered nonaromatic heterocyclic group (e.g., 1,1-dioxidoisothiazolidin-2-yl, 1-morpholinyl) optionally having 1 or 2 oxo,
   (22) carboxy,
   (23) C₁₋₆ alkoxycarbonyl (e.g., methoxycarbonyl, tert-butoxycarbonyl),
   (24) C₁₋₆ alkylsulfonyl (e.g., methylsulfonyl),
   (25) aminosulfonyl,
   (26) C₁₋₆ alkyl-aminosulfonyl (e.g., methylaminosulfonyl, ethylaminosulfonyl, isobutylaminosulfonyl, tert-butylaminosulfonyl, specifically, 2-hydroxyethylaminosulfonyl, 2-hydroxy-2-methylpropylaminosulfonyl) optionally having hydroxy,
   (27) C₃₋₆ cycloalkyl-aminosulfonyl (e.g., cyclopropylaminosulfonyl),
   (28) 4- to 6-membered non-aromatic heterocyclyl-sulfonyl (e.g., azetidin-1-ylsulfonyl, pyrrolidin-1-ylsulfonyl, morpholin-4-ylsulfonyl, specifically, 3-hydroxyazetidin-1-ylsulfonyl) optionally having hydroxy,
   (29) C₁₋₆ alkyl (C₁₋₆ alkoxycarbonyl)aminosulfonyl (e.g., methyl(tert-butoxycarbonyl)aminosulfonyl),
   (30) sulfo,
   (31) C₆₋₁₀ aryloxy-sulfonyl (e.g., phenyloxysulfonyl),
   (32) halogenated sulfonyl (e.g., chlorosulfonyl),
   (33) C₁₋₆ alkylsulfonylamino (e.g., methylsulfonylamino, propylsulfonylamino, isopropylsulfonylamino, specifically, 3-chloropropylsulfonylamino) optionally having 1 to 3 halogen atoms (e.g., chlorine atom),
   (34) C₆₋₁₀ arylsulfonylamino (e.g., phenylsulfonylamino, specifically, 4-fluorophenylsulfonylamino) optionally having 1 to 3 halogen atoms (e.g., fluorine atom),
   (35) C₁₋₆ alkyl (C₁₋₆ alkylsulfonyl)amino (e.g., methyl(isopropylsulfonyl)amino), and
   (36) thiocarbamoyl,
(B) a 5- or 6-membered aromatic heterocyclic group (e.g., 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrimidyl, 5-imidazolyl, 3-pyrazolyl, 4-pyrazolyl, 5-pyrazolyl, 4-isoxazolyl, 5-isoxazolyl, 4-oxazolyl, 1,2,3-triazol-4-yl, 5-thiadiazolyl) optionally substituted by 1 to 3 substituents selected from
   (1) C₁₋₆ alkyl (e.g., methyl, propyl, isopropyl, tert-butyl, specifically methyl, hydroxymethyl, trifluoromethyl, dimethylaminomethyl, propyl, 1-hydroxy-1-methylethyl, tert-butyl) optionally having 1 to 3 substituents selected from hydroxy, a halogen atom (e.g., fluorine atom) and di-C₁₋₆ alkylamino (e.g., dimethylamino),
   (2) C₆₋₁₀ aryl-C₁₋₆ alkyl (e.g., benzyl),
   (3) C₂₋₆ alkenyl (e.g., ethenyl, specifically, 2-(4-(trifluoromethyl)phenyl)ethenyl) optionally having C₆₋₁₀ aryl (e.g., phenyl) optionally having C₁₋₆ alkyl (e.g., methyl) optionally having 1 to 3 halogen atoms (e.g., fluorine atom),
   (4) a halogen atom (e.g., fluorine atom, chlorine atom, bromine atom),
   (5) cyano,
   (6) nitro,
   (7) C₆₋₁₀ aryl (e.g., phenyl, specifically, phenyl, 4-fluorophenyl, 4-nitrophenyl) optionally having 1 to 3 substituents selected from a halogen atom (e.g., fluorine atom) and nitro,
   (8) C₁₋₆ alkoxy (e.g., ethoxy, specifically, 2,2,2-trifluoroethoxy) optionally having 1 to 3 halogen atoms (e.g., fluorine atom),
   (9) C₆₋₁₀ aryloxy (e.g., phenoxy),
   (10) carbamoyl,
   (11) C₆₋₁₀ aryl-C₁₋₆ alkoxy (e.g., benzyloxy),
   (12) C₁₋₆ alkyl-aminocarbonyl (e.g., methylcarbamoyl, ethylcarbamoyl, propylcarbamoyl, isobutylcarbamoyl, specifically, 2-hydroxy-2-methylpropylcarbamoyl) optionally having hydroxy,
   (13) di-C₁₋₆ alkyl-aminocarbonyl (e.g., dimethylcarbamoyl),
   (14) C₃₋₈ cycloalkyl-aminocarbonyl (e.g., cyclopropylcarbamoyl, cyclobutylcarbamoyl),
   (15) 5- or 6-membered non-aromatic heterocyclyl-carbonyl (e.g., pyrrolidin-1-ylcarbonyl, morpholin-1-ylcarbonyl),
   (16) amino,
   (17) a 5- or 6-membered aromatic heterocyclic group (e.g., 2-thienyl, 2-pyridyl, 4-thiazolyl, 1,3,4-oxadiazol-2-yl, specifically, 2-thienyl, 2-pyridyl, 5-trifluoromethyl-2-pyridyl, 2-methylthiazol-4-yl, 5-methyl-1,3,4-oxadiazol-2-yl) optionally having C₁₋₆ alkyl (e.g., methyl) optionally having 1 to 3 halogen atoms (e.g., fluorine atom),
   (18) a 5- or 6-membered nonaromatic heterocyclic group (e.g., 1-pyrrolidinyl, 1-piperidinyl, 1-morpholinyl),
   (19) C₁₋₆ alkoxycarbonyl (e.g., methoxycarbonyl, ethoxycarbonyl),
   (20) carboxy, and
   (21) hydrazinocarbonyl,
(C) a condensed aromatic heterocyclic group (e.g., 3-indolyl, 6-quinolyl, 5-benzotriazolyl, 2-benzothienyl, 3-benzothienyl, thienopyrazol-5-yl) optionally substituted by 1 to 3 substituents selected from
   (1) C₁₋₆ alkyl (e.g., methyl), and
   (2) a halogen atom (e.g., chlorine atom),
(D) a 5- or 6-membered nonaromatic heterocyclic group (e.g., 2-tetrahydrofuryl, 2-tetrahydropyranyl, piperidin-3-yl) optionally substituted by 1 to 3 C₁₋₆ alkyl (e.g., methyl), or
(E) a nonaromatic fused heterocyclic group (e.g., 4-dihydrobenzofuranyl, 7-dihydrobenzofuranyl, 1,3-benzodioxol-5-yl, 1,4-benzodioxan-5-yl, 1,4-benzodioxan-6-yl, 1,5-benzodioxepin-6-yl, 1,5-benzodioxepin-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl, 3,4-dihydro-2H-1,5-benzodioxepin-7-yl) optionally substituted by 1 to 3 substituents selected from
   (1) C₁₋₆ alkyl (e.g., methyl),
   (2) C₁₋₆ alkoxy (e.g., methoxy), and
   (3) oxo; and
      X is methylene.

More specifically, preferred as compound (I') are the following compounds or salts thereof.
(1) 3-{[4-(3-chloro-4-cyanophenyl)-3,5-dimethyl-1H-pyrazol-1-yl]methyl}benzamide (Example 68),
(2) 4-{[4-(3-chloro-4-cyanophenyl)-3,5-dimethyl-1H-pyrazol-1-yl]methyl}benzamide (Example 69),
(3) 5-{[4-(3-chloro-4-cyanophenyl)-3,5-dimethyl-1H-pyrazol-1-yl]methyl}-2-fluorobenzamide (Example 77),
(4) 5-{[4-(3-chloro-4-cyanophenyl)-3,5-dimethyl-1H-pyrazol-1-yl]methyl}pyridine-2-carboxamide (Example 105),
(5) 5-{[4-(3-chloro-4-cyanophenyl)-3,5-dimethyl-1H-pyrazol-1-yl]methyl}-N-methylpyridine-2-carboxamide (Example 106),
(6) 4-{[4-(3-chloro-4-cyanophenyl)-3,5-dimethyl-1H-pyrazol-1-yl]methyl}-N-(2-hydroxy-2-methylpropyl)benzamide (Example 282),
(7) 2-chloro-4-{3,5-dimethyl-1-[4-(5-methyl-1,3,4-oxadiazol-2-yl)benzyl]-1H-pyrazol-4-yl}benzonitrile (Example 286),
(8) 4-{[4-(3-chloro-4-cyanophenyl)-3,5-dimethyl-1H-pyrazol-1-yl]methyl}-N-cyclopropylbenzamide (Example 304),
(9) 5-{[4-(3-chloro-4-cyanophenyl)-3,5-dimethyl-1H-pyrazol-1-yl]methyl}-N-cyclopropylpyridine-2-carboxamide (Example 310),
(10) 5-({4-[4-cyano-3-(trifluoromethyl)phenyl]-3,5-dimethyl-1H-pyrazol-1-yl}methyl)pyridine-2-carboxamide (Example 311),
(11) 2-chloro-4-(3,5-dimethyl-1-{[6-(5-methyl-1,3,4-oxadiazol-2-yl)pyridin-3-yl]methyl}-1H-pyrazol-4-yl)benzonitrile (Example 319),
(12) 4-{[4-(3-chloro-4-cyanophenyl)-3,5-dimethyl-1H-pyrazol-1-yl]methyl}benzenesulfonamide (Example 322),
(13) 4-{[4-(3-chloro-4-cyano-2-fluorophenyl)-3,5-dimethyl-1H-pyrazol-1-yl]methyl}benzamide (Example 337).

Compound (IA) is now explained in detail in the following.

In compound (IA), ring A is a benzene ring optionally further having substituent(s) besides cyano.
Preferred as ring A is a benzene ring optionally further having 1 or 2 substituents besides cyano, which is/are selected from (1) a halogen atom, (2) a C₁₋₆ alkyl group, (3) a halogeno-C₁₋₆ alkyl group, and (4)-OR_{c} wherein R_{c} is a hydrogen atom, a C₁₋₆ alkyl group optionally having substituent(s) or a C₃₋₇ cycloalkyl group, and more preferred is a benzene ring optionally further having 1 or 2 substituents besides cyano, which is/are selected from a fluorine atom, a chlorine atom, trifluoromethyl and methoxy.
Specific examples of ring A as a phenyl group derived from ring A include 4-cyanophenyl, 3-chloro-4-cyanophenyl, 3-cyano-4-chlorophenyl, 4-cyano-3-fluorophenyl, 4-cyano-3-(trifluoromethyl)phenyl, 5-chloro-4-cyano-2-fluorophenyl, 4-cyano-3-chloro-2-fluorophenyl, 3-methoxy-4-cyanophenyl and the like.

In compound (IA), ring B is an aromatic hydrocarbon ring optionally having substituent(s) or heterocycle optionally having substituent(s).
Preferred as ring B is a benzene ring optionally having substituent(s), a pyridine ring optionally having substituent(s), a thiazole ring optionally having substituent(s) or a pyrazole ring optionally having substituent(s), and more preferred is a benzene ring, a pyridine ring, a thiazole ring or a pyrazole ring, each of which optionally has 1 to 5 (preferably 1 to 3) substituents selected from (1) a halogen atom, (2) a C₁₋₆ alkyl group optionally having substituent(s), (3) a C₂₋₆ alkenyl group optionally having substituent(s), (4) a C₂₋₆ alkynyl group optionally having substituent(s), (5) a nitro group, (6) a phenyl group optionally having substituent(s), (7) a heterocyclic group optionally having substituent(s), (8) a cyano group, (9) -CONR_{d}Rₑ, (10) -CO₂R_{d}, (11) -NR_{d}CORₑ, (12) - SO₂NR_{d}Rₑ (13) -NR_{d}SO₂Rₑ, (14) -C(OH)R_{d}Rₑ, (15) -NR_{d}Rₑ, (16) -OR_{d}, and (17) -SR_{d} wherein R_{d} and Rₑ are the same or different and each is a hydrogen atom, a C₁₋₆ alkyl group optionally having substituent(s), a C₃₋₇ cycloalkyl group or a phenyl group optionally having substituent(s).

Specific examples of the substituent of the above-mentioned benzene ring, pyridine ring, thiazole ring and pyrazole ring include methyl, propyl, isopropyl, tert-butyl, trifluoromethyl, hydroxymethyl, 1-hydroxy-1-methylethyl, ethynyl, fluorine atom, chlorine atom, bromine atom, cyano, nitro, phenyl, methoxy, ethoxy, trifluoromethoxy, 2-(N-methyl-N-(2-pyrazyl)amino)ethoxy, quinolin-2-ylmethoxy, phenoxy, benzyloxy, carbamoyl, thiocarbamoyl, methylcarbamoyl, ethylcarbamoyl, propylcarbamoyl, isobutylcarbamoyl, tert-butylcarbamoyl, 2-ethylbutylcarbamoyl, 2-hydroxy-2-methylpropylcarbamoyl, 2-hydroxyethylcarbamoyl, 1-hydroxycyclopropylmethylcarbamoyl, methoxycarbonylmethylcarbamoyl, 2-hydroxy-2-ethylbutylcarbamoyl, carboxymethylcarbamoyl, carbamoylmethylcarbamoyl, methylcarbamoylmethylcarbamoyl, dimethylcarbamoylmethylcarbamoyl, cyanomethylcarbamoyl, 2-pyridylmethylcarbamoyl, 3-pyridylmethylcarbamoyl, 4-pyridylmethylcarbamoyl, 2-thienylmethylcarbamoyl, (1-oxidopyridin-2-yl)methylcarbamoyl, (6-cyanopyridin-2-yl)methylcarbamoyl, dimethylcarbamoyl, cyclopropylcarbamoyl, cyclobutylcarbamoyl, azetidin-1-ylcarbonyl, pyrrolidin-1-ylcarbonyl, piperidin-1-ylcarbonyl, morpholin-1-ylcarbonyl, amino, tert-butoxycarbonylamino, acetylamino, tert-butylcarbonylamino, cyclopropylcarbonylamino, methylsulfanyl, imidazolyl, pyrazolyl, thiazolyl, triazolyl, 1,3,4-oxadiazolyl, 4-carboxy-1,3-thiazol-2-yl, 4-ethoxycarbonyl-1,3-thiazol-2-yl, 4-(1-hydroxy-1-methylethyl)-1,3-thiazol-2-yl, 4-carbamoyl-1,3-thiazol-2-yl, 4,5-dimethyl-1,3-thiazol-2-yl, 1,3,4-oxadiazol-2-yl, 5-methyl-1,3,4-oxadiazol-2-yl, 1,1-dioxidoisothiazolidin-2-yl, 1-morpholinyl, carboxy, methoxycarbonyl, ethoxycarbonyl, tert-butoxycarbonyl, methylsulfonyl, aminosulfonyl, methylaminosulfonyl, ethylaminosulfonyl, isobutylaminosulfonyl, tert-butylaminosulfonyl, 2-hydroxyethylaminosulfonyl, 2-hydroxy-2-methylpropylaminosulfonyl, cyclopropylaminosulfonyl, azetidin-1-ylsulfonyl, pyrrolidin-1-ylsulfonyl, morpholin-4-ylsulfonyl, 3-hydroxyazetidin-1-ylsulfonyl, methyl(tert-butoxycarbonyl)aminosulfonyl, sulfo, phenyloxysulfonyl, chlorosulfonyl, methylsulfonylamino, propylsulfonylamino, isopropylsulfonylamino, 3-chloropropylsulfonylamino, phenylsulfonylamino, 4-fluorophenylsulfonylamino, methyl(isopropylsulfonyl)amino, dimethylaminomethyl, benzyl, ethenyl, 2-(4-(trifluoromethyl)phenyl)ethenyl, 4-fluorophenyl, 4-nitrophenyl, 2,2,2-trifluoroethoxy, hydrazinocarbonyl, 1-pyrrolidinyl, 1-piperidinyl, 2-thienyl, 2-pyridyl, 4-thiazolyl, 5-trifluoromethyl-2-pyridyl, 2-methylthiazol-4-yl and the like.

In compound (IA), R⁵ and R⁶ are the same or different and each is a hydrogen atom, a cyano group, a C₁₋₆ alkyl group optionally having substituent(s), -COORₐ, -CONRₐR_{b} or -NRₐCOR_{b}, wherein Rₐ and R_{b} are the same or different and each is a hydrogen atom, a C₁₋₆ alkyl group optionally having substituent(s), a C₃₋₇ cycloalkyl group or a phenyl group optionally having substituent(s).
R⁵ and R⁶ are the same or different and each is preferably a hydrogen atom, a cyano group, a C₁₋₆ alkyl group, a halogeno-C₁₋₆ alkyl group, -CONRₐR_{b} or -NRₐCOR_{b}.
Specific examples of R⁵ and R⁶ include cyano, methyl, carboxy, ethoxycarbonyl, carbamoyl and the like.

In compound (IA), R⁷ is a hydrogen atom, a C₁₋₆ alkyl group, a C₃₋₇ cycloalkyl group or a phenyl group optionally having substituent(s).
Preferred as R⁷ is a hydrogen atom.

Preferred as compound (IA) is a compound wherein
ring A is a benzene ring optionally having, besides cyano, 1 or 2 substituents selected from (1) a halogen atom, (2) a C₁₋₆ alkyl group, (3) a halogeno-C₁₋₆ alkyl group and (4) -OR_{c} wherein Rₑ is a hydrogen atom, a C₁₋₆ alkyl group optionally having substituent(s) or a C₃₋₇ cycloalkyl group;
ring B is a benzene ring optionally having substituent(s), a pyridine ring optionally having substituent(s), a thiazole ring optionally having substituent(s) or a pyrazole ring optionally having substituent(s);
R⁵ and R⁶ are the same or different and each is a hydrogen atom, a cyano group, a C₁₋₆ alkyl group, a halogeno-C₁₋₆ alkyl group, - CONRₐR_{b} or -NRₐCOR_{b}; and
R⁷ is a hydrogen atom.

Furthermore, preferred as compound (IA) is a compound wherein
ring A is a benzene ring optionally further having, besides cyano, 1 or 2 substituents selected from a fluorine atom, a chlorine atom, trifluoromethyl and methoxy;
ring B is a benzene ring, a pyridine ring, a thiazole ring or a pyrazole ring, each of which optionally has 1 to 5 (preferably 1 to 3) substituents selected from (1) a halogen atom, (2) a C₁₋₆ alkyl group optionally having substituent(s), (3) a C₂₋₆ alkenyl group optionally having substituent(s), (4) a C₂₋₆ alkynyl group optionally having substituent(s), (5) a nitro group, (6) a phenyl group optionally having substituent(s), (7) a heterocyclic group optionally having substituent(s), (8) a cyano group, (9) -CONR_{d}Rₑ, (10) -CO₂R_{d}, (11) -NR_{d}CORₑ, (12) - SO₂NR_{d}Rₑ, (13) -NR_{d}SO₂Rₑ, (14) -C(OH)R_{d}Rₑ, (15) -NR_{d}Rₑ, (16) -OR_{d}, and (17) -SR_{d} wherein R_{d} and Rₑ are the same or different and each is a hydrogen atom, a C₁₋₆ alkyl group optionally having substituent(s), a C₃₋₇ cycloalkyl group or a phenyl group optionally having substituent(s);
R⁵ and R⁶ are the same or different and each is a hydrogen atom, a cyano group, a C₁₋₆ alkyl group, a halogeno-C₁₋₆ alkyl group, - CONRₐR_{b} or -NRₐCOR_{b}; and
R⁷ is a hydrogen atom.

More specifically, preferred as compound (IA) are compounds of Example 68, Example 69, Example 77, Example 105, Example 106, Example 282, Example 286, Example 304, Example 310, Example 311, Example 319, Example 322 and Example 337 or salts thereof.

As salts of compound (I), compound (I') and compound (IA) of the present invention, for example, metal salts, ammonium salts, salts with organic bases, salts with inorganic acids, salts with organic acids, salts with basic or acidic amino acids and the like can be mentioned. As preferable examples of the metal salt, alkali metal salts such as sodium salt, potassium salt and the like; alkaline earth metal salts such as calcium salt, magnesium salt, barium salt and the like; aluminum salt and the like can be mentioned. Preferable examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salts with inorganic acids include salts with hydrochloric acid, hydrobromic acid, nitric acid, sulfuric acid, phosphoric acid and the like. Preferable examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salts with basic amino acids include salts with arginine, lysine, ornithine and the like. Preferable examples of the salts with acidic amino acids include salts with aspartic acid, glutamic acid and the like.
Of these, pharmaceutically acceptable salts are preferable. For example, when a compound has an acidic functional group therein, inorganic salts such as alkali metal salts (e.g., sodium salt, potassium salt and the like), alkaline earth metal salts (e.g., calcium salt, magnesium salt, barium salt and the like) and the like, ammonium salt and the like can be mentioned. When a compound has a basic functional group therein, salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like can be mentioned.

A prodrug of the compound (I), compound (I') or compound (IA) of the present invention means a compound which is converted to compound (I), compound (I') or compound (IA) by a reaction due to an enzyme, gastric acid, etc. under the physiological conditions in the living body, that is, a compound which is converted to compound (I), compound (I') or compound (IA) by oxidation, reduction, hydrolysis, etc. according to an enzyme; a compound which is converted to compound (I), compound (I') or compound (IA) by hydrolysis etc. due to gastric acid, etc. A prodrug of compound (I), compound (I') or compound (IA) may be a compound obtained by subjecting an amino group in compound (I), compound (I') or compound (IA) to an acylation, alkylation or phosphorylation (e.g., a compound obtained by subjecting an amino group in compound (I), compound (I') or compound (IA) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I), compound (I') or compound (IA) to an acylation, alkylation, phosphorylation or boration (e.g., a compound obtained by subjecting an hydroxy group in compound (I), compound (I') or compound (IA) to an acetylation, palmitoylation, propanoylation, pivaloylation, succinylation, fumarylation, alanylation, dimethylaminomethylcarbonylation, etc.); a compound obtained by subjecting a carboxyl group in compound (I), compound (I') or compound (IA) to an esterification or amidation (e.g., a compound obtained by subjecting a carboxyl group in compound (I), compound (I') or compound (IA) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. Any of these compounds can be produced from compound (I), compound (I') or compound (IA) by a method known *per se*.
A prodrug of compound (I), compound (I') or compound (IA) may also be one which is converted to compound (I), compound (I') or compound (IA) under physiological conditions, such as those described in IYAKUHIN no KAIHATSU (Development of Pharmaceuticals), Vol. 7, Design of Molecules, pp. 163-198, Published by HIROKAWA SHOTEN (1990).

The production methods of compound (I), compound (I') and compound (IA) or salts thereof are now explained in the following.
In each production method below, when alkylation reaction, hydrolysis, amination reaction, esterification reaction, amidation reaction, etherification reaction, oxidation reaction, reduction reaction and the like are carried out, these reactions are carried out according to methods known per se. Examples of such method include the methods described in ORGANIC FUNCTIONAL GROUP PREPARATIONS, 2nd edition, ACADEMIC PRESS,INC., 1989; Comprehensive Organic Transformations, VCH Publishers Inc., 1989 etc. and the like.
In addition, the resultant product can be used for the next reaction directly as a reaction mixture or a crude purification product. They can also be isolated from a reaction mixture according to a conventional method, and can be easily produced by a general separation means (e.g., recrystallization, distillation, chromatography and the like).

### Production methods

Compound (I) of the present invention or a salt thereof can be obtained by the method shown in the following reaction scheme 1 or a method analogous thereto and the like, or a known method or a method analogous thereto and the like. A compound represented by the formula (V) is encompassed in the compound represented by the formula (I). By converting a functional group of the compound of the formula (V) by a method known per se or a method analogous thereto (e.g., reduction reaction, acylation reaction, sulfonylation reaction, oxidation reaction, alkylation reaction, hydrolysis, amidation reaction, cyanation reaction, carbonylation reaction, Curtius rearrangement reaction, sulfuration reaction etc.), a compound encompassed in the formula (I) can also be obtained. When compound (V) has a protecting group, compound (I) can be obtained by eliminating the protecting group by a method known per se or a method analogous thereto.
The compound in the reaction scheme includes salts and examples of the salt include those similar to the salt of compound (I) and the like.
Examples of ethers in the production method include diethyl ether, dioxane, tetrahydrofuran and the like.
Examples of saturated hydrocarbons in the production method include hexane, pentane and the like.
Examples of halogenated hydrocarbons in the production method include dichloromethane, chloroform and the like.
Examples of amides in the production method include N,N-dimethylformamide and the like.
Examples of aromatic hydrocarbons in the production method include benzene, toluene and the like.
Examples of alcohols in the production method include methanol, ethanol, 2-propanol and the like.
Examples of ketones in the production method include acetone, methylethylketone and the like.
Examples of nitriles in the production method include acetonitrile and the like.
Examples of esters in the production method include ethyl acetate and the like.
Examples of aromatic amines in the production method include aniline and the like.
Examples of heterocycles in the production method include pyridine and the like.
Examples of organic acids in the production method include formic acid, acetic acid and the like.
Examples of sulfoxides in the production method include dimethyl sulfoxide and the like.

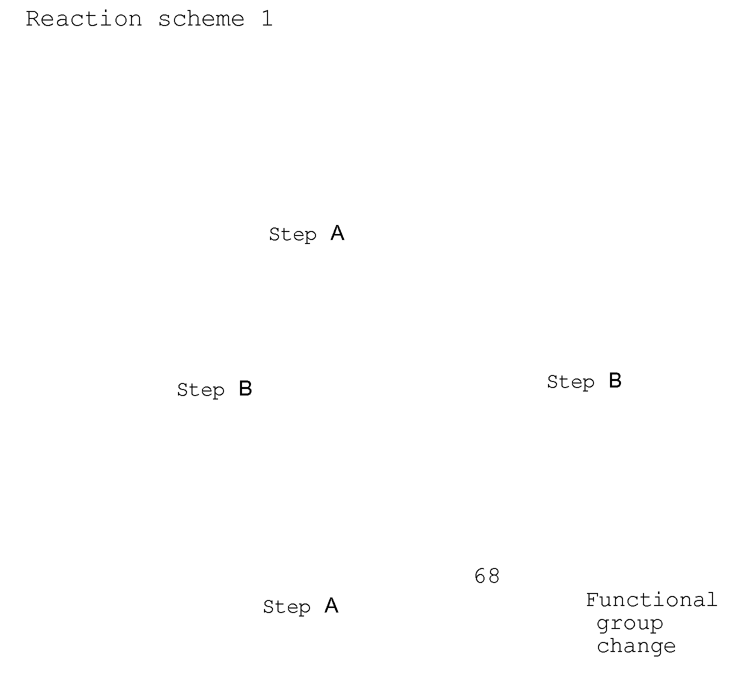

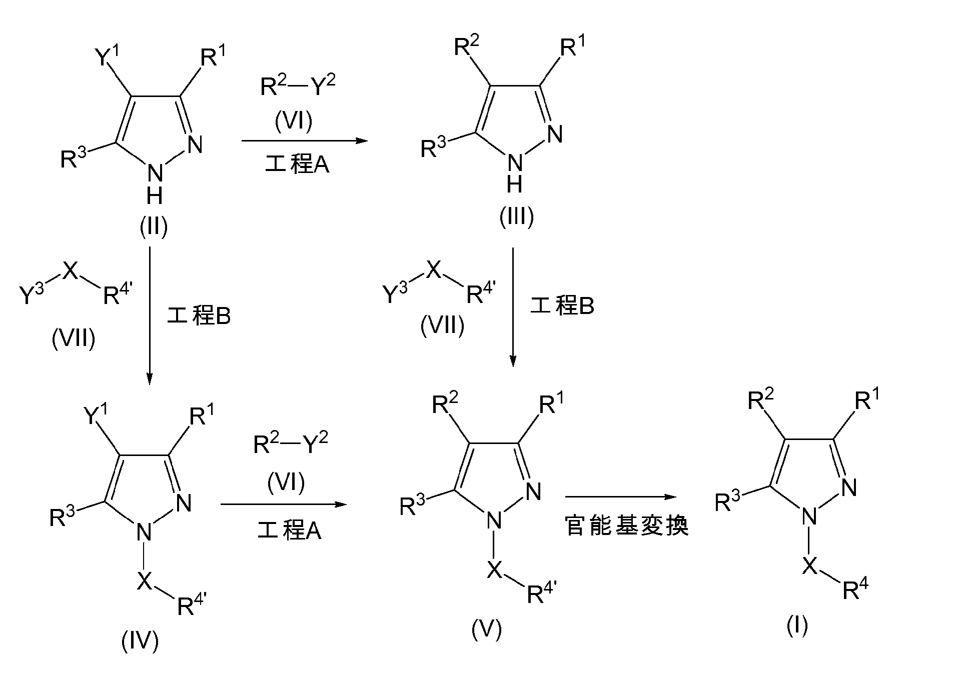

wherein Y¹ and Y² are each a halogen atom, optionally substituted boryl, optionally substituted stannyl, Y³ is a halogen atom or hydroxy (provided that when Y¹ is a halogen atom, then Y² should be boryl optionally having substituent(s) or stannyl optionally having substituent(s); and when Y¹ is boryl optionally having substituent(s) or stannyl optionally having substituent(s), then Y² should be a halogen atom), R^{4'} is a cyclic group optionally having substituent(s), and other symbols are as defined above.
Examples of the halogen atom include a fluorine atom, a chlorine atom, a bromine atom, an iodine atom.
Examples of the optionally substituted boryl include dihydroxyboryl, diethylboryl, 4,4,5,5-tetramethyl-1,3,2-dioxaborolan-2-yl.
Examples of the optionally substituted stannyl include trimethylstannyl, tri(n-butyl)stannyl.
Examples of the "cyclic group optionally having substituent(s)" for R^{4'} include those similar to the "cyclic group optionally having substituent(s)" for R⁴.
Compounds (II), (III), (IV), (VI) and (VII) to be used as starting materials can be synthesized according to a known method or a method analogous thereto. For example, they can be produced according to method below-mentioned Reference Example. In addition, when they are commercially available, the commercially available products can also be used as they are.

### [Step A]

Compound (III) or compound (V) can be synthesized by subjecting compound (II) or compound (IV) with compound (VI) to a coupling reaction generally used for organic synthesis, such as Suzuki coupling reaction, Stille coupling reaction and the like. When compound (II) or compound (IV) has a protecting group, compound (III) or compound (V) can be obtained by removing the protecting group according to a method known per se or a method analogous thereto.
The reaction can be carried out according to the method described in the reference (Suzuki et al., Synth. Commum. 1981, Vol. 11, pp 513 or Stille et al., J. Am. Chem. Soc. 1987, Vol. 109, pp 5478), or a method analogous thereto.
The reaction temperature is from about 20°C to about 150°C, preferably from about 60°C to about 120°C. The reaction time is from about 1 hr to about 50 hr.

### [Step B]

When Y³ of compound (VII) is a halogen atom, compound (IV) or (V) can be synthesized by reacting compound (II) or (III) with compound (VII) in the presence of a base according to a conventional method.
Examples of the base include alkali metal hydride such as sodium hydride, potassium hydride and the like; alkali metal amides such as sodium amide and the like; potassium tert-butoxide, potassium carbonate and the like.
The amount of the base to be used is about 1.0 to about 10 mol, preferably about 1.0 to 3.0 mol, per 1.0 mol of compound (II) or (III).
The amount of compound (VII) to be used is about 1.0 to about 10 mol, preferably about 1.0 to 2.0 mol, per 1.0 mol of compound (II) or (III).
The reaction temperature is generally about -70°C to about 100°C, preferably about 0°C to about 50°C. The reaction time is generally from about 5 min to about 48 hr, preferably from about 5 min to about 24 hr.
This reaction is generally carried out in an organic solvent that does not adversely influence the reaction. As the organic solvent that does not adversely influence the reaction, for example, ethers, saturated hydrocarbons, halogenated hydrocarbons, amides, aromatic hydrocarbons and the like can be used. These solvents may be used alone or in a mixture of two or more kinds thereof at an appropriate ratio.
When Y³ of compound (VII) is hydroxy, compound (IV) or (V) can be synthesized by subjecting compound (VII) to Mitsunobu reaction with compound (II) or (III)in the presence of a phosphine compound and an azodicarboxylic acid derivative, or in the presence of a phosphorane compound.
Examples of the phosphine compound include triarylphosphines such as triphenylphosphine and the like; trialkylphosphines such as tributylphosphine and the like; alkylarylphosphines such as dicyclohexylphenylphosphine and the like; a resin supported phosphine reagent such as diphenylphosphinopolystyrene resin etc. and the like.
The amount of the phosphine compound to be used is about 1.0 to about 10 mol, preferably about 1.0 to 3.0 mol, per 1.0 mol of compound (II) or (III).
Examples of the azodicarboxylic acid derivative include azodicarboxylates such as diethyl azodicarboxylate and the like; azodicarboxylic amides such as 1,1'-azobis(N,N-dimethylformamide) and the like.
The amount of the azodicarboxylic acid derivative to be used is about 1.0 to about 10 mol, preferably about 1.0 to 3.0 mol, per 1.0 mol of compound (II) or (III).
Examples of the phosphorane compound include a compound such as cyanomethylene tri(n-butyl)phosphorane and the like described in the reference (Tsunoda et al., Tetrahedron. Lett. 1995, Vol. 36, pp 2531).
The amount of the phosphorane compound to be used is about 1.0 to about 10 mol, preferably about 1.0 to 3.0 mol, per 1.0 mol of compound (II) or (III).
The amount of compound (VII) to be used is about 0.5 to about 10 mol, preferably about 1.0 to 3.0 mol, per 1.0 mol of compound (II) or (III).
When the phosphine compound and azodicarboxylic acid derivative are used, the reaction temperature is generally about -70°C to about 100°C, preferably about 0°C to about 50°C. The reaction time is generally from about 5 min to about 48 hr, preferably from about 5 min to about 20 hr.
When the phosphorane compound is used, the reaction temperature is from about 20°C to about 150°C, preferably from about 60°C to about 120°C. The reaction time is from about 1 hr to about 50 hr.
This reaction is generally carried out in an organic solvent that does not adversely influence the reaction. As the organic solvent that does not adversely influence the reaction, for example, ethers, saturated hydrocarbons, aromatic hydrocarbons and the like can be used. These solvents may be used alone or in a mixture of two or more kinds thereof at an appropriate ratio.

### [Conversion of functional group]

The reduction reaction can be carried out using a reducing agent generally used for organic synthesis, such as sodium borohydride, sodium cyanoborohydride, sodium triacetoxyborohydride, lithium borohydride, lithium aluminum hydride and the like. In addition, a metal salt such as calcium chloride and the like may be added. The amount of the reducing agent to be used is about 1.0 to about 10 mol, preferably about 1.0 to 5.0 mol, per 1.0 mol of compound (V). The amount of the metal salt to be used is about 0.5 to about 10 mol, preferably about 1.0 to 3.0 mol, per 1.0 mol of compound (V).
The reaction temperature is generally about -70°C to about 100°C, preferably about 0°C to about 50°C. The reaction time is generally from about 30 min to about 50 hr, preferably from 30 min to about 20 hr.
This reaction is generally carried out in an organic solvent that does not adversely influence the reaction. As the organic solvent that does not adversely influence the reaction, for example, alcohols, ethers, saturated hydrocarbons, aromatic hydrocarbons and the like can be used. These solvents may be used alone or in a mixture of two or more kinds thereof at an appropriate ratio.

The acylation reaction can be carried out using an acylating agent such as an organic acid, an acyl halide, an acid anhydride and the like, in the presence of a base where necessary, according to a method generally used for organic synthesis.
As the base, for example, alkali metal salts such as sodium hydride, potassium carbonate, sodium carbonate, potassium hydroxide, sodium hydroxide and the like, and organic bases such as triethylamine, diisopropylethylamine, pyridine and the like can be used.
The amount of the acylating agent to be used is generally 1 to 20 mol, preferably 2 to 10 mol, per 1.0 mol of compound (V). The amount of the base to be used is generally 1 to 10 mol, preferably 1 to 5 mol, per 1.0 mol of compound (V). When an organic base (e.g., triethylamine, diisopropylethylamine, pyridine etc.) is used, an excess amount thereof can also be used as a solvent.
The reaction temperature is generally 0 to 120°C, preferably 20 to 100°C. The reaction time is generally 0.5 to 100 hr, preferably 1 to 48 hr.
This reaction is generally carried out in an organic solvent that does not adversely influence the reaction. As the organic solvent that does not adversely influence the reaction, for example, ethers, saturated hydrocarbons, halogenated hydrocarbons, aromatic hydrocarbons, ketones, nitriles, amides, esters, aromatic amines, heterocycles and the like can be used. Preferable examples thereof include ethers, hydrocarbons, halogenated hydrocarbons, amides, aromatic amines and heterocycles. These solvents may be used alone or in a mixture of two or more kinds thereof at an appropriate ratio.

The sulfonylation reaction can be carried out using a sulfonylating agent such as sulfonylhalide, sulfonic acid anhydride and the like, in the presence of a base where necessary, according to a method generally used for organic synthesis.
As the base, for example, alkali metal salts such as sodium hydride, potassium carbonate, sodium carbonate, potassium hydroxide, sodium hydroxide and the like; and organic bases such as triethylamine, diisopropylethylamine, pyridine and the like can be used.
The amount of the sulfonylating agent to be used is generally 1 to 20 mol, preferably 2 to 10 mol, per 1.0 mol of compound (IV) or (VI). The amount of the base to be used is generally 1 to 10 mol, preferably 1 to 5 mol, per 1.0 mol of compound (V). When an organic base (e.g., triethylamine, diisopropylethylamine, pyridine etc.) is used, an excess amount thereof can also be used as a solvent.
The reaction temperature is generally 0 to 120°C, preferably 20 to 100°C. The reaction time is generally 0.5 to 100 hr, preferably 1 to 48 hr.
This reaction is generally carried out in an organic solvent that does not adversely influence the reaction. As the organic solvent that does not adversely influence the reaction, for example, ethers, hydrocarbons, halogenated hydrocarbons, ketones, nitriles, amides, esters, aromatic amines, heterocycles and the like can be used. Preferable examples thereof include ethers, hydrocarbons, halogenated hydrocarbons, amides, aromatic amines and heterocycles. These solvents may be used alone or in a mixture of two or more kinds thereof at an appropriate ratio.

The oxidation reaction can be carried out using an oxidant generally used for organic synthesis, such as manganese compounds (e.g., potassium permanganate, manganese dioxide etc.), chrome compounds (e.g., chromic acid etc.), sulfur compounds (e.g., dimethylsulfoxide etc.), cerium compounds (e.g., cerium (IV) diammonium nitrate etc.), in a solvent that does not adversely influence the reaction, in the presence of an acid, a base and the like where necessary. Examples of the solvent include water, saturated hydrocarbons, aromatic hydrocarbons, halogenated hydrocarbons, alcohols, ketones, organic acids, amides, esters, sulfoxides, nitriles and the like. Preferable examples thereof include water, hydrocarbons, halogenated hydrocarbons, ketones, organic acids, amides, esters, sulfoxides and nitriles. These solvents may be used alone or in a mixture of two or more kinds thereof at an appropriate ratio.
As the acid, for example, mineral acids such as sulfuric acid and the like, organic acids such as acetic acid and the like, and the like can be used. As the base, for example, alkali metal salts such as potassium hydroxide, sodium hydroxide and the like, amines such as triethylamine, diisopropylethylamine, piperidine and the like can be used. Where necessary, a dehydrating agent such as dicyclohexylcarbodiimide and the like, oxalyl chloride, pyridine sulfur trioxide and the like may be added.
The amount of the oxidant to be used is generally 1 to 20 mol, preferably 1 to 10 mol, per 1.0 mol of compound (V). When dimethylsulfoxide is used, an excess amount thereof can also be used as a solvent. The amount of the acid or base to be used is generally 1 to 20 mol, preferably 1 to 10 mol, per 1.0 mol of compound (V). The amount of the other additive to be used is generally 1 to 20 mol, preferably 1 to 10 mol, per 1.0 mol of compound (V).
The reaction temperature is generally -70 to 120°C, preferably -70 to 100°C. The reaction time is generally 0.1 to 100 hr, preferably 0.1 to 48 hr.

The alkylation reaction can be carried out by reacting compound (V) with an alkylating agent such as alkylhalide and the like in the presence of a base according to a conventional method.
As the base, for example, alkali metal hydride such as sodium hydride, potassium hydride and the like; alkali metal amides such as sodium amide and the like; potassium tert-butoxide, potassium carbonate and the like can be used.
The amount of the base to be used is about 1.0 to about 10 mol, preferably about 1.0 to 2.0 mol, per 1.0 mol of compound (V).
The amount of the alkylating agent to be used is about 1.0 to about 10 mol, preferably about 1.0 to 2.0 mol, per 1.0 mol of compound (V).
The reaction temperature is generally about -70°C to about 100°C, preferably about 0°C to about 50°C. The reaction time is generally about 5 min to about 48 hr, preferably about 5 min to about 20 hr.
This reaction is generally carried out in an organic solvent that does not adversely influence the reaction. As the organic solvent that does not adversely influence the reaction, for example, ethers, saturated hydrocarbons, halogenated hydrocarbons, amides, aromatic hydrocarbons and the like can be used. These solvents may be used alone or in a mixture of two or more kinds thereof at an appropriate ratio.

The alkylation reaction of carbonyl can be carried out by reacting compound (V) with an alkylating agent in a solvent that does not adversely influence the reaction, in the presence of an additive such as cerium (III) chloride and the like where necessary.
As the alkylating agent, for example, organic magnesium reagents such as alkylmagnesium halide and the like; organic lithium reagents such as alkyllithium and the like, and the like can be used.
As the solvent, for example, hydrocarbons, ethers and the like can be used. These solvents may be used alone or in a mixture of two or more kinds thereof at an appropriate ratio.
The amount of the organic magnesium reagent or organic lithium reagent to be used is generally 1 to 20 mol, preferably 1 to 10 mol, per 1.0 mol of compound (V). The amount of the additive to be used is generally 0.1 to 10 mol, preferably 1.0 to 5.0 mol, per 1.0 mol of compound (V).
The reaction temperature is generally about -70 to about 100°C, preferably about -70 to about 50°C. The reaction time is generally about 0.5 to about 24 hr.

The hydrolysis is carried out using an acid or a base generally used for organic synthesis.
As the acid, for example, mineral acids such as hydrochloric acid and the like; Lewis acids such as boron tribromide and the like; a combination of a Lewis acid and a thiol or a sulfide; organic acids such as trifluoroacetic acid, p-toluenesulfonic acid and the like, and the like can be used.
As the base, for example, metal hydroxides such as sodium hydroxide, potassium hydroxide, barium hydroxide and the like; basic salts such as sodium carbonate, potassium carbonate and the like; metal alkoxides such as sodium methoxide, sodium ethoxide, potassium tert-butoxide and the like; organic bases such as triethylamine, imidazole, formamidine and the like, and the like can be used.
The amount of the acid or base to be used is generally 0.1 to about 50 mol, preferably about 1 to about 20 mol, per 1.0 mol of compound (V).
This reaction is generally carried out in an organic solvent that does not adversely influence the reaction. As the organic solvent that does not adversely influence the reaction, for example, alcohols, ethers, aromatic hydrocarbons, saturated hydrocarbons, halogenated hydrocarbons, sulfoxides, or water a mixture of two or more kinds thereof and the like can be used.
The reaction time is generally about 10 min to about 50 hr, preferably about 30 min to about 12 hr. The reaction temperature is generally about 0 to about 200°C, preferably about 20 to about 120°C.

The amidation reaction can be carried out according to a method generally used for organic synthesis, which includes treating a carboxylic acid derivative or a sulfonic acid derivative with an activator to give an activated derivative, and reacting the activated derivative with an aromatic or aliphatic amine in the presence of a base or an additive where necessary. The amidation reaction can also be carried out by reacting an ester derivative with an aromatic or aliphatic amine in the presence of a base or an additive where necessary.
As the activator, for example, chlorinating agents generally used for organic synthesis, such as thionyl chloride, oxalyl chloride and the like; acylating agents generally used for organic synthesis, such as acid anhydride, acid chloride and the like; condensation agents generally used for organic synthesis, such as 1,3-dicyclohexylcarbodiimide, 1-ethyl-3-(3'-dimethylaminopropyl)carbodiimide, diethylphosphoryl cyanide, N,N-carbonyldiimidazole and the like, and the like can be used.
As the additive, for example, N-hydroxybenzotriazole, N-hydroxysuccinimide and the like can be used.
As the base, for example, organic bases such as diisopropylethylamine, triethylamine, pyridine and the like, and the like can be used.
The amount of the aromatic or aliphatic amine to be used is generally 0.5 to 5.0 mol, preferably 0.8 to 2.0 mol, per 1.0 mol of compound (V). The amount of the activator to be used is generally 1 to 10 mol, preferably 1 to 5 mol, per 1.0 mol of compound (V). The amount of the additive to be used is generally 1 to 20 mol, preferably 2 to 10 mol, per 1.0 mol of compound (V). The amount of the base to be used is generally 1 to 10 mol, preferably 1 to 5 mol, per 1.0 mol of compound (V). In addition, an excess amount thereof can also be used as a solvent.
The reaction time is generally about 10 min to about 50 hr, preferably about 30 min to about 12 hr. The reaction temperature is generally about 0 to about 100°C, preferably about 20 to about 80°C.
This reaction is generally carried out in an organic solvent that does not adversely influence the reaction. As the organic solvent that does not adversely influence the reaction, for example, ethers, hydrocarbons, halogenated hydrocarbons, ketones, nitriles, amides, heterocycles and the like can be used. Preferable examples thereof include ethers, hydrocarbons, halogenated hydrocarbons and amides. These solvents may be used alone or in a mixture of two or more kinds thereof at an appropriate ratio.

The cyanation reaction can be carried out by reacting aromatic iodide, bromide or chloride with metal cyanide in the presence of, where necessary, a metal catalyst by a method generally used for organic syntheses.
As the metal catalyst, for example, palladium reagents such as tetrakis(triphenylphosphine)palladium and the like can be used.
As the metal cyanide, for example, zinc cyanide, copper cyanide, sodium cyanide, potassium cyanide and the like can be used.
The amount of the metal catalyst to be used is generally 0.01 - 0.2 mol, preferably 0.05 - 0.1 mol, per 1.0 mol of compound (V). The amount of the metal cyanide to be used is generally 1 - 20 mol, preferably 2 - 10 mol, per 1.0 mol of compound (V).
The reaction time is generally about 10 min to about 50 hr, preferably about 30 min to about 12 hr. The reaction temperature is generally about 0 to about 100°C, preferably about 20 to about 80°C.
This reaction is generally carried out in an organic solvent that does not adversely influence the reaction. As the organic solvent that does not adversely influence the reaction, for example, ethers, hydrocarbons, halogenated hydrocarbons, ketones, nitriles, amides, heterocycles and the like can be used. Preferable examples thereof include ethers, hydrocarbons, halogenated hydrocarbons and amides. These solvents may be used alone or in a mixture of two or more kinds thereof at an appropriate ratio.

The carbonylation reaction can be carried out by reacting aromatic iodide, bromide or chloride with carbon monoxide in the presence of a metal catalyst and a base, and where necessary, alcohol such as methanol, ethanol and the like by a method generally used for organic syntheses.
As the metal catalyst, for example, palladium reagents such as palladium acetate and the like can be used in combination with, where necessary, a suitable ligand such as bis(diphenylphosphino)ferrocene and the like.
As the base, for example, organic bases such as triethylamine, diisopropylethylamine, pyridine and the like can be used.
As the carbon monoxide, gaseous carbon monoxide can be used by directly introducing same into the reaction system. In addition, a reagent that produces carbon monoxide in the system, such as molybdenum hexacarbonyl and the like can also be used.
The amount of the metal catalyst and ligand to be used is each generally 0.01 - 0.5 mol, preferably 0.1 - 0.3 mol, per 1.0 mol of compound (V). The amount of the alcohol and base to be used is each generally 1 - 20 mol, preferably 2 - 10 mol, per 1.0 mol of compound (V).
The reaction time is generally about 10 min to about 50 hr, preferably about 30 min to about 12 hr. The reaction temperature is generally about 0 to about 100°C, preferably about 20 to about 80°C.
This reaction is generally carried out in an organic solvent that does not adversely influence the reaction. As the organic solvent that does not adversely influence the reaction, for example, ethers, hydrocarbons, halogenated hydrocarbons, ketones, nitriles, amides, heterocycles and the like can be used. Preferable examples thereof include ethers, hydrocarbons, halogenated hydrocarbons and amides. These solvents may be used alone or in a mixture of two or more kinds thereof at an appropriate ratio.

The Curtius rearrangement reaction can be carried out by reacting an aromatic carboxylic acid with an azide compound such as diphenylphosphorylazide and the like in the presence of a base, as necessary in the presence of an alcohol, according to a method generally used for organic synthesis.
As the base, for example, organic bases such as triethylamine, diisopropylethylamine, pyridine and the like can be used. As the alcohol, for example, 2-methyl-2-propanol, ethanol, benzyl alcohol and the like can be used.
The amount of the azide compound to be used is generally 0.5 to 5.0 mol, preferably 1.0 to 2.0 mol, per 1.0 mol of compound (V). The amount of the base to be used is generally 1.0 to 10 mol, preferably 1.0 to 3.0 mol, per 1.0 mol of compound (V). The amount of the alcohol to be used is generally 1.0 to 50 mol, preferably 1.0 to 10 mol, per 1.0 mol of compound (V).
The reaction time is generally about 10 min to about 50 hr, preferably about 30 min to about 12 hr. The reaction temperature is generally about 0 to about 100°C, preferably about 20 to about 80°C.
This reaction is generally carried out in an organic solvent that does not adversely influence the reaction. As the organic solvent that does not adversely influence the reaction, for example, ethers, hydrocarbons, halogenated hydrocarbons, ketones, nitriles, amides, heterocycles and the like can be used. Preferable examples thereof include ethers, hydrocarbons, halogenated hydrocarbons and amides. These solvents may be used alone or in a mixture of two or more kinds thereof at an appropriate ratio.

The sulfuration reaction of compound (V) is carried out using phosphorus pentasulfide, a Lawesson reagent and the like.
This reaction can be carried out in a solvent that does not adversely influence the reaction. As the solvent, for example, ethers, aromatic hydrocarbons, saturated hydrocarbons, halogenated hydrocarbons or a mixture of two or more kinds thereof and the like can be used.
The amount of the phosphorus pentasulfide, Lawesson reagent and the like to be used is generally 0.5 to 30 mol, preferably 0.5 to 10 mol, per 1.0 mol of compound (V).
The reaction temperature is generally about 0 - about 150°C, preferably about 20 to about 120°C. The reaction time is generally 10 min to about 50 hr, preferably about 30 min to about 12 hr.

When the protecting group is t-butyl, triphenylmethyl, t-butoxycarbonyl, benzyloxycarbonyl, tetrahydropyranyl, 4-methoxybenzyl, 2,4-dimethoxybenzyl and the like, the deprotection can be carried out, for example, by treating compound (V) with an acid in a solvent that does not adversely influence the reaction. As the solvent, for example, ethers, saturated hydrocarbons, aromatic hydrocarbons, halogenated hydrocarbons, ketones, nitriles, amides, esters, organic acids and the like can be used. Preferable examples thereof include ethers, hydrocarbons and halogenated hydrocarbons. These solvents may be used alone or in a mixture of two or more kinds thereof at an appropriate ratio. As the acid, for example, mineral acids such as hydrochloric acid, hydrobromic acid, sulfuric acid, nitric acid and the like; organic acids such as formic acid, acetic acid, trifluoroacetic acid, p-toluenesulfonic acid and the like; Lewis acids such as boron tribromide and the like; and silica gel can be used. These acids may be used alone or in a mixture of two or more kinds thereof.
The amount of the acid to be used is generally 1 to 100 mol, preferably 1 to 50 mol, per 1.0 mol of compound (V). In addition, an excess amount thereof can also be used as a solvent.
The reaction temperature is generally -72 to 100°C, preferably 0 to 60°C. The reaction time is generally 0.5 to 100 hr, preferably 0.5 to 48 hr.
When the protecting group is benzyloxycarbonyl, benzyl, benzyloxymethyl and the like, the deprotection can be carried out by subjecting compound (V) to catalytic hydrogenation reaction. The catalytic hydrogenation reaction is carried out by reacting compound (V) with hydrogen in the presence of a metal catalyst generally used for organic synthesis, such as palladium-carbon, platinum-carbon and the like. In addition, a mineral acid (e.g., hydrochloric acid etc.), an organic acid (e.g., acetic acid etc.) and the like may be added.
The amount of the metal catalyst to be used is generally about 0.01 to about 1 mol, preferably about 0.01 to 0.5 mol, per 1.0 mol of compound (V).
The amount of the mineral acid (e.g., hydrochloric acid), organic acid (e.g., acetic acid) and the like to be used is generally about 1.0 to about 50 mol, preferably about 1.0 to 5.0 mol, per 1.0 mol of compound (V).
The reaction temperature is generally about -10°C to about 100°C, preferably about 0°C to about 50°C. The reaction time is generally about 30 min to about 50 hr, preferably 30 min to about 20 hr.
This reaction is generally carried out in an organic solvent that does not adversely influence the reaction. As the organic solvent that does not adversely influence the reaction, for example, alcohols, ethers, saturated hydrocarbons, aromatic hydrocarbons and the like can be used. These solvents may be used alone or in a mixture of two or more kinds thereof at an appropriate ratio.

Compound (I') and compound (IA) can be produced according to the above-mentioned production method of compound (I).

When the object product is obtained as a free form by the above-mentioned reaction, it can be converted to a salt according to a conventional method. When it is obtained as a salt, it can also be converted to a free form or other salt according to a conventional method.
The compound (I), compound (I') and compound (IA) or salts thereof obtained by the above-mentioned production methods can be isolated and purified by a known means, such as solvent extraction, liquid conversion, phase transfer, crystallization, recrystallization, chromatography and the like.
When compound (I), compound (I') and compound (IA) or salts thereof contain an optical isomer, a stereoisomer, a positional isomer or rotational isomer, they are also encompassed in compound (I), compound (I') and compound (IA) or salts thereof, as well as can be obtained as single products by synthesis methods and separation methods known per se. For example, compound (I), compound (I') and compound (IA) or salts thereof contain an optical isomer, an optical isomer resolved from the compound is also encompassed in compound (I), compound (I') and compound (IA) or salts thereof.
Here, the optical isomer can be produced by a method known per se.
The compound (I), compound (I') and compound (IA) or salts thereof may be a solvate or a non-solvate.
The compound (I), compound (I') and compound (IA) or salts thereof may be labeled with an isotope (e.g., ³H, ¹⁴C, ³⁵S) and the like.
The compound (I), compound (I') and compound (IA) or salts thereof may be a crystal.
The crystal of compound (I), compound (I') and compound (IA) or salts thereof (hereinafter sometimes to be abbreviated as the crystal of the present invention) can be produced by crystallization of compound (I), compound (I') and compound (IA) or salts thereof by applying a crystallization method known per se.

Since compound (I), compound (I') and compound (IA) or salts thereof of the present invention show superior androgen receptor antagonistic action and the like, low toxicity and a fewer side effects, they are useful as safe pharmaceutical products, androgen receptor antagonists and the like.
Since a pharmaceutical composition containing compound (I), compound (I') or compound (IA) or a salt thereof of the present invention show a superior androgen receptor antagonistic action and/or a prostate specific antigen (PSA) production inhibitory action in mammals (e.g., mouse, rat, hamster, rabbit, cat, dog, bovine, sheep, monkey, human and the like), and superior (oral) absorbability, superior (metabolism) stability and the like, it can be used an agent for the prophylaxis or treatment of androgen receptor-associated diseases, for example, hormone sensitive diseases in the androgen-dependent phase and/or the androgen-independent phase, particularly hormone sensitive cancer in the androgen-dependent phase and/or the androgen-independent phase (e.g., prostate cancer (e.g., hormone-dependent prostate cancer, hormone independent prostate cancer etc.), uterine cancer, breast cancer (including progressive breast cancer, for example, invasive ductal carcinoma, non-invasive ductal carcinoma, inflammatory breast cancer etc.), pituitary gland tumor, liver cancer (e.g., primary liver cancer, extrahepatic bile duct cancer etc.) and the like), and sex hormone sensitive diseases such as prostatomegaly, endometriosis, hysteromyoma, early puberty, dysmenorrhea, amenorrhea, premenstrual syndrome, polycystic ovary syndrome and the like, contraceptive (or agent for the prophylaxis or treatment of infertility when the rebound effect after cessation of the drug is utilized) and the like.
Particularly, since compound (I), compound (I') and compound (IA) or salts thereof of the present invention show an antagonistic action on normal androgen receptor and/or mutant receptor, they can exhibit a superior prophylactic or therapeutic effect on hormone sensitive cancers in the androgen-dependent phase and/or the androgen-independent phase.
Of the androgen receptor antagonists, a drug showing an antagonistic action on mutant androgen receptors and a drug showing an antagonistic action on androgen receptors with enhanced sensitivity are also useful as agents for the prophylaxis or treatment of hormone sensitive cancers in the androgen-dependent phase and/or the androgen-independent phase.

A pharmaceutical agent containing compound (I), compound (I') or compound (IA) or a salt thereof of the present invention can be safely administered orally or parenterally (e.g., topical, rectal, intravenous administration etc.), for example, after admixing the androgen receptor antagonist of the present invention with a pharmacologically acceptable carrier to give a pharmaceutical composition such as tablets (including sugar-coated tablets and film-coated tablets), powders, granules, capsules (including soft capsules), liquids, injections, suppositories, sustained-release agents and the like, according to a method known *per se*. Injection can be administered by intravenous, intramuscular, subcutaneous or intraorgan administration or directly to the lesion.
Examples of the pharmacologically acceptable carrier that can be used for the production of the pharmaceutical agent of the present invention include various organic or inorganic carriers conventionally used as preparation materials. For example, excipient, lubricant, binder and disintegrant for solid preparations, or solvent, solubilizing agents, suspending agent, isotonic agent, buffer, soothing agent and the like for liquid preparations can be mentioned. Furthermore, where necessary, suitable amount of conventional preservative, antioxidant, colorant, sweetening agent, adsorbent, wetting agent and the like can be used as appropriate.

Examples of the excipient include lactose, sucrose, D-mannitol, starch, cornstarch, crystalline cellulose, light anhydrous silicic acid and the like.
Examples of the lubricant include magnesium stearate, calcium stearate, talc, colloidal silica and the like.
Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose and the like.
Examples of the disintegrant include starch, carboxymethylcellulose, carboxymethylcellulose calcium, sodium carboxymethylstarch, L-hydroxypropylcellulose and the like.
Examples of the solvent include water for injection, alcohol, propylene glycol, macrogol, sesame oil, corn oil, olive oil and the like.
Examples of the solubilizer include polyethylene glycol, propylene glycol, D-mannitol, benzyl benzoate, ethanol, trisaminomethane, cholesterol, triethanolamine, sodium carbonate, sodium citrate and the like.
Examples of the suspending agent include surfactants such as stearyl triethanolamine, sodium laurylsulfate, laurylaminopropionic acid, lecithin, benzalkonium chloride, benzethonium chloride, glycerin monostearate, etc.; hydrophilic polymers such as polyvinyl alcohol, polyvinylpyrrolidone, sodium carboxymethyl cellulose, methyl cellulose, hydroxymethyl cellulose, hydroxyethyl cellulose, hydroxypropyl cellulose and the like.
Examples of the isotonic agent include glucose, D-sorbitol, sodium chloride, glycerol, D-mannitol and the like.
Examples of the buffer include a buffer solution of phosphate, acetate, carbonate, citrate and the like.
Examples of the soothing agent include benzyl alcohol and the like.
Examples of the antiseptic include paraoxybenzoates, chlorobutanol, benzyl alcohol, phenethyl alcohol, dehydroacetic acid, sorbic acid and the like.
Examples of the antioxidant include sulfite, ascorbic acid, α-tocopherol and the like.

The content of compound (I), compound (I') or compound (IA) or a salt thereof of the present invention in the pharmaceutical agent of the present invention can be appropriately determined in consideration of the administration subject, administration route, diseases and the like. For example, it is usually from about 0.01 to 100% by weight, preferably from about 0.1 to 50% by weight, further preferably from about 0.5 to 20% by weight, based on the whole preparation, though subject to variation depending on the form of the preparation.
While the content of additives such as a carrier and the like in the pharmaceutical composition of the present invention varies depending on the form of the preparation, it is usually from about 1 to 99.99% by weight, preferably from about 10 to 90% by weight, based on the whole preparation.

The compound (I), compound (I') and compound (IA) or salts thereof of the present invention are low toxic and can be used safely. The daily dose thereof varies depending on the kind of the compound, age, body weight and symptom of patients, dosage form, administration method and the like. For intravenous administration to a patient for, for example, the purpose of treating prostate cancer, the daily dose for an adult (body weight about 60 kg) is about 0.01 - about 1000 mg/kg, preferably about 0.01 - about 100 mg/kg, more preferably about 0.1 - about 100 mg/kg, particularly about 0.1 - about 50 mg/kg, especially about 1.5 - about 30 mg/kg, which is intravenously administered once a day or in several portions a day. It is needless to say that the dose varies depending on various conditions as mentioned above. Therefore, a dose smaller than the aforementioned dose may be sufficient or an excess dose may be necessary in some cases.

As a drug that can be concurrently used along with compound (I), compound (I') and compound (IA) or salts thereof of the present invention, for example, hormonal therapeutic agent, anti-cancer agent (e.g., chemotherapeutic agent, immunotherapeutic agent (including vaccine), antibody, gene therapy drug, pharmaceutical agent that inhibits the action of a cell growth factor and receptor thereof, pharmaceutical agent that inhibits angiogenesis) and the like (hereinafter to be abbreviated as concomitant drug) can be used.
Although the compound (I), compound (I') or compound (IA) or a salt thereof in the present invention exhibits excellent anticancer action even when used as a simple agent or its effect can be enhanced by using it in combination with one or more of the combination drug(s) mentioned above (multi-agent co-administration).

As examples of the "hormonal therapeutic agents", there can be used fosfestrol, diethylstylbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, tadenan, mepartricin, raloxifene, ormeloxifene, levormeloxifene, anti-estrogens (e.g., tamoxifen citrate, toremifene citrate etc.), ER down-regulator (e.g., fulvestrant etc.), human postmenopausal gonadotropin, follitropin, pill preparations, mepitiostane, testrolactone, aminoglutethimide, LH-RH derivative (e.g., LH-RH agonists (e.g., goserelin acetate, buserelin, leuprorelin etc.), LH-RH antagonist), droloxifene, epitiostanol, ethinylestradiol sulfonate, aromatase inhibitors (e.g., fadrozole hydrochloride, anastrozole, retrozole, exemestane, vorozole, formestane etc.), anti-androgens (e.g., flutamide, bicartamide, nilutamide etc.), 5α-reductase inhibitors (e.g., finasteride, dutasteride, epristeride etc.), adrenocorticohormone drugs (e.g., dexamethasone, prednisolone, betamethasone, triamcinolone etc.), androgen synthesis inhibitors (e.g., abiraterone etc.), retinoid and drugs that retard retinoid metabolism (e.g., liarozole etc.), and the like.

As examples of the "chemotherapeutic agents", there can be used alkylating agents, antimetabolites, anticancer antibiotics, plant-derived anticancer agents, and the other chemotherapeutic agents.
The "alkylating agents" include nitrogen mustard, nitrogen mustard N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin etc.

The "antimetabolites" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emitefur, etc.), aminopterine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, and ambamustine, etc.
The "anticancer antibiotics" include actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, etc.

The "plant-derived anticancer agents" include etoposide, etoposide phosphate, vinblastine sulfate, vincristine sulfate, vindesine sulfate, teniposide, paclitaxel, docetaxel, vinorelbine, irinotecan, topotecan etc.
As "other chemotherapeutic agent", for example, sobuzoxane and the like can be used.
The "immunotherapeutic agents (BRM)" include picibanil, krestin, sizofiran, lentinan, ubenimex, interferons, interleukins, macrophage colony-stimulating factor, granulocyte colony-stimulating factor, erythropoietin, lymphotoxin, Corynebacterium parvum, levamisole, polysaccharide K, procodazole, etc. As the vaccine, BCG vaccine, PROVENGE, Onyvax-P, PROSTVAC-VF, GVAX, DCVax-Prostate, SAPOIMMUNE, VPM-4-001 and the like can be used.
As the "antibody", an antibody to EpiCAM, an antibody to PSCA, an antibody to PSMA and the like are used.

The "cell growth factor" in the "drugs that inhibit the activity of cell growth factors or cell growth factor receptors" includes any substances that promote cell proliferation, which are normally peptides having a molecular weight of not more than 20,000 that are capable of exhibiting their activity at low concentrations by binding to a receptor, including (1) EGF (epidermal growth factor) or substances possessing substantially the same activity as it [e.g., EGF, heregulin, TGF-α, HB-EGF etc.], (2) insulin or substances possessing substantially the same activity as it [e.g., insulin, IGF (insulin-like growth factor)-1, IGF-2, etc.], (3) FGF (fibroblast growth factor) or substances possessing substantially the same activity as it [e.g., acidic FGF, basic FGF, KGF (keratinocyte growth factor), FGF-10, etc.], (4) other cell proliferation factors [e.g., CSF (colony stimulating factor), EPO (erythropoietin), IL-2 (interleukin-2), NGF (nerve growth factor), PDGF (platelet-derived growth factor), TGF β (transforming growth factor β), HGF (hepatocyte growth factor), VEGF (vascular endothelial growth factor), etc.], etc.
The "cell growth factor receptors" include any receptors capable of binding to the aforementioned cell growth factors and, specifically, EGF receptor and HER2, HER3 and HER4, which are the receptors belonging to the same family, insulin receptor, IGF receptor, FGF receptor-1, FGF receptor-2 and the like can be mentioned.
The "drugs that inhibit the activity of cell proliferation factor" include trastuzumab (Herceptin (trademark): (HER2 antibody)), imatinib mesylate, ZD1839, cetuximab, gefitinib, erlotinib and the like.
As the "pharmaceutical agent that inhibits angiogenesis", antibody to VEGF (e.g., bevacizumab), antibody to VEGF receptor, VEGF receptor kinase inhibitor (e.g., SU11248 etc.), PDGF receptor kinase inhibitor, Tie2 kinase inhibitor, thalidomide and the like can be used.
In addition to the aforementioned drugs, L-asparaginase, aceglatone, procarbazine hydrochloride, protoporphyrin-cobalt complex salt, mercuric hematoporphyrin-sodium, differentiation inducers (e.g., retinoid, vitamin D, etc.), α-blockers (e.g., tamsulosin hydrochloride, naftopidil, urapidil, alfuzosin, terazosin, prazosin, silodosin etc.) serine/threonine kinase inhibitor, endothelin receptor antagonist (e.g., atrasentan etc.), proteasome inhibitor (e.g., bortezomib etc.), Hsp 90 inhibitor (e.g., 17-AAG etc.), spironolactone, minoxidil, 11α-hydroxyprogesterone, bone resorption inhibitory ·metastasis suppressing agent (e.g., zoledronic acid, alendronic acid, pamidronic acid, etidronic acid, ibandronic acid, clodronic acid) and the like can be used.

Particularly preferable concomitant drug is, for example, LH-RH derivative and the like.
The LH-RH derivative includes an LH-RH derivative or salt thereof which is effective against hormone-dependent diseases, especially sex hormone-dependent diseases such as sex hormone-dependent cancers (e.g., prostate cancer, uterine cancer, breast cancer, hypophyseal tumor, liver cancer, etc.), prostatic hypertrophy, endometriosis, uterine myoma, precocious puberty, dysmenorrhea, amenorrhea, premenstrual syndrome, multilocular ovary syndrome, etc., and contraception (or infertility when rebound effect after drug withdrawal is applied). Further it includes an LH-RH derivative or salt thereof which is effective against benign tumor or malignant tumor which is sex hormone-independent and LH-RH sensitive.
Specific examples of the LH-RH derivatives or salt thereof include peptides described in "Treatment with GnRH analogs: Controversies and perspectives" issued in 1996 by The Parthenon Publishing Group Ltd., PCT Japanese Translation Patent Publication No. 3-503165, JP-A 3-101695, JP-A 7-97334 and JP-A 8-259460, etc.

The LH-RH derivative includes LH-RH agonists and LH-RH antagonists. The LH-RH antagonist includes, for example, a physiologically active peptide represented by the formula:

X-D2Nal-D4ClPhe-D3Pal-Ser-A-B-Leu-C-Pro-DAlaNH₂

[wherein X is N(4H₂-furoyl)Gly or NAc, A is a residue selected from NMeTyr, Tyr, Aph(Atz) and NMeAph(Atz), B is a residue selected from DLys(Nic), DCit, DLys(AzaglyNic), DLys(AzaglyFur), DhArg(Et₂), DAph(Atz) and DhCi, and C is Lys(Nisp), Arg or hArg(Et₂)] or a salt thereof, etc.
The LH-RH agonist includes, for example, a physiologically active peptide represented by the formula:

5-oxo-Pro-His-Trp-Ser-Tyr-Y-Leu-Arg-Pro-Z

[wherein Y is a residue selected from DLeu, DAla, DTrp, DSer(tBu), D2Nal and DHis(ImBzl) and Z is NH-C₂H₅ or Gly-NH₂] or a salt thereof, etc, especially, suitably, a peptide wherein Y is DLeu, and Z is NH-C₂H₅ (that is, Peptide A represented by 5-oxo-Pro-His-Trp-Ser-Tyr-DLeu-Leu-Arg-Pro-NH-C₂H₅; leuprorelin) or a salt thereof (e.g., acetate).

A pharmaceutical agent containing compound (I), compound (I') or compound (IA) or a salt thereof of the present invention and a concomitant drug in combination (hereinafter a combination drug of the present invention) is low toxic and can be safely administered orally or parenterally (e.g., topical, rectal, intravenous administration etc.), for example, after admixing the androgen receptor antagonist of the present invention and/or the aforementioned concomitant drug with a pharmacologically acceptable carrier to give a pharmaceutical composition such as tablets (including sugar-coated tablets and film-coated tablets), powders, granules, capsules (including soft capsules), liquids, injections, suppositories, sustained-release agents and the like, according to a method known *per se*. Injection can be administered by intravenous, intramuscular, subcutaneous or intraorgan administration or directly to the lesion.
Examples of the pharmacologically acceptable carrier that can be used for the production of the combination drug in the present invention include various organic or inorganic carriers conventionally used as preparation materials. For example, excipient, lubricant, binder and disintegrant for solid preparations, or solvent, solubilizing agents, suspending agent, isotonic agent, buffer, soothing agent and the like for liquid preparations can be mentioned. Furthermore, where necessary, suitable amount of conventional preservative, antioxidant, colorant, sweetening agent, adsorbent, wetting agent and the like can be used as appropriate.

Examples of the excipient include lactose, sucrose, D-mannitol, starch, cornstarch, crystalline cellulose, light anhydrous silicic acid and the like.
Examples of the lubricant include magnesium stearate, calcium stearate, talc, colloidal silica and the like.
Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose and the like.
Examples of the disintegrant include starch, carboxymethylcellulose, calcium carboxymethylcellulose, sodium carboxymethyl starch, L-hydroxypropylcellulose and the like.
Examples of the solvent include water for injection, alcohol, propylene glycol, macrogol, sesame oil, corn oil, olive oil and the like.
Examples of the solubilizing agents include polyethylene glycol, propylene glycol, D-mannitol, benzyl benzoate, ethanol, trisaminomethane, cholesterol, triethanolamine, sodium carbonate, sodium citrate and the like.
Example of the suspending agent include a surfactant such as stearyltriethanolamine, sodium lauryl sulfate, lauryl aminopropionic acid, lecithin, benzalkonium chloride, benzethonium chloride, glycerol monostearate and the like; a hydrophilic polymer such as polyvinyl alcohol, polyvinylpyrrolidone, sodium carboxymethylcellulose, methylcellulose, hydroxymethylcellulose, hydroxyethylcellulose, hydroxypropylcellulose etc. and the like.
Examples of the isotonic agent include glucose, D-sorbitol, sodium chloride, glycerol, D-mannitol and the like.
Examples of the buffer include a buffer solution of phosphate, acetate, carbonate, citrate etc. and the like.
Examples of the soothing agent include benzyl alcohol and the like.
Examples of the preservative include paraoxybenzoates, chlorobutanol, benzyl alcohol, phenethyl alcohol, dehydroacetic acid, sorbic acid and the like.
Examples of the antioxidant include sulfite, ascorbic acid, α-tocopherol and the like.

The mixing ratio of the androgen receptor antagonist of the present invention and a concomitant drug in the combination drug in the present invention can be appropriately determined according to the subject of administration, administration route, disease and the like.
For example, the content of the androgen receptor antagonists of the present invention in the combination drug of the present invention varies depending on the form of preparation, and is usually from about 0.01% by weight to 99.9% by weight, preferably from about 0.1% by weight to 50% by weight, more preferably from about 0.5% by weight to 20% by weight, relative to the total of the preparation.
The content of the concomitant drug in the combination drug in the present invention varies depending on the form of preparation, and is usually from about 0.01% by weight to 99.9% by weight, preferably from about 0.1% by weight to 50% by weight, more preferably from about 0.5% by weight to 20% by weight, relative to the total of the preparation.
The content of additives such as a carrier etc. in the combination drug of the present invention varies depending on the form of preparation, and is usually from about 1% by weight to 99.98% by weight, preferably from about 10% by weight to 90% by weight, to the total of the preparation.
When the androgen receptor antagonist of the present invention and the concomitant drug are formulated separately, the same contents may be adopted.

These preparations can be manufactured by a per se known method commonly used in the pharmaceutical manufacturing process.
For example, the androgen receptor antagonists of the present invention and the combination drug can be made as an injection such as an aqueous injection together with a dispersing agent (e.g., Tween 80 (manufactured by Atlas Powder, US), HCO 60 (manufactured by Nikko Chemicals Co., Ltd.), polyethylene glycol, carboxymethyl cellulose, sodium alginate, hydroxypropylmethyl cellulose, dextrin etc.), a stabilizer (e.g., ascorbic acid, sodium pyrosulfite, etc.), a surfactant (e.g., Polysorbate 80, macrogol etc.), a solubilizer (e.g., glycerin, ethanol etc.), a buffer (e.g., phosphoric acid and alkali metal salt thereof, citric acid and alkali metal salt thereof, etc.), an isotonizing agent (e.g., sodium chloride, potassium chloride, mannitol, sorbitol, glucose etc.), a pH regulator (e.g., hydrochloric acid, sodium hydroxide etc.), an antiseptic (e.g., ethyl paraoxybenzoate, benzoic acid, methylparaben, propylparaben, benzyl alcohol etc.), a dissolving agent (e.g., conc. glycerin, meglumine etc.), a solubilizing agent (e.g., propylene glycol, sucrose etc.), a soothing agent (e.g., glucose, benzyl alcohol etc.), etc., or an oily injection by dissolving, suspending or emulsifying them in a vegetable oil such as olive oil, sesame oil, cotton seed oil, corn oil etc. or a solubilizing agent such as propylene glycol, and processing them.

In the case of a preparation for oral administration, the androgen receptor antagonists of the present invention and the combination drug can be made as a preparation for oral administration by adding an excipient (e.g., lactose, sucrose, starch etc.), a disintegrating agent (e.g., starch, calcium carbonate etc.), a binder (e.g., starch, arabic gum, carboxymethyl cellulose, polyvinylpyrrolidone, hydroxypropyl cellulose etc.), a lubricant (e.g., talc, magnesium stearate, polyethylene glycol 6000 etc.) etc., to the compound of the present invention or the combination drug, according to a per se known method, and compressing and molding the mixture, then if desired, coating the molded product by a per se known method for the purpose of masking of taste, enteric property or sustained release. The film forming agent includes, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (methacrylic acid/acrylic acid copolymer, manufactured by Rohm, DE), pigment (e.g., iron oxide red, titanium dioxide, etc.) etc. The preparation for oral administration may be either a rapid release preparation or a sustained release preparation.

For example, in the case of a suppository, the androgen receptor antagonists of the present invention and the combination drug can be made into an oily or aqueous solid, semisolid or liquid suppository according to a per se known method. The oily base used in the above-mentioned composition includes, for example, glycerides of higher fatty acids [e.g., cacao butter, Witepsols (manufactured by Dynamite Nobel, DE), etc.], middle-chain fatty acids [e.g., Miglyols (manufactured by Dynamite Nobel, DE), etc.], or vegetable oils (e.g., sesame oil, soy bean oil, cotton seed oil etc.), etc. Further, the aqueous base includes, for example, polyethylene glycols and propylene glycol, and the aqueous gel base includes, for example, natural gums, cellulose derivatives, vinyl polymers, acrylic acid polymers, etc.
The above-mentioned sustained release agent includes sustained release microcapsules, etc.
For obtaining a sustained release microcapsule, a per se known method can be adopted. For example, it is preferable to mold into a sustained release preparation shown in [2] below.
The androgen receptor antagonist of the present invention is preferably molded into an oral administration preparation such as a solid preparation (e.g., powder, granule, tablet, capsule, etc.) etc., or molded into a rectal administration preparation such as a suppository. Particularly, an oral administration preparation is preferable.
The combination drug can be made into the above-mentioned drug form depending on the kind of the drug.

In the following, there will be shown specifically [1] an injection of the compound (I), compound (I') or compound (IA) or a salt thereof in the present invention or the combination drug and preparation thereof, [2] a rapid release preparation or sustained release preparation of the compound (I), compound (I') or compound (IA) or a salt thereof in the present invention or the combination drug and preparation thereof and [3] a sublingual tablet, a buccal or an intraoral quick integrating agent of the compound (I), compound (I') or compound (IA) or a salt thereof in the present invention or the combination drug and preparation thereof.

### [1] Injection and preparation thereof

It is preferred that an injection is prepared by dissolving the compound (I), compound (I') or compound (IA) or a salt thereof in the present invention or the combination drug in water. This injection may be allowed to contain a benzoate and/or a salicylate.

The injection is obtained by dissolving the compound (I), compound (I') or compound (IA) or a salt thereof in the present invention or the combination drug, and if desired, a benzoate and/or a salicylate, into water.

The above-mentioned salts of benzoic acid and salicylic acid include, for example, salts of alkali metals such as sodium, potassium etc., salts of alkaline earth metals such as calcium, magnesium etc., ammonium salts, meglumine salts, organic acid salts such as tromethamol, etc.

The concentration of the compound (I), compound (I') or compound (IA) or a salt thereof in the present invention or the combination drug is from 0.5 w/v% to 50 w/v%, preferably from about 3 w/v% to about 20 w/v%. The concentration of a salt of benzoic acid or/and a salt of salicylic acid is from 0.5 w/v% to 50 w/v%, preferably from 3 w/v% to 20 w/v%.

Conventional additives to be used in an injection may be appropriately added in a preparation of the present invention. Examples of the additives include a stabilizer (e.g., ascorbic acid, sodium pyrosulfite, etc.), a surfactant (e.g., Polysorbate 80, macrogol etc.), a solubilizer (e.g., glycerin, ethanol etc.), a buffer (e.g., phosphoric acid and alkali metal salt thereof, citric acid and alkali metal salt thereof, etc.), an isotonizing agent (e.g., sodium chloride, potassium chloride, etc.), a dispersing agent (e.g., hydroxypropylmethyl cellulose, dextrin), a pH regulator (e.g., hydrochloric acid, sodium hydroxide etc.), an antiseptic (e.g., ethyl paraoxybenzoate, benzoic acid etc.), a dissolving agent (e.g., conc. glycerin, meglumine etc.), a solubilizing agent (e.g., propylene glycol, sucrose etc.), a soothing agent (e.g., glucose, benzyl alcohol etc.), etc. These additives are blended in a usual proportion generally employed in an injection.

It is advantageous that the pH of the injection is controlled from pH 2 to 12, preferably from 2.5 to 8.0 by addition of a pH regulator.

An injection is obtained by dissolving the compound (I), compound (I') or compound (IA) or a salt thereof in the present invention or the combination drug and if desired, a salt of benzoic acid and/or a salt of salicylic acid, and if necessary, the above-mentioned additives into water. These may be dissolved in any order, and can be appropriately dissolved in the same manner as in a conventional method of producing an injection.

An aqueous solution for injection may be advantageously heated, alternatively, for example, filter sterilization, high pressure heat sterilization, etc. can be conducted in the same manner as those for a usual injection, to provide an injection.

It may be advantageous that an aqueous solution for injection is subjected to high pressure heat sterilization at 100°C to 121°C for 5 minutes to 30 minutes.

Further, a preparation endowed with the antibacterial property of a solution may also be produced so that it can be used as a preparation which is divided and administered multiple-times.

### [2] Sustained release preparation or rapid release preparation, and preparation thereof

Preferred is a sustained release preparation which is obtained, by coating a core containing the compound (I), compound (I') or compound (IA) or a salt thereof in the present invention or the combination drug with a film forming agent such as a water-insoluble substance, swellable polymer, etc., if desired. For example, a sustained release preparation for oral once-a-day administration is preferable.
The water insoluble substance used in a film forming agent includes, for example, a cellulose ether such as ethyl cellulose, butyl cellulose, etc.; a cellulose ester such as cellulose acetate, cellulose propionate, etc.; a polyvinyl ester such as polyvinyl acetate, polyvinyl butyrate, etc.; an acrylic acid polymer such as acrylic acid/methacrylic acid copolymer, methylmethacrylate copolymer, ethoxyethyl methacrylate/cinnamoethylmethacrylate/aminoalkyl methacrylate copolymer, polyacrylic acid, polymethacrylic acid, methacrylic acid alkyl amide copolymer, poly(methyl methacrylate), polymethacrylate, polymethacryl amide, amino alkyl methacrylate copolymer, poly(methacrylic acid anhydride), glycidyl methacrylate copolymer, specially an Eudragit (manufactured by Rohm Pharma) such as Eudragit RS-100, RL-100, RS-30D, RL-30D, RL-PO, RS-PO (copolymer of ethyl acylate/methyl methacrylate/trimethyl chloride methacrylate/ammonium ethyl), Eudragit NE-30D (copolymer of methyl methacrylate/ethyl acrylate), etc., a hydrogenated oil such as hardened caster oil (e.g., Lovely wax (Freund Corporation), etc.), etc.; a wax such as carnauba wax, fatty acid glycerin ester, paraffin, etc.; polyglycerin fatty acid ester, etc.

The swellable polymer is preferably a polymer having acidic dissociating group and pH-dependent swelling property, and a polymer having acidic dissociating group which swells little in an acidic area such as stomach and swells greatly in a neutral area such as the small intestine or the large intestine.
The polymer having acidic dissociating group and pH-dependent swelling property includes, for example, crosslinkable polyacrylic polymer such as Carbomer 934P, 940, 941, 974P, 980, 1342 etc., polycarbophil, calcium polycarbophil (all are manufactured by BF Goodrich.), Hibiswako 103, 104, 105, 304 (all are manufactured by Wako Pure Chemical Industries, Ltd.), etc.

The film forming agent used in a sustained release preparation may further contain a hydrophilic substance.
The hydrophilic substance includes, for example, a polysaccharide optionally having sulfuric acid group such as pullulans, dextrin, arginic acid alkali metal salt, etc.; a polysaccharide having a hydroxyalkyl group or a carboxyalkyl group such as hydroxypropyl cellulose, hydroxypropyl methyl cellulose, sodium carboxymethyl cellulose, etc.; methyl cellulose; polyvinyl pyrrolidone; polyvinyl alcohol; polyethylene glycol; etc.
The content of water-insoluble substance in the film forming agent of sustained release preparation is about 30% (w/w) to about 90% (w/w), preferably about 35% (w/w) to about 80% (w/w), and more preferably about 40% (w/w) to about 75% (w/w). The content of swellable polymer is about 3% (w/w) to about 30% (w/w), preferably about 3% (w/w) to about 15% (w/w). The film forming agent may further contain a hydrophilic substance, in this case, the content of the hydrophilic substance in the film forming agent is about 50% (w/w) or less, preferably about 5% (w/w) to about 40% (w/w), and more preferably about 5% (w/w) to about 35% (w/w). This % (w/w) indicates % by weight based on a film forming agent composition which is obtained by removing a solvent (e.g., water, lower alcohols such as methanol, ethanol etc.) from a film forming agent liquid.

The sustained release preparation is manufactured by preparing a core containing drug, then, coating the resultant core with a film forming agent liquid prepared by heating and dissolving a water-insoluble substance, swellable polymer, etc. or by dissolving or dispersing it in a solvent as exemplified below.

### I. Preparation of core containing a drug

The form of a core containing a drug to be coated with a film forming agent (hereinafter, sometimes simply referred to as the core) is not particularly limited, and preferably, the core is formed into particles such as granules or fine particles.

When the core is composed of granules or fine particles, the average particle size thereof is preferably from about 150 to about 2,000 µm, further preferably, from about 500 µm to about 1,400 µm.

Preparation of the core can be conducted by a usual preparation method. For example, it can be prepared by mixing a suitable excipient, binding agent, disintegrating agent, lubricant, stabilizer, etc. with a drug, and subjecting the mixture to wet-extrusion granulating method or fluidized bed granulating method, etc.

The content of drugs in a core is from about 0.5% (w/w) to about 95% (w/w), preferably from about 5.0% (w/w) to about 80% (w/w), further preferably from about 30% (w/w) to about 70% (w/w).

The excipient contained in the core includes, for example, saccharides such as sucrose, lactose, mannitol, glucose etc., starch, crystalline cellulose, calcium phosphate, corn starch etc. Among them, crystalline cellulose, corn starch are preferable.

The binders include, for example, polyvinyl alcohol, hydroxypropyl cellulose, polyethylene glycol, polyvinyl pyrrolidone, Pluronic F68, gum arabic, gelatin, starch, etc. The disintegrant include, for example, carboxymethyl cellulose calcium (ECG505), croscarmellose sodium (Ac-Di-Sol), crosslinkable polyvinyl pyrrolidone (crospovidone), low-substituted hydroxypropyl cellulose (L-HPC), etc. Among these, hydroxypropyl cellulose, polyvinyl pyrrolidone and low-substituted hydroxypropyl cellulose are preferable. The lubricants or the aggregation inhibitor includes, for example, talc, magnesium stearate and an inorganic salt thereof. The lubricant includes a polyethylene glycol, etc. The stabilizing agent includes an acid such as tartaric acid, citric acid, succinic acid, fumaric acid, maleic acid, etc.

In addition to the above-mentioned production method, the core can also be prepared by, for example, a rolling granulation method in which a drug or a mixture of the drug with an excipient, lubricant, etc. is added portionwise onto an inert carrier particle which is the core of the core while spraying a binder dissolved in a suitable solvent such as water, lower alcohol (e.g., methanol, ethanol, etc.) etc., a pan coating method, a fluidized bed coating method or a melt granulating method. The inert carrier particle includes, for example, those made of sucrose, lactose, starch, crystalline cellulose or waxes, and the average particle size thereof is preferably from about 100 µm to about 1,500 µm.
For the purpose of separating the drug contained in the core from the film forming agent, the surface of the core may be coated with a protective agent. The protective agent includes, for example, the above-mentioned hydrophilic substances, water-insoluble substances etc. The protective agent includes, preferably polyethylene glycol, and polysaccharides having hydroxyalkyl or carboxyalkyl, more preferably, hydroxypropylmethyl cellulose and hydroxypropyl cellulose. The protective agent may contain a stabilizer such as acids such as tartaric acid, citric acid, succinic acid, fumaric acid, maleic acid etc., and a lubricant such as talc etc. When the protective agent is used, the coating amount is from about 1% (w/w) to about 15% (w/w), preferably from about 1% (w/w) to about 10% (w/w), further preferably from about 2% (w/w) to about 8% (w/w), based on the core.
The coating of the protective agent can be carried out by a usual coating method, and specifically, the coating can be carried out by spraying the protective agent onto a core by a fluidized bed coating method, pan coating method etc.

### II. Coating of core with a film forming agent

A core obtained in the above-mentioned step I is coated with a film forming agent liquid obtained by heating and dissolving the above-mentioned water-insoluble substance and pH-dependent swellable polymer, and a hydrophilic substance, or by dissolving or dispersing them in a solvent, to give a sustained release preparation.
The method for coating a core with a film forming agent liquid includes, for example, a spray coating method etc.
The composition ratio of a water-insoluble substance, swellable polymer and hydrophilic substance in a film forming agent liquid is appropriately selected so that the contents of these components in a coated film are the above-mentioned contents, respectively.
The coating amount of a film forming agent is from about 1% (w/w) to about 90% (w/w), preferably from about 5% (w/w) to about 50% (w/w), further preferably from about 5% (w/w) to 35% (w/w), based on a core (exclusive of the coating amount of the protective agent).
The solvent in the film forming agent liquid includes water or an organic solvent, alone or in admixture thereof. In the case of use in admixture, the mixing ratio of water to an organic solvent (water/organic solvent: weight ratio) can be varied in the range from 1 to 100%, and preferably from 1% to about 30%. The organic solvent is not particularly limited as long as it dissolves a water-insoluble substance, and for example, it includes lower alcohols such as methyl alcohol, ethyl alcohol, isopropyl alcohol, n-butyl alcohol, etc., lower alkanones such as acetone, etc., acetonitrile, chloroform, methylene chloride, etc. Among them, lower alcohols are preferable, and ethyl alcohol and isopropyl alcohol are particularly preferable. Water, and a mixture of water with an organic solvent are preferably used as a solvent for a film forming agent. In this case, if necessary, an acid such as tartaric acid, citric acid, succinic acid, fumaric acid, maleic acid, etc. may also be added into a film forming agent liquid for stabilizing the film forming agent liquid.
An operation of coating by spray coating can be conducted by a usual coating method, and specifically, it can be conducted by spray-coating a film forming agent liquid onto a core, for example, by a fluidized bed coating method, pan coating method etc. In this case, if necessary, talc, titanium oxide, magnesium stearate, calcium stearate, light anhydrous silicic acid etc. may also be added as a lubricant, and glycerin fatty acid ester, hydrogenated castor oil, triethyl citrate, cetyl alcohol, stearyl alcohol etc. may also be added as a plasticizer.
After coating with a film forming agent, if necessary, an antistatic agent such as talc etc. may be mixed.

The rapid release preparation may be liquid (solution, suspension, emulsion etc.) or solid (particle, pill, tablet etc.). It may be oral agents or parenteral agents such as an injection, etc., and preferably, oral agents.
The rapid release preparation, usually, may contain, in addition to an active component drug, also carriers, additives and excipients conventionally used in the field of formulation (hereinafter, sometimes abbreviated as the excipient). The preparation excipient used is not particularly limited as long as it is an excipient ordinarily used as a preparation excipient. For example, the excipient for an oral solid preparation includes lactose, starch, corn starch, crystalline cellulose (Avicel PH101, manufactured by Asahi Kasei Corporation, etc.), powder sugar, granulated sugar, mannitol, light anhydrous silicic acid, magnesium carbonate, calcium carbonate, L-cysteine, etc., and preferably, corn starch and mannitol, etc. These excipients can be used alone or in combination of two or more. The content of the excipient is, for example, from about 4.5 w/w% to about 99.4 w/w%, preferably from about 20 w/w% to about 98.5 w/w%, further preferably from about 30 w/w% to about 97 w/w%, based on the total amount of the rapid release preparation.
The content of a drug in the rapid release preparation can be appropriately selected in the range from about 0.5% to about 95%, preferably from about 1% to about 60% based on the total amount of the rapid release preparation.

When the rapid release preparation is an oral solid preparation, it usually contains a disintegrating agent in addition to the above-mentioned components. The disintegrating agent includes, for example, carboxymethyl cellulose calcium (ECG-505, manufactured by GOTOKU CHEMICAL COMPANY LTD.), croscarmellose sodium (e.g., acjizol, manufactured by Asahi Kasei Corporation), crospovidone (e.g., colidone CL, manufactured by BASF), low-substituted hydroxypropyl cellulose (manufactured by Shin-Etsu Chemical Co., Ltd.), carboxymethylstarch (manufactured by Matsutani Chemical Industry Co., Ltd.), carboxymethylstarch sodium (Exprotab, manufactured by Kimura Sangyo), partially α-starch (PCS, manufactured by Asahi Kasei Corporation), etc., and for example, includes those which disintegrate a granule by absorbing water in contact with water, causing swelling, or making a channel between an effective ingredient constituting the core and an excipient. These disintegrating agents can be used alone or in combinations of two or more. The amount of the disintegrating agent used is appropriately selected depending on the kind and blending amount of a drug used, formulation design for release property, etc., and for example, from about 0.05 w/w% to about 30 w/w%, preferably from about 0.5 w/w% to about 15 w/w%, based on the total amount of the rapid release preparation.

When the rapid release preparation is an oral solid preparation, it may further contain if desired, additives conventional in solid preparations in addition to the above-mentioned composition. Such an additive includes, for example, a binder (e.g., sucrose, gelatin, arabic gum powder, methyl cellulose, hydroxypropyl cellulose, hydroxypropylmethyl cellulose, carboxylmethyl cellulose, polyvinylpyrrolidone, pullulans, dextrin, etc.), a lubricant (e.g., polyethylene glycol, magnesium stearate, talc, light anhydrous silicic acid (e.g., aerosil (Nippon Aerosil)), a surfactant (e.g., anionic surfactants such as sodium alkylsulfate, etc., nonionic surfactants such as polyoxyethylene fatty acid ester and polyoxyethylene sorbitan fatty acid ester, polyoxyethylene castor oil derivatives, etc.), a colorant (e.g., tar coloring matter, caramel, iron oxide red, titanium oxide, riboflavins), if necessary, a corrigent (e.g., sweetening agent, flavor, etc.), an adsorbent, an antiseptic, a wetting agent, an antistatic agent, etc. Further, a stabilizer such as an organic acid such as tartaric acid, citric acid, succinic acid, fumaric acid, etc. may also be added.
The above-mentioned binder includes preferably hydroxypropyl cellulose, polyethylene glycol and polyvinylpyrrolidone, etc.
The rapid release preparation can be prepared by mixing the above-mentioned components, and if necessary, further kneading the mixture, and molding it based on a usual technology of producing preparations. The above-mentioned mixing is conducted by generally used methods, for example, mixing, kneading, etc. Specifically, when a rapid release preparation is formed, for example, into a particle, it can be prepared, according to the same means as in the above-mentioned method for preparing a core of a sustained release preparation, by mixing the components using a vertical granulator, universal kneader (manufactured by Hata Iron Works Co., Ltd.), fluidized bed granulator FD-5S (manufactured by Powrex Corporation), etc., then, subjecting the mixture to a wet extrusion granulation method, fluidized bed granulation method, etc.
Thus obtained rapid release preparation and sustained release preparation may be themselves made into products or made into products appropriately together with preparation excipients etc., separately, by an ordinary method, then, may be administered simultaneously or may be administered in combination at any administration interval, or they may be themselves made into one oral administration preparation (e.g., granule, fine particle, tablet, capsule etc.) or made into one oral administration preparation together with preparation excipients etc. It may also be permissible that they are made into granules or fine particles, and filled in the same capsule to be used as a preparation for oral administration.

### [3] Sublingual, buccal or intraoral quick disintegrating agent and preparation thereof

Sublingual, buccal or intraoral quick disintegrating agents may be a solid preparation such as tablet etc., or may be an oral mucosa membrane patch (film).
The sublingual, buccal or intraoral quick disintegrating agent is preferably a preparation containing the androgen receptor antagonists of the present invention or the combination drug and an excipient. It may contain also auxiliary agents such as a lubricant, isotonizing agent, hydrophilic carrier, water-dispersible polymer, stabilizer etc. Further, for easy absorption and increased bioavailability, β-cyclodextrin or β-cyclodextrin derivatives (e.g., hydroxypropyl-β-cyclodextrin etc.), etc. may also be contained.
The above-mentioned excipient includes lactose, sucrose, D-mannitol, starch, crystalline cellulose, light anhydrous silicic acid, etc. The lubricant includes magnesium stearate, calcium stearate, talc, colloidal silica, etc., and particularly preferably, magnesium stearate and colloidal silica. The isotonizing agent includes sodium chloride, glucose, fructose, mannitol, sorbitol, lactose, saccharose, glycerin, urea, etc., and particularly preferably, mannitol. The hydrophilic carrier includes swellable hydrophilic carriers such as crystalline cellulose, ethyl cellulose, crosslinkable polyvinylpyrrolidone, light anhydrous silicic acid, silicic acid, dicalcium phosphate, calcium carbonate etc., and particularly preferably, crystalline cellulose (e.g., microcrystalline cellulose, etc.). The water-dispersible polymer includes gums (e.g., gum tragacanth, acacia gum, guar gum), alginates (e.g., sodium alginate), cellulose derivatives (e.g., methyl cellulose, carboxymethyl cellulose, hydroxymethyl cellulose, hydroxypropyl cellulose, hydroxypropylmethyl cellulose), gelatin, aqueous starch, polyacrylic acids (e.g., Carbomer), polymethacrylic acid, polyvinyl alcohol, polyethylene glycol, polyvinylpyrrolidone, polycarbophil, ascorbate, palmitates, etc., and preferably, hydroxypropylmethyl cellulose, polyacrylic acid, alginate, gelatin, carboxymethyl cellulose, polyvinylpyrrolidone, polyethylene glycol, etc., particularly preferably, hydroxypropylmethyl cellulose. The stabilizer includes cysteine, thiosorbitol, tartaric acid, citric acid, sodium carbonate, ascorbic acid, glycine, sodium sulfite, etc., and particularly preferably, citric acid and ascorbic acid.

The sublingual, buccal or intraoral quick disintegrating agent can be manufactured by mixing the androgen receptor antagonists of the present invention or the combination drug and an excipient by a per se known method. Further, if desired, auxiliary agents such as a lubricant, isotonizing agent, hydrophilic carrier, water-dispersible polymer, stabilizer, colorant, sweetening agent, antiseptic etc. may be mixed. The sublingual, buccal or intraoral quick disintegrating agent is obtained by mixing the above-mentioned components simultaneously or at a time interval, then subjecting the mixture to tablet-making molding under pressure. For obtaining suitable hardness, it may also be permissible that the materials are moistened by using a solvent such as water, alcohol etc. if desired before and after the tablet making process, and after the molding, the materials are dried, to obtain a product.

In the case of molding into a mucosa membrane patch (film), the androgen receptor antagonists of the present invention or the combination drug and the above-mentioned water-dispersible polymer (preferably, hydroxypropyl cellulose, hydroxypropylmethyl cellulose), excipient etc. are dissolved in a solvent such as water etc., and the resulted solution is cast to give a film. Further, additives such as a plasticizer, a stabilizer, an antioxidant, an antiseptic, a colorant, a buffer, a sweetening agent etc. may also be added. For imparting suitable elasticity to the film, glycols such as polyethylene glycol, propylene glycol, etc. may be contained, or for enhancing adhesion of the film to an intraoral mucosa membrane lining, a bio-adhesive polymer (e.g., polycarbophil, carbopol) may also be contained. In the casting, a solution is poured on the non-adhesive surface, spread to uniform thickness (preferably, about 10 micron to about 1,000 micron) by an application tool such as a doctor blade etc., then, the solution is dried to form a film. It may be advantageous that thus formed film is dried at room temperature or under heat, and cut into given area.

The intraoral quick disintegrating preparation is preferably solid quick diffuse preparation composed of a network body comprising the androgen receptor antagonists of the present invention or the combination drug, and a watersoluble or water-diffusible carrier which is inert to the androgen receptor antagonists of the present invention or the combination drug. This network body is obtained by sublimating a solvent from the composition constituted of a solution prepared by dissolving the androgen receptor antagonists of the present invention or the combination drug in a suitable solvent.
The composition of an intraoral quick disintegrating agent preferably contains a matrix forming agent and a secondary component in addition to the androgen receptor antagonists of the present invention or the combination drug.
The matrix forming agent includes animal proteins or vegetable proteins such as gelatins, dextrins, soybean, wheat and psyllium seed protein etc.; rubber substances such as arabic gum, guar gum, agar, xanthane, etc.; polysaccharides; alginic acids; carboxymethyl celluloses; carrageenans; dextrans; pectins; synthetic polymers such as polyvinylpyrrolidone, etc.; substances derived from a gelatin-arabic gum complex, etc. Further, it includes saccharides such as mannitol, dextrose, lactose, galactose, trehalose, etc.; cyclic saccharides such as cyclodextrin etc.; inorganic salts such as sodium phosphate, sodium chloride and aluminum silicate, etc.; amino acids having 2 to 12 carbon atoms such as glycine, L-alanine, L-aspartic acid, L-glutamic acid, L-hydroxyproline, L-isoleucine, L-leucine, L-phenylalanine, etc.
One or more of the matrix forming agent(s) can be introduced in a solution or suspension before solidification. Such matrix forming agent may be present in addition to a surfactant, or may be present with the surfactant excluded. The matrix forming agents may help to keep the androgen receptor antagonists of the present invention or the combination drug diffused in the solution or suspension, in addition to formation of the matrix.

The composition may contain secondary components such as a preservative, an antioxidant, a surfactant, a thickening agent, a colorant, a pH controlling agent, a flavoring agent, a sweetening agent, a food taste masking agent, etc. The suitable colorant includes red, black and yellow iron oxides, and FD & C dyes such as FD & C Blue 2, FD & C Red 40, etc. manufactured by Elis and Eberald. Examples of the suitable flavoring agent include mint, raspberry, licorice, orange, lemon, grape fruit, caramel, vanilla, cherry, grape flavor and combinations thereof. Examples of the suitable pH controlling agent include citric acid, tartaric acid, phosphoric acid, hydrochloric acid and maleic acid. Examples of the suitable sweetening agent include aspartame, acesulfame K and thaumatine, etc. Examples of the suitable food taste masking agent include sodium bicarbonate, ion exchange resin, cyclodextrin-inclusion compounds, adsorbent substances and microcapsulated apomorphine.
The preparation contains the androgen receptor antagonists of the present invention or the combination drug in an amount usually from about 0.1% by weight to about 50% by weight, preferably from about 0.1% by weight to about 30% by weight, and is preferably a preparation (such as the above-mentioned sublingual agent, buccal etc.) which can dissolve 90% or more the androgen receptor antagonists of the present invention or the combination drug (into water) within the time range of about 1 minute to about 60 minutes, preferably of about 1 minute to 15 minutes, more preferably of about 2 minutes to about 5 minutes, and intraoral quick disintegrating preparations which are disintegrated within the range of 1 second to 60 seconds, preferably of 1 to 30 seconds, further preferably of 1 to 10 seconds after being placed in the oral cavity.

The content of the above-mentioned excipient in the whole preparation is from about 10% by weight to about 99% by weight, preferably from about 30% by weight to about 90% by weight. The content of β-cyclodextrin or β-cyclodextrin derivative in the whole preparation is from 0 to about 30% by weight. The content of the lubricant in the whole preparation is from about 0.01% by weight to about 10% by weight, preferably from about 1% by weight to about 5% by weight. The content of the isotonizing agent in the whole preparation is from about 0.1% by weight to about 90% by weight, preferably, from about 10% by weight to about 70% by weight. The content of the hydrophilic carrier in the whole preparation is from about 0.1% by weight to about 50% by weight, preferably, from about 10% by weight to about 30% by weight. The content of the water-dispersible polymer in the whole preparation is from about 0.1 to about 30% by weight, preferably, from about 10% by weight to about 25% by weight. The content of the stabilizer in the whole preparation is from about 0.1% by weight to about 10% by weight, preferably, from about 1% by weight to about 5% by weight. The above-mentioned preparation may further contain additives such as a colorant, a sweetening agent, an antiseptic, etc., if necessary.

The dose of a combination preparation of the present invention differs depending on the kind of the compound (I), compound (I') and compound (IA), age, body weight, condition, drug form, administration method, administration period etc., and for example, for a prostate cancer patient (adult, body weight: about 60 kg), the combination preparation is administered intravenously, at a dose of about 0.01 to about 1,000 mg/kg/day, preferably about 0.01 to about 100 mg/kg/day, more preferably about 0.1 to about 100 mg/kg/day, particularly about 0.1 to about 50 mg/kg/day, especially about 1.5 to about 30 mg/kg/day, in terms of the androgen receptor antagonists of the present invention or the combination drug, respectively, once or several times a day in divided portions. Of course, since the dose as described above varies depending on various conditions, it may be sometimes sufficient to administer smaller amounts than the above-mentioned dosage, and further, it may be sometimes necessary to administer greater amounts than that.
The amount of the combination drug can be set at any value unless side effects are problematical. The daily dosage in terms of the combination drug differs depending on the severity of symptoms, age, sex, body weight, sensitivity difference of the subject, administration time and interval, property, prescription, and kind of the pharmaceutical preparation, kind of effective ingredient, etc., and not particularly limited; for example, in the case of oral administration, the dose of the drug is usually from about 0.001 mg to 2,000 mg, preferably from about 0.01 mg to 500 mg, further preferably from about 0.1 mg to 100 mg, per 1 kg body weight of a mammal, which is usually administered once to four times a day in divided portions.

In administration of the combination preparation of the present invention, the androgen receptor antagonists of the present invention may be administered after administration of the combination drug or the combination drug may be administered after administration of the androgen receptor antagonists of the present invention, though they may be administered simultaneously. When administered at a time interval, the interval differs depending on the effective ingredient to be administered, drug form and administration method. For example, when the combination drug is administered first, the androgen receptor antagonists of the present invention is administered within time range of from 1 minute to 3 days, preferably from 10 minutes to 1 day, more preferably from 15 minutes to 1 hour after administration of the combined drug. When the androgen receptor antagonists of the present invention is administered first, the combined drug is administered within time range of from 1 minute to 1 day, preferably from 10 minutes to 6 hours, more preferably from 15 minutes to 1 hour after administration of the androgen receptor antagonists of the present invention.
In a preferable administration method, for example, the combination drug formulated into an oral administration preparation is administered orally at a daily dose of about 0.001 mg/kg to 200 mg/kg, and 15 minutes later, the androgen receptor antagonists of the present invention formulated into an oral administration preparation is administered orally at a daily dose of about 0.005 mg/kg to 100 mg/kg.

### Examples

The present invention is explained in more detail in the following by referring to Reference Examples, Examples, Preparation Examples and Experimental Examples, which are not to be construed as limitative.
The "room temperature" in the following Reference Examples means a temperature of generally from about 10°C to 35°C. Unless otherwise specified, "%" means weight % and the yield is in mol/mol%.
NMR spectra are shown by proton NMR with tetramethylsilane as the internal standard, using 200 MHz or 300 MHz spectrometer; δ values are expressed in ppm.
In the present specification, the melting point means one measured, for example, using a trace melting point measurement device (Yanaco, Buchi, B-545 and the like) or DSC (Differential Scanning Calorimetory) apparatus (SEIKO, EXSTAR6000 and the like) and the like.
In general, the melting points may vary depending on the measurement apparatuses, the measurement conditions and the like. The crystal in the present specification may show different values from the melting point described in the present specification as long as they are within general error range.
Other abbreviations used in the description mean the following.
s: singlet
brs: broad singlet
d: doublet
t: triplet
q: quartet
ddd: double double doublet
dt: double triplet
td: triple doublet
m: multiplet
br: broad
CDCl₃: deuterated chloroform
DMSO-d₆: deuterated dimethyl sulfoxide
CD₃OD: deuterated methanol
¹H-NMR: proton nuclear magnetic resonance
Me: methyl
Et: ethyl
i-Pr: isopropyl
boc: tert-butyloxycarbonyl
THF: tetrahydrofuran
TFA: trifluoroacetic acid
AIBN: 2,2'-azobis(isobutyronitrile)
DMF: N,N-dimethylformamide
NMP: N-methylpyrrolidone
NBS: N-bromosuccinimide
WSC: 1-ethyl-3-(3-dimethylaminopropyl)carbodiimide hydrochloride
DMT-MM: 4-(4,6-dimethoxy-1,3,5-triazin-2-yl)-4-methylmorpholin-4-ium chloride
HOBt: 1-hydroxybenzotriazole

### Reference Example 1

### tert-butyl 3,5-dimethyl-4-(4,4,5,5-tetramethyl-1,3,2-dioxaborolan-2-yl)-1H-pyrazole-1-carboxylate

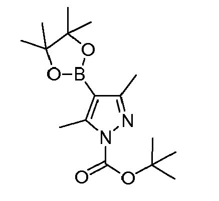

3,5-Dimethyl-4-(4,4,5,5-tetramethyl-1,3,2-dioxaborolan-2-yl)-1H-pyrazole (3.05 g) was dissolved in acetonitrile (80 mL), and N,N-dimethylaminopyridine (1.09 g) and di-tert-butyl bicarbonate (4.1 mL) were added. The reaction mixture was stirred at room temperature for 3 hr. Saturated brine was added, and the mixture was extracted with ethyl acetate. The organic layer was washed with saturated brine, dried over anhydrous magnesium sulfate and concentrated. The obtained residue was washed with hexane-diisopropyl ether to give the title compound (3.40 g) as colorless crystals.
¹H-NMR (DMSO-d₆) δ: 1.27 (12H, s), 1.55 (9H, s), 2.21 (3H, s), 2.59 (3H, s).

### Reference Example 2

### tert-butyl 4-iodo-3,5-dimethyl-1H-pyrazole-1-carboxylate

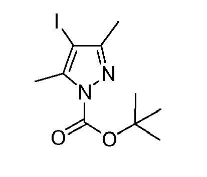

The title compound was obtained in the same manner as in Reference Example 1 and using 4-iodo-3,5-dimethyl-1H-pyrazole as a starting material.
¹H-NMR (CDCl₃) δ: 1.64 (9H, s), 2.28 (3H, s), 2.55 (3H, s).

### Reference Example 3

### 3,5-dimethyl-1-(tetrahydro-2H-pyran-2-yl)-4-(4,4,5,5-tetramethyl-1,3,2-dioxaborolan-2-yl)-1H-pyrazole

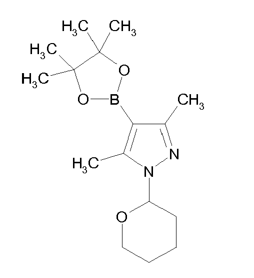

3,5-Dimethyl-4-(4,4,5,5-tetramethyl-1,3,2-dioxaborolan-2-yl)-1H-pyrazole (4.95 g) was dissolved in THF (45 mL), and 3,4-dihydro-2H-pyran (3.05 mL) and TFA (1.99 mL) were added. The reaction mixture was stirred with heating under reflux for 8 hr, poured into saturated aqueous sodium hydrogen carbonate, and the mixture was extracted with ethyl acetate. The organic layer was washed with saturated brine, dried over anhydrous magnesium sulfate and concentrated. The obtained residue was washed with diisopropyl ether-hexane (1:2) to give the title compound (5.34 g) as colorless crystals.
¹H-NMR (CDCl₃) δ: 1.28 (12H, s), 1.45-1.59 (4H, m), 1.83-1.92 (1H, m), 2.00-2.14 (1H, m), 2.35 (3H, s), 2.45 (3H, s), 3.52-3.75 (1H, m), 3.95-4.16 (1H, m), 5.18 (1H, dd).

### Reference Example 4

### 2-chloro-4-(3,5-dimethyl-1H-pyrazol-4-yl)benzonitrile

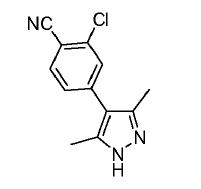

(Synthesis method 1) A mixture of tert-butyl 3,5-dimethyl-4-(4,4,5,5-tetramethyl-1,3,2-dioxaborolan-2-yl)-1H-pyrazole-1-carboxylate (1.70 g) synthesized in Reference Example 1, 4-bromo-2-chlorobenzonitrile (1.14 g), tetrakis(triphenylphosphine)palladium(0) (0.58 g), sodium carbonate (1.4 g), 1,2-dimethoxyethane (40 mL) and water (7 mL) was stirred at 90°C for 4 hr. Water and ethyl acetate were added to the reaction mixture, and the organic layer was separated. The organic layer was washed with saturated brine, dried over anhydrous magnesium sulfate and concentrated. The residue was purified by column chromatography (hexane-ethyl acetate), and each fraction was suspended in diisopropyl ether and collected by filtration to give the title compound (193 mg) and tert-butyl 4-(3-chloro-4-cyanophenyl)-3,5-dimethyl-1H-pyrazole-1-carboxylate (620 mg) as a solid. A mixture of tert-butyl 4-(3-chloro-4-cyanophenyl)-3,5-dimethyl-1H-pyrazole-1-carboxylate (600 mg) and TFA was stirred at room temperature for 3 hr, neutralized with saturated aqueous sodium hydroxide solution and extracted with ethyl acetate. The organic layer was washed with saturated brine, dried over anhydrous magnesium sulfate and concentrated. The obtained residue was washed with diisopropyl ether to give the title compound (328 mg) as colorless crystals.
(Synthesis method 2) 3,5-Dimethyl-1-(tetrahydro-2H-pyran-2-yl)-4-(4,4,5,5-tetramethyl-1,3,2-dioxaborolan-2-yl)-1H-pyrazole (28.3 g) synthesized in Reference Example 3, 4-bromo-2-chlorobenzonitrile (22.9 g) and triethylamine (25.8 mL) were dissolved in THF-water (6:1)(217 mL), and 1,1'-bis(diphenylphosphino)ferrocene palladium (II) dichloride dichloromethane complex (1:1)(2.26 g) was added under a nitrogen atmosphere. The reaction mixture was stirred with heating under reflux for 24 hr, and concentrated. The obtained residue was poured into saturated aqueous ammonium chloride solution, and the mixture was extracted with ethyl acetate. The organic layer was washed with saturated brine, dried over anhydrous magnesium sulfate and concentrated. The obtained residue was purified by column chromatography to give 2-chloro-4-[3,5-dimethyl-1-(tetrahydro-2H-pyran-2-yl)-1H-pyrazol-4-yl]benzonitrile (20.0 g) as colorless crystals. A mixture of the obtained 2-chloro-4-[3,5-dimethyl-1-(tetrahydro-2H-pyran-2-yl)-1H-pyrazol-4-yl]benzonitrile (18.7 g) and 10% hydrochloric acid methanol solution (180 mL) was stirred at room temperature for 4 hr. The reaction mixture was poured into ice-cooled 1 mol/L aqueous sodium hydroxide solution to quench the reaction, methanol was evaporated and the obtained aqueous layer was extracted with ethyl acetate. The organic layer was washed with saturated brine, dried over anhydrous magnesium sulfate and concentrated. The obtained residue was washed with diisopropyl ether-hexane to give the title compound (13.7 g) as colorless crystals.
¹H-NMR (DMSO-d₆) δ: 2.33 (6H, s), 7.24-7.30 (1H, m), 7.42 (1H, d), 7.70 (1H, d).

### Reference Example 5

### 4-(3,5-dimethyl-1H-pyrazol-4-yl)benzonitrile

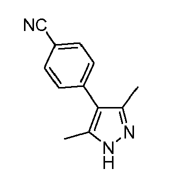

(Synthesis method 1) The title compound was obtained in the same manner as in Reference Example 4 and using tert-butyl 3,5-dimethyl-4-(4,4,5,5-tetramethyl-1,3,2-dioxaborolan-2-yl)-1H-pyrazole-1-carboxylate synthesized in Reference Example 3 and 4-bromobenzonitrile as starting materials.
(Synthesis method 2) tert-butyl 3,5-dimethyl-4-(4,4,5,5-tetramethyl-1,3,2-dioxaborolan-2-yl)-1H-pyrazole-1-carboxylate (14.4 g) synthesized in Reference Example 1, 4-bromobenzonitrile (8.13 g) and sodium carbonate (11.8 g) were dissolved in dimethoxyethane-water (6:1), and tetrakis(triphenylphosphine)palladium(0)(5.16 g) was added under a nitrogen atmosphere. The reaction mixture was stirred at 90°C for 4 hr, poured into water, and the mixture was extracted with ethyl acetate. The organic layer was washed with saturated brine, dried over anhydrous magnesium sulfate and concentrated. The obtained residue was applied to column chromatography to remove the impurities, whereby a mixture of tert-butyl 4-(4-cyanophenyl)-3,5-dimethyl-1H-pyrazole-1-carboxylate and the title compound was obtained. TFA (50 mL) was added to the obtained mixture, and the mixture was stirred at room temperature for 15 min. The reaction mixture was concentrated, and azeotroped with toluene. The obtained residue was purified by column chromatography (hexane-ethyl acetate), and recrystallized from diethyl ether-hexane to give the title compound (2.00 g) as colorless crystals.
¹H-NMR (CDCl₃) δ: 2.33 (6H, s), 7.39 (2H, d), 7.71 (2H, d).

### Reference Example 6

### 4-(3,5-dimethyl-1H-pyrazol-4-yl)-2-fluorobenzonitrile

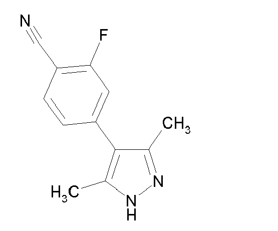

The title compound was obtained in the same manner as in Reference Example 4, Synthesis method 2 and using 3,5-dimethyl-1-(tetrahydro-2H-pyran-2-yl)-4-(4,4,5,5-tetramethyl-1,3,2-dioxaborolan-2-yl)-1H-pyrazole synthesized in Reference Example 3 and 4-bromo-2-fluorobenzonitrile as starting materials.
¹H-NMR (CDCl₃) δ: 2.33 (6H, s), 7.13 (1H, dd), 7.17 (1H, dd), 7.65 (1H, dd).

### Reference Example 7

### 4-(3,5-dimethyl-1H-pyrazol-4-yl)-2-trifluoromethylbenzonitrile

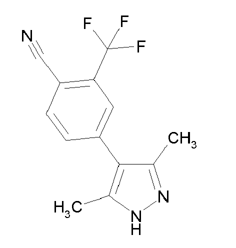

The title compound was obtained in the same manner as in Reference Example 4, Synthesis method 2 and using 3,5-dimethyl-1-(tetrahydro-2H-pyran-2-yl)-4-(4,4,5,5-tetramethyl-1,3,2-dioxaborolan-2-yl)-1H-pyrazole synthesized in Reference Example 3 and 4-bromo-2-trifluoromethylbenzonitrile as starting materials.
¹H-NMR (CDCl₃) δ: 2.34 (6H, s), 7.57 (1H, dd), 7.69 (1H, dd), 7.88 (1H, dd).

### Reference Example 8

### 2-chloro-5-(3,5-dimethyl-1H-pyrazol-4-yl)benzonitrile

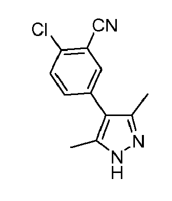

The title compound was obtained in the same manner as in Reference Example 4, Synthesis method 1 and using tert-butyl 4-iodo-3,5-dimethyl-1H-pyrazole-1-carboxylate synthesized in Reference Example 2 and (4-chloro-3-cyanophenyl)boronic acid as starting materials.
¹H-NMR (CDCl₃) δ: 2.30 (6H, s), 7.40 - 7.46 (1H, m), 7.52 - 7.57 (2H, m).

### Reference Example 9

### 1-(4-fluorobenzyl)-3,5-dimethyl-4-(4,4,5,5-tetramethyl-1,3,2-dioxaborolan-2-yl)-1H-pyrazole

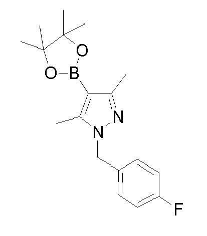

To a solution of 3,5-dimethyl-4-(4,4,5,5-tetramethyl-1,3,2-dioxaborolan-2-yl)-1H-pyrazole (1.19 g) in DMF (40 mL) was added sodium hydride (60% oil dispersion, 0.26 g) and, after stirring the mixture at room temperature for 20 min, 4-fluorobenzyl bromide (0.80 mL) was added. The reaction mixture was stirred at room temperature for 3 hr, the reaction mixture was poured into water, and the mixture was extracted with ethyl acetate. The ethyl acetate layer was washed with water, dried over anhydrous magnesium sulfate and concentrated. The residue was washed with diethyl ether to give the title compound (1.14 g) as a solid.
¹H-NMR (CDCl₃) δ: 1.29 (12H, s), 2.32 (3H, s), 2.36 (3H, s), 5.17 (2H, s), 6.94 - 7.09 (4H, m).

### Reference Example 10

### 5-bromo-1-methyl-2-oxo-1,2-dihydropyridine-3-carbonitrile

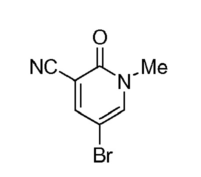

To a solution of 5-bromo-2-oxo-1,2-dihydropyridine-3-carbonitrile (507 mg) in DMF (5 mL) were added cesium carbonate (1.66 g) and methyl iodide (0.19 mL), and the mixture was stirred at 0°C for 2 hr. The reaction mixture was poured into water, and the mixture was extracted with ethyl acetate. The ethyl acetate layer was washed with water, dried over anhydrous magnesium sulfate and concentrated. The residue was purified by column chromatography (hexane-ethyl acetate) to give the title compound (317 mg) as a solid.
¹H-NMR (CDCl₃) δ: 3.61 (3H, s), 7.69 (1H, d), 7.85 (1H, d).

### Reference Example 11

### 4-bromo-2-chloro-5-fluorobenzamide

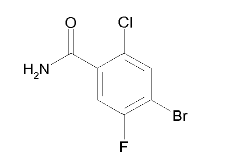

4-Bromo-2-chloro-5-fluorobenzoic acid (1.69 g) and HOBt ammonium salt (1.52 g) were dissolved in DMF (30 mL), and WSC (1.92 g) was added. The reaction mixture was stirred at room temperature for 40 min, poured into 1 mol/L hydrochloric acid, and the mixture was extracted with ethyl acetate. The organic layer was washed with saturated aqueous sodium hydrogen carbonate and then saturated brine, dried over anhydrous magnesium sulfate and concentrated. The obtained residue was washed with diisopropyl ether to give the title compound (1.48 g) as colorless crystals.
¹H-NMR (CDCl₃) δ: 5.89 (1H, brs), 6.46 (1H, brs), 7.65 (1H, d), 7.67 (1H, d).

### Reference Example 12

### 4-bromo-2-chloro-5-fluorobenzonitrile

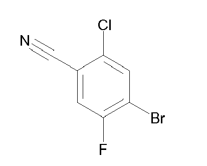

4-Bromo-2-chloro-5-fluorobenzamide (1.48 g) and pyridine (1.48 mL) were dissolved in DMF (30 mL), cooled in an ice bath, oxalyl chloride (1.02 mL) was added dropwise. The reaction mixture was stirred at the same temperature for 15 min, poured into water, and extracted with diethyl ether. The organic layer was washed with saturated aqueous sodium hydrogen carbonate and then saturated brine, dried over anhydrous magnesium sulfate and concentrated to give the title compound (1.16 g) as colorless crystals.
¹H-NMR (CDCl₃) δ: 7.43 (1H, d), 7.77 (1H, d).

### Reference Example 13

### 2-chloro-4-(3,5-dimethyl-1H-pyrazol-4-yl)-5-fluorobenzonitrile

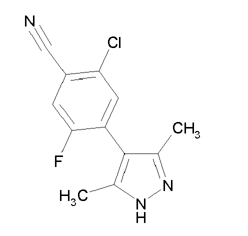

The title compound was obtained in the same manner as in Reference Example 4, Synthesis method 2 and using 3,5-dimethyl-1-(tetrahydro-2H-pyran-2-yl)-4-(4,4,5,5-tetramethyl-1,3,2-dioxaborolan-2-yl)-1H-pyrazole synthesized in Reference Example 3 and 4-bromo-2-chloro-5-fluorobenzonitrile as starting materials.
¹H-NMR (CDCl₃) δ: 2.27 (6H, d), 7.40 (1H, d), 7.49 (1H, d).

### Reference Example 14

### 2-chloro-4-(1H-pyrazol-4-yl)benzonitrile

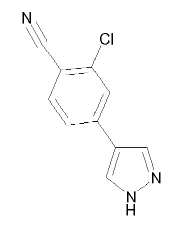

tert-Butyl 4-(4,4,5,5-tetramethyl-1,3,2-dioxaborolan-2-yl)-1H-pyrazole-1-carboxylate (1.53 g), 4-bromo-2-chlorobenzonitrile (1.43 g) and triethylamine (1.45 mL) were dissolved in THF-water (6:1, 11.7 mL), and 1,1'-bis(diphenylphosphino)ferrocene palladium (II) dichloride dichloromethane complex (1:1)(127.4 mg) was added under a nitrogen atmosphere. The reaction mixture was stirred with heating under reflux for 3 hr, and the mixture was cooled to room temperature and TFA (20 mL) was added. The reaction mixture was stirred at room temperature for 1.5 hr, and concentrated. 1 mol/L Aqueous sodium hydroxide solution was added to the obtained residue, and the mixture was extracted with ethyl acetate. The organic layer was washed with saturated brine, dried over anhydrous magnesium sulfate and concentrated. The obtained residue was purified by column chromatography (hexane-ethyl acetate) to give the title compound (808.6 mg) as pale-orange crystals.
¹H-NMR (CDCl₃) δ: 7.80 (1H, dd), 7.93 (1H, d), 8.05 (1H, d), 8.22 (1H, brs), 8.44 (1H, brs), 13.27 (1H, brs).

### Reference Example 15

### 2-(bromomethyl)-1,3-thiazole

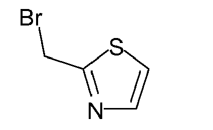

2-(Hydroxymethyl)-1,3-thiazole (337.6 mg) was dissolved in DMF (15 mL), and triphenylphosphine (923.2 mg) and NBS (626.5 mg) were added. The reaction mixture was stirred at room temperature for 1 hr, poured into saturated sodium sulfite aqueous solution, and the mixture was extracted with ethyl acetate. The organic layer was washed with saturated aqueous sodium hydrogen carbonate and then saturated brine, dried over anhydrous magnesium sulfate and concentrated. Hexane-ethyl acetate (3:1) was added to the obtained residue, and the precipitated triphenylphosphine oxide was filtered off. The filtrate was concentrated, and the obtained residue was purified by column chromatography (hexane-ethyl acetate) to give the title compound (249.3 mg) as a brown oil.
¹H-NMR (CDCl₃) δ: 4.77 (2H, s), 7.39 (1H, d), 7.76 (1H, d).

### Reference Example 16

### 2-bromo-5-(chloromethyl)pyridine hydrochloride

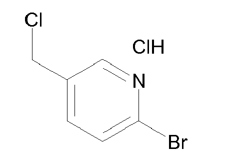

2-Bromo-5-(hydroxymethyl)pyridine (2.64 g) was dissolved in THF (70 mL) and, after ice-cooling, thionyl chloride (5.11 mL) was added dropwise. The reaction mixture was stirred at the same temperature for 30 min, and concentrated. The obtained residue was washed with diethyl ether to give the title compound (2.65 g) as yellow crystals.
¹H-NMR (CDCl₃) δ: 4.55 (2H, s), 7.52 (1H, d), 7.63 (1H, dd), 8.40 (1H, d).

### Reference Example 17

### 3-bromo-5-(chloromethyl)pyridine hydrochloride

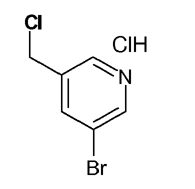

The title compound was obtained in the same manner as in Reference Example 16 and using 3-bromo-5-(hydroxymethyl)pyridine as a starting material.
¹H-NMR (CDCl₃) δ: 4.77 (2H, s), 8.51 (1H, s), 8.82 (1H, d), 8.93 (1H, d).

### Reference Example 18

### Ethyl 4-formyl-1-trityl-1H-pyrazole-3-carboxylate

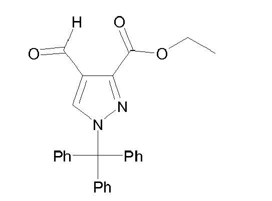

A mixture of ethyl 4-formyl-1H-pyrazole-3-carboxylate (5.00 g), triphenylchloromethane (8.71 g), triethylamine (6.2 mL), 4-dimethylaminopyridine (0.38 g) and dichloromethane (50 mL) was stirred at room temperature for 3 hr. The reaction mixture was poured into saturated brine, and extracted with ethyl acetate. The ethyl acetate layer was washed with saturated brine, dried over anhydrous magnesium sulfate and concentrated to give the title compound (7.01 g).
¹H-NMR (CDCl₃) δ: 1.39 (3H, t), 4.42 (2H, q), 7. 08 - 7.15 (6H, m), 7.31 (1H, s), 7.32 - 7.37 (8H, m), 7.96 (1H, s), 10.39 (1H, s).

### Reference Example 19

### Ethyl 4-(hydroxymethyl)-1-trityl-1H-pyrazole-3-carboxylate

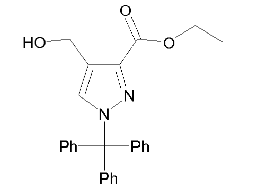

A mixture of ethyl 4-formyl-1-trityl-1H-pyrazole-3-carboxylate (4.0 g) synthesized in Reference Example 18, sodium borohydride (0.45 g), THF (20 mL) and methanol (20 mL) was stirred at 0°C for 1 hr. The reaction mixture was poured into aqueous citric acid solution, and extracted with ethyl acetate. The ethyl acetate layer was washed with saturated brine, dried over anhydrous magnesium sulfate and concentrated to give the title compound as a colorless solid (4.26 g).
¹H-NMR (CDCl₃) δ: 1.37 (3H, t), 3.75 (1H, t), 4.38 (2H, q), 4.62 (2H, d), 7.10 - 7.16 (6H, m), 7.24 (1H, s), 7.32 (9H, td).

### Reference Example 20

### Ethyl 4-(iodomethyl)-1-trityl-1H-pyrazole-3-carboxylate

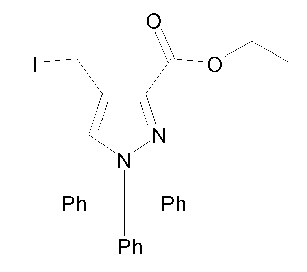

A mixture of ethyl 4-(hydroxymethyl)-1-trityl-1H-pyrazole-3-carboxylate (2.30 g) synthesized in Reference Example 19, triphenylphosphine (1.61 g), iodine (1.56 g), imidazole (0.42 g) and dichloromethane (20 mL) was stirred at room temperature for 4 hr. The reaction mixture was partitioned between saturated aqueous sodium hydrogen carbonate solution and ethyl acetate and the organic layer was washed with saturated brine, dried (anhydrous magnesium sulfate), and concentrated. The residue was suspended in diethyl ether and filtrated to give the title compound (1.12 g) as a colorless solid.
¹H-NMR (CDCl₃): 1.40 (3H, t), 4.39 (2H, q), 4.59 (2H, s), 7.08 - 7.15 (6H, m), 7.32 (9H, td), 7.37 (1H, s).

### Reference Example 21

### 4-(3,5-dimethyl-1H-pyrazol-4-yl)-2-methoxybenzonitrile

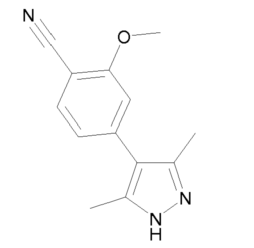

The title compound (1.05 g) was obtained as colorless crystals in the same manner as in Reference Example 4, Synthesis method 2 and using 3,5-dimethyl-1-(tetrahydro-2H-pyran-2-yl)-4-(4,4,5,5-tetramethyl-1,3,2-dioxaborolan-2-yl)-1H-pyrazole (6.19 g) synthesized in Reference Example 3 and 4-bromo-2-methoxybenzonitrile (4.72 g) as starting materials. ¹H-NMR (CDCl₃) δ: 2.34 (6H, s), 3.96 (3H, s), 6.87 (1H, d), 6.93 (1H, dd), 7.60 (1H, d).

### Reference Example 22

### 2-chloro-3-fluoro-4-iodobenzonitrile

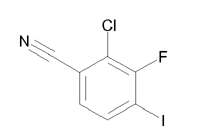

Concentrated sulfuric acid (60 mL) was poured into ice (60 g) and a solution (5.62 g/60 mL) of 4-amino-2-chloro-3-fluorobenzonitrile in acetonitrile was slowly added dropwise to the obtained mixture under ice-cooling. Then, aqueous sodium nitrite solution (4.58 g/40 mL) was slowly added dropwise under ice-cooling. The obtained solution was poured into aqueous potassium iodide solution (21.4 g/60 mL), which was prepared separately, under ice-cooling. After stirring at room temperature for 40 min, the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate, basic silica gel) to give the title compound (5.73 g) as colorless crystals.
¹H-NMR (CDCl₃) δ: 7.22 (1H, dd), 7.80 (1H, dd).

### Reference Example 23

### 2-chloro-4-(3,5-dimethyl-1H-pyrazol-4-yl)-3-fluorobenzonitrile

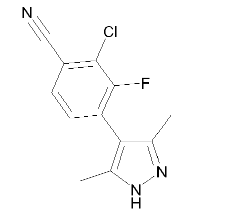

The title compound (700.6 mg) was obtained as colorless crystals in the same manner as in Reference Example 4, Synthesis method 2 and using 3,5-dimethyl-1-(tetrahydro-2H-pyran-2-yl)-4-(4,4,5,5-tetramethyl-1,3,2-dioxaborolan-2-yl)-1H-pyrazole (3.29 g) synthesized in Reference Example 3 and 2-chloro-3-fluoro-4-iodobenzonitrile (1.50 g) synthesized in Reference Example 22 as starting materials.
¹H-NMR (CDCl₃) δ: 2.25 (6H, d), 7.21-7.30 (1H, m), 7.52 (1H, dd).

### Reference Example 24

### Ethyl 4-bromo-1-[4-(tert-butoxycarbonyl)benzyl]-5-methyl-1H-pyrazole-3-carboxylate

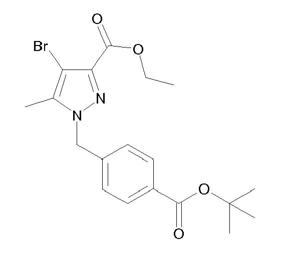

Ethyl 4-bromo-5-methyl-1H-pyrazole-3-carboxylate (25.5 g) was dissolved in DMF (500 mL), ice-cooled, and potassium tert-butoxide (14.7 g) was added. The reaction mixture was stirred for 10 min, and a solution (14.7 g/50 mL) of tert-butyl 4-(bromomethyl)benzoate in DMF was added dropwise slowly. The reaction mixture was stirred at 0°C for 1.5 hr, poured into saturated aqueous ammonium chloride solution, and the mixture was extracted with ethyl acetate. The organic layer was washed with saturated brine, dried over anhydrous magnesium sulfate and concentrated. The obtained residue was purified by column chromatography (hexane-ethyl acetate) to give two kinds of positional isomers. A high polar compound was washed with diisopropyl ether-hexane to give the title compound (11.6 g) as colorless crystals.
¹H-NMR (CDCl₃) δ: 1.43 (3H, t), 1.58 (9H, s), 2.16 (3H, s), 4.45 (2H, q), 5.45 (2H, s), 7.13 (2H, d), 7.95 (2H, d).

### Reference Example 25

### 2-chloro-4-(4,4,5,5-tetramethyl-1,3,2-dioxaborolan-2-yl)benzonitrile

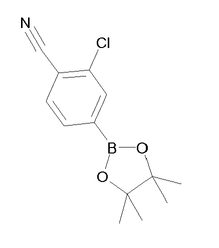

4-Bromo-2-chlorobenzonitrile (19.43 g), 4,4,4',4',5,5,5',5'-octamethyl-2,2'-bi-1,3,2-dioxaborolan (25.08 g), potassium acetate (26.5 g), and 1,1'-bis(diphenylphosphino)ferrocene palladium (II) dichloride dichloromethane complex (1:1)(2.20 g) were dissolved in DMSO (300 mL), and the mixture was stirred at 80°C for 14 hr. Saturated brine was added to the reaction mixture, and the mixture was extracted with ethyl acetate. The organic layer was washed with saturated brine, dried over anhydrous magnesium sulfate and concentrated. The obtained residue was suspended in diisopropyl ether-hexane and filtrated to give the title compound (17.74 g) as a brown powder.
¹H-NMR (CDCl₃) δ: 1.35 (12H, s), 7. 63 - 7.68 (1H, m), 7.72 - 7.80 (1H, m), 7.91 (1H, s).

### Example 1

### 4-[1-(4-fluorobenzyl)-3,5-dimethyl-1H-pyrazol-4-yl]benzonitrile

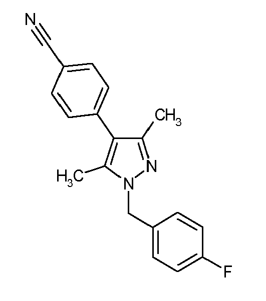

To a solution of 4-(3,5-dimethyl-1H-pyrazol-4-yl)benzonitrile synthesized in Reference Example 5 in DMF (5 mL) was added sodium hydride (60% oil dispersion, 37 mg) and, after stirring at room temperature for 20 min, 4-fluorobenzyl bromide (106 µL) was added. The reaction mixture was stirred at room temperature for 3 hr. The reaction mixture was poured into water, and the mixture was extracted with ethyl acetate, and the ethyl acetate layer was washed with water, dried over anhydrous magnesium sulfate and concentrated. The residue was purified by column chromatography (hexane-ethyl acetate) and crystallized from diethyl ether to give the title compound (203 mg) as colorless crystals.
¹H-NMR (CDCl₃) δ: 2.20 (3H, s), 2.29 (3H, s), 5.25 (2H, s), 6.99 - 7.08 (2H, m), 7.10 - 7.15 (2H, m), 7.35 (2H, d), 7.69 (2H, d).

### Example 2

### 2-chloro-4-[3,5-dimethyl-1-(pyridin-2-ylmethyl)-1H-pyrazol-4-yl]benzonitrile

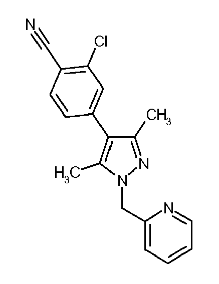

The title compound was obtained in the same manner as in Example 1 and using 2-chloro-4-(3,5-dimethyl-1H-pyrazol-4-yl)benzonitrile synthesized in Reference Example 4 and 2-(bromomethyl)pyridine hydrobromide as starting materials.
¹H-NMR (CDCl₃) δ: 2.26 (3H, s), 2.30 (3H, s), 5.41 (2H, s), 6.99 (1H, d), 7.22 (1H, dd), 7.24 - 7.28 (1H, m), 7.41 (1H, d), 7.65 - 7.73 (2H, m), 8.58 (1H, d).

### Example 3

### 2-chloro-4-[1-(4-fluorobenzyl)-3,5-dimethyl-1H-pyrazol-4-yl]benzonitrile

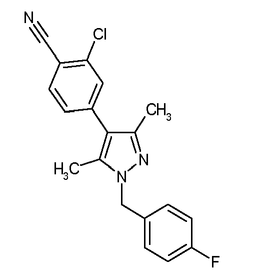

The title compound was obtained in the same manner as in Example 1 and using 2-chloro-4-(3,5-dimethyl-1H-pyrazol-4-yl)benzonitrile synthesized in Reference Example 4 and 4-fluorobenzyl bromide as starting materials.
¹H-NMR (CDCl₃) δ: 2.21 (3H, s), 2.29 (3H, s), 5.25 (2H, s), 7.03 (2H, t), 7.11 - 7.14 (1H, m), 7.15 (1H, d), 7.24 (1H, dd), 7.39 (1H, d), 7.69 (1H, d).

### Example 4

### Methyl 4-{[4-(4-cyanophenyl)-3,5-dimethyl-1H-pyrazol-1-yl]methyl}benzoate

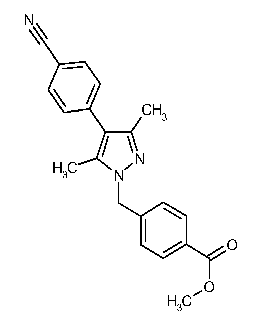

The title compound was obtained in the same manner as in Example 1 and using 4-(3,5-dimethyl-1H-pyrazol-4-yl)benzonitrile synthesized in Reference Example 5 and methyl 4-(bromomethyl)benzoate as starting materials.
¹H-NMR (CDCl₃) δ: 2.18 (3H, s), 2.30 (3H, s), 3.91 (3H, s), 5.34 (2H, s), 7.19 (2H, d), 7.36 (2H, d), 7.69 (2H, d), 8.01 (2H, d).

### Example 5

### Ethyl 4-{[4-(3-chloro-4-cyanophenyl)-3,5-dimethyl-1H-pyrazol-1-yl]methyl}-1H-pyrazole-5-carboxylate

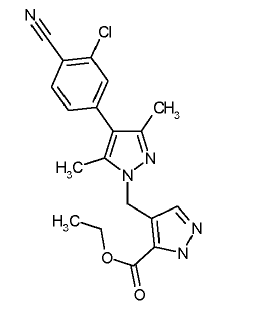

Ethyl 4-{[4-(3-chloro-4-cyanophenyl)-3,5-dimethyl-1H-pyrazol-1-yl]methyl}-1-trityl-1H-pyrazole-5-carboxylate (1.21 g) was obtained in the same manner as in Example 1 and using 2-chloro-4-(3,5-dimethyl-1H-pyrazol-4-yl)benzonitrile (0.60 g) synthesized in Reference Example 4 and ethyl 4-(iodomethyl)-1-trityl-1H-pyrazole-3-carboxylate (1.49 g) synthesized in Reference Example 20 as starting materials. A mixture of the compound (1.20 g) obtained above and TFA (20 mL) was stirred at room temperature for 20 hr. The reaction solution was neutralized with saturated aqueous sodium hydrogen carbonate solution and extracted with ethyl acetate. The ethyl acetate layer was washed with water, dried over anhydrous magnesium sulfate and concentrated. The obtained residue was suspended in diethyl ether and filtrated to give the title compound (427 mg).
¹H-NMR (CDCl₃) δ: 1.42 (3H, t), 2.27 (3H, s), 2.29 (3H, s), 4.45 (2H, q), 5.49 (2H, s), 7.23 - 7.29 (1H, m), 7.37 - 7.43 (2H, m), 7.69 (1H, d), 11.59 (1H, s).

### Example 6

### 2-chloro-5-{[4-(3-chloro-4-cyanophenyl)-3,5-dimethyl-1H-pyrazol-1-yl]methyl}nicotinic acid

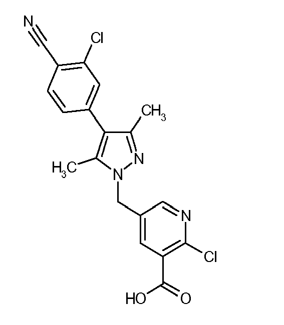

Ethyl 2-chloro-5-{[4-(3-chloro-4-cyanophenyl)-3,5-dimethyl-1H-pyrazol-1-yl]methyl}nicotinate (435 mg) was obtained as an oil in the same manner as in Example 1 and using 2-chloro-4-(3,5-dimethyl-1H-pyrazol-4-yl)benzonitrile (600 mg) synthesized in Reference Example 4 and ethyl 5-(bromomethyl)-2-chloronicotinate (870 mg) as starting materials. The compound (431 mg) obtained above was dissolved in ethanol (2 mL), 1 mol/L aqueous sodium hydroxide solution (2 mL) was added, and the mixture was stirred at 50°C for 2 hr. The reaction solution was neutralized with 1 mol/L hydrochloric acid, and extracted with ethyl acetate. The ethyl acetate layer was washed with water, dried over anhydrous magnesium sulfate and concentrated. The residue was purified by column chromatography (hexane-ethyl acetate), suspended in diethyl ether and filtrated to give the title compound (357 mg).
¹H-NMR (DMSO-d₆) δ: 2.19 (3H, s), 2.32 (3H, s), 5.41 (2H, s), 7.48 (1H, dd), 7.68 (1H, d), 7.99 (1H, d), 8.08 (1H, d), 8.42 (1H, d), 13.86 (1H, s).

### Example 7

### 2-chloro-4-[1-(4-cyanobenzyl)-3,5-dimethyl-1H-pyrazol-4-yl]benzonitrile

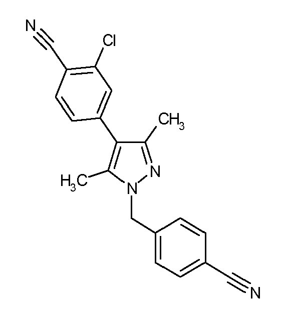

The title compound was obtained in the same manner as in Example 1 and using 2-chloro-4-(3,5-dimethyl-1H-pyrazol-4-yl)benzonitrile synthesized in Reference Example 4 and 4-cyanobenzyl bromide as starting materials.
¹H-NMR (CDCl₃) δ: 2.20 (3H, s), 2.29 (3H, s), 5.33 (s, 2H), 7.19-7.25 (3H, m), 7.39 (1H, d), 7.64 (2H, d), 7.70 (1H, d).

### Example 8

### Methyl 4-{[4-(3-chloro-4-cyanophenyl)-3,5-dimethyl-1H-pyrazol-1-yl]methyl}benzoate

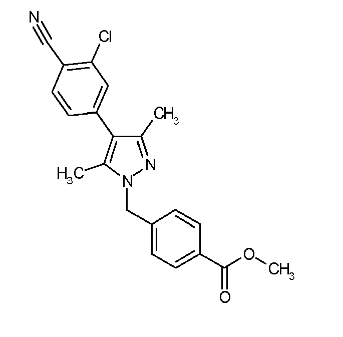

The title compound was obtained in the same manner as in Example 1 and using 2-chloro-4-(3,5-dimethyl-1H-pyrazol-4-yl)benzonitrile synthesized in Reference Example 4 and methyl 4-(bromomethyl)benzoate as starting materials.
¹H-NMR (CDCl₃) δ: 2.19 (3H, s), 2.30 (3H, s), 3.91 (3H, s), 5.34 (2H, s), 7.19 (2H, d), 7.22-7.27 (1H, m), 7.39(1H, d), 7.69 (1H, d), 8.01 (2H, d).

### Example 9

### 2-chloro-4-[1-(4-chlorobenzyl)-3,5-dimethyl-1H-pyrazol-4-yl]benzonitrile

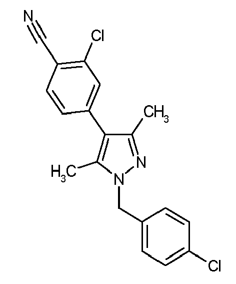

The title compound was obtained in the same manner as in Example 1 and using 2-chloro-4-(3,5-dimethyl-1H-pyrazol-4-yl)benzonitrile synthesized in Reference Example 4 and 4-chlorobenzyl chloride as starting materials.
¹H-NMR (CDCl₃) δ: 2.20 (3H, s), 2.29 (3H, s), 5.25 (2H, s), 7.08 (2H, d), 7.24 (1H, dd), 7.31 (2H, d), 7.38 (1H, d), 7.69 (1H, d).

### Example 10

### 2-chloro-4-[3,5-dimethyl-1-(pyridin-4-ylmethyl)-1H-pyrazol-4-yl]benzonitrile

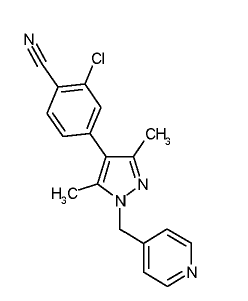

4-(Chloromethyl)pyridine hydrochloride (83.5 mg) was dissolved in DMF (2.3 mL), cooled in an ice bath, and sodium hydride (50% oil dispersion, 48.9 mg) was added. Sequentially, 2-chloro-4-(3,5-dimethyl-1H-pyrazol-4-yl)benzonitrile (119.0 mg) synthesized in Reference Example 4 was added. The reaction mixture was stirred at room temperature for 1.5 hr, sodium hydride (50% oil dispersion, 24.5 mg) and 4-(chloromethyl)pyridine hydrochloride (15.2 mg) were added, and the mixture was further stirred for 1.5 hr. The reaction mixture was poured into water, and the mixture was extracted with ethyl acetate. The organic layer was washed with saturated brine, dried over anhydrous magnesium sulfate and concentrated. The obtained residue was purified by column chromatography (hexane-ethyl acetate) and recrystallized from ethyl acetate-hexane to give the title compound (69.6 mg) as colorless crystals.
¹H-NMR (CDCl₃) δ: 2.20 (3H, s), 2.30 (3H, s), 5.29 (2H, s), 7.01 (2H, d), 7.26 (1H, dd), 7.41 (1H, d), 7.71 (1H, d), 8.58 (2H, d).

### Example 11

### 2-chloro-4-[3,5-dimethyl-1-(pyridin-3-ylmethyl)-1H-pyrazol-4-yl]benzonitrile

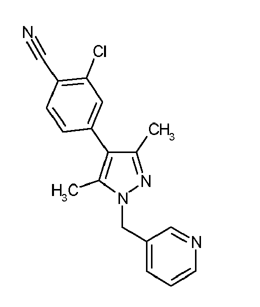

The title compound was obtained in the same manner as in Example 10 and using 2-chloro-4-(3,5-dimethyl-1H-pyrazol-4-yl)benzonitrile synthesized in Reference Example 4 and 3-(bromomethyl)pyridine hydrobromide as starting materials.
¹H-NMR (CDCl₃) δ: 2.23 (3H, s), 2.29 (3H, s), 5.30 (2H, s), 7.24 (1H, dd), 7.27-7.32 (1H, m), 7.38 (1H, d), 7.50 (1H, dt), 7.69 (1H, d), 8.47 (1H, d), 8.56 (1H, dd).

### Example 12

### 2-chloro-4-[1-(4-methoxybenzyl)-3,5-dimethyl-1H-pyrazol-4-yl]benzonitrile

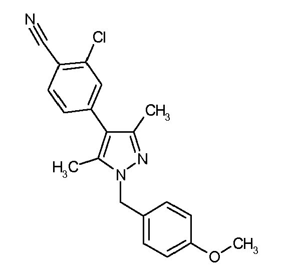

The title compound was obtained in the same manner as in Example 1 and using 2-chloro-4-(3,5-dimethyl-1H-pyrazol-4-yl)benzonitrile synthesized in Reference Example 4 and 4-methoxybenzyl chloride as starting materials.
¹H-NMR (CDCl₃) δ: 2.21 (3H, s), 2.29 (3H, s), 3.79 (3H, s), 5.22 (2H, s), 6,87 (2H, d), 7.11 (2H, d), 7.23 (1H, dd), 7.38 (1H, d), 7.68 (1H, d).

### Example 13

### 2-chloro-4-[3,5-dimethyl-l-(1,3-thiazol-2-ylmethyl)-1H-pyrazol-4-yl]benzonitrile

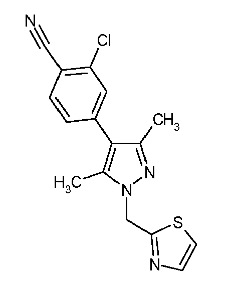

The title compound was obtained in the same manner as in Example 1 and using 2-chloro-4-(3,5-dimethyl-1H-pyrazol-4-yl)benzonitrile synthesized in Reference Example 4 and 2-(bromomethyl)-1,3-thiazole synthesized in Reference Example 15 as starting materials.
¹H-NMR (CDCl₃) δ: 2.29 (3H, s), 2.32 (3H, s), 5.58 (2H, s), 7.23 (1H, d), 7.33 (1H, d), 7.39 (1H, d), 7.69(1H, d), 7.76 (1H, s).

### Example 14

### 2-chloro-4-[3,5-dimethyl-l-(1,3-thiazol-2-ylmethyl)-1H-pyrazol-4-yl]benzonitrile

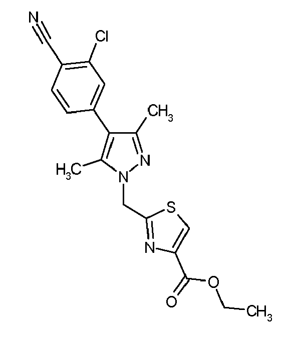

The title compound was obtained in the same manner as in Example 1 and using 2-chloro-4-(3,5-dimethyl-1H-pyrazol-4-yl)benzonitrile synthesized in Reference Example 4 and ethyl 2-(bromomethyl)-1,3-thiazole-4-carboxylate as starting materials.
¹H-NMR (CDCl₃) δ: 1.41 (3H, t), 2.29 (6H, s), 4.40 (2H, q), 5.61 (2H, s), 7.22-7.26 (1H, m), 7.39 (1H, d), 7.70 (1H, d), 8.14 (1H, s).

### Example 15

### 2-chloro-4-[1-(2-cyanobenzyl)-3,5-dimethyl-1H-pyrazol-4-yl]benzonitrile

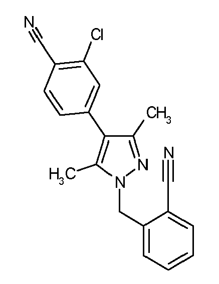

The title compound was obtained in the same manner as in Example 1 and using 2-chloro-4-(3,5-dimethyl-1H-pyrazol-4-yl)benzonitrile synthesized in Reference Example 4 and 2-cyanobenzyl bromide as starting materials.
¹H-NMR (CDCl₃) δ: 2.25 (3H, s), 2.29 (3H, s), 5.49 (2H, s), 7.06 (1H, d), 7.24-7.29 (1H, m), 7.37-7.46 (2H, m), 7.57 (1H, dt), 7.70 (2H, s).

### Example 16

### 2-chloro-4-[1-(3-cyanobenzyl)-3,5-dimethyl-1H-pyrazol-4-yl]benzonitrile

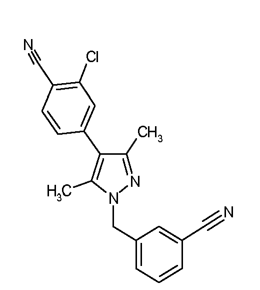

The title compound was obtained in the same manner as in Example 1 and using 2-chloro-4-(3,5-dimethyl-1H-pyrazol-4-yl)benzonitrile synthesized in Reference Example 4 and 3-cyanobenzyl bromide as starting materials.
¹H-NMR (CDCl₃) δ: 2.21 (3H, s), 2.30 (3H, s), 5.31 (2H, s), 7.24-7.29 (1H, m), 7.37-7.43 (3H, m), 7.48 (1H, dt), 7.57-7.63 (1H, m), 7.71 (1H, d).

### Example 17

### 2-chloro-4-{3,5-dimethyl-1-[4-(1H-1,2,4-triazol-1-yl)benzyl]-1H-pyrazol-4-yl}benzonitrile

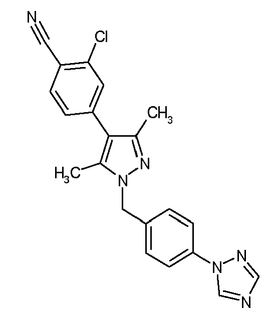

The title compound was obtained in the same manner as in Example 1 and using 2-chloro-4-(3,5-dimethyl-1H-pyrazol-4-yl)benzonitrile synthesized in Reference Example 4 and 1-[4-(bromomethyl)phenyl]-1H-1,2,4-triazole as starting materials.
¹H-NMR (CDCl₃) δ: 2.23 (3H, s), 2.31 (3H, s), 5.34 (2H, s), 7.22-7.27 (1H, m), 7.30 (2H, d), 7.40 (1H, d), 7.65-7.75 (3H, m), 8.10 (1H, s), 8.53 (1H, s).

### Example 18

### 4-{1-[(6-bromopyridin-3-yl)methyl]-3,5-dimethyl-1H-pyrazol-4-yl}-2-chlorobenzonitrile

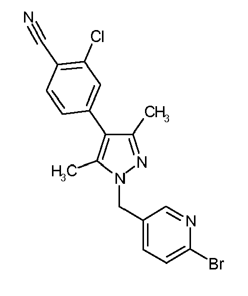

2-Chloro-4-(3,5-dimethyl-1H-pyrazol-4-yl)benzonitrile (1.24 g) synthesized in Reference Example 4 and 2-bromo-5-(chloromethyl)pyridine hydrochloride (1.43 g) synthesized in Reference Example 16 were dissolved in DMF (2.5 mL), cooled in an ice bath, and sodium hydride (50% oil dispersion, 513.6 mg) was added. The reaction mixture was stirred at room temperature for 20 min. After stirring, the mixture was poured into water, and extracted with diethyl ether. The organic layer was washed with saturated brine, dried over anhydrous magnesium sulfate and concentrated. The obtained residue was purified by column chromatography (hexane-ethyl acetate), and recrystallized from ethyl acetate-hexane to give the title compound (2.02 g) as colorless crystals.
¹H-NMR (CDCl₃) δ: 2.22 (3H, s), 2.27 (3H, s), 5.24 (2H, s), 7.23 (1H, dd), 7.37 (2H, d), 7.45-7.50 (1H, d), 7.70 (1H, d), 8.24 (1H, s).

### Example 19

### 4-{1-[(5-bromopyridin-3-yl)methyl]-3,5-dimethyl-1H-pyrazol-4-yl}-2-chlorobenzonitrile

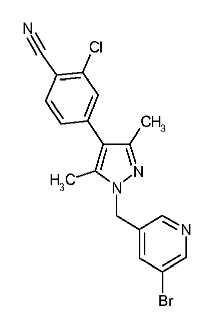

The title compound was obtained in the same manner as in Example 18 and using 2-chloro-4-(3,5-dimethyl-1H-pyrazol-4-yl)benzonitrile synthesized in Reference Example 4 and 3-bromo-5-(chloromethyl)pyridine hydrochloride synthesized in Reference Example 17 as starting materials.
¹H-NMR (CDCl₃) δ: 2.24 (3H, s), 2.29 (3H, s), 5.27 (2H, s), 7.21-7.28 (1H, m), 7.39 (1H, d), 7.66 (1H, t), 7.70 (1H, d), 8.38 (1H, d), 8.63 (1H, d).

### Example 20

### Methyl 5-{[4-(3-chloro-4-cyanophenyl)-3,5-dimethyl-1H-pyrazol-1-yl]methyl}pyridine-2-carboxylate

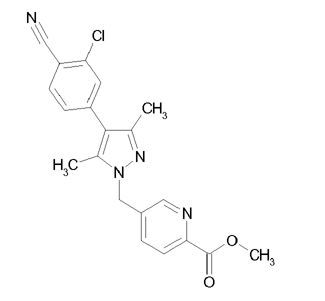

4-{1-[(6-Bromopyridin-3-yl)methyl]-3,5-dimethyl-1H-pyrazol-4-yl}-2-chlorobenzonitrile (1.41 g) obtained in Example 18 and triethylamine (0.59 mL) were dissolved in methanol (35 mL), and 1,1'-bis(diphenylphosphino)ferrocene palladium (II) dichloride dichloromethane complex (1:1) was added under a nitrogen atmosphere. The inside of the flask was substituted with carbon monoxide, and the mixture was stirred at 60°C for 6 hr. The reaction mixture was poured into water, and the mixture was extracted with ethyl acetate. The organic layer was washed with saturated brine, dried over anhydrous magnesium sulfate and concentrated. The obtained residue was purified by column chromatography (hexane-ethyl acetate) to give the title compound (0.94 g) as a yellow powder.
¹H-NMR (CDCl₃) δ: 2.22 (3H, s), 2.28 (3H, s), 4.01 (3H, s), 5.37 (2H, s), 7.23 (1H, dd), 7.38 (1H, d), 7.61 (1H, dd), 7.70 (1H, d), 8.12 (1H, d), 8.57 (1H, d).

### Example 21

### Methyl 2-{[4-(3-chloro-4-cyanophenyl)-3,5-dimethyl-1H-pyrazol-1-yl]methyl}benzoate

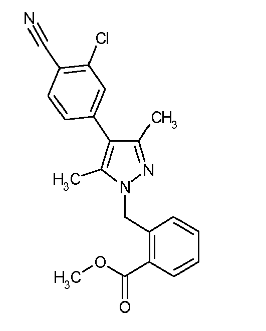

The title compound was obtained in the same manner as in Example 1 and using 2-chloro-4-(3,5-dimethyl-1H-pyrazol-4-yl)benzonitrile synthesized in Reference Example 4 and methyl 2-(chloromethyl)benzoate as starting materials.
¹H-NMR (CDCl₃) δ: 2.19 (3H, s), 2.32 (3H, s), 3.95 (3H, s), 5.76 (2H, s), 6.51 (1H, d), 7.30 (1H, dd), 7.35 (1H, t), 7.40-7.49 (2H, m), 7.71 (1H, d), 8.05 (1H, dd).

### Example 22

### Methyl 3-{[4-(3-chloro-4-cyanophenyl)-3,5-dimethyl-1H-pyrazol-1-yl]methyl}benzoate

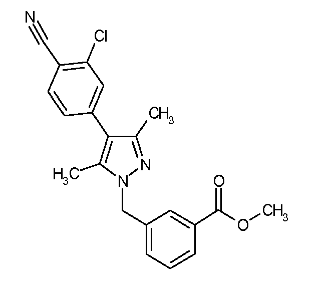

The title compound was obtained in the same manner as in Example 1 and using 2-chloro-4-(3,5-dimethyl-1H-pyrazol-4-yl)benzonitrile synthesized in Reference Example 4 and methyl 3-(bromomethyl)benzoate as starting materials.
¹H-NMR (CDCl₃) δ: 2.21 (3H, s), 2.30 (3H, s), 3.92 (3H, s), 5.33 (2H, s), 7.22-7.28 (1H, m), 7.29-7.35 (1H, m), 7.37-7.46 (2H, m), 7.69 (1H, d), 7.88 (1H, s), 7.97 (1H, d).

### Example 23

### 4-[1-(4-cyanobenzyl)-3,5-dimethyl-1H-pyrazol-4-yl]benzonitrile

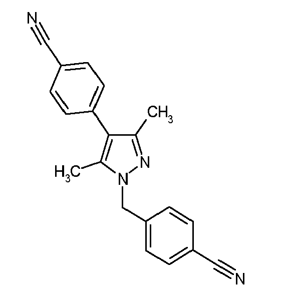

The title compound was obtained in the same manner as in Example 1 and using 4-(3,5-dimethyl-1H-pyrazol-4-yl)benzonitrile synthesized in Reference Example 5 and 4-cyanobenzyl bromide as starting materials.
¹H-NMR (CDCl₃) δ: 2.19 (3H, s), 2.29 (3H, s), 5.34 (2H, s), 7.23 (2H, d), 7.36 (2H, d), 7.64 (2H, d), 7.70 (2H, d).

### Example 24

### 3-{[4-(4-cyanophenyl)-3,5-dimethyl-1H-pyrazol-1-yl]methyl}benzonitrile

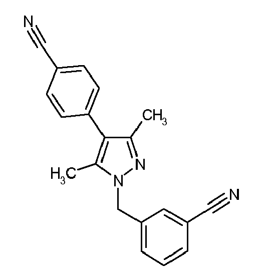

The title compound was obtained in the same manner as in Example 1 and using 4-(3,5-dimethyl-1H-pyrazol-4-yl)benzonitrile synthesized in Reference Example 5 and 3-cyanobenzyl bromide as starting materials.
¹H-NMR (CDCl₃) δ: 2.20 (3H, s), 2.30 (3H, s), 5.31 (2H, s), 7.34-7.43 (4H, m), 7.47 (1H, t), 7.60 (1H, d), 7.71 (2H, d).

### Example 25

### 4-{1-[(6-bromopyridin-3-yl)methyl]-3,5-dimethyl-1H-pyrazol-4-yl}benzonitrile

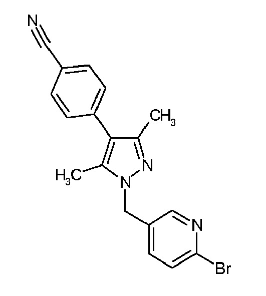

The title compound was obtained in the same manner as in Example 18 and using 4-(3,5-dimethyl-1H-pyrazol-4-yl)benzonitrile synthesized in Reference Example 5 and 2-bromo-5-(chloromethyl)pyridine hydrochloride synthesized in Reference Example 16 as starting materials.
¹H-NMR (CDCl₃) δ: 2.22 (3H, s), 2.27 (3H, s), 5.26 (2H, d), 7.29-7.41 (3H, m), 7.44-7.53 (1H, m), 7.67-7.73 (2H, m), 8.25 (1H, dd).

### Example 26

### 4-{1-[(5-bromopyridin-3-yl)methyl]-3,5-dimethyl-1H-pyrazol-4-yl}benzonitrile

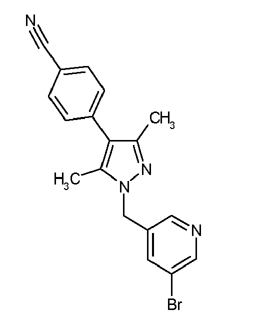

The title compound was obtained in the same manner as in Example 18 and using 4-(3,5-dimethyl-1H-pyrazol-4-yl)benzonitrile synthesized in Reference Example 5 and 3-bromo-5-(chloromethyl)pyridine hydrochloride synthesized in Reference Example 17 as starting materials.
¹H-NMR (CDCl₃) δ: 2.23 (3H, s), 2.28 (3H, s), 5.27 (2H, d), 7.36 (2H, d), 7.65-7.67 (1H, m), 7.70 (2H, d), 8.39 (1H, d), 8.63 (1H, d).

### Example 27

### Ethyl 2-{[4-(4-cyanophenyl)-3,5-dimethyl-1H-pyrazol-1-yl]methyl}-1,3-thiazole-4-carboxylate

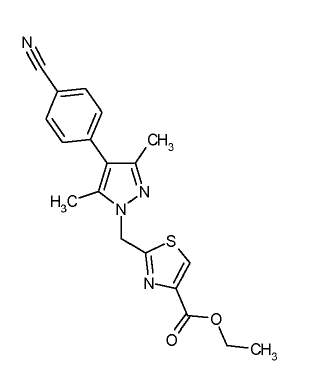

The title compound was obtained in the same manner as in Example 1 and using 4-(3,5-dimethyl-1H-pyrazol-4-yl)benzonitrile synthesized in Reference Example 5 and ethyl 2-(bromomethyl)-1,3-thiazole-4-carboxylate as starting materials.
¹H-NMR (CDCl₃) δ: 1.41 (3H, t), 2.28 (3H, s), 2.30 (3H, s), 4.44 (2H, q), 5.61 (2H, s), 7.35 (2H, d), 7.70 (2H, d), 8.14 (1H, s).

### Example 28

### 4-[1-(4-cyanobenzyl)-3,5-dimethyl-1H-pyrazol-4-yl]-2-fluorobenzonitrile

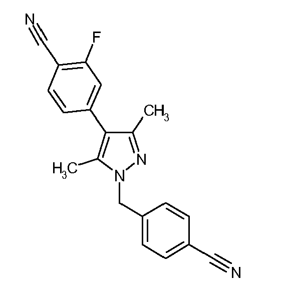

The title compound was obtained in the same manner as in Example 1 and using 4-(3,5-dimethyl-1H-pyrazol-4-yl)-2-fluorobenzonitrile synthesized in Reference Example 6 and 4-cyanobenzyl bromide as starting materials.
¹H-NMR (CDCl₃) δ: 2.21 (3H, s), 2.30 (3H, s), 5.34 (2H, s), 7.11 (1H, dd), 7.14 (1H, dd), 7.23 (2H, d), 7.62-7.69 (3H, m).

### Example 29

### 4-{3,5-dimethyl-1-[4-(1H-pyrazol-1-yl)benzyl]-1H-pyrazol-4-yl}-2-fluorobenzonitrile

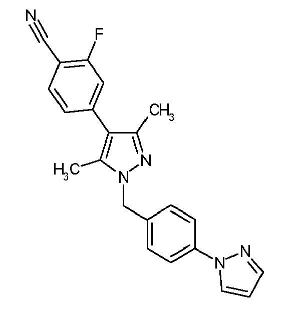

The title compound was obtained in the same manner as in Reference Example 1 and using 4-(3,5-dimethyl-1H-pyrazol-4-yl)-2-fluorobenzonitrile synthesized in Reference Example 6 and 1-[4-(bromomethyl)phenyl]-1H-pyrazole as starting materials.
¹H-NMR (CDCl₃) δ: 2.23 (3H, s), 2.32 (3H, s), 5.31 (2H, s), 6.47 (1H, t), 7.06-7.17 (2H, m), 7.25 (2H, d), 7.63 (1H, d), 7.67 (2H, d), 7.72 (1H, d), 7.90 (1H, d).

### Example 30

### 4-{[4-(4-cyano-3-fluorophenyl)-3,5-dimethyl-1H-pyrazol-1-yl]methyl}benzamide

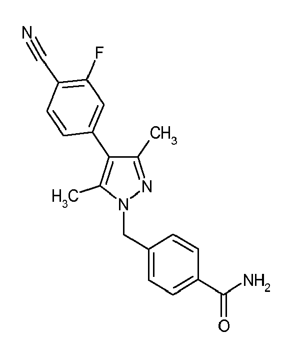

The title compound was obtained in the same manner as in Example 1 and using 4-(3,5-dimethyl-1H-pyrazol-4-yl)-2-fluorobenzonitrile synthesized in Reference Example 6 and 4-(chloromethyl)benzamide as starting materials.
¹H-NMR (CDCl₃) δ: 2.21 (3H, s), 2.31 (3H, s), 5.34 (2H, s), 5.64 (1H, brs), 5.98 (1H, brs), 7.11 (1H, dd), 7.14 (1H, dd), 7.22 (2H, d), 7.65 (1H, dd), 7.79 (2H, d).

### Example 31

### Methyl 2-chloro-5-{[4-(3-chloro-4-cyanophenyl)-3,5-dimethyl-1H-pyrazol-1-yl]methyl}benzoate

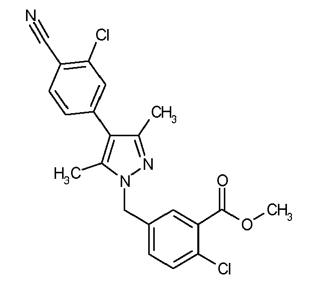

The title compound was obtained in the same manner as in Example 18 and using 2-chloro-4-(3,5-dimethyl-1H-pyrazol-4-yl)benzonitrile synthesized in Reference Example 4 and methyl 5-(bromomethyl)-2-chlorobenzoate as starting materials.
¹H-NMR (CDCl₃) δ: 2.21 (3H, s), 2.29 (3H, s), 3.93 (3H, s), 5.26 (2H, s), 7.18 (1H, dd), 7.24 (1H, dd), 7.39 (1H, d), 7.43 (1H, d), 7.67 (1H, d), 7.70 (1H, d).

### Example 32

### Methyl 5-{[4-(3-chloro-4-cyanophenyl)-3,5-dimethyl-1H-pyrazol-1-yl]methyl}-2-fluorobenzoate

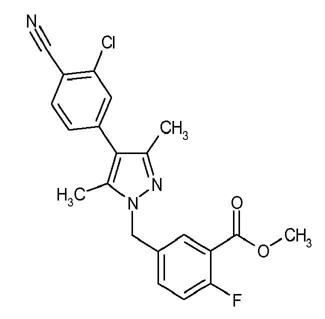

The title compound was obtained in the same manner as in Example 18 and using 2-chloro-4-(3,5-dimethyl-1H-pyrazol-4-yl)benzonitrile synthesized in Reference Example 4 and methyl 5-(bromomethyl)-2-fluorobenzoate as starting materials.
¹H-NMR (CDCl₃) δ: 2.22 (3H, s), 2.29 (3H, s), 3.93 (3H, s), 5.27 (2H, s), 7.12 (1H, dd), 7.22-7.26 (1H, m), 7.27-7.34 (1H, m), 7.39 (1H, d), 7.69 (1H, d), 7.79 (1H, dd).

### Example 33

### Dimethyl 4-{[4-(3-chloro-4-cyanophenyl)-3,5-dimethyl-1H-pyrazol-1-yl]methyl}phthalate

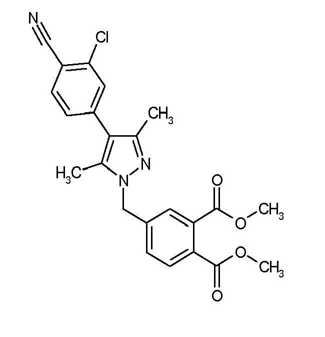

The title compound was obtained in the same manner as in Example 18 and using 2-chloro-4-(3,5-dimethyl-1H-pyrazol-4-yl)benzonitrile synthesized in Reference Example 4 and dimethyl 4-(bromomethyl)phthalate as starting materials.
¹H-NMR (CDCl₃) δ: 2.19 (3H, s), 2.29 (3H, s), 3.90 (3H, s), 3.91 (3H, s), 5.33 (2H, s), 7.22-7.30 (2H, m), 7.39 (1H, d), 7.52 (1H, d), 7.70 (1H, d), 7.73 (1H, d).

### Example 34

### 4-[l-(4-cyanobenzyl)-3,5-dimethyl-1H-pyrazol-4-yl]-2-(trifluoromethyl)benzonitrile

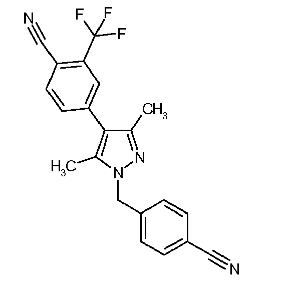

The title compound was obtained in the same manner as in Example 1 and using 4-(3,5-dimethyl-1H-pyrazol-4-yl)-2-(trifluoromethyl)benzonitrile synthesized in Reference Example 7 and 4-cyanobenzyl bromide as starting materials.
¹H-NMR (CDCl₃) δ: 2.22 (3H, s), 2.31 (3H, s), 5.35 (2H, s), 7.24 (2H, d), 7.55 (1H, dd), 7.63-7.69 (3H, m), 7.88 (1H, d).

### Example 35

### 4-({4-[4-cyano-3-(trifluoromethyl)phenyl]-3,5-dimethyl-1H-pyrazol-1-yl}methyl)benzamide

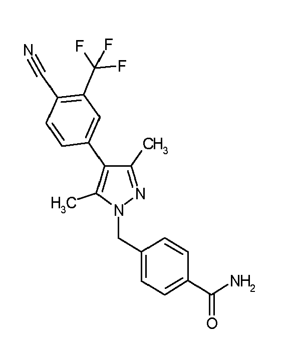

The title compound was obtained in the same manner as in Example 1 and using 4-(3,5-dimethyl-1H-pyrazol-4-yl)-2-(trifluoromethyl)benzonitrile synthesized in Reference Example 7 and 4-(chloromethyl)benzamide as starting materials.
¹H-NMR (CDCl₃) δ: 2.21 (3H, s), 2.32 (3H, s), 5.35 (2H, s), 5.56 (1H, brs), 6.00 (1H, brs), 7.23 (2H, d), 7.55 (1H, dd), 7.66 (1H, s), 7.80 (2H, d), 7.87 (1H, d).

### Example 36

### 2-chloro-4-[1-(4-cyanobenzyl)-3,5-dimethyl-1H-pyrazol-4-yl]-5-fluorobenzonitrile

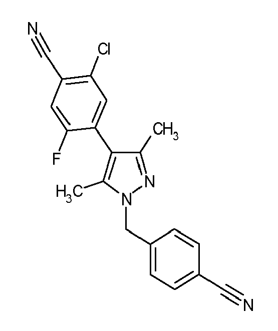

The title compound was obtained in the same manner as in Example 1 and using 2-chloro-4-(3,5-dimethyl-1H-pyrazol-4-yl)-5-fluorobenzonitrile synthesized in Reference Example 13 and 4-cyanobenzyl bromide as starting materials.
¹H-NMR (CDCl₃) δ: 2.12 (3H, d), 2.23 (3H, d), 5.34 (2H, s), 7.22 (2H, d), 7.37 (1H, d), 7.47 (1H, d), 7.65 (2H, d).

### Example 37

### 4-{[4-(5-chloro-4-cyano-2-fluorophenyl)-3,5-dimethyl-1H-pyrazol-1-yl]methyl}benzamide

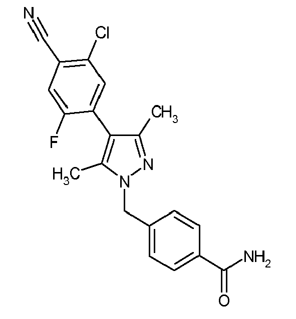

The title compound was obtained in the same manner as in Example 1 and using 2-chloro-4-(3,5-dimethyl-1H-pyrazol-4-yl)-5-fluorobenzonitrile synthesized in Reference Example 13 and 4-(chloromethyl)benzamide as starting materials.
¹H-NMR (CDCl₃) δ: 2.12 (3H, d), 2.23 (3H, d), 5.34 (2H, s), 5.60 (1H, brs), 5.99 (1H, brs), 7.21 (2H, d), 7.37 (1H, d), 7.46 (1H, d), 7.79 (2H, d).

### Example 38

### 4-[1-(3-cyanobenzyl)-3,5-dimethyl-1H-pyrazol-4-yl]-2-(trifluoromethyl)benzonitrile

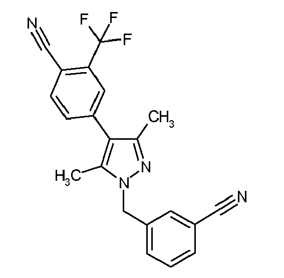

The title compound was obtained in the same manner as in Example 1 and using 4-(3,5-dimethyl-1H-pyrazol-4-yl)-2-(trifluoromethyl)benzonitrile synthesized in Reference Example 7 and 3-cyanobenzyl bromide as starting materials.
¹H-NMR (CDCl₃) δ: 2.23 (3H, s), 2.31 (3H, s), 5.32 (2H, s), 7.38-7.45 (2H, m), 7.49 (1H, dd), 7.56 (1H, dd), 7.59-7.63 (1H, d), 7.68 (1H, d), 7.89 (1H, d).

### Example 39

### 4-[1-(2-cyanobenzyl)-3,5-dimethyl-1H-pyrazol-4-yl]-2-(trifluoromethyl)benzonitrile

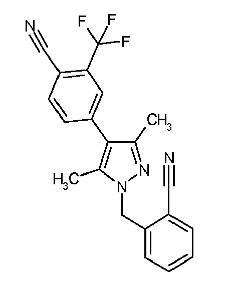

The title compound was obtained in the same manner as in Example 1 and using 4-(3,5-dimethyl-1H-pyrazol-4-yl)-2-(trifluoromethyl)benzonitrile synthesized in Reference Example 7 and 2-cyanobenzyl bromide as starting materials.
¹H-NMR (CDCl₃) δ: 2.27 (3H, s), 2.31 (3H, s), 5.51 (2H, s), 7.09 (1H, d), 7.44 (1H, d), 7.52-7.62 (2H, m), 7.68 (1H, s), 7.71 (1H, dd), 7.88 (1H, d).

### Example 40

### 4-[1-(4-chlorobenzyl)-3,5-dimethyl-1H-pyrazol-4-yl]-2-(trifluoromethyl)benzonitrile

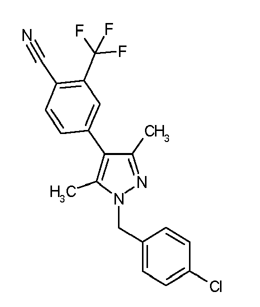

The title compound was obtained in the same manner as in Example 1 and using 4-(3,5-dimethyl-1H-pyrazol-4-yl)-2-(trifluoromethyl)benzonitrile synthesized in Reference Example 7 and 4-chlorobenzyl chloride as starting materials.
¹H-NMR (CDCl₃) δ: 2.21 (3H, s), 2.30 (3H, s), 5.26 (2H, s), 7.10 (2H, d), 7.32 (2H, d), 7.54 (1H, d), 7.65 (1H, d), 7.87 (1H, d).

### Example 41

### 4-[3,5-dimethyl-1-(pyridin-3-ylmethyl)-1H-pyrazol-4-yl]-2-(trifluoromethyl)benzonitrile

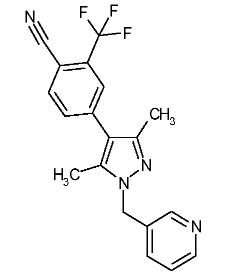

The title compound was obtained in the same manner as in Example 18 and using 4-(3,5-dimethyl-1H-pyrazol-4-yl)-2-(trifluoromethyl)benzonitrile synthesized in Reference Example 7 and 3-(chloromethyl)pyridine hydrochloride as starting materials.
¹H-NMR (CDCl₃) δ: 2.25 (3H, s), 2.30 (3H, s), 5.31 (2H, s), 7.28-7.34 (m, 1H), 7.48-7.57 (m, 2H), 7.65 (1H, d), 7.87 (1H, d), 8.48 (1H, s), 8.57 (1H, d).

### Example 42

### 2-chloro-4-{3,5-dimethyl-1-[4-(1H-pyrazol-1-yl)benzyl]-1H-pyrazol-4-yl}benzonitrile

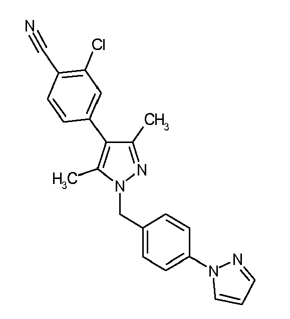

The title compound was obtained in the same manner as in Example 1 and using 2-chloro-4-(3,5-dimethyl-1H-pyrazol-4-yl)benzonitrile synthesized in Reference Example 4 and 1-[4-(bromomethyl)phenyl]-1H-pyrazole as starting materials.
¹H-NMR (CDCl₃) δ: 2.22 (3H, s), 2.31 (3H, s), 5.31 (2H, s), 6.47 (dd, 1H), 7.22-7.28 (m, 3H), 7.39 (1H, d), 7.64-7.74 (4H, m), 7.90 (1H, d).

### Example 43

### Ethyl 5-{[4-(3-chloro-4-cyanophenyl)-3,5-dimethyl-1H-pyrazol-1-yl]methyl}-2-furoate

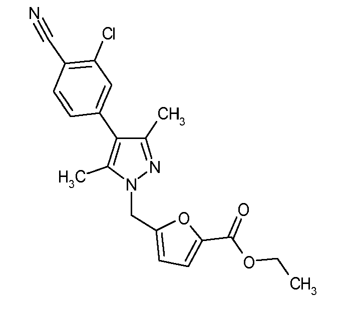

The title compound was obtained in the same manner as in Example 1 and using ethyl 2-chloro-4-(3,5-dimethyl-1H-pyrazol-4-yl)benzonitrile synthesized in Reference Example 4 and ethyl 5-chloromethyl-2-furoate as starting materials.
¹H-NMR (CDCl₃) δ: 1.36 (3H, t), 2.25 (3H, s), 2.36 (3H, s), 4.35 (2H, q), 5.29 (2H, s), 6.33 (1H, d), 7.10 (1H, d), 7.24 (1H, dd), 7.39 (1H, d), 7.70 (1H, d).

### Example 44

### Methyl 4-{[4-(3-chloro-4-cyanophenyl)-3,5-dimethyl-1H-pyrazol-1-yl]methyl}-2-fluorobenzoate

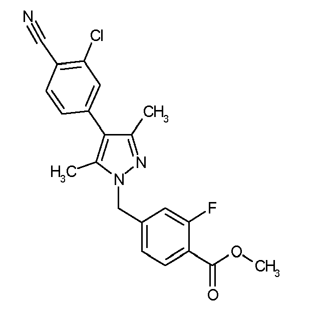

The title compound was obtained in the same manner as in Example 18 and using 2-chloro-4-(3,5-dimethyl-1H-pyrazol-4-yl)benzonitrile synthesized in Reference Example 4 and methyl 4-(bromomethyl)-2-fluorobenzoate as starting materials.
¹H-NMR (CDCl₃) δ: 2.20 (3H, s), 2.30 (3H, s), 3.92 (3H, s), 5.31 (2H, s), 6.86 (dd, 1H), 6.98 (dd, 1H), 7.26 (1H, dd), 7.40 (1H, d), 7.70 (1H, d), 7.92 (1H, t).

### Example 45

### Methyl 2-bromo-4-{[4-(3-chloro-4-cyanophenyl)-3,5-dimethyl-1H-pyrazol-1-yl]methyl}benzoate

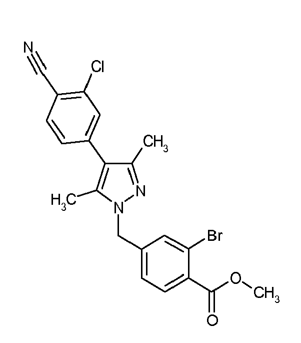

The title compound was obtained in the same manner as in Example 18 and using 2-chloro-4-(3,5-dimethyl-1H-pyrazol-4-yl)benzonitrile synthesized in Reference Example 4 and methyl 4-(bromomethyl)-2-bromobenzoate as starting materials.
¹H-NMR (CDCl₃) δ: 2.20 (3H, s), 2.30 (3H, s), 3.92 (3H, s), 5.27 (2H, s), 7.11 (1H, dd), 7.25 (1H, dd), 7.40 (1H, d), 7.46 (1H, d), 7.70 (1H, d), 7.77 (1H, d).

### Example 46

### 4-{1-[(3,5-dimethylisoxazol-4-yl)methyl]-3,5-dimethyl-1H-pyrazol-4-yl}-2-(trifluoromethyl)benzonitrile

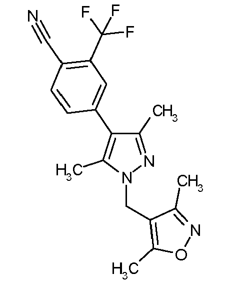

The title compound was obtained in the same manner as in Example 1 and using 4-(3,5-dimethyl-1H-pyrazol-4-yl)-2-(trifluoromethyl)benzonitrile synthesized in Reference Example 7 and 4-(chloromethyl)-3,5-dimethylisoxazole as starting materials.
¹H-NMR (CDCl₃) δ: 2.24 (3H, s), 2.26 (3H, s), 2.28 (3H, s), 2.39 (3H, s), 5.03 (2H, s), 7.53 (1H, dd), 7.64 (1H, d), 7.89 (1H, d).

### Example 47

### 2-chloro-4-(3,5-dimethyl-1-{[6-(trifluoromethyl)pyridin-3-yl]methyl}-1H-pyrazol-4-yl)benzonitrile

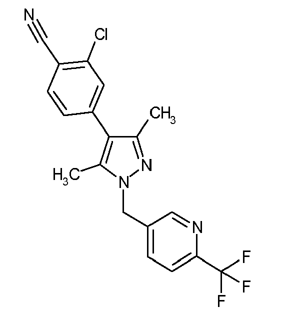

The title compound was obtained in the same manner as in Example 1 and using 2-chloro-4-(3,5-dimethyl-1H-pyrazol-4-yl)benzonitrile synthesized in Reference Example 4 and 5-(chloromethyl)-2-(trifluoromethyl)pyridine as starting materials.
¹H-NMR (CDCl₃) δ: 2.25 (3H, s), 2.28 (3H, s), 5.37 (2H, s), 7.24 (1H, dd), 7.38 (1H, d), 7.67-7.68 (2H, m), 7.71 (1H, d), 8.57 (1H, s).

### Example 48

### Methyl 4-{[4-(4-chloro-3-cyanophenyl)-3,5-dimethyl-1H-pyrazol-1-yl]methyl}benzoate

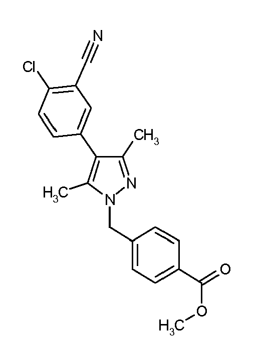

The title compound was obtained in the same manner as in Example 1 and using 2-chloro-5-(3,5-dimethyl-1H-pyrazol-4-yl)benzonitrile synthesized in Reference Example 8 and methyl 4-(bromomethyl)benzoate as starting materials.
¹H-NMR (CDCl₃) δ: 2.15 (3H, s), 2.27 (3H, s), 3.91 (3H, s), 5.33 (2H, s), 7.19 (2H, d), 7.38 - 7.45 (1H, m), 7.52 - 7.57 (2H, m), 8.01 (2H, d).

### Example 49

### 4-{1-[4-(3-chloro-4-cyanophenyl)-3,5-dimethyl-1H-pyrazol-1-yl]ethyl}benzoic acid

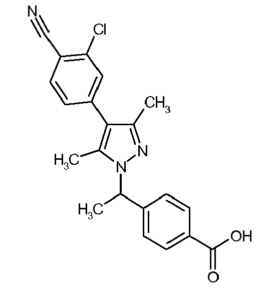

2-Chloro-4-(3,5-dimethyl-1H-pyrazol-4-yl)benzonitrile (389.1 mg) synthesized in Reference Example 4 and ethyl 4-(1-bromoethyl)benzoate (647.9 mg) were dissolved in DMF (1.9 mL), cooled in an ice bath, and sodium hydride (50% oil dispersion, 96.8 mg) was added. The reaction mixture was stirred at room temperature for 2.5 days. After stirring, the mixture was poured into 1 mol/L aqueous sodium hydroxide solution, and washed with diethyl ether. The aqueous layer was neutralized with 1 mol/L hydrochloric acid, and the mixture was extracted with ethyl acetate. The organic layer was washed with saturated brine, dried over anhydrous magnesium sulfate and concentrated. The obtained residue was purified by column chromatography (hexane-ethyl acetate) to give the title compound (434.5 mg) as a colorless powder.
¹H-NMR (DMSO-d₆) δ: 1.80 (3H, d), 2.22 (3H, s), 2.23 (3H, s), 5.72 (1H, q), 7.35 (2H, dd), 7.46 (1H, dd), 7.65 (1H, d), 7.90 (2H, d), 7.98 (1H, d).

### Example 50

### 4-{[4-(4-cyanophenyl)-3,5-dimethyl-1H-pyrazol-1-yl]methyl}benzoic acid

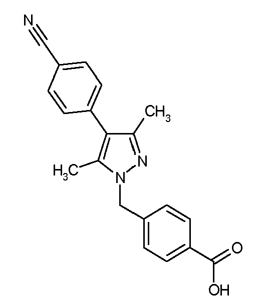

Methyl 4-{[4-(4-cyanophenyl)-3,5-dimethyl-1H-pyrazol-1-yl]methyl}benzoate (151.5 mg) synthesized in Example 4 was dissolved in ethanol (2 mL), 1 mol/L aqueous sodium hydroxide solution (2 mL) was added, and the mixture was stirred at 50°C for 2 hr. The reaction solution was neutralized with 1 mol/L hydrochloric acid, and extracted with ethyl acetate. The ethyl acetate layer was washed with water, dried over anhydrous magnesium sulfate and concentrated. The residue was washed with diethyl ether and filtrated to give the title compound (357 mg) as a solid.
¹H-NMR (DMSO-d₆) δ: 2.20 (3H, s), 2.24 (3H, s), 5.39 (2H, s), 7.28 (2H, d), 7.51 (2H, d), 7.86 (2H, d), 7.89 - 7.95 (2H, m) 12.93 (1H, s).

### Example 51

### 4-{[4-(3-chloro-4-cyanophenyl)-3,5-dimethyl-1H-pyrazol-1-yl]methyl}-1H-pyrazole-5-carboxylic acid

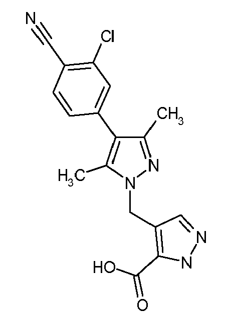

The title compound was obtained in the same manner as in Example 50 and using ethyl 4-{[4-(3-chloro-4-cyanophenyl)-3,5-dimethyl-1H-pyrazol-1-yl]methyl}-1H-pyrazole-5-carboxylate synthesized in Example 5 as a starting material.
¹H-NMR (DMSO-d₆) δ: 2.18 (3H, s), 2.29 (3H, s), 5.38 (2H, s), 7.30 - 7.42 (1H, m), 7.47 (1H, dd), 7.66 (1H, d), 7.99 (1H, d), 13.27 (1H, s).

### Example 52

### 4-{[4-(3-chloro-4-cyanophenyl)-3,5-dimethyl-1H-pyrazol-1-yl]methyl}benzoic acid

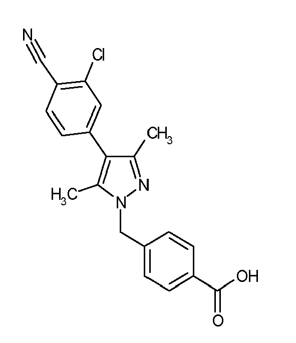

The title compound was obtained in the same manner as in Example 50 and using methyl 4-{[4-(3-chloro-4-cyanophenyl)-3,5-dimethyl-1H-pyrazol-1-yl]methyl}benzoate synthesized in Example 8 as a starting material.
¹H-NMR (DMSO-d₆) δ: 2.21 (3H, s), 2.25 (3H, s), 5.39 (2H, s), 7.27 (2H, d), 7.48 (1H, dd), 7.67 (1H, d), 7.92 (2H, d), 7.98 (1H, d), 12.94 (1H, s).

### Example 53

### 2-{[4-(3-chloro-4-cyanophenyl)-3,5-dimethyl-1H-pyrazol-1-yl]methyl}-1,3-thiazole-4-carboxylic acid

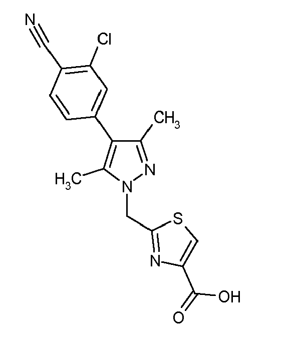

The title compound was obtained in the same manner as in Example 50 and using ethyl 2-{[4-(3-chloro-4-cyanophenyl)-3,5-dimethyl-1H-pyrazol-1-yl]methyl}-1,3-thiazole-4-carboxylate synthesized in Example 14 as a starting material.
¹H-NMR (CDCl₃) δ: 2.30 (3H, s), 2.32 (3H, s), 5.63 (2H, s), 7.22-7.26 (1H, m), 7.39 (1H, d), 7.71 (1H, d), 8.26 (1H, s).

### Example 54

### 5-{[4-(3-chloro-4-cyanophenyl)-3,5-dimethyl-1H-pyrazol-1-yl]methyl}pyridine-2-carboxylic acid

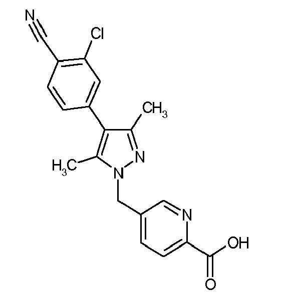

Methyl 5-{[4-(3-chloro-4-cyanophenyl)-3,5-dimethyl-1H-pyrazol-1-yl]methyl}pyridine-2-carboxylate (688.2 mg) synthesized in Example 20 was dissolved in methanol (7.2 mL), and 1 mol/L aqueous sodium hydroxide solution (3.61 mL) was added. The reaction mixture was stirred at room temperature for 10 min, and neutralized with ammonium chloride (250 mg). The insoluble component was filtered off and the filtrate was concentrated and azeotroped with toluene. The obtained residue was washed with diethyl ether to give the title compound (881.4 mg, containing sodium chloride as impurity) as a pale-orange powder.
¹H-NMR (CD₃OD) δ: 2.34 (3H, s), 2.36 (3H, s), 5.49 (2H, s), 7.50 (1H, dd), 7.62-7.74 (2H, m), 7.91 (1H, d), 8.05 (1H, d), 8.46 (1H, s).

### Example 55

### 2-{[4-(3-chloro-4-cyanophenyl)-3,5-dimethyl-1H-pyrazol-1-yl]methyl}benzoic acid

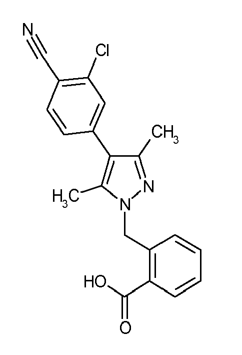

The title compound was obtained in the same manner as in Example 50 and using methyl 2-{[4-(3-chloro-4-cyanophenyl)-3,5-dimethyl-1H-pyrazol-1-yl]methyl}benzoate synthesized in Example 21 as a starting material.
¹H-NMR (CDCl₃) δ: 2.28 (3H, s), 2.33 (3H, s), 5.61 (2H, s), 6.79 (1H, d), 7.22-7.30 (1H, m), 7.37-7.43 (2H, m), 7.44-7.53 (1H, m), 7.72 (1H, d), 8.03 (1H, dd).

### Example 56

### 3-{[4-(3-chloro-4-cyanophenyl)-3,5-dimethyl-1H-pyrazol-1-yl]methyl}benzoic acid

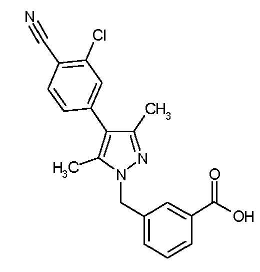

The title compound was obtained in the same manner as in Example 50 and using methyl 3-{[4-(3-chloro-4-cyanophenyl)-3,5-dimethyl-1H-pyrazol-1-yl]methyl}benzoate synthesized in Example 22 as a starting material.
¹H-NMR (CDCl₃) δ: 2.25 (3H, s), 2.31 (3H, s), 5.35 (2H, s), 7.22-7.28 (1H, m), 7.33-7.41 (2H, m), 7.46 (1H, t), 7.70 (1H, d), 7.94-8.08 (2H, m).

### Example 57

### 2-{[4-(4-cyanophenyl)-3,5-dimethyl-1H-pyrazol-1-yl]methyl}-1,3-thiazole-4-carboxylic acid

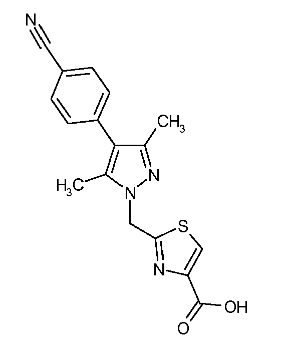

The title compound was obtained in the same manner as in Example 50 and using ethyl 2-{[4-(4-cyanophenyl)-3,5-dimethyl-1H-pyrazol-1-yl]methyl}-1,3-thiazole-4-carboxylate synthesized in Example 27 as a starting material.
¹H-NMR (CDCl₃) δ: 2.31 (3H, s), 2.33 (3H, s), 5.63 (2H, s), 7.37 (2H, d), 7.72 (2H, d), 8.27 (1H, s).

### Example 58

### 2-chloro-5-{[4-(3-chloro-4-cyanophenyl)-3,5-dimethyl-1H-pyrazol-1-yl]methyl}benzoic acid

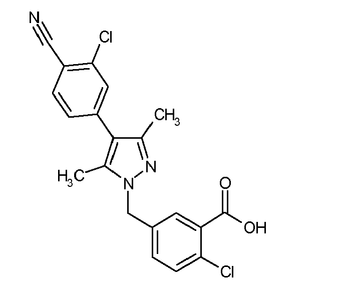

The title compound was obtained in the same manner as in Example 50 and using methyl 2-chloro-5-{[4-(3-chloro-4-cyanophenyl)-3,5-dimethyl-1H-pyrazol-1-yl]methyl}benzoate synthesized in Example 31 as a starting material.
¹H-NMR (DMSO-d₆) δ: 2.20 (3H, s), 2.27 (3H, s), 5.35 (2H, s), 7.30 (1H, dd), 7.48 (1H, dd), 7.53 (1H, d), 7.67 (2H, d), 7.98 (1H, d), 13.48 (1H, brs).

### Example 59

### 5-{[4-(3-chloro-4-cyanophenyl)-3,5-dimethyl-1H-pyrazol-1-yl]methyl}-2-fluorobenzoic acid

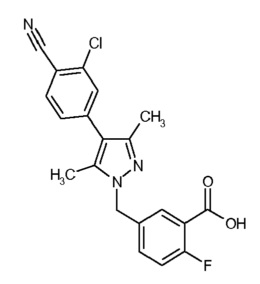

The title compound was obtained in the same manner as in Example 50 and using methyl 5-{[4-(3-chloro-4-cyanophenyl)-3,5-dimethyl-1H-pyrazol-1-yl]methyl}-2-fluorobenzoate synthesized in Example 32 as a starting material.
¹H-NMR (CDCl₃) δ: 2.21 (3H, s), 2.28 (3H, s), 5.34 (2H, s), 7.30 (1H, dd), 7.39-7.45 (1H, m), 7.47 (1H, dd), 7.67 (1H, d), 7.77 (1H, dd), 7.98 (1H, d).

### Example 60

### 4-{[4-(3-chloro-4-cyanophenyl)-3,5-dimethyl-1H-pyrazol-1-yl]methyl}phthalic acid

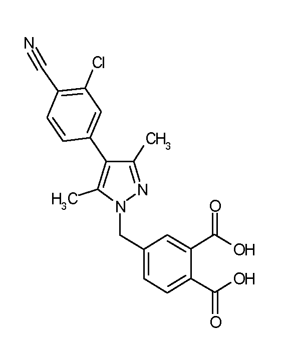

The title compound was obtained in the same manner as in Example 50 and using dimethyl 4-{[4-(3-chloro-4-cyanophenyl)-3,5-dimethyl-1H-pyrazol-1-yl]methyl}phthalate synthesized in Example 33 as a starting material.
¹H-NMR (DMSO-d₆) δ: 2.21 (3H, s), 2.27 (3H, s), 5.40 (2H, s), 7.34 (1H, dd), 7.49 (1H, dd), 7.54 (1H, s), 7.64-7.74 (2H, m), 7.99 (1H, d).

### Example 61

### 5-{[4-(3-chloro-4-cyanophenyl)-3,5-dimethyl-1H-pyrazol-1-yl]methyl}-2-furoic acid

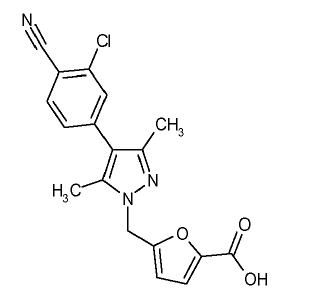

The title compound was obtained in the same manner as in Example 50 and using ethyl 5-{[4-(3-chloro-4-cyanophenyl)-3,5-dimethyl-1H-pyrazol-1-yl]methyl}-2-furoate synthesized in Example 43 as a starting material.
¹H-NMR (CDCl₃) δ: 2.26 (3H, s), 2.37 (3H, s), 5.32 (2H, s), 6.40 (1H, d), 7.24 (2H, m), 7.39 (1H, d), 7.70 (1H, d).

### Example 62

### 4-{[4-(3-chloro-4-cyanophenyl)-3,5-dimethyl-1H-pyrazol-1-yl]methyl}-2-fluorobenzoic acid

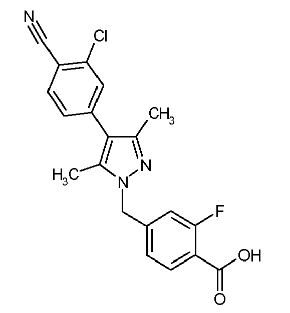

The title compound was obtained in the same manner as in Example 50 and using methyl 4-{[4-(3-chloro-4-cyanophenyl)-3,5-dimethyl-1H-pyrazol-1-yl]methyl}-2-fluorobenzoate synthesized in Example 44 as a starting material.
¹H-NMR (CDCl₃) δ: 2.21 (3H, s), 2.31 (3H, s), 5.33 (2H, s), 6.89 (1H, dd), 7.01 (1H, dd), 7.26 (1H, dd), 7.41 (1H, d), 7.71 (1H, d), 7.99 (1H, t).

### Example 63

### 4-{[4-(3-chloro-4-cyanophenyl)-3,5-dimethyl-1H-pyrazol-1-yl]methyl}-2-cyanobenzoic acid

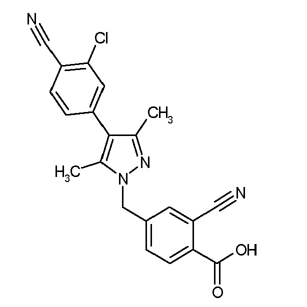

The title compound was obtained in the same manner as in Example 50 and using methyl 4-{[4-(3-chloro-4-cyanophenyl)-3,5-dimethyl-1H-pyrazol-1-yl]methyl}-2-cyanobenzoate synthesized in Example 122 as a starting material.
¹H-NMR (DMSO-d₆) δ: 2.21 (3H, s), 2.28 (3H, s), 5.45 (2H, s), 7.46-7.57 (2H, m), 7.70 (1H, d), 7.78 (1H, s), 8.00 (1H, d), 8.08 (1H, d).

### Example 64

### 4-{[4-(4-chloro-3-cyanophenyl)-3,5-dimethyl-1H-pyrazol-1-yl]methyl}benzoic acid

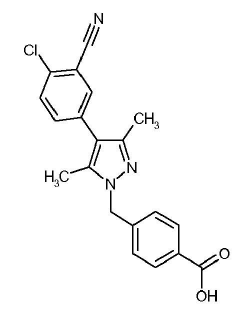

The title compound was obtained in the same manner as in Example 50 and using methyl 4-{[4-(4-chloro-3-cyanophenyl)-3,5-dimethyl-1H-pyrazol-1-yl]methyl}benzoate synthesized in Example 48 as a starting material.
¹H-NMR (CDCl₃) δ: 2.18 (3H, s), 2.22 (3H, s), 5.38 (2H, s), 7.28 (2H, d), 7.66 (1H, dd), 7.75 - 7.80 (1H, m), 7.90 - 7.95 (3H, s), 12.95 (1H, s).

### Example 65

### 4-{[4-(4-cyanophenyl)-3,5-dimethyl-1H-pyrazol-1-yl]methyl}benzamide

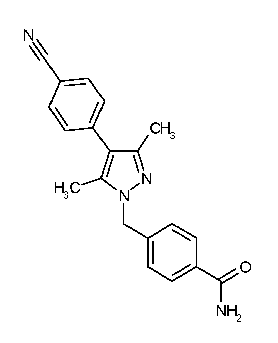

A solution of 4-{[4-(4-cyanophenyl)-3,5-dimethyl-1H-pyrazol-1-yl]methyl}benzoic acid (92 mg) synthesized in Example 50, WSC (80 mg) and HOBt ammonium salt (63 mg) in DMF (5 mL) was stirred at room temperature for 20 hr. The reaction solution was neutralized with 1 mol/L hydrochloric acid, and extracted with ethyl acetate. The ethyl acetate layer was washed with water, dried over anhydrous magnesium sulfate and concentrated. The residue was washed with diethyl ether and filtrated to give the title compound (53 mg) as a solid.
¹H-NMR (DMSO-d₆) δ: 2.20 (3H, s), 2.24 (3H, s), 5.35 (2H, s), 7.24 (2H, d), 7.35 (1H, s), 7.51 (2H, d), 7.81-7.89 (4H, m), 7.93 (1H, s).

### Example 66

### 4-{[4-(3-chloro-4-cyanophenyl)-3,5-dimethyl-1H-pyrazol-1-yl]methyl}-1H-pyrazole-5-carboxamide

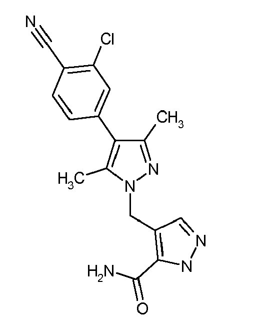

The title compound was obtained in the same manner as in Example 65 and using 4-{[4-(3-chloro-4-cyanophenyl)-3,5-dimethyl-1H-pyrazol-1-yl]methyl}-1H-pyrazole-5-carboxylic acid synthesized in Example 51 as a starting material.
¹H-NMR (DMSO-d₆) δ: 2.18 (3H, s), 2.30 (3H, s), 5.42 (2H, s), 7.27 (1H, s), 7.42 - 7.51 (2H, m), 7.55 (1H, s), 7.65 (1H, d), 7.98 (1H, d), 13.19 (1H, s).

### Example 67

### 2-chloro-5-{[4-(3-chloro-4-cyanophenyl)-3,5-dimethyl-1H-pyrazol-1-yl]methyl}nicotinamide

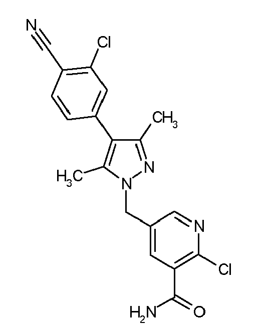

The title compound was obtained in the same manner as in Example 65 and using 2-chloro-5-{[4-(3-chloro-4-cyanophenyl)-3,5-dimethyl-1H-pyrazol-1-yl]methyl}nicotinic acid synthesized in Example 6 as a starting material.
¹H-NMR (DMSO-d₆) δ: 2.19 (3H, s), 2.32 (3H, s), 5.38 (2H, s), 7.48 (1H, dd), 7.68 (1H, d), 7.73 (1H, d), 7.79 (1H, s), 7.99 (1H, d), 8.06 (1H, s), 8.34 (1H, d).

### Example 68

### 3-{[4-(3-chloro-4-cyanophenyl)-3,5-dimethyl-1H-pyrazol-1-yl]methyl}benzamide

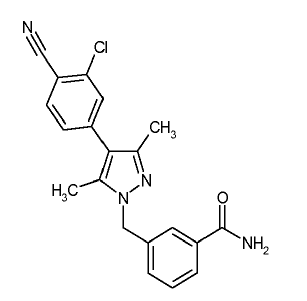

The title compound was obtained in the same manner as in Example 65 and using 3-{[4-(3-chloro-4-cyanophenyl)-3,5-dimethyl-1H-pyrazol-1-yl]methyl}benzoic acid synthesized in Example 56 as a starting material.
¹H-NMR (CDCl₃) δ: 2.21 (3H, s), 2.29 (3H, s), 5.32 (2H, s), 5.60 (1H, brs), 6.05 (1H, brs), 7.22-7.27 (1H, m), 7.30 (1H, d), 7.39 (1H, d), 7.43 (1H, t), 7.63-7.74 (3H, m).

### Example 69

### 4-{[4-(3-chloro-4-cyanophenyl)-3,5-dimethyl-1H-pyrazol-1-yl]methyl}benzamide

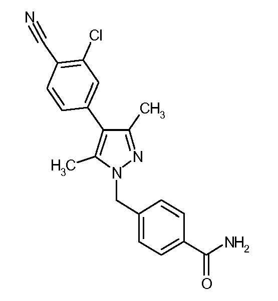

The title compound was obtained in the same manner as in Example 65 and using 4-{[4-(3-chloro-4-cyanophenyl)-3,5-dimethyl-1H-pyrazol-1-yl]methyl}benzoic acid synthesized in Example 52 as a starting material.
¹H-NMR (CDCl₃) δ: 2.19 (3H, s), 2.30 (3H, s), 5.34 (2H, s), 5.61 (1H, brs), 5.98 (1H, brs), 7.18-7.27 (3H, m), 7.39 (1H, d), 7.70 (1H, d), 7.79 (2H, d).

### Example 70

### 2-{[4-(4-cyanophenyl)-3,5-dimethyl-1H-pyrazol-1-yl]methyl}-1,3-thiazole-4-carboxamide

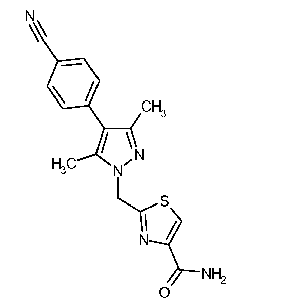

The title compound was obtained in the same manner as in Example 65 and using 2-{[4-(4-cyanophenyl)-3,5-dimethyl-1H-pyrazol-1-yl]methyl}-1,3-thiazole-4-carboxylic acid synthesized in Example 57 as a starting material.
¹H-NMR (CDCl₃) δ: 2.29 (3H, s), 2.32 (3H, s), 5.54 (2H, s), 5.57 (1H, brs), 7.08 (1H, brs), 7.36 (2H, d), 7.70 (2H, d), 8.13 (1H, s).

### Example 71

### 2-chloro-5-{[4-(3-chloro-4-cyanophenyl)-3,5-dimethyl-1H-pyrazol-1-yl]methyl}benzamide

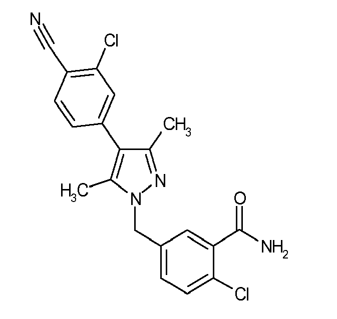

The title compound was obtained in the same manner as in Example 65 and using 2-chloro-5-{[4-(3-chloro-4-cyanophenyl)-3,5-dimethyl-1H-pyrazol-1-yl]methyl}benzoic acid synthesized in Example 58 as a starting material.
¹H-NMR (CDCl₃) δ: 2.21 (3H, s), 2.28 (3H, s), 5.27 (2H, s), 5.87 (1H, brs), 6.41 (1H, brs), 7.17 (1H, dd), 7.24 (1H, dd), 7.39 (1H, d), 7.40 (1H, d), 7.67 (1H, d), 7.69 (1H, d).

### Example 72

### 2-{[4-(3-chloro-4-cyanophenyl)-3,5-dimethyl-1H-pyrazol-1-yl]methyl}benzamide

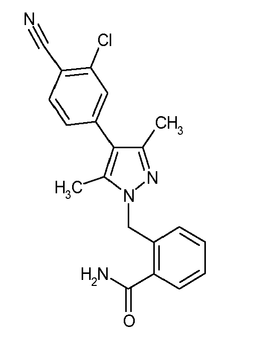

The title compound was obtained in the same manner as in Example 65 and using 2-{[4-(3-chloro-4-cyanophenyl)-3,5-dimethyl-1H-pyrazol-1-yl]methyl}benzoic acid synthesized in Example 55 as a starting material.
¹H-NMR (CDCl₃) δ: 2.33 (3H, s), 2.36 (3H, s), 5.48 (2H, s), 5.74 (1H, brs), 6.91-6.99 (1H, m), 7.22-7.29 (1H, m), 7.32-7.45 (3H, m), 7.61-7.75 (3H, m).

### Example 73

### 5-{[4-(3-chloro-4-cyanophenyl)-3,5-dimethyl-1H-pyrazol-1-yl]methyl}-2-furamide

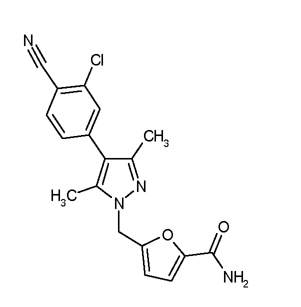

The title compound was obtained in the same manner as in Example 65 and using 5-{[4-(3-chloro-4-cyanophenyl)-3,5-dimethyl-1H-pyrazol-1-yl]methyl}-2-furoic acid synthesized in Example 61 as a starting material.
¹H-NMR (CDCl₃) δ: 2.26 (3H, s), 2.33 (3H, s), 5.27 (2H, s), 5.50 (1H, brs), 6.19 (1H, brs), 6.38 (1H, d), 7.11 (1H, d), 7.24 (1H, dd), 7.38 (1H, d), 7.70 (1H, d).

### Example 74

### 4-{[4-(3-chloro-4-cyanophenyl)-3,5-dimethyl-1H-pyrazol-1-yl]methyl}-2-fluorobenzamide

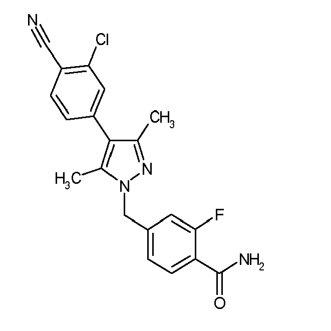

The title compound was obtained in the same manner as in Example 65 and using 4-{[4-(3-chloro-4-cyanophenyl)-3,5-dimethyl-1H-pyrazol-1-yl]methyl}-2-fluorobenzoic acid synthesized in Example 62 as a starting material.
¹H-NMR (CDCl₃) δ: 2.21 (3H, s), 2.30 (3H, s), 5.32 (2H, s), 5.74 (1H, brs), 6.64 (1H, brs), 6.88 (1H, d), 7.06 (1H, d), 7.23-7.29 (1H, m), 7.40 (1H, s), 7.71 (1H, d), 8.12 (1H, t).

### Example 75

### 4-{[4-(3-chloro-4-cyanophenyl)-3,5-dimethyl-1H-pyrazol-1-yl]methyl}-2-cyanobenzamide

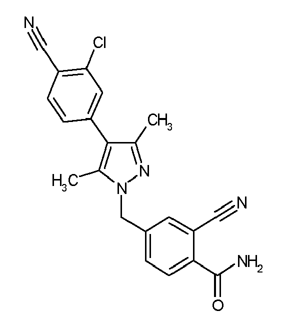

The title compound was obtained in the same manner as in Example 65 and using 4-{[4-(3-chloro-4-cyanophenyl)-3,5-dimethyl-1H-pyrazol-1-yl]methyl}-2-cyanobenzoic acid synthesized in Example 63 as a starting material.
¹H-NMR (CDCl₃) δ: 2.22 (3H, s), 2.30 (3H, s), 5.35 (2H, s), 5.86 (1H, brs), 6.29 (1H, brs), 7.26 (1H, dd), 7.41 (1H, d), 7.46-7.51 (2H, m), 7.72 (1H, d), 7.93 (1H, d).

### Example 76

### 4-{1-[4-(3-chloro-4-cyanophenyl)-3,5-dimethyl-1H-pyrazol-1-yl]ethyl}benzamide

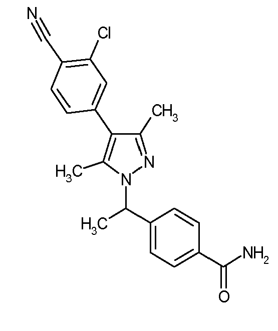

The title compound was obtained in the same manner as in Example 65 and using 4-{1-[4-(3-chloro-4-cyanophenyl)-3,5-dimethyl-1H-pyrazol-1-yl]ethyl}benzoic acid synthesized in Example 49 as a starting material.
¹H-NMR (CDCl₃) δ: 1.96 (3H, d), 2.15 (3H, s), 2.32 (s, 3H), 5.45 (1H, q), 5.57 (1H, brs), 6.05 (1H, brs), 7.20-7.28 (3H, m), 7.37 (1H, d), 7.68 (1H, d), 7.78 (2H, d).

### Example 77

### 5-{[4-(3-chloro-4-cyanophenyl)-3,5-dimethyl-1H-pyrazol-1-yl]methyl}-2-fluorobenzamide

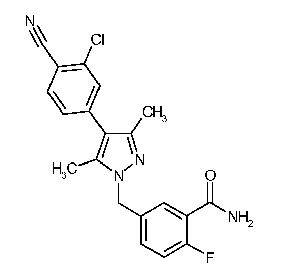

The title compound was obtained in the same manner as in Example 65 and using 5-{[4-(3-chloro-4-cyanophenyl)-3,5-dimethyl-1H-pyrazol-1-yl]methyl}-2-fluorobenzoic acid synthesized in Example 64 as a starting material.
¹H-NMR (CDCl₃) δ: 2.22 (3H, s), 2.28 (3H, s) 5.29 (2H, s), 5.79 (1H, brs), 5.79 (1H, brs), 7.13 (1H, dd), 7.24 (1H, dd), 7.28-7.32 (1H, m), 7.39 (1H, d), 7.69 (1H, d), 8.00 (1H, dd).

### Example 78

### 4-{[4-(3-chloro-4-cyanophenyl)-3,5-dimethyl-1H-pyrazol-1-yl]methyl}-N-methylbenzamide

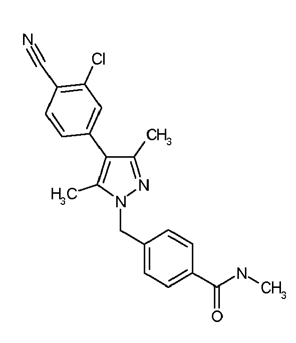

4-{[4-(3-Chloro-4-cyanophenyl)-3,5-dimethyl-1H-pyrazol-1-yl]methyl}benzoic acid (129.4 mg) synthesized in Example 52 was dissolved in THF (1.8 mL), ice-cooled, and thionyl chloride (0.13 mL) and DMF (9.2 µL) were added. The reaction mixture was stirred at room temperature for 30 min and concentrated to give 4-{[4-(3-chloro-4-cyanophenyl)-3,5-dimethyl-1H-pyrazol-1-yl]methyl}benzoyl chloride (136.2 mg). The obtained 4-{[4-(3-chloro-4-cyanophenyl)-3,5-dimethyl-1H-pyrazol-1-yl]methyl}benzoyl chloride (68.0 mg) was dissolved in THF (1.77 mL), 2 mol/L methylamine THF solution was added under ice-cooling. The reaction mixture was stirred at the same temperature for 5 min. After stirring, the mixture was poured into water, and extracted with ethyl acetate. The organic layer was washed with saturated brine, dried over anhydrous magnesium sulfate and concentrated. The obtained residue was purified by column chromatography (hexane-ethyl acetate), and recrystallized from ethyl acetate-heptane to give the title compound (55.2 mg) as colorless crystals.
¹H-NMR (DMSO-d₆) δ: 2.21 (3H, s), 2.26 (3H, s) 2.77 (3H, d), 5.35 (2H, s), 7.25 (2H, d), 7.48 (1H, dd), 7.67 (1H, d), 7.79 (2H, d), 7.99 (1H, d), 8.39 (1H, d).

### Example 79

### 4-{[4-(3-chloro-4-cyanophenyl)-3,5-dimethyl-1H-pyrazol-1-yl]methyl}-N,N-dimethylbenzamide

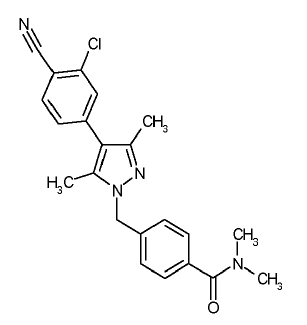

The title compound (46.1 mg) was obtained in the same manner as in Example 78 and using, as starting materials, 2 mol/L dimethylamine-THF solution and 4-{[4-(3-chloro-4-cyanophenyl)-3,5-dimethyl-1H-pyrazol-1-yl]methyl}benzoyl chloride (68.0 mg) prepared in the same manner as in Example 78 from 4-{[4-(3-chloro-4-cyanophenyl)-3,5-dimethyl-1H-pyrazol-1-yl]methyl}benzoic acid synthesized in Example 52. ¹H-NMR (DMSO-d₆) δ: 2.21 (3H, s), 2.27 (3H, s) 2.89 (3H, s), 2.96 (3H, s), 5.35 (2H, s), 7.23 (2H, d), 7.38 (2H, d), 7.48 (1H, dd), 7.68 (1H, d), 7.98 (1H, d).

### Example 80

### 2-{[4-(3-chloro-4-cyanophenyl)-3,5-dimethyl-1H-pyrazol-1-yl]methyl}-N-methylbenzamide

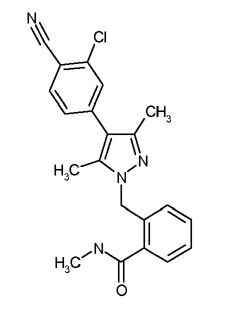

2-{[4-(3-Chloro-4-cyanophenyl)-3,5-dimethyl-1H-pyrazol-1-yl]methyl}-N-methylbenzoic acid (125.6 mg) synthesized in Example 55 and 2 mol/L methylamine THF solution (0.21 mL) were dissolved in THF (1.7 mL), and WSC (79.2 mg) was added. The reaction mixture was stirred at room temperature for 25 min. After stirring, the mixture was poured into 1 mol/L hydrochloric acid and extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate) and recrystallized from ethyl acetate-heptane to give the title compound (57.8 mg) as colorless crystals.
¹H-NMR (CDCl₃) δ: 2.24 (3H, s), 2.35 (3H, s), 3.01 (3H, d), 5.41 (2H, s), 6.87-6.98 (1H, m), 7.21-7.30 (1H, m), 7.33-7.39 (2H, m), 7.40 (1H, d), 7.47 (1H, brs), 7.53-7.59 (m, 1H), 7.70 (1H, d).

### Example 81

### 2-{[4-(3-chloro-4-cyanophenyl)-3,5-dimethyl-1H-pyrazol-1-yl]methyl}-N,N-dimethylbenzamide

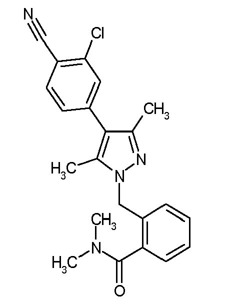

The title compound was obtained in the same manner as in Example 80 and using 2-{[4-(3-chloro-4-cyanophenyl)-3,5-dimethyl-1H-pyrazol-1-yl]methyl}benzoic acid synthesized in Example 55 and 2 mol/L dimethylamine THF solution as starting materials.
¹H-NMR (CDCl₃) δ: 2.25 (3H, s), 2.26 (3H, s), 2.79 (3H, s), 3.05 (3H, s), 5.30 (2H, s), 6.97-7.03 (1H, m), 7.20-7.28 (2H, m), 7.31-7.36 (2H, m), 7.39 (1H, d), 7.69 (1H, d).

### Example 82

### 3-{[4-(3-chloro-4-cyanophenyl)-3,5-dimethyl-1H-pyrazol-1-yl]methyl}-N,N-dimethylbenzamide

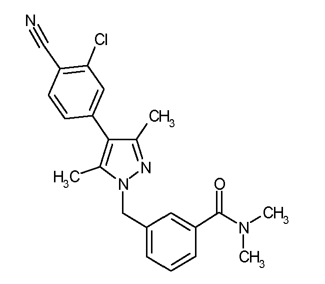

The title compound was obtained in the same manner as in Example 80 and using 3-{[4-(3-chloro-4-cyanophenyl)-3,5-dimethyl-1H-pyrazol-1-yl]methyl}benzoic acid synthesized in Example 56 and 2 mol/L dimethylamine THF solution as starting materials.
¹H-NMR (CDCl₃) δ: 2.20 (3H, s), 2.29 (3H, s), 2.95 (3H, s), 3.10 (3H, s), 5.30 (2H, s), 7.16 (1H, d), 7.21-7.29 (2H, m), 7.32-7.36 (1H, m), 7.36-7.41 (2H, m), 7.69 (1H, d).

### Example 83

### 5-{[4-(3-chloro-4-cyanophenyl)-3,5-dimethyl-1H-pyrazol-1-yl]methyl}-N,N-dimethylpyridine-2-carboxamide

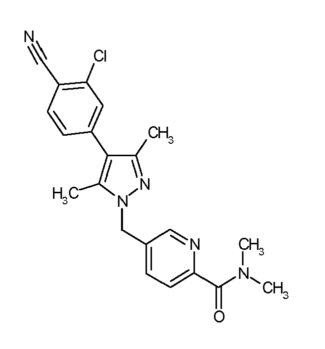

5-{[4-(3-Chloro-4-cyanophenyl)-3,5-dimethyl-1H-pyrazol-1-yl]methyl}pyridine-2-carboxylic acid (purity 75%, containing sodium chloride as impurity, 128.4 mg) synthesized in Example 54 was suspended in THF (1.3 mL), and thionyl chloride (95.8 µL) and DMF (6.8 µL) were added. The reaction mixture was stirred at room temperature for 20 min. After stirring, the mixture was concentrated to remove excess thionyl chloride. The obtained residue was dissolved in THF (1.3 mL), 2 mol/L dimethylamine THF solution was added, and the mixture was stirred at room temperature for 30 min. The reaction mixture was poured into saturated aqueous sodium hydrogen carbonate, and extracted with ethyl acetate. The organic layer was washed with saturated brine, dried over anhydrous magnesium sulfate and concentrated. The obtained residue was purified by column chromatography (ethyl acetate-methanol), and recrystallized from ethyl acetate-hexane to give the title compound (45.7 mg) as colorless crystals.
¹H-NMR (CDCl₃) δ: 2.24 (3H, s), 2.29 (3H, s), 3.09 (3H, s), 3.14 (3H, s), 5.33 (2H, s), 7.22-7.26 (1H, m), 7.39 (1H, d), 7.54-7.60 (1H, m), 7.62-7.67 (1H, m), 7.70 (1H, d), 8.41 (1H, d).

### Example 84

### 5-{[4-(3-chloro-4-cyanophenyl)-3,5-dimethyl-1H-pyrazol-1-yl]methyl}-N,N-dimethylnicotinamide

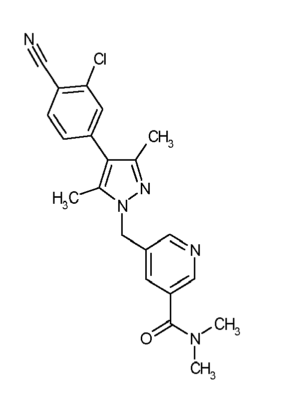

The title compound was obtained in the same manner as in Example 83 and using 5-{[4-(3-chloro-4-cyanophenyl)-3,5-dimethyl-1H-pyrazol-1-yl]methyl}nicotinic acid synthesized in Example 270 and 2 mol/L dimethylamine THF solution as starting materials.
¹H-NMR (CDCl₃) δ: 2.27 (3H, s), 2.28 (3H, s), 3.03 (3H, s), 3.15 (3H, s), 5.41 (2H, s), 7.21-7.28 (1H, m), 7.40 (1H, d), 7.72 (1H, d), 7.94 (1H, s), 8.55 (1H, s), 8.68 (1H, s).

### Example 85

### 2-chloro-4-{3,5-dimethyl-1-[2-(morpholin-4-ylcarbonyl)benzyl]-1H-pyrazol-4-yl}benzonitrile

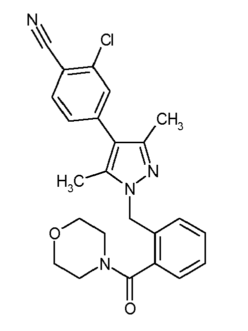

The title compound was obtained in the same manner as in Example 80 and using 2-{[4-(3-chloro-4-cyanophenyl)-3,5-dimethyl-1H-pyrazol-1-yl]methyl}benzoic acid synthesized in Example 55 and morpholine as starting materials.
¹H-NMR (CDCl₃) δ: 2.26 (3H, s), 2.27 (3H, s), 3. 18-3.83 (8H, m), 5.33 (2H, s), 7.04 (1H, dd), 7.19-7.30 (2H, m), 7.32-7.45 (3H, m), 7.70 (1H, d).

### Example 86

### 2-chloro-4-{3,5-dimethyl-1-[3-(morpholin-4-ylcarbonyl)benzyl]-1H-pyrazol-4-yl}benzonitrile

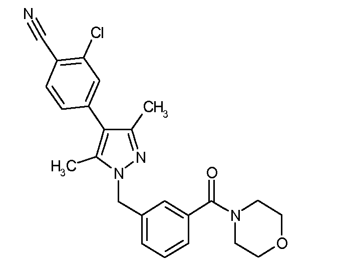

The title compound was obtained in the same manner as in Example 80 and using 3-{[4-(3-chloro-4-cyanophenyl)-3,5-dimethyl-1H-pyrazol-1-yl]methyl}benzoic acid synthesized in Example 56 and morpholine as starting materials.
¹H-NMR (CDCl₃) δ: 2.26 (3H, s), 2.27 (3H, s), 3. 18-3.83 (8H, m), 5.33 (2H, s), 7.04 (1H, dd), 7.19-7.30 (2H, m), 7.32-7.45 (3H, m), 7.70 (1H, d).

### Example 87

### 2-chloro-4-(3,5-dimethyl-1-{[5-(morpholin-4-ylcarbonyl)pyridin-3-yl]methyl}-1H-pyrazol-4-yl)benzonitrile

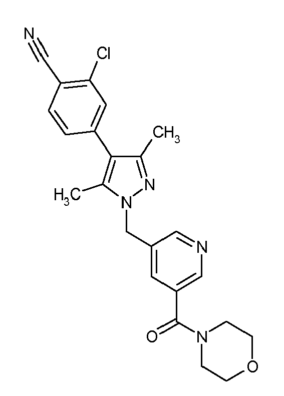

The title compound was obtained in the same manner as in Example 83 and using 5-{[4-(3-chloro-4-cyanophenyl)-3,5-dimethyl-1H-pyrazol-1-yl]methyl}nicotinic acid synthesized in Example 270 and morpholine as starting materials.
¹H-NMR (CDCl₃) δ: 2.25 (3H, s), 2.27 (3H, s), 3.31-3.91 (8H, m), 5.32 (2H, s), 7.17-7.30 (1H, m), 7.39 (1H, d), 7.60 (1H, s), 7.71 (1H, d), 8.51 (1H, s), 8.60 (1H, s).

### Example 88

### 2-chloro-4-(3,5-dimethyl-1-{[6-(morpholin-4-ylcarbonyl)pyridin-3-yl]methyl}-1H-pyrazol-4-yl)benzonitrile

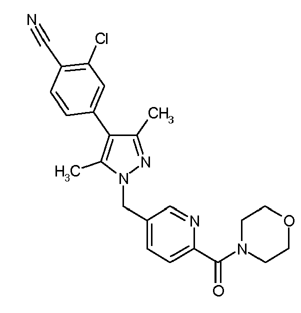

The title compound was obtained in the same manner as in Example 83 and using 5-{[4-(3-chloro-4-cyanophenyl)-3,5-dimethyl-1H-pyrazol-1-yl]methyl}pyridine-2-carboxylic acid synthesized in Example 54 and morpholine as starting materials.
¹H-NMR (CDCl₃) δ: 2.24 (3H, s), 2.28 (3H, s), 3.65-3.71 (4H, m), 3.78-3.83 (4H, m), 5.33 (2H, s), 7.17-7.30 (1H, m), 7.39 (1H, d), 7.57-7.62 (1H, m), 7.67-7.73 (2H, d), 8.40 (1H, d).

### Example 89

### 5-{[4-(3-chloro-4-cyanophenyl)-3,5-dimethyl-1H-pyrazol-1-yl]methyl}-N-methylnicotinamide

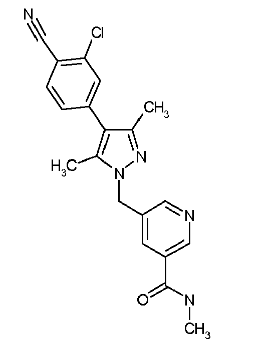

The title compound was obtained in the same manner as in Example 83 and using 5-{[4-(3-chloro-4-cyanophenyl)-3,5-dimethyl-1H-pyrazol-1-yl]methyl}nicotinic acid synthesized in Example 270 and 2 mol/L methylamine THF solution as starting materials.
¹H-NMR (CDCl₃) δ: 2.25 (3H, s), 2.28 (3H, s), 3.04 (3H, d), 5.34 (2H, s), 6.32 (1H, brs), 7.20-7.28 (1H, m), 7.39 (1H, d), 7.70 (1H, d), 7.92-8.10 (1H, m), 8.49-8.66 (1H, m), 8.89-8.98 (1H, m).

### Example 90

### 2-chloro-4-{3,5-dimethyl-1-[4-(morpholin-4-ylcarbonyl)benzyl]-1H-pyrazol-4-yl}benzonitrile

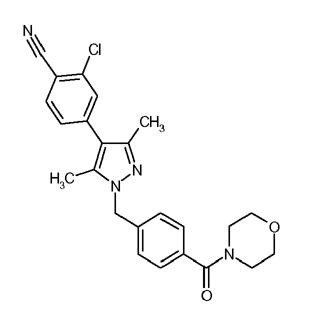

The title compound was obtained in the same manner as in Example 78 and using, as starting materials, 4-{[4-(3-chloro-4-cyanophenyl)-3,5-dimethyl-1H-pyrazol-1-yl]methyl}benzoyl chloride prepared in the same manner as in Example 78 from 4-{[4-(3-chloro-4-cyanophenyl)-3,5-dimethyl-1H-pyrazol-1-yl]methyl}benzoic acid synthesized in Example 52, and morpholine.
¹H-NMR (CDCl₃) δ: 2.20 (3H, s), 2.30 (3H, s), 3.23-4.01 (8H, m), 5.31 (2H, s), 7.13-7.29 (3H, m), 7.34-7.42 (3H, m), 7.69 (1H, d).

### Example 91

### 4-{[4-(3-chloro-4-cyanophenyl)-3,5-dimethyl-1H-pyrazol-1-yl]methyl}-N-(2-hydroxyethyl)benzamide

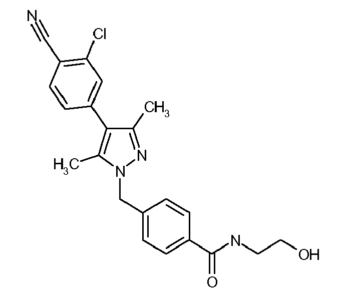

The title compound was obtained in the same manner as in Example 78 and using, as starting materials, 4-{[4-(3-chloro-4-cyanophenyl)-3,5-dimethyl-1H-pyrazol-1-yl]methyl}benzoyl chloride prepared in the same manner as in Example 78 from 4-{[4-(3-chloro-4-cyanophenyl)-3,5-dimethyl-1H-pyrazol-1-yl]methyl}benzoic acid synthesized in Example 52, and 2-aminoethanol.
¹H-NMR (CDCl₃) δ: 2.19 (3H, s), 2.30 (3H, s), 3.54-3.70 (2H, m), 3.79-3.90 (2H, m), 5.33 (2H, s), 7.18-7.25 (3H, m), 7.39 (1H, m), 7.69 (1H, d), 7.76 (2H, d).

### Example 92

### 3-{[4-(3-chloro-4-cyanophenyl)-3,5-dimethyl-1H-pyrazol-1-yl]methyl}-N-methylbenzamide

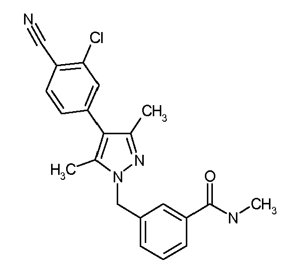

The title compound was obtained in the same manner as in Example 80 and using 3-{[4-(3-chloro-4-cyanophenyl)-3,5-dimethyl-1H-pyrazol-1-yl]methyl}benzoic acid synthesized in Example 56 and 2 mol/L methylamine THF solution as starting materials.
¹H-NMR (CDCl₃) δ: 2.21 (3H, s), 2.29 (3H, s), 3.01 (3H, d), 5.31 (2H, s), 6.14 (1H, brs), 7.20-7.30 (2H, m), 7.35-7.46 (2H, m), 7.61-7.66 (2H, m), 7.69 (1H, d).

### Example 93

### 5-{[4-(3-chloro-4-cyanophenyl)-3,5-dimethyl-1H-pyrazol-1-yl]methyl}-N-methyl-2-furamide

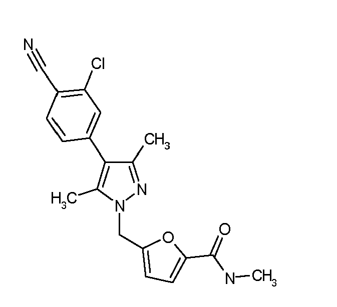

5-{[4-(3-Chloro-4-cyanophenyl)-3,5-dimethyl-1H-pyrazol-1-yl]methyl}-2-furoic acid (344.5 mg) synthesized in Example 61, WSC (57.0 mg) and HOBt (26.8 mg) were dissolved in DMF (1 mL), and 2 mol/L methylamine THF solution (0.15 mL) was added. The reaction mixture was stirred at room temperature for 3.5 hr, poured into 1 mol/L hydrochloric acid, and the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate), and recrystallized from ethyl acetate-hexane to give the title compound (54.2 mg) as colorless crystals.
¹H-NMR (CDCl₃) δ: 2.27 (3H, s), 2.32 (3H, s), 2.97 (3H, d), 5.25 (2H, s), 6.31 (1H, brs), 6.36 (1H, d), 7.04 (1H, d), 7.24 (1H, dd), 7.38 (1H, d), 7.70 (1H, d).

### Example 94

### 4-{[4-(3-chloro-4-cyanophenyl)-3,5-dimethyl-1H-pyrazol-1-yl]methyl}-2-fluoro-N-methylbenzamide

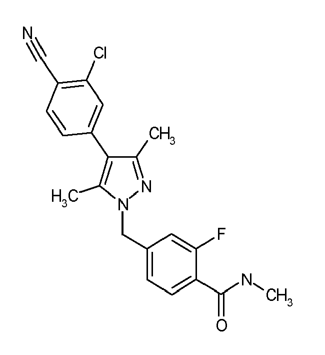

The title compound was obtained in the same manner as in Example 93 and using 4-{[4-(3-chloro-4-cyanophenyl)-3,5-dimethyl-1H-pyrazol-1-yl]methyl}-2-fluorobenzoic acid synthesized in Example 74 as a starting material.
¹H-NMR (CDCl₃) δ: 2.22 (3H, s), 2.32 (3H, s), 3.05 (3H, dd), 5.32 (2H, s), 6.70 (1H, brs), 6.87 (1H, dd), 7.06 (1H, dd), 7.28 (1H, dd), 7.42 (1H, d), 7.72 (1H, d), 8.12 (1H, t).

### Example 95

### 2-chloro-5-{[4-(3-chloro-4-cyanophenyl)-3,5-dimethyl-1H-pyrazol-1-yl]methyl}-N-methylnicotinamide

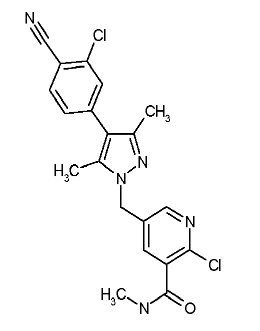

A mixture of 2-chloro-5-{[4-(3-chloro-4-cyanophenyl)-3,5-dimethyl-1H-pyrazol-1-yl]methyl}nicotinic acid synthesized in Example 6, N,N-carbonyldiimidazole (3 mL) and THF (1 mL) was stirred at 0°C for 30 min, 1 mol/L methylamine THF solution (1 mL) was added and the mixture was stirred at the same temperature for 2 hr. The reaction mixture was poured into saturated brine, and the mixture was extracted with ethyl acetate. The ethyl acetate layer was washed with water, dried over anhydrous magnesium sulfate and concentrated. The residue was purified by column chromatography (hexane-ethyl acetate) to give the title compound (26 mg) as a solid.
¹H-NMR (CDCl₃) δ: 2.24 (3H, s), 2.26 (3H, s), 3.05 (3H, d), 5.29 (2H, s), 6.51 (1H, s), 7.24 (1H, dd), 7.38 (1H, d), 7.70 (1H, d), 7.97 (1H, d), 8.30 (1H, d).

### Example 96

### 2-chloro-4-(1-{[6-chloro-5-(pyrrolidin-1-ylcarbonyl)pyridin-3-yl]methyl}-3,5-dimethyl-1H-pyrazol-4-yl)benzonitrile

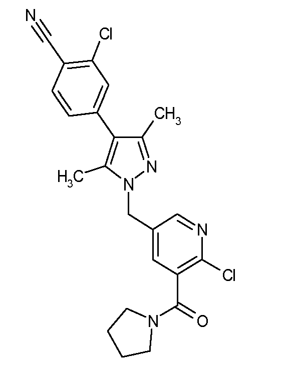

The title compound was obtained in the same manner as in Example 95 and using 2-chloro-5-{[4-(3-chloro-4-cyanophenyl)-3,5-dimethyl-1H-pyrazol-1-yl]methyl}nicotinic acid synthesized in Example 6 and pyrrolidine as starting materials.
¹H-NMR (CDCl₃) δ: 1.89 - 2.03 (4H, m), 2.23 (3H, s), 2.27 (3H, s), 3.19 - 3.27 (2H, m), 3.61 - 3.69 (2H, m), 5.28 (2H, s), 7.23 (1H, dd), 7.38 (1H, d), 7.54 (1H, d), 7.71 (1H, d), 8.27 (1H, d).

### Example 97

### 2-{[4-(3-chloro-4-cyanophenyl)-3,5-dimethyl-1H-pyrazol-1-yl]methyl}-N-methyl-1,3-thiazole-4-carboxamide

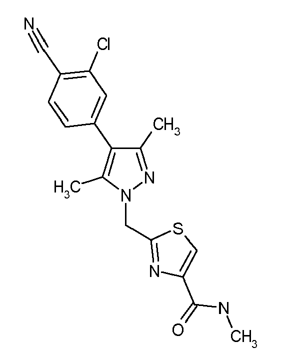

The title compound was obtained in the same manner as in Example 78 and using, as starting materials, 2 mol/L methylamine THF solution and 2-{[4-(3-chloro-4-cyanophenyl)-3,5-dimethyl-1H-pyrazol-1-yl]methyl}-1,3-thiazole-4-carbonyl chloride prepared in the same manner as in Example 78 from 2-{[4-(3-chloro-4-cyanophenyl)-3,5-dimethyl-1H-pyrazol-1-yl]methyl}-1,3-thiazole-4-carboxylic acid synthesized in Reference Example 53.
¹H-NMR (CDCl₃) δ: 2.29 (3H, s), 2.32 (3H, s), 3.01 (3H, dd), 5.52 (2H, s), 7.22-7.28 (1H, m), 7.39 (1H, d), 7.70 (1H, d), 8.08 (1H, s).

### Example 98

### 2-{[4-(3-chloro-4-cyanophenyl)-3,5-dimethyl-1H-pyrazol-1-yl]methyl}-1,3-thiazole-4-carboxamide

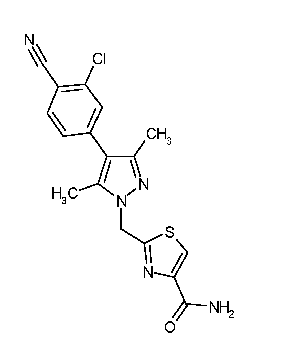

The title compound was obtained in the same manner as in Example 78 and using, as starting materials, 2-{[4-(3-chloro-4-cyanophenyl)-3,5-dimethyl-1H-pyrazol-1-yl]methyl}-1,3-thiazole-4-carbonyl chloride prepared in the same manner as in Example 78 from 2-{[4-(3-chloro-4-cyanophenyl)-3,5-dimethyl-1H-pyrazol-1-yl]methyl}-1,3-thiazole-4-carboxylic acid synthesized in Example 53, and 28% aqueous ammonia.
¹H-NMR (CDCl₃) δ: 2.29 (3H, s), 2.33 (3H, s), 5.54 (2H, s), 5.60 (1H, brs), 7.07 (1H, brs), 7.20-7.29 (1H, m), 7.39 (1H, d), 7.71 (1H, d), 8.14 (1H, s).

### Example 99

### 2-chloro-4-{3,5-dimethyl-1-[4-(pyrrolidin-1-ylcarbonyl)benzyl]-1H-pyrazol-4-yl}benzonitrile

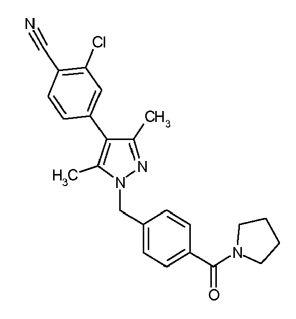

The title compound was obtained in the same manner as in Example 78 and using, as starting materials, 4-{[4-(3-chloro-4-cyanophenyl)-3,5-dimethyl-1H-pyrazol-1-yl]methyl}benzoyl chloride prepared in the same manner as in Example 78 from 4-{[4-(3-chloro-4-cyanophenyl)-3,5-dimethyl-1H-pyrazol-1-yl]methyl}benzoic acid synthesized in Example 52, and pyrrolidine.
¹H-NMR (CDCl₃) δ: 1.78-2.00 (4H, m), 2.19 (3H, s), 2.30 (3H, s), 3.31-3.48 (2H, m), 3.54-3.72 (2H, m), 5.30 (2H, s), 7.02-7.30 (3H, m), 7.36-7.41 (1H, m), 7.49 (2H, d), 7.69 (1H, d).

### Example 100

### 2-chloro-4-{3,5-dimethyl-1-[4-(piperidin-1-ylcarbonyl)benzyl]-1H-pyrazol-4-yl}benzonitrile

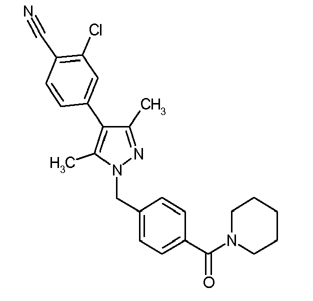

The title compound was obtained in the same manner as in Example 78 and using, as starting materials, 4-{[4-(3-chloro-4-cyanophenyl)-3,5-dimethyl-1H-pyrazol-1-yl]methyl}benzoyl chloride prepared in the same manner as in Example 78 from 4-{[4-(3-chloro-4-cyanophenyl)-3,5-dimethyl-1H-pyrazol-1-yl]methyl}benzoic acid synthesized in Example 52, and piperidine.
¹H-NMR (CDCl₃) δ: 1.45-1.54 (2H, m), 1.61-1.72 (4H, m), 2.20 (3H, s), 2.30 (3H, s), 3.25-3.42 (2H, m), 3.61-3.79 (2H, m), 5.30 (2H, s), 7.15 (2H, d), 7.25 (1H, m), 7.37 (2H, d), 7.40 (1H, d), 7.69 (1H, d).

### Example 101

### 2-{[4-(3-chloro-4-cyanophenyl)-3,5-dimethyl-1H-pyrazol-1-yl]methyl}-N,N-dimethyl-1,3-thiazole-4-carboxamide

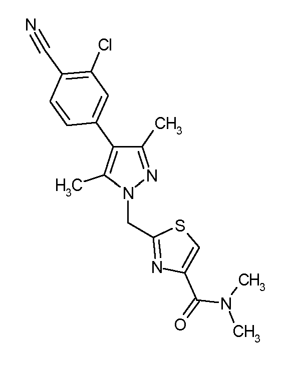

The title compound was obtained in the same manner as in Example 78 and using, as starting materials, 2 mol/L dimethylamine THF solution and 2-{[4-(3-chloro-4-cyanophenyl)-3,5-dimethyl-1H-pyrazol-1-yl]methyl}-1,3-thiazole-4-carbonyl chloride prepared in the same manner as in Example 78 from 2-{[4-(3-chloro-4-cyanophenyl)-3,5-dimethyl-1H-pyrazol-1-yl]methyl}-1,3-thiazole-4-carboxylic acid synthesized in Example 53.
¹H-NMR (CDCl₃) δ: 2.28 (3H, s), 2.32 (3H, s), 3.11 (3H, s), 3.24 (3H, s), 5.55 (2H, s), 7.22-7.27 (1H, m), 7.70 (2H, d), 7.84 (1H, s).

### Example 102

### 5-{[4-(3-chloro-4-cyanophenyl)-3,5-dimethyl-1H-pyrazol-1-yl]methyl}nicotinamide

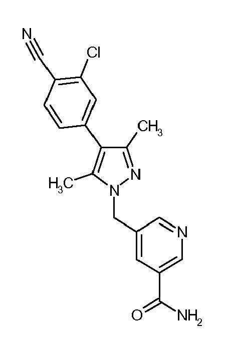

The title compound was obtained in the same manner as in Example 83 and using 5-{[4-(3-chloro-4-cyanophenyl)-3,5-dimethyl-1H-pyrazol-1-yl]methyl}nicotinic acid synthesized in Example 270 and aqueous ammonia as starting materials.
¹H-NMR (CDCl₃) 8:, 2.26 (3H, s), 2.28 (3H, s), 5.35 (2H, s), 5.74 (1H, brs), 6.17 (1H, brs), 7.24 (1H, dd), 7.39 (1H, d), 7.70 (1H, d), 8.05 (1H, dd), 8.61 (1H, dd), 8.95 (1H, d).

### Example 103

### 2-chloro-5-{[4-(3-chloro-4-cyanophenyl)-3,5-dimethyl-1H-pyrazol-1-yl]methyl}-N-methylbenzamide

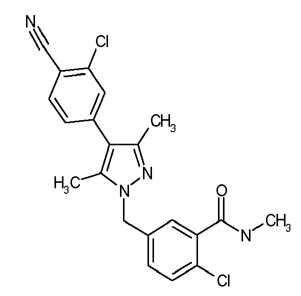

The title compound was obtained in the same manner as in Example 78 and using, as starting materials, 2 mol/L methylamine THF solution and 2-chloro-5-{[4-(3-chloro-4-cyanophenyl)-3,5-dimethyl-1H-pyrazol-1-yl]methyl}benzoyl chloride prepared in the same manner as in Example 78 from 2-chloro-5-{[4-(3-chloro-4-cyanophenyl)-3,5-dimethyl-1H-pyrazol-1-yl]methyl}benzoic acid synthesized in Example 58.
¹H-NMR (CDCl₃) δ: 2.21 (3H, s), 2.28 (3H, s), 3.03 (3H, d), 5.25 (2H, s), 6.27 (1H, brs), 7.13 (1H, dd), 7.22-7.26 (1H, m), 7.33-7.41 (2H, m), 7.53 (1H, d), 7.69 (1H, d).

### Example 104

### 5-{[4-(3-chloro-4-cyanophenyl)-3,5-dimethyl-1H-pyrazol-1-yl]methyl}-2-fluoro-N-methylbenzamide

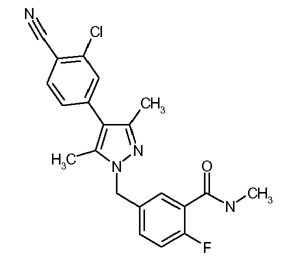

The title compound was obtained in the same manner as in Example 78 and using, as starting materials, 2 mol/L methylamine THF solution and 5-{[4-(3-chloro-4-cyanophenyl)-3,5-dimethyl-1H-pyrazol-1-yl]methyl}-2-fluorobenzoyl chloride prepared in the same manner as in Example 78 from 5-{[4-(3-chloro-4-cyanophenyl)-3,5-dimethyl-1H-pyrazol-1-yl]methyl}-2-fluorobenzoic acid synthesized in Example 59.
¹H-NMR (CDCl₃) δ: 2.21 (3H, s), 2.28 (3H, s), 3.04 (3H, dd), 5.29 (2H, s), 6.74 (1H, brs), 7.08 (1H, d), 7.12 (1H, d), 7.22-7.26 (1H, m), 7.38 (1H, d), 7.69 (1H, d), 7.97 (1H, dd).

### Example 105

### 5-{[4-(3-chloro-4-cyanophenyl)-3,5-dimethyl-1H-pyrazol-1-yl]methyl}pyridine-2-carboxamide

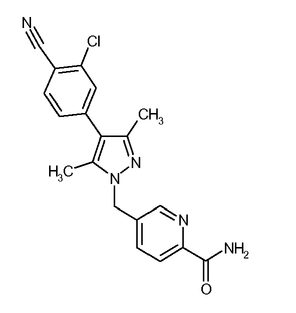

The title compound was obtained in the same manner as in Example 93 and using 5-{[4-(3-chloro-4-cyanophenyl)-3,5-dimethyl-1H-pyrazol-1-yl]methyl}pyridine-2-carboxylic acid synthesized in Example 54 as a starting material.
¹H-NMR (CDCl₃) δ: 2.24 (3H, s), 2.29 (3H, s), 5.36 (2H, s), 5.52 (1H, brs), 7.22-7.27 (1H, m), 7.39 (1H, d), 7.64 (1H, dd), 7.70 (1H, d), 7.78 (1H, brs), 8.19 (1H, d), 8.41 (1H, d).

### Example 106

### 5-{[4-(3-chloro-4-cyanophenyl)-3,5-dimethyl-1H-pyrazol-1-yl]methyl}-N-methylpyridine-2-carboxamide

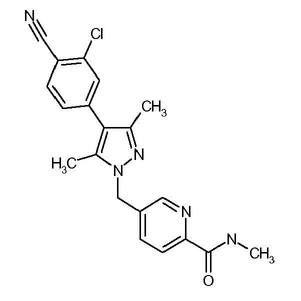

The title compound was obtained in the same manner as in Example 83 and using 5-{[4-(3-chloro-4-cyanophenyl)-3,5-dimethyl-1H-pyrazol-1-yl]methyl}pyridine-2-carboxylic acid synthesized in Example 54 and 2 mol/L methylamine THF solution as starting materials.
¹H-NMR (CDCl₃) δ: 2.23 (3H, s), 2.29 (3H, s), 3.03 (3H, d), 5.35 (2H, s), 7.22-7.28 (m, 1H), 7.39 (1H, d), 7.62 (1H, dd), 7.70 (1H, d), 7.94 (1H, brs), 8.18 (1H, d), 8.38 (1H, dd).

### Example 107

### 4-{[4-(4-cyanophenyl)-3,5-dimethyl-1H-pyrazol-1-yl]methyl}-1H-pyrazole-5-carboxamide

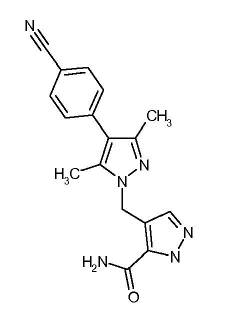

A mixture containing ethyl 4-{[4-(4-cyanophenyl)-3,5-dimethyl-1H-pyrazol-1-yl]methyl}-1-trityl-1H-pyrazole-5-carboxylate was obtained in the same manner as in Example 1 and using 4-(3,5-dimethyl-1H-pyrazol-4-yl)benzonitrile (0.21 g) synthesized in Reference Example 5 and ethyl 4-(iodomethyl)-1-trityl-1H-pyrazole-3-carboxylate (0.52 g) synthesized in Reference Example 20 as starting materials. TFA (2 mL) was added to the mixture obtained above, and the mixture was stirred at room temperature for 20 hr. The reaction solution was neutralized with saturated aqueous sodium hydrogen carbonate solution, and extracted with ethyl acetate. The ethyl acetate layer was washed with water, dried over anhydrous magnesium sulfate and concentrated. The residue was suspended in diethyl ether and filtrated to give ethyl 4-{[4-(4-cyanophenyl)-3,5-dimethyl-1H-pyrazol-1-yl]methyl}-1H-pyrazole-5-carboxylate (99 mg). 4-{[4-(4-Cyanophenyl)-3,5-dimethyl-1H-pyrazol-1-yl]methyl}-1H-pyrazole-5-carboxylic acid (73 mg) was obtained in the same manner as in Example 50 and using ethyl 4-{[4-(4-cyanophenyl)-3,5-dimethyl-1H-pyrazol-1-yl]methyl}-1H-pyrazole-5-carboxylate (90 mg) as a starting material. The title compound (17 mg) was obtained as a solid in the same manner as in Example 65 and using 4-{[4-(4-cyanophenyl)-3,5-dimethyl-1H-pyrazol-1-yl]methyl}-1H-pyrazole-5-carboxylic acid (71 mg) as a starting material.
¹H-NMR (DMSO-d₆) δ: 2.17 (3H, s), 2.29 (3H, s), 5.41 (2H, s), 7.29 (1H, s), 7.45 - 7.53 (3H, m), 7.56 (1H, s), 7.86 (2H, d) 13.20 (1H, s).

### Example 108

### 2-{[4-(4-cyanophenyl)-3,5-dimethyl-1H-pyrazol-1-yl]methyl}-1,3-thiazole-4-carbonitrile

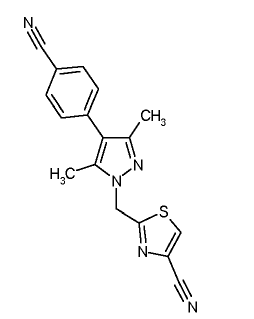

2-{[4-(4-Cyanophenyl)-3,5-dimethyl-1H-pyrazol-1-yl]methyl}-1,3-thiazole-4-carboxamide (90.9 mg) synthesized in Example 70 and pyridine (32.6 µL) were dissolved in DMF (1.3 mL), cooled in an ice bath, and oxalyl chloride (28.2 µL) was added dropwise. The reaction mixture was stirred at the same temperature for 1 hr, water was added to quench the reaction, and the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate), and recrystallized from ethyl acetate-hexane to give the title compound (34.1 mg) as colorless crystals.
¹H-NMR (CDCl₃) δ: 2.30 (3H, s), 2.33 (3H, s), 5.58 (2H, s), 7.37 (2H, d), 7.73 (2H, d), 8.00 (1H, s).

### Example 109

### 2-{[4-(3-chloro-4-cyanophenyl)-3,5-dimethyl-1H-pyrazol-1-yl]methyl}-1,3-thiazole-4-carbonitrile

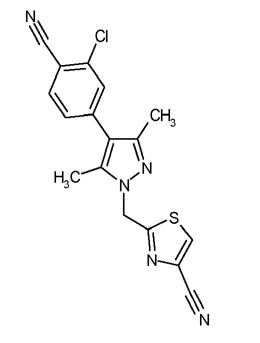

The title compound was obtained in the same manner as in Example 108 and using 2-{[4-(3-chloro-4-cyanophenyl)-3,5-dimethyl-1H-pyrazol-1-yl]methyl}-1,3-thiazole-4-carboxamide synthesized in Example 98 as a starting material.
¹H-NMR (CDCl₃) δ: 2.28 (3H, s), 2.33 (3H, s), 5.55 (2H, s), 7.22-7.26 (1H, m), 7.39 (1H, d), 7.71 (1H, d), 7.99 (1H, s).

### Example 110

### 2-chloro-4-{3,5-dimethyl-1-[(2-methyl-1,3-dioxo-2,3-dihydro-1H-isoindol-5-yl)methyl]-1H-pyrazol-4-yl}benzonitrile

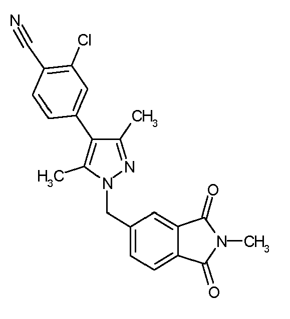

A mixture of 4-{[4-(3-chloro-4-cyanophenyl)-3,5-dimethyl-1H-pyrazol-1-yl]methyl}phthalic acid (940.3 mg) synthesized in Example 60 and acetic anhydride (11.5 mL) was stirred at 60°C for 15 min, concentrated and azeotroped with toluene. The obtained residue was crystallized from hexane to give 2-chloro-4-{1-[(1,3-dioxo-1,3-dihydro-2-benzofuran-5-yl)methyl]-3,5-dimethyl-1H-pyrazol-4-yl}benzonitrile (606.8 mg). The obtained 2-chloro-4-{1-[(1,3-dioxo-1,3-dihydro-2-benzofuran-5-yl)methyl]-3,5-dimethyl-1H-pyrazol-4-yl}benzonitrile (220.2 mg) was dissolved in THF (3 mL), and 2 mol/L methylamine THF solution (0.30 mL) was added. After stirring at room temperature for 5 min, acetic anhydride (0.11 mL) and triethylamine (0.16 mL) were added. The reaction mixture was stirred at 60°C for 20 min. After stirring, the mixture was poured into saturated aqueous ammonium chloride solution, and the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate) to give the title compound (145.8 mg) as a colorless powder.
¹H-NMR (CDCl₃) δ: 2.22 (3H, s), 2.30 (3H, s), 3.18 (3H, s), 5.40 (2H, s), 7.27 (1H, dd), 7.41 (1H, d), 7.49 (1H, dd), 7.58 (1H, d), 7.71 (1H, d), 7.83 (1H, d).

### Example 111

### N-(4-{[4-(3-chloro-4-cyanophenyl)-3,5-dimethyl-1H-pyrazol-1-yl]methyl}phenyl)cyclopropanecarboxamide

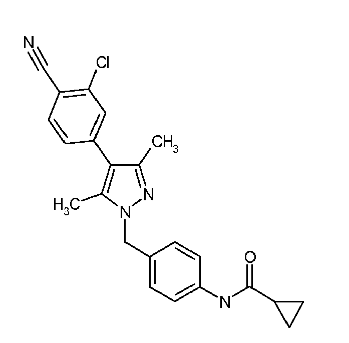

4-[1-(4-Aminobenzyl)-3,5-dimethyl-1H-pyrazol-4-yl]-2-chlorobenzonitrile (50.5 mg) synthesized in Example 114 and triethylamine (31.4 µL) were dissolved in THF (0.7 mL), and cyclopropanecarbonyl chloride (16.3 µL) was added. After stirring at room temperature for 10 min, it was poured into saturated aqueous ammonium chloride solution, and the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate), and recrystallized from ethyl acetate-methanol-hexane to give the title compound (145.8 mg) as colorless crystals.
¹H-NMR (CDCl₃) δ: 0.81-0.90 (2H, m), 1.04-1.13 (2H, m), 1.49 (1H, d), 2.19 (3H, s), 2.29 (3H, s), 5.23 (2H, s), 7.12 (2H, d), 7.23 (1H, dd), 7.38 (1H, d), 7.48 (2H, d), 7.68 (1H, d).

### Example 112

### N-(4-{[4-(3-chloro-4-cyanophenyl)-3,5-dimethyl-1H-pyrazol-1-yl]methyl}phenyl)-2,2-dimethylpropanamide

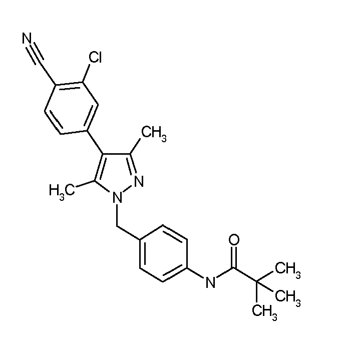

The title compound was obtained in the same manner as in Example 111 and using 4-[1-(4-aminobenzyl)-3,5-dimethyl-1H-pyrazol-4-yl]-2-chlorobenzonitrile synthesized in Example 114 and pivalic acid chloride as starting materials.
¹H-NMR (CDCl₃) δ: 1.31 (9H, s), 2.18 (3H, s), 2.29 (3H, s), 5.24 (2H, s), 7.13 (2H, d), 7.23 (1H, dd), 7.37 (1H, d), 7.50 (2H, d), 7.68 (1H, d).

### Example 113

### tert-butyl (4-{[4-(3-chloro-4-cyanophenyl)-3,5-dimethyl-1H-pyrazol-1-yl]methyl}phenyl)carbamate

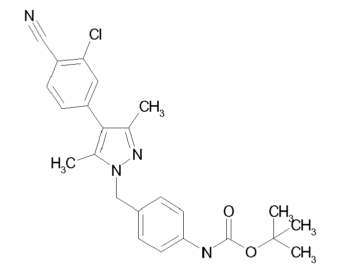

4-{[4-(3-Chloro-4-cyanophenyl)-3,5-dimethyl-1H-pyrazol-1-yl]methyl}benzoic acid (460.6 mg) synthesized in Example 52 and triethylamine (0.22 mL) were dissolved in toluene (5 mL), and diphenylphosphorylazide (0.31 mL) was added. After stirring at room temperature for 30 min, tert-butanol (0.35 mL) was added to the reaction mixture, and the mixture was stirred at 90°C for 2 hr. The reaction mixture was poured into saturated aqueous ammonium chloride solution, and the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate) to give the title compound (277.0 mg) as a colorless powder.
¹H-NMR (CDCl₃) δ: 1.51 (9H, d), 2.18 (3H, s), 2.29 (3H, s), 5.22 (2H, s), 6.46 (1H, brs), 7.10 (2H, d), 7.23 (1H, dd), 7.33 (2H, d), 7.37 (1H, d), 7.68 (1H, d).

### Example 114

### 4-[1-(4-aminobenzyl)-3,5-dimethyl-1H-pyrazol-4-yl]-2-chlorobenzonitrile

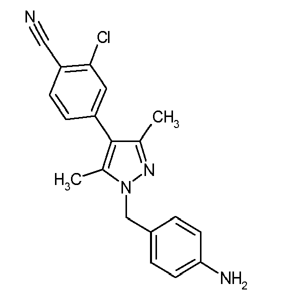

A mixture of tert-butyl (4-{[4-(3-chloro-4-cyanophenyl)-3,5-dimethyl-1H-pyrazol-1-yl]methyl}phenyl)carbamate (241.3 mg) synthesized in Example 113 and TFA (1 mL) was stirred at room temperature for 30 min. The reaction mixture was concentrated, neutralized with 1 mol/L aqueous sodium hydroxide solution, and the mixture was extracted with ethyl acetate. The organic layer was washed with saturated brine, dried over anhydrous magnesium sulfate and concentrated to give the title compound (186.2 mg) as a pale-yellow powder.
¹H-NMR (CDCl₃) δ: 2.20 (3H, s), 2.29 (3H, s), 5.17 (2H, s), 6.66 (2H, d), 7.00 (2H, d), 7.23 (1H, dd), 7.37 (1H, d), 7.67 (1H, d).

### Example 115

### 5-{[4-(3-chloro-4-cyanophenyl)-3,5-dimethyl-1H-pyrazol-1-yl]methyl}pyridine-2-carbonitrile

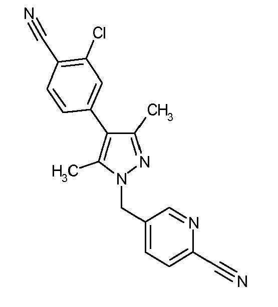

4-{1-[(6-Bromopyridin-3-yl)methyl]-3,5-dimethyl-1H-pyrazol-4-yl}-2-chlorobenzonitrile (103.2 mg) synthesized in Example 18 was dissolved in DMF (1 mL), and zinc cyanide and tetrakis(triphenylphosphine)palladium(0) were added under a nitrogen atmosphere. The reaction mixture was stirred under a nitrogen atmosphere at 100°C for 5 hr, poured into water, and the mixture was extracted with diethyl ether. The organic layer was washed with saturated brine, dried over anhydrous magnesium sulfate and concentrated. The obtained residue was purified by column chromatography (hexane-ethyl acetate), and recrystallized from ethyl acetate-hexane to give the title compound (23.0 mg) as colorless crystals.
¹H-NMR (CDCl₃) δ: 2.24 (3H, s), 2.27 (3H, s), 5.35 (2H, s), 7.20-7.27 (1H, m), 7.38 (1H, d), 7.58-7.63 (1H, m), 7.65-7.73 (2H, m), 8.56 (1H, s).

### Example 116

### 5-{[4-(4-cyanophenyl)-3,5-dimethyl-1H-pyrazol-1-yl]methyl}pyridine-2-carbonitrile

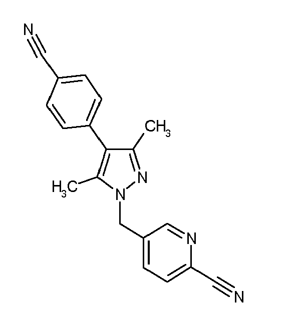

The title compound was obtained in the same manner as in Example 115 and using 4-{1-[(6-bromopyridin-3-yl)methyl]-3,5-dimethyl-1H-pyrazol-4-yl}benzonitrile synthesized in Example 25 as a starting material.
¹H-NMR (CDCl₃) δ: 2.23 (3H, s), 2.27 (3H, s), 5.36 (2H, s), 7.35 (2H, d), 7.58-7.63 (1H, m), 7.67 (1H, d), 7.71 (2H, d), 8.56 (1H, s).

### Example 117

### 5-{[4-(4-cyanophenyl)-3,5-dimethyl-1H-pyrazol-1-yl]methyl}nicotinonitrile

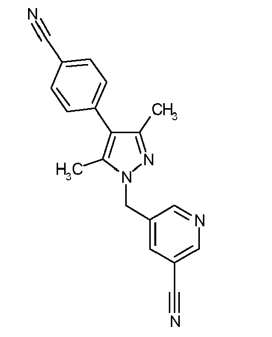

The title compound was obtained in the same manner as in Example 115 and using 4-{1-[(5-bromopyridin-3-yl)methyl]-3,5-dimethyl-1H-pyrazol-4-yl}benzonitrile synthesized in Example 26 as a starting material.
¹H-NMR (CDCl₃) δ: 2.24 (3H, s), 2.28 (3H, s), 5.34 (2H, s), 7.36 (2H, d), 7.71 (2H, d), 7.72-7.74 (1H, m), 8.69 (1H, d), 8.83 (1H, d).

### Example 118

### 5-{[4-(3-chloro-4-cyanophenyl)-3,5-dimethyl-1H-pyrazol-1-yl]methyl}nicotinonitrile

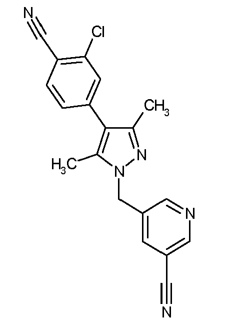

The title compound was obtained in the same manner as in Example 115 and using 4-{1-[(5-bromopyridin-3-yl)methyl]-3,5-dimethyl-1H-pyrazol-4-yl}-2-chlorobenzonitrile synthesized in Example 19 as a starting material.
¹H-NMR (CDCl₃) δ: 2.25 (3H, s), 2.29 (3H, s), 5.33 (2H, s), 7.22-7.28 (1H, m), 7.40 (1H, d), 7.66-7.75 (2H, m), 8.69 (1H, d), 8.83 (1H, d).

### Example 119

### N-(4-{[4-(3-chloro-4-cyanophenyl)-3,5-dimethyl-1H-pyrazol-1-yl]methyl}phenyl)acetamide

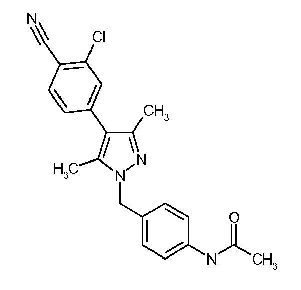

A mixture of 4-[1-(4-aminobenzyl)-3,5-dimethyl-1H-pyrazol-4-yl]-2-chlorobenzonitrile (50.2 mg) synthesized in Example 114, pyridine (0.50 mL) and acetic anhydride (0.25 mL) was stirred at room temperature for 1 hr. The reaction mixture was poured into 1 mol/L hydrochloric acid, and the mixture was extracted with ethyl acetate. The organic layer was washed with saturated aqueous sodium hydrogen carbonate and then saturated brine, dried over anhydrous magnesium sulfate and concentrated. The obtained residue was and recrystallized from ethyl acetate-methanol-hexane to give the title compound (36.4 mg) as colorless crystals.
¹H-NMR (CDCl₃) δ: 2.17 (3H, s), 2.19 (3H, s), 2.29 (3H, s), 5.24 (2H, s), 7.09-7.15 (3H, m), 7.24 (1H, dd), 7.38 (1H, d), 7.47 (2H, d), 7.68 (1H, d).

### Example 120

### Methyl 5-{[4-(3-chloro-4-cyanophenyl)-3,5-dimethyl-1H-pyrazol-1-yl]methyl}nicotinate

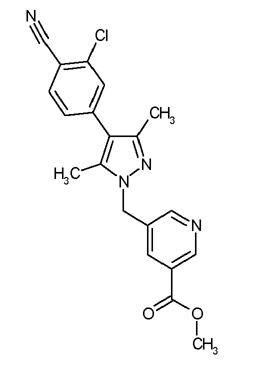

The title compound was obtained in the same manner as in Example 20 and using 4-{1-[(5-bromopyridin-3-yl)methyl]-3,5-dimethyl-1H-pyrazol-4-yl}-2-chlorobenzonitrile synthesized in Example 19 as a starting material.
¹H-NMR (CDCl₃) δ: 2.25 (3H, s), 2.28 (3H, s), 3.96 (3H, s), 5.34 (2H, s), 7.24 (1H, dd), 7.39 (1H, d), 7.70 (1H, d), 8.13 (1H, dd), 8.61 (1H, d), 9.16 (1H, d).

### Example 121

### 2-chloro-4-{1-[(1,3-dioxo-2,3-dihydro-1H-isoindol-5-yl)methyl]-3,5-dimethyl-1H-pyrazol-4-yl}benzonitrile

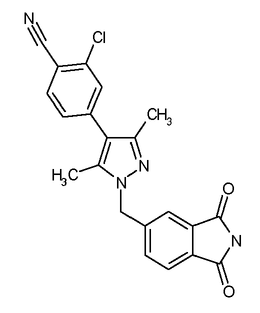

2-Chloro-4-{1-[(1,3-dioxo-1,3-dihydro-2-benzofuran-5-yl)methyl]-3,5-dimethyl-1H-pyrazol-4-yl}benzonitrile (150.0 mg) prepared by the method of Example 110 from 4-{[4-(3-chloro-4-cyanophenyl)-3,5-dimethyl-1H-pyrazol-1-yl]methyl}phthalic acid synthesized in Example 60 was dissolved in THF (2 mL), and 2,4-dimethoxybenzylamine (62.3 µL) was added. The mixture was stirred at room temperature for 1 hr, and acetic anhydride (72.4 µL) and triethylamine (0.10 mL) were added. The reaction mixture was stirred at 60°C for 3 hr, poured into saturated aqueous ammonium chloride solution, and the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate) to give a white powder (175.5 mg). The obtained compound (123.3 mg) was dissolved in acetonitrile-water (4:1) (1.2 mL), and cerium (IV) diammonium nitrate (624.8 mg) was added. The reaction mixture was stirred at room temperature for 1 hr, poured into water, and the mixture was extracted with ethyl acetate. The organic layer was washed successively with saturated aqueous sodium sulfite solution, saturated aqueous sodium hydrogen carbonate and saturated brine, dried over anhydrous magnesium sulfate and concentrated. The obtained residue was purified by column chromatography (hexane-ethyl acetate) and recrystallized from ethyl acetate-hexane to give the title compound (13.5 mg) as colorless crystals.
¹H-NMR (CDCl₃) δ: 2.23 (3H, s), 2.30 (3H, s), 5.42 (2H, s), 7.24-7.29 (1H, m), 7.41 (1H, d), 7.52-7.61 (2H, m), 7.71 (1H, d), 7.86 (1H, d).

### Example 122

### Methyl 4-{[4-(3-chloro-4-cyanophenyl)-3,5-dimethyl-1H-pyrazol-1-yl]methyl}-2-cyanobenzoate

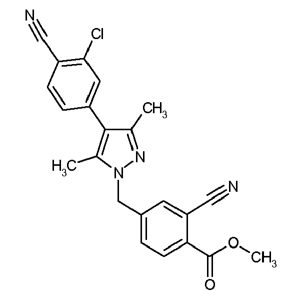

Methyl 2-bromo-4-{[4-(3-chloro-4-cyanophenyl)-3,5-dimethyl-1H-pyrazol-1-yl]methyl}benzoate (450.2 mg) synthesized in Example 45 was dissolved in NMP (5 mL), and copper (I) cyanide was added. The reaction mixture was stirred at 180°C for 1 hr, poured into water, 10% aqueous ammonia was added, and the mixture was extracted with ethyl acetate. The organic layer was washed with 10% aqueous ammonia and then saturated brine, dried over anhydrous magnesium sulfate and concentrated. The obtained residue was purified by column chromatography (hexane-ethyl acetate) to give the title compound (368.1 mg) as a colorless powder.
¹H-NMR (CDCl₃) δ: 2.21 (3H, s), 2.30 (3H, s), 4.00 (3H, s), 5.36 (2H, s), 7.27 (1H, dd), 7.41 (1H, d), 7.45 (1H, dd), 7.51 (1H, d), 7.72 (1H, d), 8.14 (1H, d).

### Example 123

### 2-chloro-4-{1-[(1,4-dioxo-1,2,3,4-tetrahydrophthalazin-6-yl)methyl]-3,5-dimethyl-1H-pyrazol-4-yl}benzonitrile

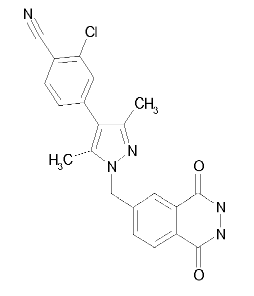

2-Chloro-4-{1-[(1,3-dioxo-1,3-dihydro-2-benzofuran-5-yl)methyl]-3,5-dimethyl-1H-pyrazol-4-yl}benzonitrile (120.5 mg) prepared by the method of Example 110 from 4-{[4-(3-chloro-4-cyanophenyl)-3,5-dimethyl-1H-pyrazol-1-yl]methyl}phthalic acid synthesized in Example 60 was dissolved in THF (1 mL), and hydrazine monohydrate was added. After stirring at room temperature for 17 hr, acetic acid (0.1 mL) was added and the mixture was further stirred with heating under reflux for 5 hr. The reaction mixture was poured into water, and the mixture was extracted with ethyl acetate. The organic layer was washed with saturated brine, dried over anhydrous magnesium sulfate and concentrated. The obtained residue was purified by column chromatography (ethyl acetate-methanol), and recrystallized from ethyl acetate-methanol-hexane to give the title compound (105.1 mg) as colorless crystals.
¹H-NMR (CDCl₃) δ: 2.29 (6H, s), 5.56 (2H, s), 7.45 (1H, dd), 7.61 (1H, d), 7.69 (1H, dd), 7.84 (1H, d), 7.90 (1H, d), 8.18 (1H, d).

### Example 124

### 2-chloro-4-(1-{[6-chloro-5-(1-hydroxy-1-methylethyl)pyridin-3-yl]methyl}-3,5-dimethyl-1H-pyrazol-4-yl)benzonitrile

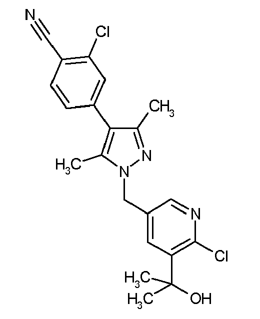

Ethyl 2-chloro-5-{[4-(3-chloro-4-cyanophenyl)-3,5-dimethyl-1H-pyrazol-1-yl]methyl}nicotinate (0.15 g) synthesized in Example 6 was dissolved in THF, and methylmagnesium bromide (3 mol/L diethyl ether solution, 0.6 mL) was added dropwise at 0°C. The reaction mixture was stirred at the same temperature for 2 hr, poured into ice water, and neutralized with citric acid. The reaction mixture was extracted with ethyl acetate, and the ethyl acetate layer was washed with water, dried over anhydrous magnesium sulfate and concentrated. The residue was purified by column chromatography (hexane-ethyl acetate), and crystallized from diethyl ether to give the title compound (30 mg) as colorless crystals.
¹H-NMR (CDCl₃) δ: 1.74 (6H, s), 2.25 (3H, s), 2.27 (3H, s), 5.28 (2H, s), 7.24 (1H, dd), 7.38 (1H, d), 7.70 (1H, d), 7.99 (1H, d), 8.08 (1H, d).

### Example 125

### 2-chloro-4-(1-{[5-(1-hydroxy-1-methylethyl)-1H-pyrazol-4-yl]methyl}-3,5-dimethyl-1H-pyrazol-4-yl)benzonitrile

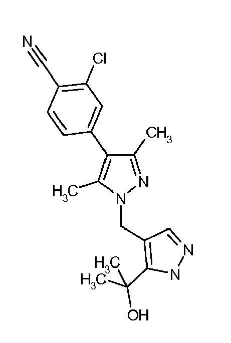

The title compound was obtained in the same manner as in Example 124 and using ethyl 4-{[4-(3-chloro-4-cyanophenyl)-3,5-dimethyl-1H-pyrazol-1-yl]methyl}-1H-pyrazole-5-carboxylate synthesized in Example 5 as a starting material.
¹H-NMR (CDCl₃) δ: 1.60 (6H, s), 2.23 (3H, s), 2.34 (3H, s), 5.31 (2H, s), 6.20 (1H, s), 7.20 (1H, dd), 7.35 (1H, d), 7.40 (1H, s), 7.68 (1H, d).

### Example 126

### 2-chloro-4-{1-[4-(1-hydroxy-1-methylethyl)benzyl]-3,5-dimethyl-1H-pyrazol-4-yl}benzonitrile

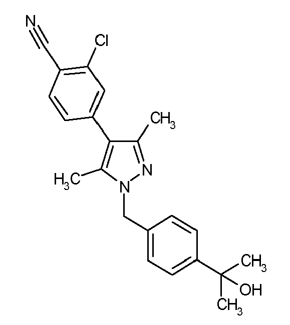

The title compound (45.2 mg) was obtained in the same manner as in Example 124 and using methyl 4-{[4-(3-chloro-4-cyanophenyl)-3,5-dimethyl-1H-pyrazol-1-yl]methyl}benzoate synthesized in Example 8 as a starting material.
¹H-NMR (CDCl₃) δ: 1.57 (6H, s), 2.21 (3H, s), 2.30 (3H, s) 5.27 (2H, s), 7.13 (2H, d), 7.21-7.28 (1H, m), 7.39 (1H, d), 7.46 (2H, d), 7.68 (1H, d).

### Example 127

### 2-chloro-4-(1-{[4-(1-hydroxy-1-methylethyl)-1,3-thiazol-2-yl]methyl}-3,5-dimethyl-1H-pyrazol-4-yl)benzonitrile

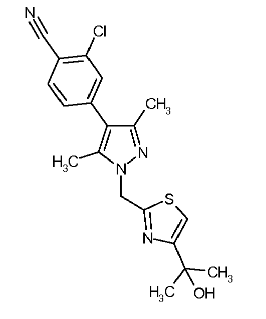

The title compound was obtained in the same manner as in Example 124 and using ethyl 2-{[4-(3-chloro-4-cyanophenyl)-3,5-dimethyl-1H-pyrazol-1-yl]methyl}-1,3-thiazole-4-carboxylate synthesized in Example 14 as a starting material.
¹H-NMR (CDCl₃) δ: 1.49 (6H, s), 2.20 (3H, s), 2.30 (3H, s) 5.52 (2H, s), 7.25 (1H, s), 7.36 (1H, dd), 7.50 (1H, d), 7.78 (1H, d).

### Example 128

### 2-chloro-4-{1-[2-(1-hydroxy-1-methylethyl)benzyl]-3,5-dimethyl-1H-pyrazol-4-yl}benzonitrile

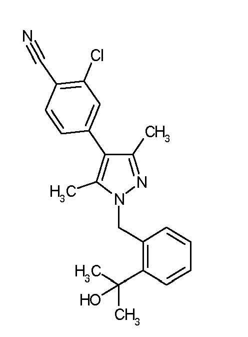

The title compound was obtained in the same manner as in Example 124 and using methyl 2-{[4-(3-chloro-4-cyanophenyl)-3,5-dimethyl-1H-pyrazol-1-yl]methyl}benzoate synthesized in Example 21 as a starting material.
¹H-NMR (CDCl₃) δ: 1.68 (6H, s), 2.24 (3H, s), 2.32 (3H, s) 4.98 (1H, s), 5.73 (2H, s), 6.90 (1H, dd), 7.13-7.31 (3H, m), 7.33-7.39 (2H, m), 7.68 (1H, d).

### Example 129

### 2-chloro-4-{1-[3-(1-hydroxy-1-methylethyl)benzyl]-3,5-dimethyl-1H-pyrazol-4-yl}benzonitrile

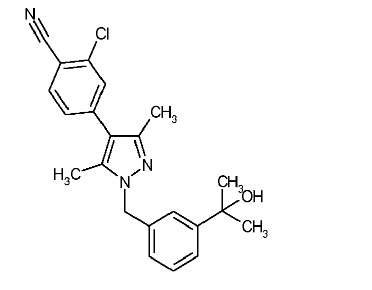

The title compound was obtained in the same manner as in Example 124 and using methyl 3-{[4-(3-chloro-4-cyanophenyl)-3,5-dimethyl-1H-pyrazol-1-yl]methyl}benzoate synthesized in Example 22 as a starting material. ¹H-NMR (CDCl₃) δ: 1.57 (6H, s), 1.70 (1H, s), 2.22 (3H, s), 2.30 (3H, s) 5.29 (2H, s), 6.97 (1H, d), 7.21-7.43 (5H, m), 7.36-7.69 (1H, d).

### Example 130

### 2-chloro-4-{1-[4-chloro-3-(1-hydroxy-1-methylethyl)benzyl]-3,5-dimethyl-1H-pyrazol-4-yl}benzonitrile

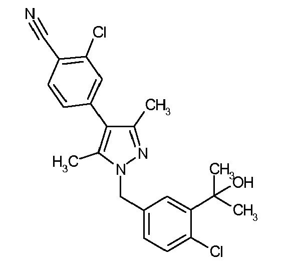

The title compound was obtained in the same manner as in Example 124 and using methyl 2-chloro-5-{[4-(3-chloro-4-cyanophenyl)-3,5-dimethyl-1H-pyrazol-1-yl]methyl}benzoate synthesized in Example 31 as a starting material.
¹H-NMR (CDCl₃) δ: 1.72 (6H, s), 2.22 (3H, s), 2.29 (3H, s), 5.25 (2H, s), 6.86-6.97 (1H, m), 7.22-7.28 (1H, m), 7.31 (1H, d), 7.39 (1H, d), 7.57 (1H, d), 7.69 (1H, d).

### Example 131

### 2-chloro-4-(1-{[6-chloro-5-(hydroxymethyl)pyridin-3-yl]methyl}-3,5-dimethyl-1H-pyrazol-4-yl)benzonitrile

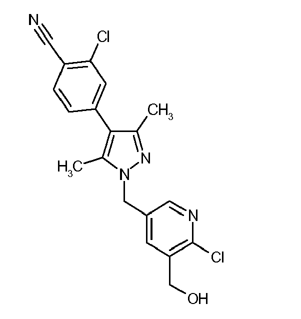

To a suspension of sodium borohydride (67 mg) and calcium chloride (198 mg) in THF (12 mL) was added ethanol (6 mL) at room temperature, and the mixture was stirred at room temperature for 10 min. Ethyl 2-chloro-5-{[4-(3-chloro-4-cyanophenyl)-3,5-dimethyl-1H-pyrazol-1-yl]methyl}nicotinate (0.38 g) synthesized in Example 6 was added to the above-mentioned mixture, and the mixture was stirred at room temperature for 24 hr. The reaction mixture was partitioned between saturated aqueous citric acid solution and ethyl acetate. The aqueous layer was extracted with ethyl acetate, and the ethyl acetate layer was washed with water, dried over anhydrous magnesium sulfate and concentrated. The residue was purified by column chromatography (hexane-ethyl acetate), and crystallized from diethyl ether to give the title compound (169 mg) as colorless crystals.
¹H-NMR (CDCl₃) δ: 2.25 (3H, s), 2.27 (3H, s), 4.77 (2H, s), 5.28 (2H, s), 7.23 (1H, dd), 7.38 (1H, d), 7.70 (1H, d), 7.75 - 7.79 (1H, m), 8.14 (1H, d).

### Example 132

### 5-[1-(4-fluorobenzyl)-3,5-dimethyl-1H-pyrazol-4-yl]-1-methyl-2-oxo-1,2-dihydropyridine-3-carbonitrile

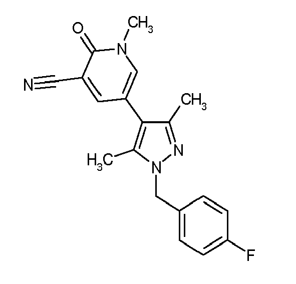

A mixture of 1-(4-fluorobenzyl)-3,5-dimethyl-4-(4,4,5,5-tetramethyl-1,3,2-dioxaborolan-2-yl)-1H-pyrazole (0.43 g) synthesized in Reference Example 9, 5-bromo-1-methyl-2-oxo-1,2-dihydropyridine-3-carbonitrile (0.28 g) synthesized in Reference Example 10, tetrakis(triphenylphosphine)palladium(0)(174 mg), sodium carbonate (0.34 g), 1,2-dimethoxyethane (15 mL) and water (3 mL) was stirred at 90°C for 4 hr. Water and ethyl acetate were added to the reaction mixture, and the organic layer was separated. The organic layer was washed with saturated brine, dried over anhydrous magnesium sulfate and concentrated. The residue was purified by column chromatography (hexane-ethyl acetate) to give the title compound (233 mg) as a solid.
¹H-NMR (CDCl₃) δ: 2.12 (3H, s), 2.21 (3H, s), 3.65 (3H, s), 5.22 (2H, s), 6.99 - 7.06 (2H, m), 7.09 - 7.16 (2H, m), 7.39 (1H, d), 7.71 (1H, d).

### Example 133

### 2-chloro-4-{1-[(2,4-dimethyl-1,3-thiazol-5-yl)methyl]-3,5-dimethyl-1H-pyrazol-4-yl}benzonitrile

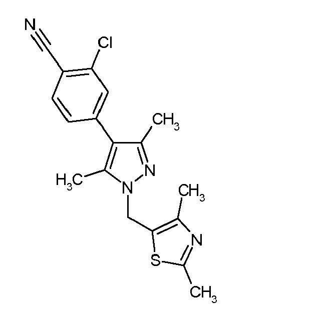

A resin reagent (PS-triphenylphosphine) (about 130 mg) was charged in a polypropylene tube equipped with a filter ·cap, and preconditioned therein. Then, a solution (0.42 mol/L, 0.357 mL) of (2,4-dimethyl-1,3-thiazol-5-yl)methanol in THF and a solution (0.28 mol/L, 0.357 mL) of 2-chloro-4-(3,5-dimethyl-1H-pyrazol-4-yl)benzonitrile synthesized in Reference Example 4 in THF were added and the mixture was stirred. di-tert-Butyl azodicarboxylate (about 60 mg) was added and the mixture was stirred at room temperature for 18 hr. The resin reagent was removed by filtering and washing the reaction mixture. The filtrate was concentrated. The residue was purified by reversed-phase preparative HPLC (Gilson, UniPoint system, YMC ODS column 30x75 mm, 0.1% TFA containing acetonitrile-water (5:95 - 100:0)) to give the title compound (26.3 mg).
MS (ESI+, m/e) 357 (M+1)
¹H-NMR (CD₃OD) δ: 2.28 (3H, s), 2.35 (3H, s), 2.48 (3H, s), 2.65 (3H, s), 5.45 (2H, s), 7.43 (1H, dd), 7.57 (1H, d), 7.87 (1H, d).

### Example 134

### 2-chloro-4-[1-(4-chloro-2-methylbenzyl)-3,5-dimethyl-1H-pyrazol-4-yl]benzonitrile

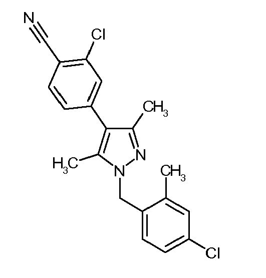

The title compound was obtained in the same manner as in Example 133 and using 4-chloro-2-methylbenzyl alcohol as a starting material.
MS (ESI+, m/e) 370 (M+1)
¹H-NMR (CD₃OD) δ: 2.27 (3H, s), 2.31 (3H, s), 2.40 (3H, s), 5.35 (2H, s), 6.57 (1H, d), 7.15-7.29 (2H, m), 7.48 (1H, dd), 7.63 (1H, d), 7.89 (1H, d).

### Example 135

### 2-chloro-4-(3,5-dimethyl-1-{[3-(trifluoromethyl)-1H-pyrazol-5-yl]methyl}-1H-pyrazol-4-yl)benzonitrile

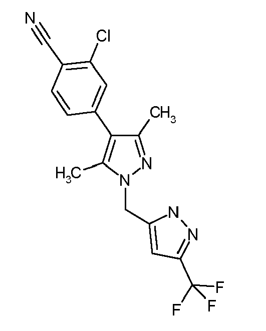

The title compound was obtained in the same manner as in Example 133 and using [3-(trifluoromethyl)-1H-pyrazol-5-yl]methanol as a starting material.
MS (ESI+, m/e) 380 (M+1)
¹H-NMR (CD₃OD) δ: 2.29 (3H, s), 2.37 (3H, s), 5.43 (2H, s), 6.56 (1H, s), 7.46 (1H, dd), 7.61 (1H, d), 7.87 (1H, d).

### Example 136

### 2-chloro-4-[1-(2-furylmethyl)-3,5-dimethyl-1H-pyrazol-4-yl]benzonitrile

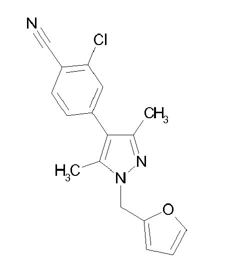

A resin reagent (PS-triphenylphosphine) (about 140 mg) was charged in a miniblock reaction apparatus (Bohdan), and preconditioned therein. Then, a solution (0.25 mol/L, 0.50 mL) of furfuryl alcohol in THF and a solution (0.20 mol/L, 0.50 mL) of 2-chloro-4-(3,5-dimethyl-1H-pyrazol-4-yl)benzonitrile synthesized in Reference Example 4 in THF were added and the mixture was stirred. A solution (0.42 mol/L, 0.50 mL) of di-tert-butyl azodicarboxylate in THF was added and the mixture was stirred at room temperature for 18 hr. The resin reagent was removed by filtering and washing the reaction mixture. The filtrate was concentrated. The residue was purified by reversed-phase preparative HPLC (Gilson, UniPoint system, YMC ODS column 30x75 mm, 0.1% TFA containing acetonitrile-water (5:95 - 100:0)) to give the title compound (9.2 mg).
MS (ESI+, m/e) 312 (M+1)

### Example 137

### 2-chloro-4-[1-(3-furylmethyl)-3,5-dimethyl-1H-pyrazol-4-yl]benzonitrile

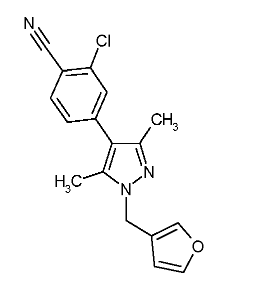

The title compound was obtained in the same manner as in Example 136 and using 3-furanmethanol as a starting material.
MS (ESI+, m/e) 312 (M+1)

### Example 138

### 2-chloro-4-[3,5-dimethyl-1-(tetrahydrofuran-2-ylmethyl)-1H-pyrazol-4-yl]benzonitrile

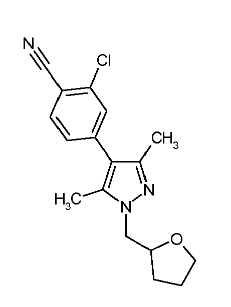

The title compound was obtained in the same manner as in Example 136 and using tetrahydrofurfuryl alcohol as a starting material.
MS (ESI+, m/e) 316 (M+1)

### Example 139

### 4-(1-benzyl-3,5-dimethyl-1H-pyrazol-4-yl)-2-chlorobenzonitrile

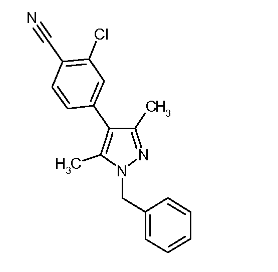

The title compound was obtained in the same manner as in Example 136 and using benzyl alcohol as a starting material.
MS (ESI+, m/e) 322 (M+1)

### Example 140

### 2-chloro-4-[3,5-dimethyl-1-(pyrazin-2-ylmethyl)-1H-pyrazol-4-yl]benzonitrile

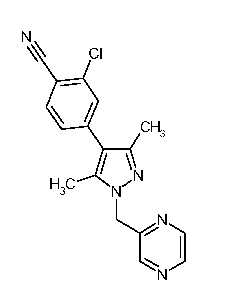

The title compound was obtained in the same manner as in Example 136 and using pyrazin-2-ylmethanol as a starting material.
MS (ESI+, m/e) 324 (M+1)

### Example 141

### 2-chloro-4-{3,5-dimethyl-1-[(1-methyl-1H-imidazol-5-yl)methyl]-1H-pyrazol-4-yl}benzonitrile

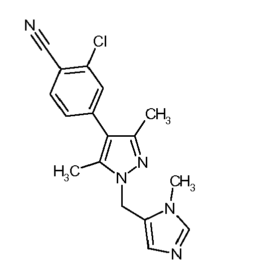

The title compound was obtained in the same manner as in Example 136 and using (1-methyl-1H-imidazol-5-yl)methanol as a starting material.
MS (ESI+, m/e) 326 (M+1)

### Example 142

### 2-chloro-4-[3,5-dimethyl-1-(2-thienylmethyl)-1H-pyrazol-4-yl]benzonitrile

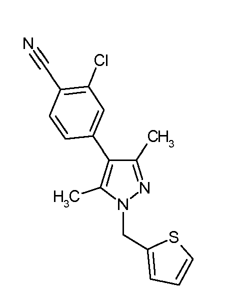

The title compound was obtained in the same manner as in Example 136 and using thiophene-2-methanol as a starting material.
MS (ESI+, m/e) 328 (M+1)

### Example 143

### 2-chloro-4-[3,5-dimethyl-1-(3-thienylmethyl)-1H-pyrazol-4-yl]benzonitrile

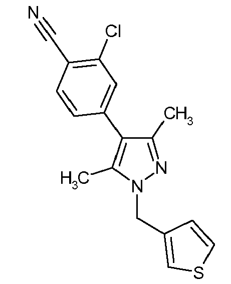

The title compound was obtained in the same manner as in Example 136 and using 3-thiophenemethanol as a starting material.
MS (ESI+, m/e) 328 (M+1)

### Example 144

### 2-chloro-4-[3,5-dimethyl-1-(1,3-thiazol-5-ylmethyl)-1H-pyrazol-4-yl]benzonitrile

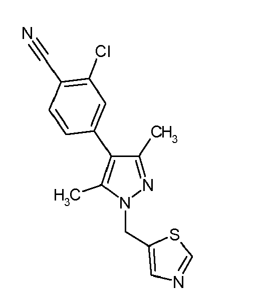

The title compound was obtained in the same manner as in Example 136 and using thiazole-5-methanol as a starting material.
MS (ESI+, m/e) 329 (M+1)

### Example 145

### 2-chloro-4-[3,5-dimethyl-1-(tetrahydro-2H-pyran-2-ylmethyl)-1H-pyrazol-4-yl]benzonitrile

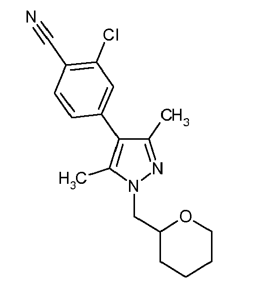

The title compound was obtained in the same manner as in Example 136 and using tetrahydropyran-2-methanol as a starting material.
MS (ESI+, m/e) 330 (M+1)

### Example 146

### 2-chloro-4-[3,5-dimethyl-1-(3-methylbenzyl)-1H-pyrazol-4-yl]benzonitrile

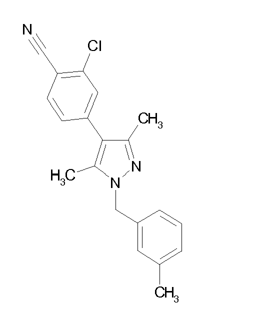

The title compound was obtained in the same manner as in Example 136 and using 3-methylbenzyl alcohol as a starting material.
MS (ESI+, m/e) 336 (M+1)

### Example 147

### 2-chloro-4-[3,5-dimethyl-1-(4-methylbenzyl)-1H-pyrazol-4-yl]benzonitrile

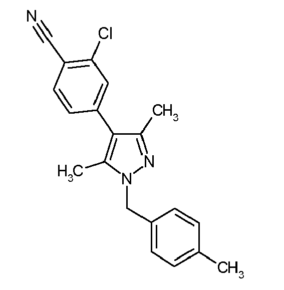

The title compound was obtained in the same manner as in Example 136 and using 4-methylbenzyl alcohol as a starting material.
MS (ESI+, m/e) 336 (M+1)

### Example 148

### 2-chloro-4-[3,5-dimethyl-1-(1-phenylethyl)-1H-pyrazol-4-yl]benzonitrile

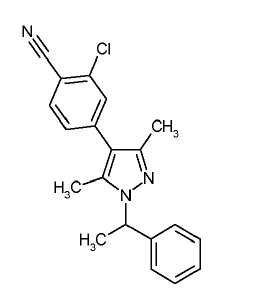

The title compound was obtained in the same manner as in Example 136 and using DL-1-phenylethanol as a starting material.
MS (ESI+, m/e) 336 (M+1)
¹H-NMR (CDCl₃) δ: 1.95 (3H, d), 2.15 (3H, s), 2.32 (3H, s), 5.43 (1H, q), 7.16-7.35 (5H, m), 7.23 (1H, dd), 7.37 (1H, d), 7.68 (1H, d).

### Example 149

### 2-chloro-4-[3,5-dimethyl-1-(1-phenylethyl)-1H-pyrazol-4-yl]benzonitrile

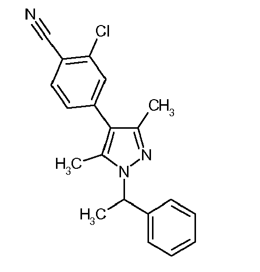

The title compound was obtained in the same manner as in Example 136 and using (S)-(-)-1-phenylethanol as a starting material.
MS (ESI+, m/e) 336 (M+1)

### Example 150

### 2-chloro-4-{3,5-dimethyl-1-[(6-methylpyridin-2-yl)methyl]-1H-pyrazol-4-yl}benzonitrile

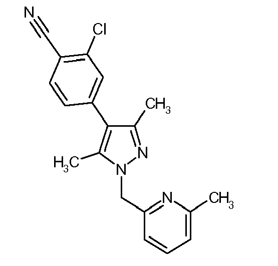

The title compound was obtained in the same manner as in Example 136 and using 6-methyl-2-pyridinemethanol as a starting material.
MS (ESI+, m/e) 337 (M+1)

### Example 151

### 2-chloro-4-{3,5-dimethyl-1-[(2-methylpyridin-3-yl)methyl]-1H-pyrazol-4-yl}benzonitrile

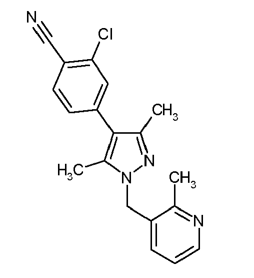

The title compound was obtained in the same manner as in Example 136 and using (2-methylpyridin-3-yl)-methanol as a starting material.
MS (ESI+, m/e) 337 (M+1)

### Example 152

### 2-chloro-4-[3,5-dimethyl-1-(1-pyridin-4-ylethyl)-1H-pyrazol-4-yl]benzonitrile

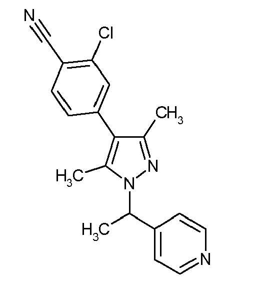

The title compound was obtained in the same manner as in Example 136 and using (+/-)-1-(4-pyridyl)ethanol as a starting material.
MS (ESI+, m/e) 337 (M+1)

### Example 153

### 2-chloro-4-[1-(3-fluorobenzyl)-3,5-dimethyl-1H-pyrazol-4-yl]benzonitrile

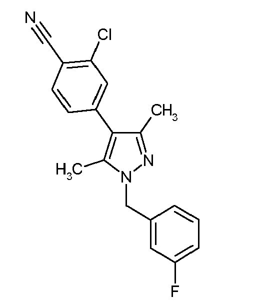

The title compound was obtained in the same manner as in Example 136 and using 3-fluorobenzyl alcohol as a starting material.
MS 340 (ESI+, m/e) (M+1)

### Example 154

### 2-chloro-4-{1-[(1,3-dimethyl-1H-pyrazol-5-yl)methyl]-3,5-dimethyl-1H-pyrazol-4-yl}benzonitrile

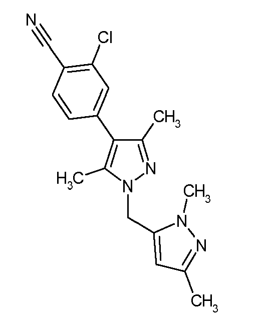

The title compound was obtained in the same manner as in Example 136 and using (1,3-dimethyl-1H-pyrazol-5-yl)methanol as a starting material.
MS (ESI+, m/e) 340 (M+1)

### Example 155

### 2-chloro-4-{1-[(1,5-dimethyl-1H-pyrazol-3-yl)methyl]-3,5-dimethyl-1H-pyrazol-4-yl)benzonitrile

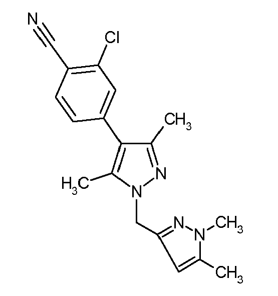

The title compound was obtained in the same manner as in Example 136 and using (1,5-dimethyl-1H-pyrazol-3-yl)methanol as a starting material.
MS (ESI+, m/e) 340 (M+1)

### Example 156

### 2-chloro-4-{1-[(2-fluoropyridin-4-yl)methyl]-3,5-dimethyl-1H-pyrazol-4-yl}benzonitrile

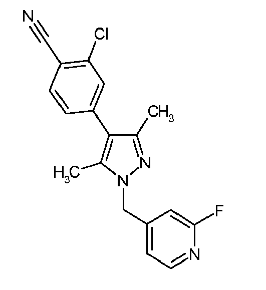

The title compound was obtained in the same manner as in Example 136 and using 2-fluoro-4-pyridinemethanol as a starting material.
MS (ESI+, m/e) 341 (M+1)

### Example 157

### 2-chloro-4-{1-[(3,5-dimethylisoxazol-4-yl)methyl]-3,5-dimethyl-1H-pyrazol-4-yl}benzonitrile

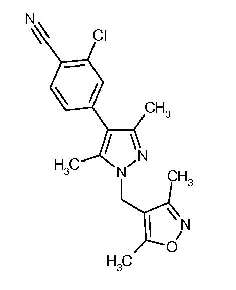

The title compound was obtained in the same manner as in Example 136 and using (3,5-dimethyl-4-isoxazolyl)methanol as a starting material.
MS (ESI+, m/e) 341 (M+1)

### Example 158

### 2-chloro-4-{3,5-dimethyl-1-[(1-methylpiperidin-3-yl)methyl]-1H-pyrazol-4-yl}benzonitrile

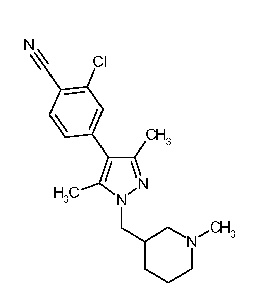

The title compound was obtained in the same manner as in Example 136 and using 1-methylpiperidine-3-methanol as a starting material.
MS (ESI+, m/e) 343 (M+1)

### Example 159

### 2-chloro-4-[1-(2-ethynylbenzyl)-3,5-dimethyl-1H-pyrazol-4-yl]benzonitrile

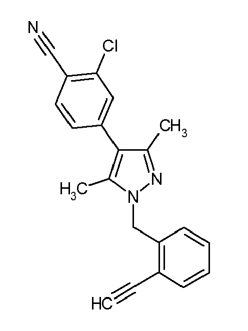

The title compound was obtained in the same manner as in Example 136 and using 2-ethynylbenzyl alcohol as a starting material.
MS (ESI+, m/e) 346 (M+1)

### Example 160

### 2-chloro-4-[1-(4-ethynylbenzyl)-3,5-dimethyl-1H-pyrazol-4-yl]benzonitrile

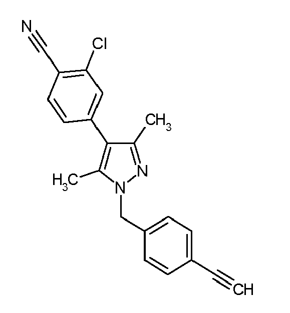

The title compound was obtained in the same manner as in Example 136 and using 4-ethynylbenzyl alcohol as a starting material.
MS (ESI+, m/e) 346 (M+1)

### Example 161

### 2-chloro-4-[1-(2-methoxybenzyl)-3,5-dimethyl-1H-pyrazol-4-yl]benzonitrile

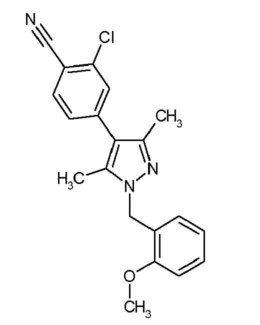

The title compound was obtained in the same manner as in Example 136 and using 2-methoxybenzyl alcohol as a starting material.
MS (ESI+, m/e) 352 (M+1)

### Example 162

### 2-chloro-4-[1-(3-methoxybenzyl)-3,5-dimethyl-1H-pyrazol-4-yl]benzonitrile

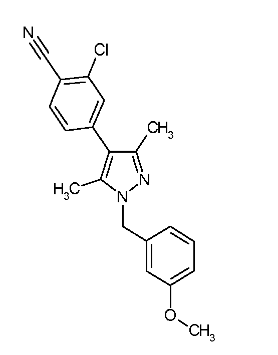

The title compound was obtained in the same manner as in Example 136 and using 3-methoxybenzyl alcohol as a starting material.
MS (ESI+, m/e) 352 (M+1)

### Example 163

### 2-chloro-4-[1-(3-chlorobenzyl)-3,5-dimethyl-1H-pyrazol-4-yl]benzonitrile

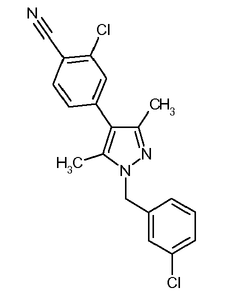

The title compound was obtained in the same manner as in Example 136 and using 3-chlorobenzyl alcohol as a starting material.
MS (ESI+, m/e) 356 (M+1)

### Example 164

### 2-chloro-4-[1-(2,4-difluorobenzyl)-3,5-dimethyl-1H-pyrazol-4-yl]benzonitrile

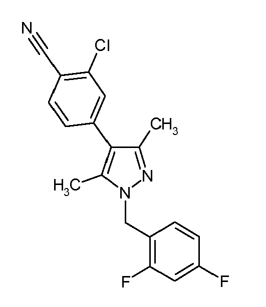

The title compound was obtained in the same manner as in Example 136 and using 2,4-difluorobenzyl alcohol as a starting material.
MS (ESI+, m/e) 358 (M+1)

### Example 165

### 2-chloro-4-[1-(1H-indol-3-ylmethyl)-3,5-dimethyl-1H-pyrazol-4-yl]benzonitrile

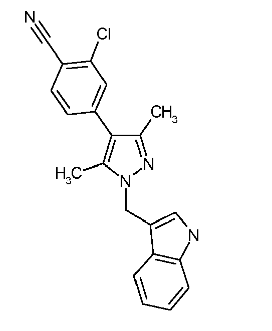

The title compound was obtained in the same manner as in Example 136 and using indole-3-carbinol as a starting material.
MS (ESI+, m/e) 361 (M+1)

### Example 166

### 2-chloro-4-{1-[cyclopropyl(phenyl)methyl]-3,5-dimethyl-1H-pyrazol-4-yl}benzonitrile

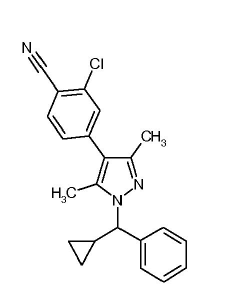

The title compound was obtained in the same manner as in Example 136 and using α-cyclopropylbenzyl alcohol as a starting material.
MS (ESI+, m/e) 362 (M+1)
¹H-NMR (CDCl₃) δ: 0.41-0.57 (2H, m), 0.74-0.91 (2H, m), 1.88-1.97 (1H, m), 2.13 (3H, s), 2.32 (3H, s), 4.42 (1H, d), 7.28-7.36 (5H, m), 7.24 (1H, dd), 7.39 (1H, d), 7.68 (1H, d).

### Example 167

### 4-[1-(1,3-benzodioxol-5-ylmethyl)-3,5-dimethyl-1H-pyrazol-4-yl]-2-chlorobenzonitrile

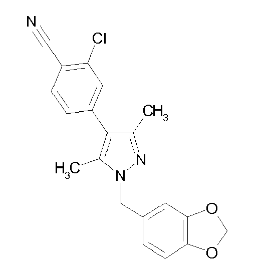

The title compound was obtained in the same manner as in Example 136 and using piperonyl alcohol as a starting material.
MS (ESI+, m/e) 366 (M+1)

### Example 168

### 2-chloro-4-[3,5-dimethyl-1-(3-nitrobenzyl)-1H-pyrazol-4-yl]benzonitrile

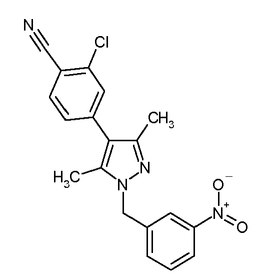

The title compound was obtained in the same manner as in Example 136 and using 3-nitrobenzyl alcohol as a starting material.
MS (ESI+, m/e) 367 (M+1)

### Example 169

### 2-chloro-4-[3,5-dimethyl-1-(4-nitrobenzyl)-1H-pyrazol-4-yl]benzonitrile

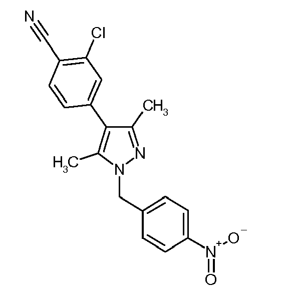

The title compound was obtained in the same manner as in Example 136 and using 4-nitrobenzyl alcohol as a starting material.
MS (ESI+, m/e) 367 (M+1)

### Example 170

### 2-chloro-4-{3,5-dimethyl-1-[4-(methylthio)benzyl]-1H-pyrazol-4-yl}benzonitrile

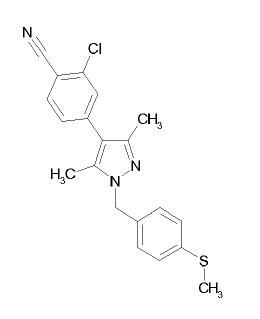

The title compound was obtained in the same manner as in Example 136 and using 4-(methylthio)benzyl alcohol as a starting material.
MS (ESI+, m/e) 368 (M+1)

### Example 171

### 2-chloro-4-[3,5-dimethyl-1-(quinolin-6-ylmethyl)-1H-pyrazol-4-yl]benzonitrile

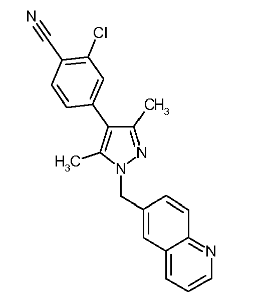

The title compound was obtained in the same manner as in Example 136 and using quinolin-6-ylmethanol as a starting material.
MS (ESI+, m/e) 373 (M+1)

### Example 172

### 2-chloro-4-[1-(2-chloro-4-fluorobenzyl)-3,5-dimethyl-1H-pyrazol-4-yl]benzonitrile

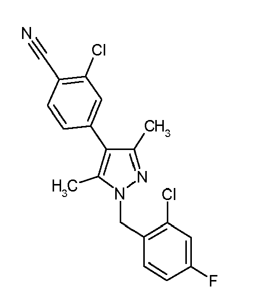

The title compound was obtained in the same manner as in Example 136 and using 2-chloro-4-fluorobenzyl alcohol as a starting material.
MS (ESI+, m/e) 374 (M+1)

### Example 173

### 2-chloro-4-{3,5-dimethyl-1-[(1-methyl-1H-1,2,3-benzotriazol-5-yl)methyl]-1H-pyrazol-4-yl}benzonitrile

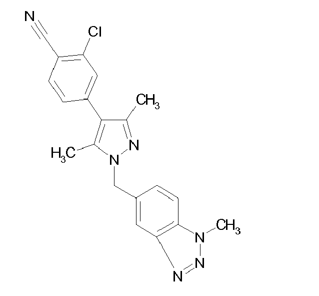

The title compound was obtained in the same manner as in Example 136 and using (1-methyl-1H-1,2,3-benzotriazol-5-yl)methanol as a starting material.
MS (ESI+, m/e) 377 (M+1)
¹H-NMR (CDCl₃) δ: 2.24 (3H, s), 2.32 (3H, s), 3.50 (3H, s), 5.48 (2H, s), 7.24-7.27 (1H, m), 7.39 (1H, d), 7.41 (1H, dd), 7.53 (1H, dd), 7.71 (1H, d), 7.73 (1H, d).

### Example 174

### 4-[1-(1-benzothien-2-ylmethyl)-3,5-dimethyl-1H-pyrazol-4-yl]-2-chlorobenzonitrile

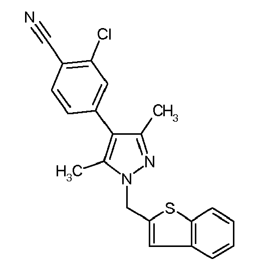

The title compound was obtained in the same manner as in Example 136 and using 1-benzothiophen-2-ylmethanol as a starting material.
MS (ESI+, m/e) 378 (M+1)

### Example 175

### 4-[1-(4-tert-butylbenzyl)-3,5-dimethyl-1H-pyrazol-4-yl]-2-chlorobenzonitrile

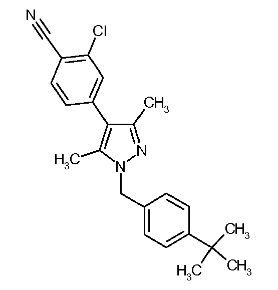

The title compound was obtained in the same manner as in Example 136 and using 4-tert-butylbenzyl alcohol as a starting material.
MS (ESI+, m/e) 378 (M+1)

### Example 176

### 2-chloro-4-[1-(2,3-dihydro-1,4-benzodioxin-5-ylmethyl)-3,5-dimethyl-1H-pyrazol-4-yl]benzonitrile

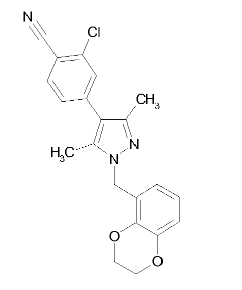

The title compound was obtained in the same manner as in Example 136 and using 2,3-dihydro-1,4-benzodioxin-5-ylmethanol as a starting material.
MS (ESI+, m/e) 380 (M+1)

### Example 177

### 2-chloro-4-[1-(2,3-dihydro-1,4-benzodioxin-6-ylmethyl)-3,5-dimethyl-1H-pyrazol-4-yl]benzonitrile

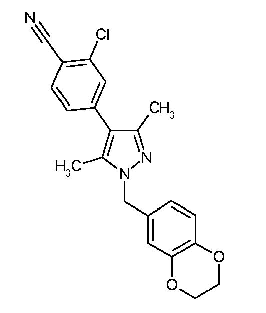

The title compound was obtained in the same manner as in Example 136 and using 2,3-dihydro-1,4-benzodioxin-6-ylmethanol as a starting material.
MS (ESI+, m/e) 380 (M+1)

### Example 178

### Methyl 2-{[4-(3-chloro-4-cyanophenyl)-3,5-dimethyl-1H-pyrazol-1-yl]methyllisonicotinate

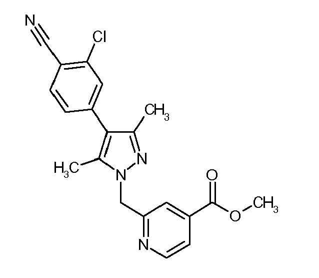

The title compound was obtained in the same manner as in Example 136 and using methyl 2-(hydroxymethyl)isonicotinate as a starting material.
MS (ESI+, m/e) 381 (M+1)

### Example 179

### 2-chloro-4-[1-(2,3-dimethoxybenzyl)-3,5-dimethyl-1H-pyrazol-4-yl]benzonitrile

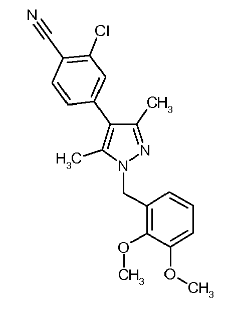

The title compound was obtained in the same manner as in Example 136 and using 2,3-dimethoxybenzyl alcohol as a starting material.
MS (ESI+, m/e) 382 (M+1)

### Example 180

### 2-chloro-4-[1-(2,4-dimethoxybenzyl)-3,5-dimethyl-1H-pyrazol-4-yl]benzonitrile

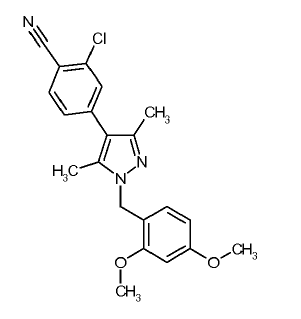

The title compound was obtained in the same manner as in Example 136 and using 2,4-dimethoxybenzyl alcohol as a starting material.
MS (ESI+, m/e) 382 (M+1)

### Example 181

### 2-chloro-4-[1-(3,4-dimethoxybenzyl)-3,5-dimethyl-1H-pyrazol-4-yl]benzonitrile

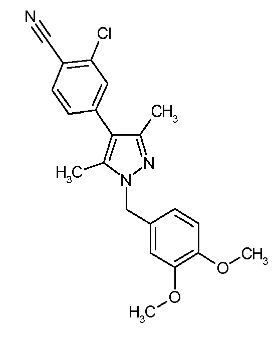

The title compound was obtained in the same manner as in Example 136 and using 3,4-dimethoxybenzyl alcohol as a starting material.
MS (ESI+, m/e) 382 (M+1)

### Example 182

### 2-chloro-4-[1-(2-fluoro-5-nitrobenzyl)-3,5-dimethyl-1H-pyrazol-4-yl]benzonitrile

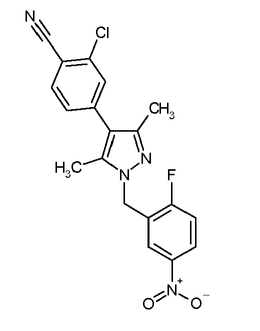

The title compound was obtained in the same manner as in Example 136 and using 2-fluoro-5-nitrobenzyl alcohol as a starting material.
MS (ESI+, m/e) 385 (M+1)

### Example 183

### 2-chloro-4-[1-(4-fluoro-3-nitrobenzyl)-3,5-dimethyl-1H-pyrazol-4-yl]benzonitrile

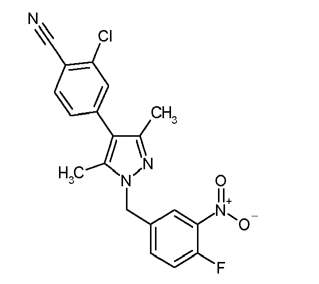

The title compound was obtained in the same manner as in Example 136 and using 4-fluoro-3-nitrobenzyl alcohol as a starting material.
MS (ESI+, m/e) 385 (M+1)

### Example 184

### 2-chloro-4-[1-(4-chloro-2-methoxybenzyl)-3,5-dimethyl-1H-pyrazol-4-yl]benzonitrile

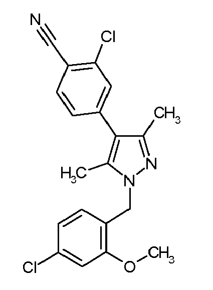

The title compound was obtained in the same manner as in Example 136 and using 4-chloro-2-methoxybenzyl alcohol as a starting material.
MS (ESI+, m/e) 386 (M+1)

### Example 185

### 2-chloro-4-{3,5-dimethyl-1-[(2-methylquinolin-6-yl)methyl]-1H-pyrazol-4-yl}benzonitrile

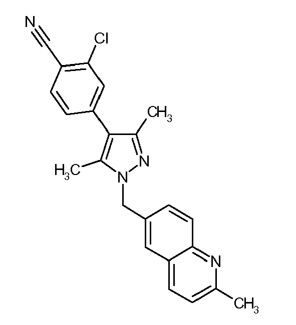

The title compound was obtained in the same manner as in Example 136 and using (2-methylquinolin-6-yl)methanol as a starting material.
MS (ESI+, m/e) 387 (M+1)

### Example 186

### 2-chloro-4-{3,5-dimethyl-1-[(2-phenyl-1H-imidazol-5-yl)methyl]-1H-pyrazol-4-yl}benzonitrile

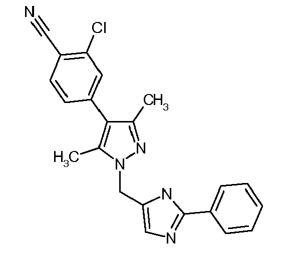

The title compound was obtained in the same manner as in Example 136 and using (2-phenyl-1H-imidazol-5-yl)methanol as a starting material.
MS (ESI+, m/e) 388 (M+1)

### Example 187

### 2-chloro-4-{1-[4-(1H-imidazol-1-yl)benzyl]-3,5-dimethyl-1H-pyrazol-4-yl}benzonitrile

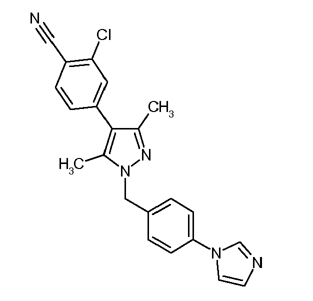

The title compound was obtained in the same manner as in Example 136 and using [4-(1H-imidazol-1-yl)phenyl]methanol as a starting material.
MS (ESI+, m/e) 388 (M+1)

### Example 188

### 2-chloro-4-{3,5-dimethyl-1-[(5-phenyl-1,3-oxazol-4-yl)methyl]-1H-pyrazol-4-yl}benzonitrile

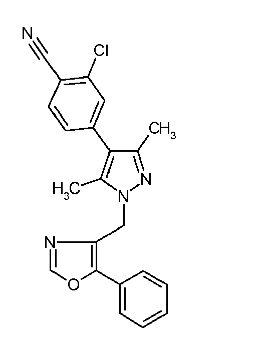

The title compound was obtained in the same manner as in Example 136 and using (5-phenyl-1,3-oxazol-4-yl)methanol as a starting material.
MS (ESI+, m/e) 389 (M+1)

### Example 189

### 2-chloro-4-{3,5-dimethyl-1-[(3-phenylisoxazol-5-yl)methyl]-1H-pyrazol-4-yl}benzonitrile

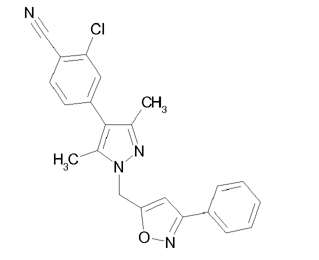

The title compound was obtained in the same manner as in Example 136 and using (3-phenyl-5-isoxazolyl)methanol as a starting material.
MS (ESI+, m/e) 389 (M+1)

### Example 190

### 2-chloro-4-{3,5-dimethyl-1-[3-(trifluoromethyl)benzyl]-1H-pyrazol-4-yl}benzonitrile

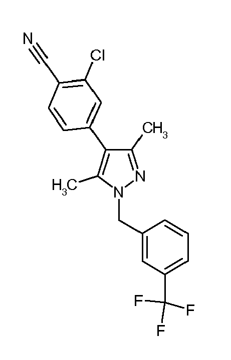

The title compound was obtained in the same manner as in Example 136 and using 3-(trifluoromethyl)benzyl alcohol as a starting material.
MS (ESI+, m/e) 390 (M+1)

### Example 191

### 2-chloro-4-{3,5-dimethyl-1-[4-(trifluoromethyl)benzyl]-1H-pyrazol-4-yl}benzonitrile

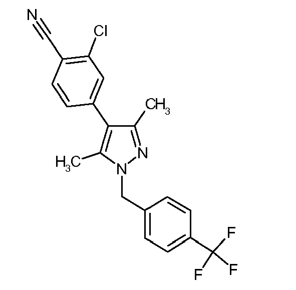

The title compound was obtained in the same manner as in Example 136 and using 4-(trifluoromethyl)benzyl alcohol as a starting material.
MS (ESI+, m/e) 390 (M+1)

### Example 192

### 2-chloro-4-[1-(2,4-dichlorobenzyl)-3,5-dimethyl-1H-pyrazol-4-yl]benzonitrile

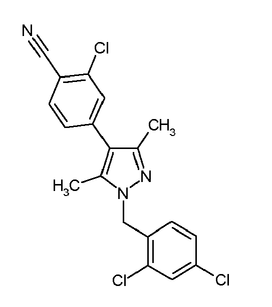

The title compound was obtained in the same manner as in Example 136 and using 2,4-dichlorobenzyl alcohol as a starting material.
MS (ESI+, m/e) 390 (M+1)

### Example 193

### 2-chloro-4-[1-(2,5-dichlorobenzyl)-3,5-dimethyl-1H-pyrazol-4-yl]benzonitrile

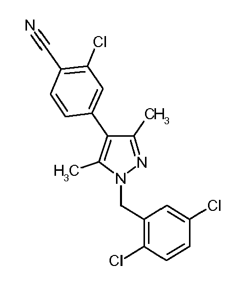

The title compound was obtained in the same manner as in Example 136 and using 2,5-dichlorobenzyl alcohol as a starting material.
MS (ESI+, m/e) 390 (M+1)

### Example 194

### 2-chloro-4-[1-(3,5-dichlorobenzyl)-3,5-dimethyl-1H-pyrazol-4-yl]benzonitrile

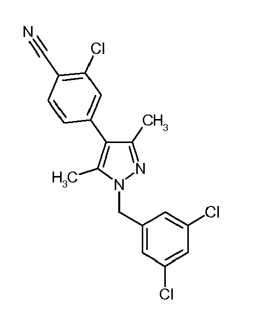

The title compound was obtained in the same manner as in Example 136 and using 3,5-dichlorobenzyl alcohol as a starting material.
MS (ESI+, m/e) 390 (M+1)

### Example 195

### 2-chloro-4-{1-[(5,6-dichloropyridin-3-yl)methyl]-3,5-dimethyl-1H-pyrazol-4-yl}benzonitrile

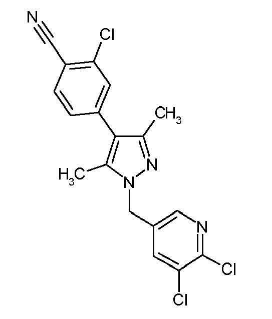

The title compound was obtained in the same manner as in Example 136 and using 5,6-dichloro-3-pyridinemethanol as a starting material.
MS (ESI+, m/e) 391 (M+1)

### Example 196

### 2-chloro-4-{1-[(2,2-dimethyl-2,3-dihydro-1-benzofuran-7-yl)methyl]-3,5-dimethyl-1H-pyrazol-4-yl}benzonitrile

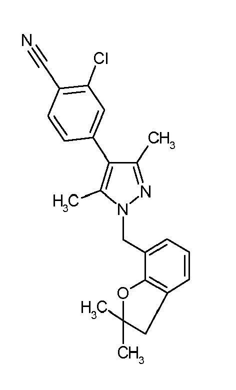

The title compound was obtained in the same manner as in Example 136 and using (2,2-dimethyl-2,3-dihydro-1-benzofuran-7-yl)methanol as a starting material.
MS (ESI+, m/e) 392 (M+1)

### Example 197

### 2-chloro-4-{3,5-dimethyl-1-[(6-pyrrolidin-1-ylpyridin-3-yl)methyl]-1H-pyrazol-4-yl}benzonitrile

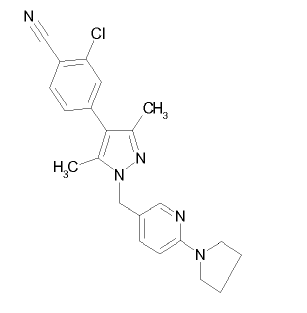

The title compound was obtained in the same manner as in Example 136 and using (6-pyrrolidin-1-ylpyridin-3-yl)methanol as a starting material.
MS (ESI+, m/e) 392 (M+1)

### Example 198

### 2-chloro-4-{3,5-dimethyl-1-[(5-methyl-1-benzothien-2-yl)methyl]-1H-pyrazol-4-yl}benzonitrile

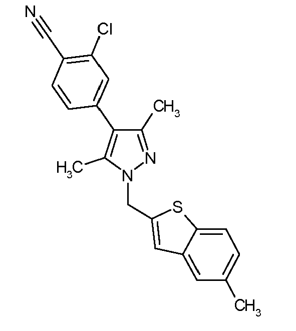

The title compound was obtained in the same manner as in Example 136 and using (5-methyl-1-benzothien-2-yl)methanol as a starting material.
MS (ESI+, m/e) 392 (M+1)

### Example 199

### 2-chloro-4-[1-(3,4-dihydro-2H-1,5-benzodioxepin-6-ylmethyl)-3,5-dimethyl-1H-pyrazol-4-yl]benzonitrile

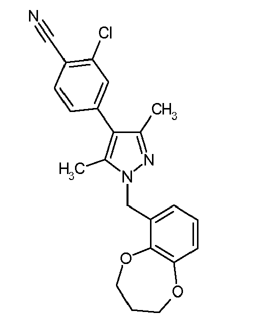

The title compound was obtained in the same manner as in Example 136 and using 3,4-dihydro-2H-1,5-benzodioxepin-6-ylmethanol as a starting material.
MS (ESI+, m/e) 394 (M+1)

### Example 200

### 2-chloro-4-[1-(3,4-dihydro-2H-1,5-benzodioxepin-7-ylmethyl)-3,5-dimethyl-1H-pyrazol-4-yl]benzonitrile

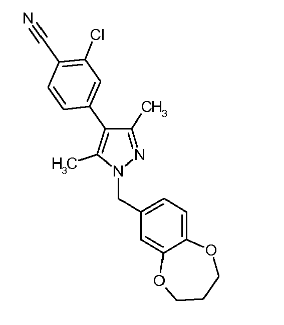

The title compound was obtained in the same manner as in Example 136 and using 3,4-dihydro-2H-1,5-benzodioxepin-7-ylmethanol as a starting material.
MS (ESI+, m/e) 394 (M+1)

### Example 201

### 2-chloro-4-{1-[(3,5-dimethyl-4-nitropyridin-2-yl)methyl]-3,5-dimethyl-1H-pyrazol-4-yl}benzonitrile

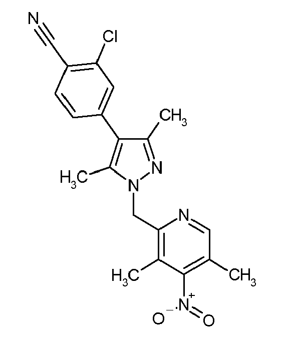

The title compound was obtained in the same manner as in Example 136 and using 3,5-dimethyl-2-hydroxymethyl-4-nitropyridine as a starting material.
MS (ESI+, m/e) 396 (M+1)

### Example 202

### 2-chloro-4-(1-{[1,3-dimethyl-1H-thieno(2,3-c)pyrazol-5-yl]methyl}-3,5-dimethyl-1H-pyrazol-4-yl)benzonitrile

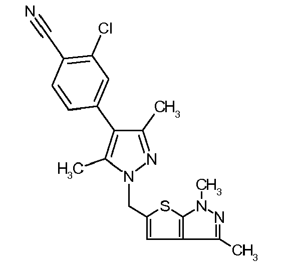

The title compound was obtained in the same manner as in Example 136 and using [1,3-dimethyl-1H-thieno(2,3-c)pyrazol-5-yl]methanol as a starting material.
MS (ESI+, m/e) 396 (M+1)
¹H-NMR (CDCl₃) δ: 2.29 (3H, s), 2.33 (3H, s), 2.41 (3H, s), 3.86 (3H, s), 5.36 (2H, s), 6.84 (1H, s), 7.23 (1H, dd), 7.38 (1H, d), 7.69 (1H, d).

### Example 203

### 2-chloro-4-[1-(3-methoxy-4-nitrobenzyl)-3,5-dimethyl-1H-pyrazol-4-yl]benzonitrile

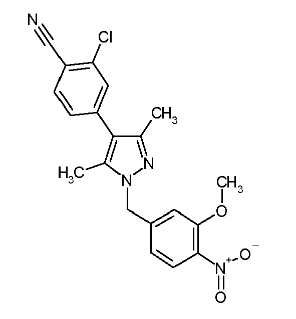

The title compound was obtained in the same manner as in Example 136 and using 3-methoxy-4-nitrobenzyl alcohol as a starting material.
MS (ESI+, m/e) 397 (M+1)

### Example 204

### 2-chloro-4-[1-(4-methoxy-3-nitrobenzyl)-3,5-dimethyl-1H-pyrazol-4-yl]benzonitrile

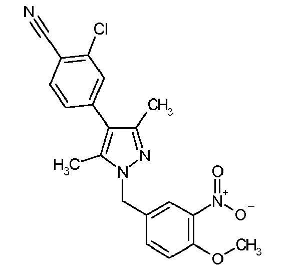

The title compound was obtained in the same manner as in Example 136 and using 4-methoxy-3-nitrobenzyl alcohol as a starting material.
MS (ESI+, m/e) 397 (M+1)

### Example 205

### 4-[1-(biphenyl-4-ylmethyl)-3,5-dimethyl-1H-pyrazol-4-yl]-2-chlorobenzonitrile

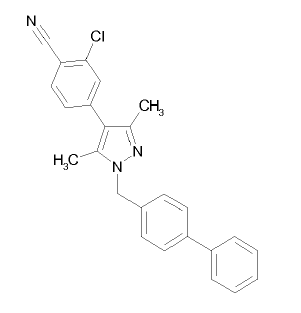

The title compound was obtained in the same manner as in Example 136 and using 4-biphenylmethanol as a starting material.
MS (ESI+, m/e) 398 (M+1)

### Example 206

### 2-chloro-4-[1-(diphenylmethyl)-3,5-dimethyl-1H-pyrazol-4-yl]benzonitrile

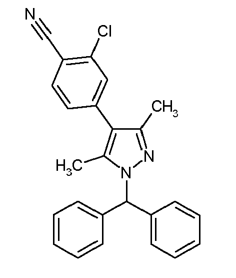

The title compound was obtained in the same manner as in Example 136 and using benzhydrol as a starting material.
MS (ESI+, m/e) 398 (M+1)

### Example 207

### 4-[1-(4-bromobenzyl)-3,5-dimethyl-1H-pyrazol-4-yl]-2-chlorobenzonitrile

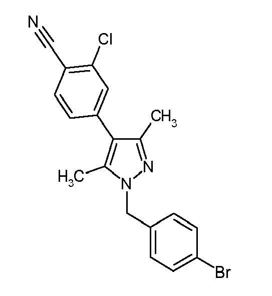

The title compound was obtained in the same manner as in Example 136 and using 4-bromobenzyl alcohol as a starting material.
MS (ESI+, m/e) 400 (M+1)

### Example 208

### 4-{1-[(5-bromopyridin-2-yl)methyl]-3,5-dimethyl-1H-pyrazol-4-yl}-2-chlorobenzonitrile

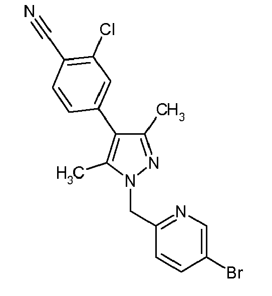

The title compound was obtained in the same manner as in Example 136 and using (5-bromopyridin-2-yl)methanol as a starting material.
MS (ESI+, m/e) 401 (M+1)

### Example 209

### 2-chloro-4-{3,5-dimethyl-1-[(2-methyl-5-phenyl-3-furyl)methyl]-1H-pyrazol-4-yl}benzonitrile

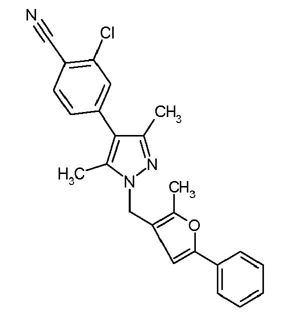

The title compound was obtained in the same manner as in Example 136 and using (2-methyl-5-phenyl-3-furyl)methanol as a starting material.
MS (ESI+, m/e) 402 (M+1)

### Example 210

### 2-chloro-4-{3,5-dimethyl-1-[(5-methyl-1-phenyl-1H-pyrazol-4-yl)methyl]-1H-pyrazol-4-yl}benzonitrile

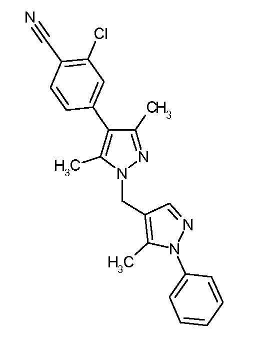

The title compound was obtained in the same manner as in Example 136 and using (5-methyl-1-phenyl-1H-pyrazol-4-yl)methanol as a starting material.
MS (ESI+, m/e) 402 (M+1)

### Example 211

### 2-chloro-4-{3,5-dimethyl-1-[(1-methyl-5-phenyl-1H-pyrazol-4-yl)methyl]-1H-pyrazol-4-yl}benzonitrile

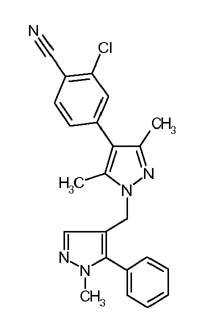

The title compound was obtained in the same manner as in Example 136 and using (1-methyl-5-phenyl-1H-pyrazol-4-yl)methanol as a starting material.
MS (ESI+, m/e) 402 (M+1)

### Example 212

### 4-{1-[(1-benzyl-1H-imidazol-5-yl)methyl]-3,5-dimethyl-1H-pyrazol-4-yl}-2-chlorobenzonitrile

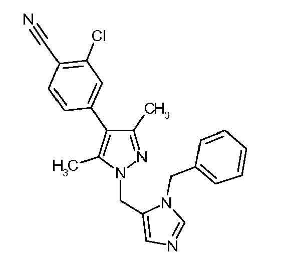

The title compound was obtained in the same manner as in Example 136 and using 1-benzyl-5-hydroxymethyl-1H-imidazole as a starting material.
MS (ESI+, m/e) 232 (M+1)

### Example 213

### 2-chloro-4-{3,5-dimethyl-1-[(3-methyl-5-phenylisoxazol-4-yl)methyl]-1H-pyrazol-4-yl}benzonitrile

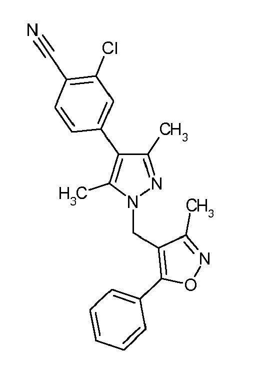

The title compound was obtained in the same manner as in Example 136 and using (3-methyl-5-phenylisoxazol-4-yl)methanol as a starting material.
MS (ESI+, m/e) 403 (M+1)

### Example 214

### 2-chloro-4-{3,5-dimethyl-1-[(5-methyl-3-phenylisoxazol-4-yl)methyl]-1H-pyrazol-4-yl}benzonitrile

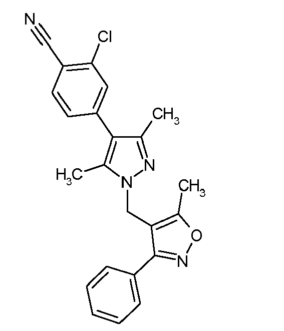

The title compound was obtained in the same manner as in Example 136 and using (5-methyl-3-phenylisoxazol-4-yl)methanol as a starting material.
MS (ESI+, m/e) 403 (M+1)

### Example 215

### 2-chloro-4-{3,5-dimethyl-1-[(5-methyl-2-phenyl-2H-1,2,3-triazol-4-yl)methyl]-1H-pyrazol-4-yl}benzonitrile

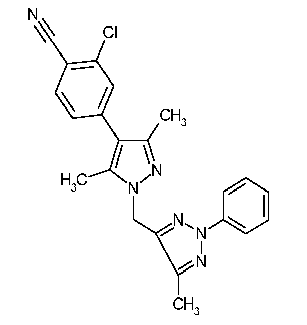

The title compound was obtained in the same manner as in Example 136 and using (5-methyl-2-phenyl-2H-1,2,3-triazol-4-yl)methanol as a starting material.
MS (ESI+, m/e) 403 (M+1)

### Example 216

### 2-chloro-4-{3,5-dimethyl-1-[(2-phenyl-1,3-thiazol-4-yl)methyl]-1H-pyrazol-4-yl}benzonitrile

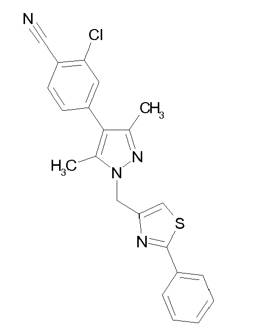

The title compound was obtained in the same manner as in Example 136 and using (2-phenyl-1,3-thiazol-4-yl)methanol as a starting material.
MS (ESI+, m/e) 405 (M+1)

### Example 217

### 2-chloro-4-{3,5-dimethyl-1-[(5-pyridin-2-yl-2-thienyl)methyl]-1H-pyrazol-4-yl}benzonitrile

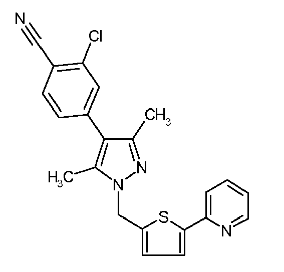

The title compound was obtained in the same manner as in Example 136 and using (5-pyridin-2-yl-2-thienyl)methanol as a starting material.
MS (ESI+, m/e) 405 (M+1)

### Example 218

### 2-chloro-4-{3,5-dimethyl-1-[(4-phenyl-1,2,3-thiadiazol-5-yl)methyl]-1H-pyrazol-4-yl}benzonitrile

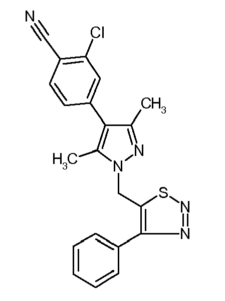

The title compound was obtained in the same manner as in Example 136 and using (4-phenyl-1,2,3-thiadiazol-5-yl)methanol as a starting material.
MS (ESI+, m/e) 406 (M+1)

### Example 219

### 2-chloro-4-[1-(2-methoxy-6,7,8,9-tetrahydro-5H-benzo[7]annulen-5-yl)-3,5-dimethyl-1H-pyrazol-4-yl]benzonitrile

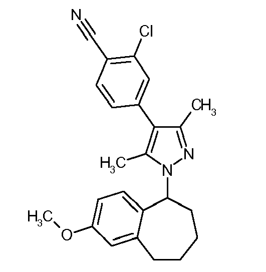

The title compound was obtained in the same manner as in Example 136 and using 2-methoxy-6,7,8,9-tetrahydro-5H-benzo[7]annulen-5-ol as a starting material.
MS (ESI+, m/e) 406 (M+1)

### Example 220

### 2-chloro-4-{3,5-dimethyl-1-[(2-piperidin-1-ylpyridin-3-yl)methyl]-1H-pyrazol-4-yl}benzonitrile

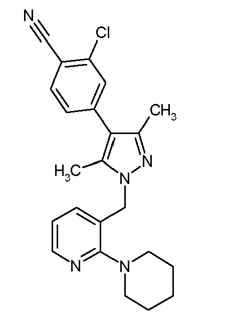

The title compound was obtained in the same manner as in Example 136 and using (2-piperidin-1-ylpyridin-3-yl)methanol as a starting material.
MS (ESI+, m/e) 406 (M+1)

### Example 221

### 2-chloro-4-[3,5-dimethyl-1-(2-morpholin-4-ylbenzyl)-1H-pyrazol-4-yl]benzonitrile

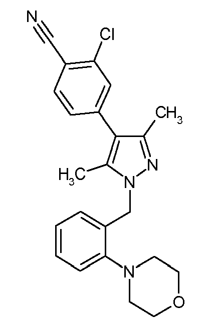

The title compound was obtained in the same manner as in Example 136 and using (2-morpholin-4-ylphenyl)methanol as a starting material.
MS (ESI+, m/e) 408 (M+1)

### Example 222

### 2-chloro-4-[3,5-dimethyl-1-(4-morpholin-4-ylbenzyl)-1H-pyrazol-4-yl]benzonitrile

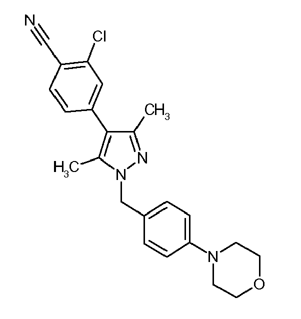

The title compound was obtained in the same manner as in Example 136 and using (4-morpholin-4-ylphenyl)methanol as a starting material.
MS (ESI+, m/e) 407 (M+1)

### Example 223

### 2-chloro-4-{3,5-dimethyl-1-[(6-morpholin-4-ylpyridin-3-yl)methyl]-1H-pyrazol-4-yl}benzonitrile

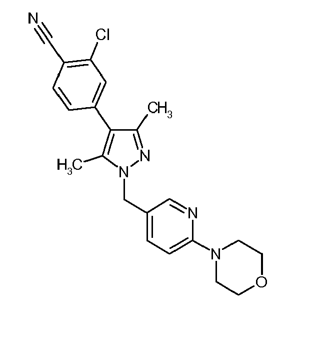

The title compound was obtained in the same manner as in Example 136 and using (6-morpholino-3-pyridinyl)methanol as a starting material.
MS (ESI+, m/e) 408 (M+1)

### Example 224

### 4-[1-(2,2'-bithien-5-ylmethyl)-3,5-dimethyl-1H-pyrazol-4-yl]-2-chlorobenzonitrile

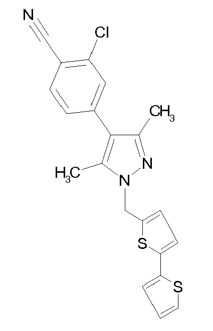

The title compound was obtained in the same manner as in Example 136 and using 2,2'-bithien-5-ylmethanol as a starting material.
MS (ESI+, m/e) 410 (M+1)

### Example 225

### 2-chloro-4-(3,5-dimethyl-1-{[2-(2-thienyl)-1,3-thiazol-4-yl]methyl}-1H-pyrazol-4-yl)benzonitrile

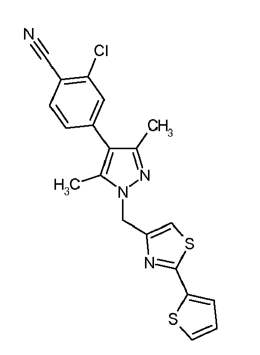

The title compound was obtained in the same manner as in Example 136 and using [2-(2-thienyl)-1,3-thiazol-4-yl]methanol as a starting material.
MS (ESI+, m/e) 411 (M+1)

### Example 226

### 2-chloro-4-{1-[(5-chloro-1-benzothien-3-yl)methyl]-3,5-dimethyl-1H-pyrazol-4-yl}benzonitrile

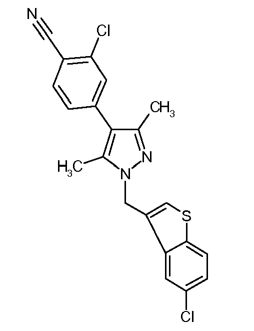

The title compound was obtained in the same manner as in Example 136 and using (5-chloro-1-benzothien-3-yl)methanol as a starting material.
MS (ESI+, m/e) 412 (M+1)
¹H-NMR (CDCl₃) δ: 2.25 (3H, s), 2.33 (3H, s), 5.48 (2H, s), 7.12 (1H, s), 7.25 (1H, dd), 7.36 (1H, dd), 7.39 (1H, d), 7.70 (1H, d), 7.78 (1H, d), 7.84 (1H, d).

### Example 227

### 2-chloro-4-[3,5-dimethyl-1-(3-phenoxybenzyl)-1H-pyrazol-4-yl]benzonitrile

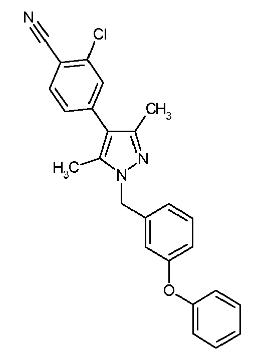

The title compound was obtained in the same manner as in Example 136 and using 3-phenoxybenzyl alcohol as a starting material.
MS (ESI+, m/e) 414 (M+1)

### Example 228

### 2-chloro-4-{3,5-dimethyl-1-[(4-methyl-2-phenylpyrimidin-5-yl)methyl]-1H-pyrazol-4-yl}benzonitrile

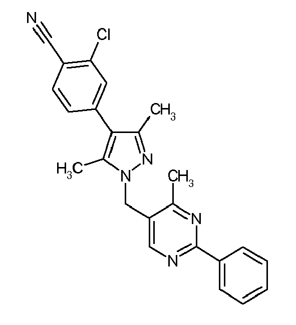

The title compound was obtained in the same manner as in Example 136 and using (4-methyl-2-phenylpyrimidin-5-yl)methanol as a starting material.
MS (ESI+, m/e) 414 (M+1)

### Example 229

### 2-chloro-4-{3,5-dimethyl-1-[(6-phenoxypyridin-3-yl)methyl]-1H-pyrazol-4-yl}benzonitrile

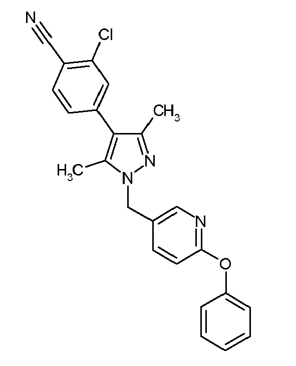

The title compound was obtained in the same manner as in Example 136 and using (6-phenoxypyridin-3-yl)methanol as a starting material.
MS (ESI+, m/e) 415 (M+1)

### Example 230

### 4-[1-(4-bromo-2-fluorobenzyl)-3,5-dimethyl-1H-pyrazol-4-yl]-2-chlorobenzonitrile

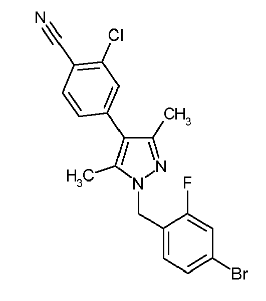

The title compound was obtained in the same manner as in Example 136 and using 4-bromo-2-fluorobenzyl alcohol as a starting material.
MS (ESI+, m/e) 418 (M+1)

### Example 231

### 2-chloro-4-{3,5-dimethyl-1-[(4-methyl-2-phenyl-1,3-thiazol-5-yl)methyl]-1H-pyrazol-4-yl}benzonitrile

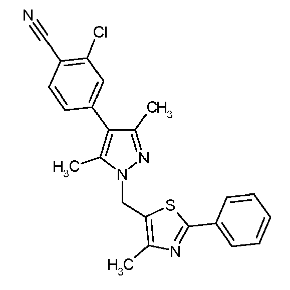

The title compound was obtained in the same manner as in Example 136 and using (4-methyl-2-phenyl-1,3-thiazol-5-yl)methanol as a starting material.
MS (ESI+, m/e) 419 (M+1)

### Example 232

### 2-chloro-4-(1-{[1-(4-fluorophenyl)-5-methyl-1H-pyrazol-4-yl]methyl}-3,5-dimethyl-1H-pyrazol-4-yl)benzonitrile

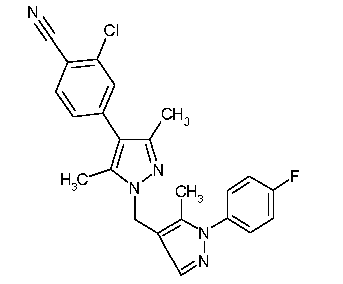

The title compound was obtained in the same manner as in Example 136 and using [1-(4-fluorophenyl)-5-methyl-1H-pyrazol-4-yl]methanol as a starting material.
MS (ESI+, m/e) 420 (M+1)

### Example 233

### 2-chloro-4-{1-[(7-methoxy-2,2-dimethyl-2,3-dihydro-1-benzofuran-4-yl)methyl]-3, 5-dimethyl-1H-pyrazol-4-yl}benzonitrile

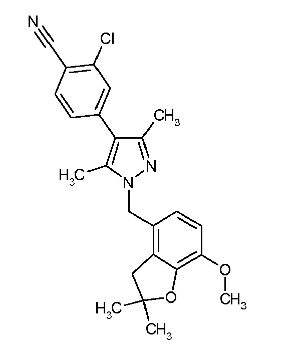

The title compound was obtained in the same manner as in Example 136 and using (7-methoxy-2,2-dimethyl-2,3-dihydro-1-benzofuran-4-yl)methanol as a starting material.
MS (ESI+, m/e) 422 (M+1)

### Example 234

### Methyl 4-{[4-(3-chloro-4-cyanophenyl)-3,5-dimethyl-1H-pyrazol-1-yl]methyl}-3-nitrobenzoate

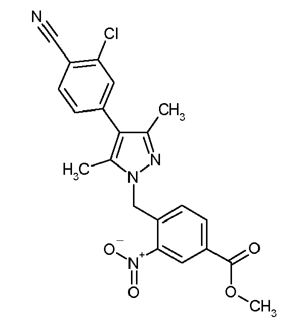

The title compound was obtained in the same manner as in Example 136 and using methyl 4-(hydroxymethyl)-3-nitrobenzoate as a starting material.
MS (ESI+, m/e) 425 (M+1)

### Example 235

### Methyl 3-{[4-(3-chloro-4-cyanophenyl)-3,5-dimethyl-1H-pyrazol-1-yl]methyl}-5-nitrobenzoate

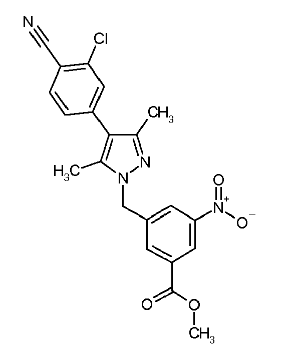

The title compound was obtained in the same manner as in Example 136 and using methyl 3-(hydroxymethyl)-5-nitrobenzoate as a starting material.
MS (ESI+, m/e) 425 (M+1)

### Example 236

### 2-chloro-4-(3,5-dimethyl-1-{[5-(2-methyl-1,3-thiazol-4-yl)-2-thienyl]methyl}-1H-pyrazol-4-yl)benzonitrile

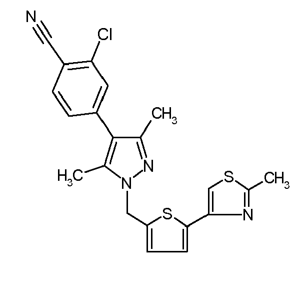

The title compound was obtained in the same manner as in Example 136 and using [5-(2-methyl-1,3-thiazol-4-yl)-2-thienyl]methanol as a starting material.
MS (ESI+, m/e) 425 (M+1)

### Example 237

### 4-{1-[3-(benzyloxy)benzyl]-3,5-dimethyl-1H-pyrazol-4-yl}-2-chlorobenzonitrile

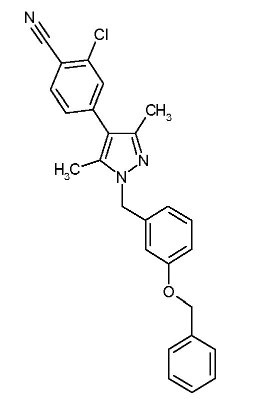

The title compound was obtained in the same manner as in Example 136 and using 3-benzyloxybenzyl alcohol as a starting material.
MS (ESI+, m/e) 428 (M+1)

### Example 238

### 4-{1-[4-(benzyloxy)benzyl]-3,5-dimethyl-1H-pyrazol-4-yl}-2-chlorobenzonitrile

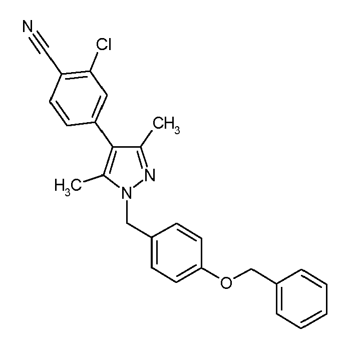

The title compound was obtained in the same manner as in Example 136 and using 4-benzyloxybenzyl alcohol as a starting material.
MS (ESI+, m/e) 428 (M+1)

### Example 239

### 4-(1-{[3-(benzyloxy)pyridin-2-yl]methyl}-3,5-dimethyl-1H-pyrazol-4-yl)-2-chlorobenzonitrile

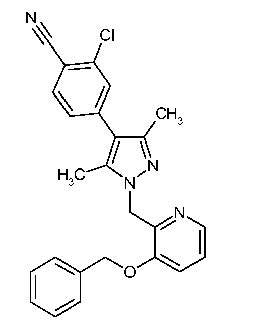

The title compound was obtained in the same manner as in Example 136 and using [3-(benzyloxy)-2-pyridinyl]methanol as a starting material.
MS (ESI+, m/e) 429 (M+1)

### Example 240

### 4-(1-{[5-(benzyloxy)pyridin-2-yl]methyl}-3,5-dimethyl-1H-pyrazol-4-yl)-2-chlorobenzonitrile

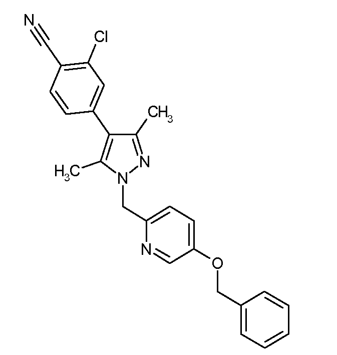

The title compound was obtained in the same manner as in Example 136 and using [5-(benzyloxy)-2-pyridinyl]methanol as a starting material.
MS (ESI+, m/e) 429 (M+1)

### Example 241

### 4-[1-(5-bromo-2-methoxybenzyl)-3,5-dimethyl-1H-pyrazol-4-yl]-2-chlorobenzonitrile

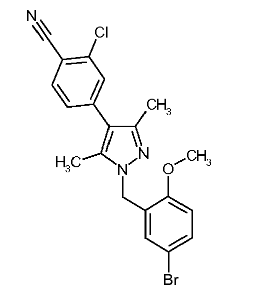

The title compound was obtained in the same manner as in Example 136 and using 5-bromo-2-methoxybenzyl alcohol as a starting material.
MS (ESI+, m/e) 430 (M+1)
¹H-NMR (CDCl₃) δ: 2.23 (3H, s), 2.31 (3H, s), 3.86 (3H, s), 5.26 (2H, s), 6.77 (1H, d), 6.87 (1H, d), 7.28 (1H, dd), 7.37 (1H, dd), 7.42 (1H, d), 7.71 (1H, d).

### Example 242

### 4-(1-{[5-(benzyloxy)-1-methyl-1H-pyrazol-3-yl]methyl}-3,5-dimethyl-1H-pyrazol-4-yl)-2-chlorobenzonitrile

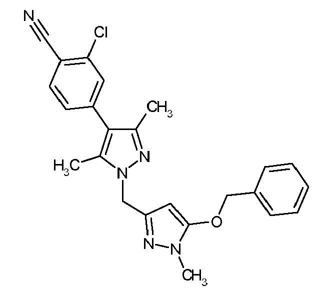

The title compound was obtained in the same manner as in Example 136 and using [5-(benzyloxy)-1-methyl-1H-pyrazol-3-yl]methanol as a starting material.
MS (ESI+, m/e) 432 (M+1)

### Example 243

### 2-chloro-4-(3,5-dimethyl-1-{[3-methyl-4-(2,2,2-trifluoroethoxy)pyridin-2-yl]methyl}-1H-pyrazol-4-yl)benzonitrile

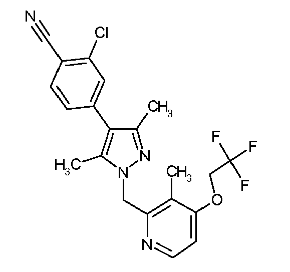

The title compound was obtained in the same manner as in Example 136 and using [3-methyl-4-(2,2,2-trifluoroethoxy)pyridin-2-yl]methanol as a starting material.
MS (ESI+, m/e) 435 (M+1)

### Example 244

### 2-chloro-4-{1-[3-chloro-4-(trifluoromethoxy)benzyl]-3,5-dimethyl-1H-pyrazol-4-yl}benzonitrile

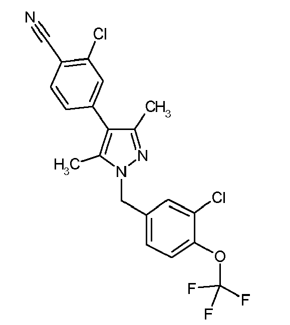

The title compound was obtained in the same manner as in Example 136 and using 3-chloro-4-(trifluoromethoxy)benzyl alcohol as a starting material.
MS (ESI+, m/e) 440 (M+1)

### Example 245

### 4-{1-[3,5-bis(trifluoromethyl)benzyl]-3,5-dimethyl-1H-pyrazol-4-yl}-2-chlorobenzonitrile

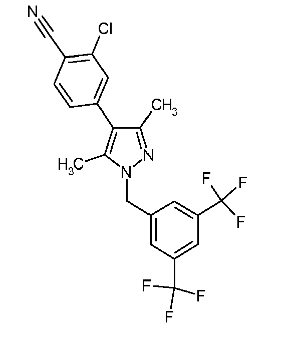

The title compound was obtained in the same manner as in Example 136 and using 3,5-bis(trifluoromethyl)benzyl alcohol as a starting material.
MS (ESI+, m/e) 458 (M+1)

### Example 246

### 4-[1-(2-bromo-4,5-dimethoxybenzyl)-3,5-dimethyl-1H-pyrazol-4-yl]-2-chlorobenzonitrile

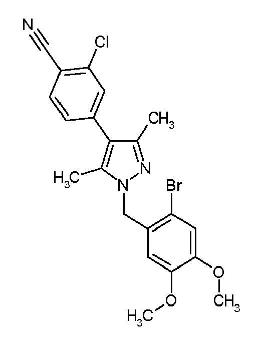

The title compound was obtained in the same manner as in Example 136 and using 2-bromo-4,5-dimethoxybenzyl alcohol as a starting material.
MS (ESI+, m/e) 460 (M+1)

### Example 247

### 4-{1-[bis(4-chlorophenyl)methyl]-3,5-dimethyl-1H-pyrazol-4-yl}-2-chlorobenzonitrile

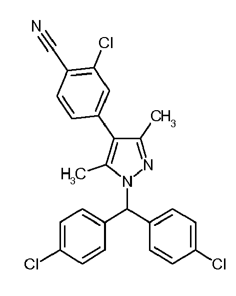

The title compound was obtained in the same manner as in Example 136 and using 4,4'-dichlorobenzhydrol as a starting material.
MS (ESI+, m/e) 466 (M+1)

### Example 248

### 2-chloro-4-[3,5-dimethyl-1-({3-methyl-1-[5-(trifluoromethyl)pyridin-2-yl]-1H-pyrazol-4-yl}methyl)-1H-pyrazol-4-yl]benzonitrile

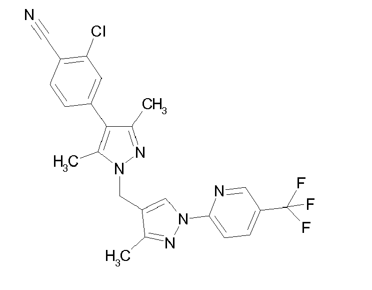

The title compound was obtained in the same manner as in Example 136 and using {3-methyl-1-[5-(trifluoromethyl)pyridin-2-yl]-1H-pyrazol-4-yl}methanol as a starting material.
MS (ESI+, m/e) 471 (M+1)

### Example 249

### 2-chloro-4-[3,5-dimethyl-1-(4-{2-[methyl(pyrimidin-2-yl)amino]ethoxy}benzyl)-1H-pyrazol-4-yl]benzonitrile

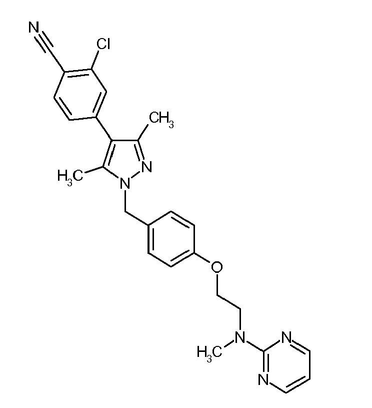

The title compound was obtained in the same manner as in Example 136 and using (4-{2-[methyl(pyrimidin-2-yl)amino]ethoxy}phenyl)methanol as a starting material.
MS (ESI+, m/e) 473 (M+1)

### Example 250

### 2-chloro-4-(3,5-dimethyl-1-{[1-(4-nitrophenyl)-5-propyl-1H-1,2,3-triazol-4-yl]methyl}-1H-pyrazol-4-yl)benzonitrile

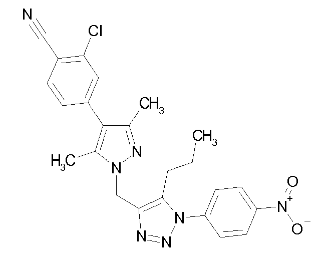

The title compound was obtained in the same manner as in Example 136 and using [1-(4-nitrophenyl)-5-propyl-1H-1,2,3-triazol-4-yl]methanol as a starting material.
MS (ESI+, m/e) 476 (M+1)

### Example 251

### 2-chloro-4-{3,5-dimethyl-1-[4-(quinolin-2-ylmethoxy)benzyl]-1H-pyrazol-4-yl}benzonitrile

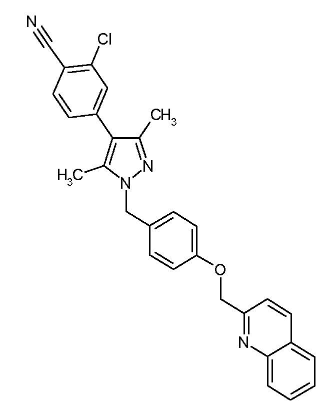

The title compound was obtained in the same manner as in Example 136 and using [4-(quinolin-2-ylmethoxy)phenyl]methanol as a starting material.
MS (ESI+, m/e) 479 (M+1)

### Example 252

### 2-chloro-4-{3,5-dimethyl-1-[(2-{(E)-2-[4-(trifluoromethyl)phenyl]vinyl}-1,3-oxazol-4-yl)methyl]-1H-pyrazol-4-yl}benzonitrile

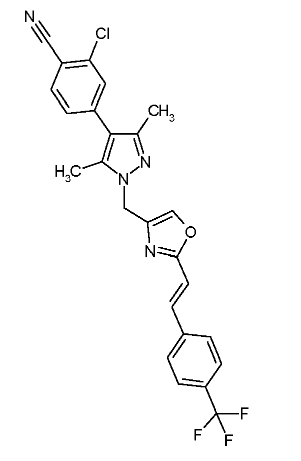

The title compound was obtained in the same manner as in Example 136 and using (2-{(E)-2-[4-(trifluoromethyl)phenyl]vinyl}-1,3-oxazol-4-yl)methanol as a starting material.
MS (ESI+, m/e) 483 (M+1)

### Example 253

### 4-{1-[(1,3-dimethyl-1H-pyrazol-5-yl)methyl]-3,5-dimethyl-1H-pyrazol-4-yl}benzonitrile

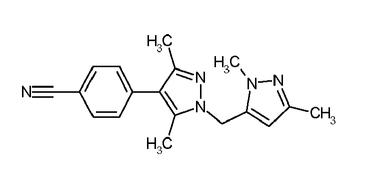

A resin reagent (PS-triphenylphosphine) (about 220 mg) was charged in a polypropylene tube equipped with a filter ·cap, and preconditioned therein. Then, a solution (0.42 mol/L, 0.643 mL) of (1,3-dimethyl-1H-pyrazol-5-yl)methanol in THF and a solution (0.28 mol/L, 0.643 mL) of 2-chloro-4-(3,5-dimethyl-1H-pyrazol-4-yl)benzonitrile synthesized in Reference Example 5 in THF were added and the mixture was stirred. A solution (0.70 mol/L, 0.643 mL) of di-tert-butyl azodicarboxylate in THF was added and the mixture was stirred at room temperature for 24 hr. The resin reagent was removed by filtering and washing the reaction mixture. The filtrate was concentrated. The residue was applied to reversed-phase preparative HPLC (Gilson, UniPoint system, YMC ODS column 30x75 mm, 0.1% TFA containing acetonitrile-water (5:95 - 100:0)) and the eluted fraction was concentrated under reduced pressure, and obtained residue was further purified by silica gel column chromatography (50 - 100% ethyl acetate/hexane) to give the title compound (17.8 mg).
MS (ESI+, m/e) 306 (M+1)
¹H-NMR (DMSO-d₆) δ: 2.07 (3H, s), 2.16 (3H, s), 2.29 (3H, s), 3.79 (3H, s), 5.32 (2H, s), 5.87 (1H, s), 7.49 (2H, d), 7.86 (2H, d).

### Example 254

### 4-{1-[(1,5-dimethyl-1H-pyrazol-3-yl)methyl]-3,5-dimethyl-1H-pyrazol-4-yl}benzonitrile

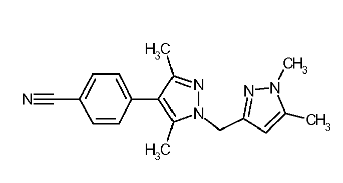

The title compound was obtained in the same manner as in Example 253 and using (1,5-dimethyl-1H-pyrazol-3-yl)methanol as a starting material.
MS (ESI+, m/e) 306 (M+1)
¹H-NMR (DMSO-d₆) δ: 2.07 (3H, s), 2.16 (3H, s), 2.29 (3H, s), 3.79 (3H, s), 5.32 (2H, s), 5.87 (1H, s), 7.49 (2H, d), 7.86 (2H, d).

### Example 255

### 4-{3,5-dimethyl-1-[(1-methyl-1H-1,2,3-benzotriazol-5-yl)methyl]-1H-pyrazol-4-yl}benzonitrile

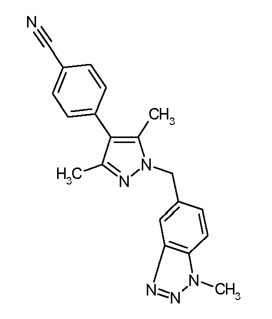

A resin reagent (PS-triphenylphosphine) (about 87 mg) was charged in a miniblock reaction apparatus (Bohdan), and preconditioned therein. Then, a solution (0.15 mol/L, 0.75 mL) of (1-methyl-1H-1,2,3-benzotriazol-5-yl)methanol in THF and a solution (0.15 mol/L, 0.50 mL) of 4-(3,5-dimethyl-1H-pyrazol-4-yl)benzonitrile synthesized in Reference Example 5 in THF were added and the mixture was stirred. A solution (0.375 mol/L, 0.50 mL) of di-tert-butyl azodicarboxylate in THF was added and the mixture was stirred at room temperature for 43 hr. The resin reagent was removed by filtering and washing the reaction mixture. The filtrate was concentrated. The residue was purified by reversed-phase preparative HPLC (Gilson, UniPoint system, YMC ODS column 30x75 mm, 0.1% TFA containing acetonitrile-water (2:98 - 100:0)) to give the title compound (5.4 mg).
MS (ESI+, m/e) 343 (M+1)

### Example 256

### 4-{1-[(5-bromopyridin-2-yl)methyl]-3,5-dimethyl-1H-pyrazol-4-yl}benzonitrile

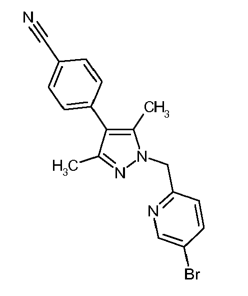

The title compound was obtained in the same manner as in Example 255 and using (5-bromopyridin-2-yl)methanol as a starting material.
MS (ESI+, m/e) 367 (M+1)

### Example 257

### 4-{1-[(6-aminopyridin-3-yl)methyl]-3,5-dimethyl-1H-pyrazol-4-yl}-2-chlorobenzonitrile

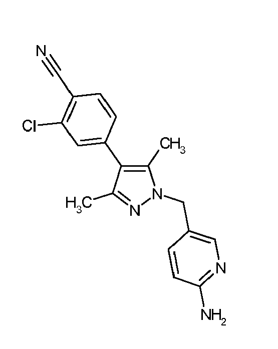

The title compound was obtained in the same manner as in Example 255 and using (6-amino-3-pyridinyl)methanol as a starting material.
MS (ESI+, m/e) 338 (M+1)

### Example 258

### 2-chloro-4-[1-({5-[(dimethylamino)methyl]-2-furyl}methyl)-3,5-dimethyl-1H-pyrazol-4-yl]benzonitrile

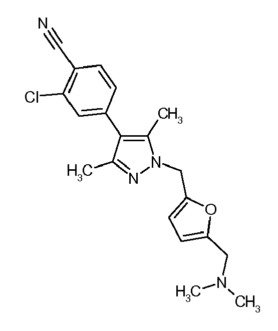

The title compound was obtained in the same manner as in Example 255 and using 5-(dimethylaminomethyl)furfurylalcohol hydrochloride as a starting material and adding about 1.2 equivalent amount of N-methylimidazole as a base.
MS (ESI+, m/e) 369 (M+1)

### Example 259

### 2-chloro-4-{3,5-dimethyl-1-[(2-morpholin-4-ylpyridin-3-yl)methyl]-1H-pyrazol-4-yl}benzonitrile

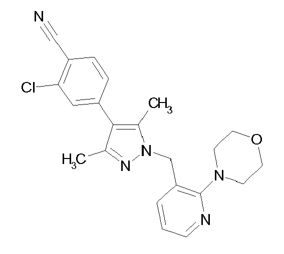

The title compound was obtained in the same manner as in Example 255 and using (2-morpholino-3-pyridinyl)methanol as a starting material.
MS (ESI+, m/e) 408 (M+1)
¹H-NMR (DMSO-d₆) δ: 2.20 (3H, s), 2.24 (3H, s), 3.10 (4H, t), 3.79 (4H, t), 5.31 (2H, s), 7.01-7.07 (2H, m), 7.49 (1H, dd), 7.69 (1H, d), 8.00 (1H, d), 8.23 (1H, dd).

### Example 260

### 4-{1-[(2-fluoropyridin-4-yl)methyl]-3,5-dimethyl-1H-pyrazol-4-yl}benzonitrile

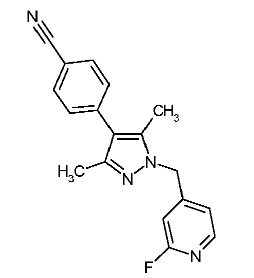

The title compound was obtained in the same manner as in Example 255 and using 2-fluoro-4-pyridinemethanol as a starting material.
MS (ESI+, m/e) 307 (M+1)

### Example 261

### 4-{1-[(3,5-dimethylisoxazol-4-yl)methyl]-3,5-dimethyl-1H-pyrazol-4-yl}benzonitrile

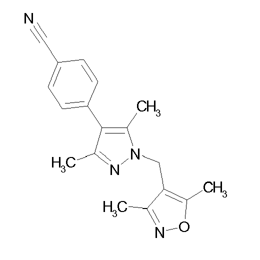

The title compound was obtained in the same manner as in Example 255 and using (3,5-dimethyl-4-isoxazolyl)methanol as a starting material.
MS (ESI+, m/e) 307 (M+1)
¹H-NMR (DMSO-d₆) δ: 2.15 (3H, s), 2.16 (3H, s), 2.29 (3H, s), 2.36 (3H, s), 5.08 (2H, s), 7.48 (2H, d), 7.86 (2H, d).

### Example 262

### 4-[3,5-dimethyl-1-(1,3-thiazol-5-ylmethyl)-1H-pyrazol-4-yl]benzonitrile

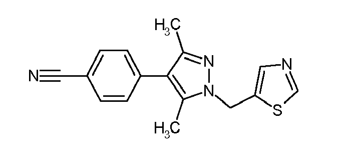

A solution (0.090 mol/L, 2.0 mL) of 4-(3,5-dimethyl-1H-pyrazol-4-yl)benzonitrile obtained in Reference Example 5 in THF was added to thiazole-5-methanol (35.6 mg) and the mixture was stirred. Cyanomethylene tri-N-butylphosphorane (about 230 mg) was added, and the mixture was stirred with heating at 60°C for 32 hr. The reaction mixture was filtered, and the filtrate was concentrated. The residue was purified by silica gel column chromatography (50 - 100% ethyl acetate/hexane), and further by reversed-phase preparative HPLC (Gilson, UniPoint system, YMC ODS column 30x75 mm, 0.1% TFA containing acetonitrile-water (5:95 - 100:0)), and the eluted fraction was passed through ion-exchange resin, whereby the TFA salt was converted to a free form to give the title compound (29.6 mg).
MS (ESI+, m/e) 295 (M+1)
¹H-NMR (DMSO-d₆) δ: 2.18 (3H, s), 2.31 (3H, s), 5.56 (2H, s), 7.48 (2H, d), 7.85 (2H, d), 7.92 (1H, d), 9.04 (1H, d).

### Example 263

### 4-{3,5-dimethyl-1-[(2-methylpyridin-3-yl)methyl]-1H-pyrazol-4-yl}benzonitrile

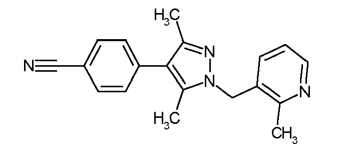

The title compound was obtained in the same manner as in Example 262 and using (2-methylpyridin-3-yl)methanol as a starting material.
MS (ESI+, m/e) 303 (M+1)
¹H-NMR (DMSO-d₆) δ: 2.20 (3H, s), 2.23 (3H, s), 2.54 (3H, s), 5.34 (2H, s), 6.95 (1H, dd), 7.18 (1H, dd), 7.53 (2H, d), 7.88 (2H, d), 8.35 (1H, dd).

### Example 264

### 4-[3,5-dimethyl-1-(pyrazin-2-ylmethyl)-1H-pyrazol-4-yl]benzonitrile

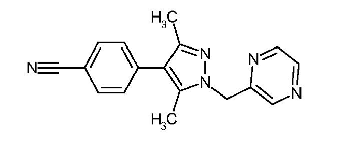

A solution (0.090 mol/L, 2.2 mL) of 4-(3,5-dimethyl-1H-pyrazol-4-yl)benzonitrile obtained in Reference Example 5 in THF was added to pyrazin-2-ylmethanol (37.1 mg) and the mixture was stirred. Cyanomethylene tri-N-butylphosphorane (about 250 mg) was added, and the mixture was stirred with heating at 60°C for 31 hr. The reaction mixture was filtered, and the filtrate was concentrated. The residue was purified by silica gel column chromatography (50 - 100% ethyl acetate/hexane, 0 - 30% methanol/ethyl acetate), and further by reversed-phase preparative HPLC (Gilson, UniPoint system, YMC ODS column 30x75 mm, 0.1% TFA containing acetonitrile-water (5:95 - 100:0)), and the eluted fraction was passed through ion-exchange resin, whereby the TFA salt was converted to a free form to give the title compound (23.1 mg).
MS (ESI+, m/e) 290 (M+1)
¹H-NMR (DMSO-d₆) δ: 2.16 (3H, s), 2.33 (3H, s), 5.47 (2H, s), 7.51 (2H, d), 7.87 (2H, d), 8.52 (1H, d), 5.89-8.63 (2H, m).

### Example 265

### 4-{3,5-dimethyl-1-[(1-methyl-1H-imidazol-5-yl)methyl]-1H-pyrazol-4-yl}benzonitrile

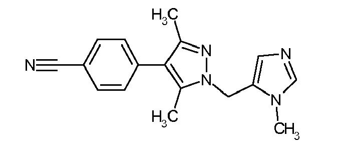

The title compound was obtained in the same manner as in Example 264 and using (1-methyl-1H-imidazol-5-yl)methanol as a starting material.
MS (ESI+, m/e) 292 (M+1)
¹H-NMR (DMSO-d₆) δ: 2.15 (3H, s), 2.31 (3H, s), 3.68 (3H, s), 5.30 (2H, s), 6.90 (1H, s), 7.47 (2H, d), 7.57 (1H, s), 7.85 (2H, d).

### Example 266

### 4-{1-[(2-fluoropyridin-4-yl)methyl]-3,5-dimethyl-1H-pyrazol-4-yl}benzonitrile

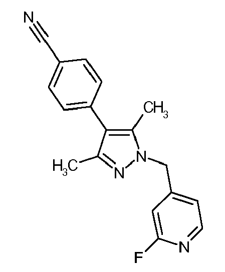

A solution (0.10 mol/L, 2.0 mL) of 4-(3,5-dimethyl-1H-pyrazol-4-yl)benzonitrile obtained in Reference Example 5 in THF was added to 2-fluoro-4-pyridinemethanol (53.9 mg) and the mixture was stirred. Cyanomethylene tri-N-butylphosphorane (about 260 mg) was added, and the mixture was stirred with heating at 60°C for 41 hr. The reaction mixture was filtered, and the filtrate was concentrated. The residue was purified by silica gel column chromatography (30 - 100% ethyl acetate/hexane), and further by reversed-phase preparative HPLC (Gilson, UniPoint system, YMC ODS column 30x75 mm, 0.1% TFA containing acetonitrile-water (5:95 - 100:0)), and the eluted fraction was passed through ion-exchange resin, whereby the TFA salt was converted to a free form to give the title compound (18.3 mg).
MS (ESI+, m/e) 307 (M+1)
¹H-NMR (DMSO-d₆) δ: 2.21 (3H, s), 2.25 (3H, s), 5.43 (2H, s), 6.88 (1H, brs), 7.07 (1H, dd), 7.54 (2H, d), 7.88 (2H, d), 8.22 (1H, d).

### Example 267

### 4-{1-[(3,5-dimethylisoxazol-4-yl)methyl]-3,5-dimethyl-1H-pyrazol-4-yl}benzonitrile

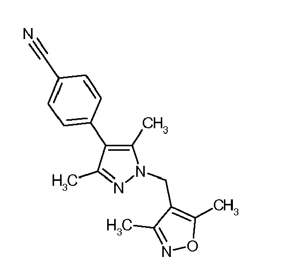

The title compound was obtained in the same manner as in Example 266 and using (3,5-dimethyl-4-isoxazolyl)methanol as a starting material.
MS (ESI+, m/e) 307 (M+1)
¹H-NMR (DMSO-d₆) δ: 2.15 (3H, s), 2.16 (3H, s), 2.29 (3H, s), 2.36 (3H, s), 5.08 (2H, s), 7.48 (2H, d), 7.86 (2H, d).

### Example 268

### 4-{[4-(4-chloro-3-cyanophenyl)-3,5-dimethyl-1H-pyrazol-1-yl]methyl}benzamide

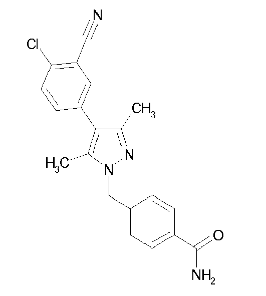

The title compound was obtained in the same manner as in Example 65 and using 4-{[4-(4-chloro-3-cyanophenyl)-3,5-dimethyl-1H-pyrazol-1-yl]methyl}benzoic acid synthesized in Example 64 as a starting material.
¹H-NMR (CDCl₃) δ: 2.18 (3H, s), 2.23 (3H, s), 5.34 (2H, s), 7.24 (2H, d), 7.35 (1H, s), 7.60 - 7.68 (1H, m), 7.73 - 7.80 (1H, m), 7.83 (2H, d), 7.89 - 7.95 (2H, m).

### Example 269

### 4-{[4-(3-chloro-4-cyanophenyl)-3,5-dimethyl-1H-pyrazol-1-yl]methyl}benzenecarbothioamide

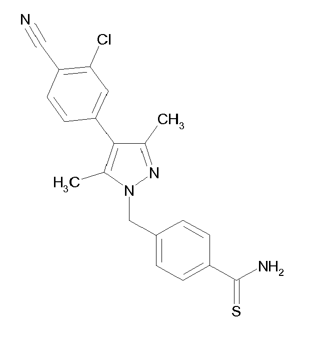

4-{[4-(3-Chloro-4-cyanophenyl)-3,5-dimethyl-1H-pyrazol-1-yl]methyl}benzamide (599.3 mg) synthesized in Example 69 was suspended in toluene (8.2 mL), and a Lawesson reagent was added. The reaction mixture was stirred with heating under reflux for 2 hr and the resulting precipitate was collected by filtration. The precipitate was washed with ethyl acetate-hexane to give the title compound (626.1 mg) as yellow crystals.
¹H-NMR (DMSO-d₆) δ: 2.21 (3H, s), 2.27 (3H, s), 5.35 (2H, s), 7.21 (2H, d), 7.49 (1H, dd), 7.68 (1H, d), 7.84 (2H, d), 7.99 (1H, d), 9.48 (1H, brs), 9.86 (1H, brs).

### Example 270

### 5-{[4-(3-chloro-4-cyanophenyl)-3,5-dimethyl-1H-pyrazol-1-yl]methyl}nicotinic acid

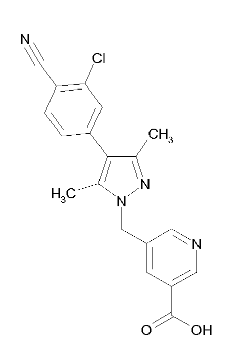

The title compound (containing sodium chloride as impurity) was obtained as a white powder in the same manner as in Example 54 and using methyl 5-{[4-(3-chloro-4-cyanophenyl)-3,5-dimethyl-1H-pyrazol-1-yl]methyl}nicotinate synthesized in Example 120 as a starting material.
¹H-NMR (CDCl₃) δ: 2.29 (6H, s), 5.36 (2H, s), 7.21-7.28 (1H, m), 7.39 (1H, d), 7.71 (1H, d), 8.26 (1H, d), 8.63 (1H, d), 9.20 (1H, d).

### Example 271

### 2-chloro-4-[1-(4-cyanobenzyl)-1H-pyrazol-4-yl]benzonitrile

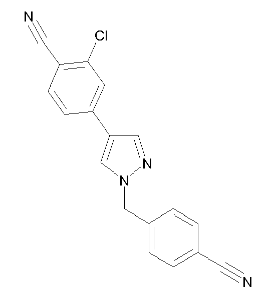

2-Chloro-4-(1H-pyrazol-4-yl)benzonitrile (99.2 mg) synthesized in Reference Example 14 was dissolved in DMF (2.4 mL), and potassium tert-butoxide (82.0 mg) was added. The reaction mixture was stirred at room temperature for 5 min and 4-cyanobenzyl bromide (105.0 mg) was added. The reaction mixture was further stirred at room temperature for 40 min. After stirring, the mixture was poured into saturated aqueous ammonium chloride solution, and extracted with ethyl acetate. The organic layer was washed with saturated brine, dried over anhydrous magnesium sulfate and concentrated. The obtained residue was purified by column chromatography (ethyl acetate-hexane), and recrystallized from ethyl acetate-methanol-hexane to give the title compound (114.8 mg) as colorless crystals.
¹H-NMR (CDCl₃) δ: 5.42 (2H, s), 7.33 (2H, d), 7.44 (1H, dd), 7.59 (1H, d), 7.62-7.77 (3H, m), 7.76 (1H, d), 7.89 (1H, d).

### Example 272

### 4-{[4-(3-chloro-4-cyanophenyl)-1H-pyrazol-1-yl]methyl}benzamide

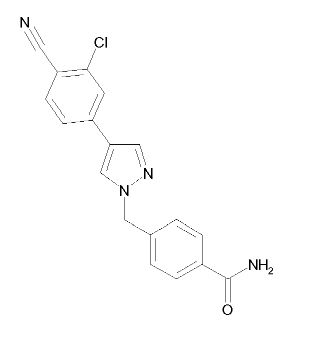

The title compound was obtained in the same manner as in Example 271 and using 2-chloro-4-(1H-pyrazol-4-yl)benzonitrile synthesized in Reference Example 14 and 4-(chloromethyl)benzamide as starting materials.
¹H-NMR (CDCl₃) δ: 5.42 (2H, s), 7.33 (2H, d), 7.77 (1H, dd), 7.85 (2H, d), 7.94 (1H, d), 8.03 (1H, d), 8.18 (1H, s), 8.59 (1H, s).

### Example 273

### 2-chloro-4-{3,5-dimethyl-1-[4-(4-methyl-1,3-thiazol-2-yl)benzyl]-1H-pyrazol-4-yl}benzonitrile

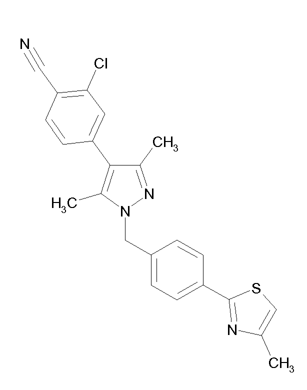

4-{[4-(3-Chloro-4-cyanophenyl)-3,5-dimethyl-1H-pyrazol-1-yl]methyl}benzenecarbothioamide (101.9 mg) synthesized in Example 269 was suspended in ethanol (1.3 mL), and bromoacetone (24.7 µL) was added. The reaction mixture was stirred with heating under reflux for 4 hr, poured into water, and the mixture was extracted with ethyl acetate. The organic layer was washed with saturated brine, dried over anhydrous magnesium sulfate and concentrated. The obtained residue was purified by column chromatography (ethyl acetate-hexane), and recrystallized from ethyl acetate-hexane to give the title compound (46.9 mg) as colorless crystals.
¹H-NMR (CDCl₃) δ: 2.20 (3H, s), 2.31 (3H, s), 2.50 (3H, d), 5.32 (2H, s), 6.88 (1H, d), 7.20 (2H, d), 7.25 (1H, dd), 7.39 (1H, d), 7.69 (1H, d), 7.90 (2H, d).

### Example 274

### 2-chloro-4-{1-[4-(4,5-dimethyl-1,3-thiazol-2-yl)benzyl]-3,5-dimethyl-1H-pyrazol-4-yl}benzonitrile

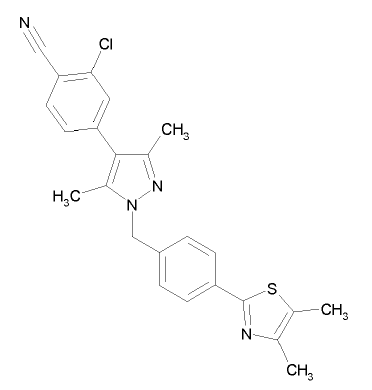

The title compound was obtained in the same manner as in Example 269 and using 4-{[4-(3-chloro-4-cyanophenyl)-3,5-dimethyl-1H-pyrazol-1-yl]methyl}benzenecarbothioamide synthesized in Example 269 and 3-bromo-2-butanone as starting materials.
¹H-NMR (CDCl₃) δ: 2.20 (3H, s), 2.30 (3H, s), 2.38 (3H, s), 2.39 (3H, s), 5.30 (2H, s), 7.17 (2H, d), 7.25 (1H, dd), 7.39 (1H, d), 7.69 (1H, d), 7.83 (2H, d).

### Example 275

### Ethyl 2-(4-{[4-(3-chloro-4-cyanophenyl)-3,5-dimethyl-1H-pyrazol-1-yl]methyl}phenyl)-1,3-thiazole-4-carboxylate

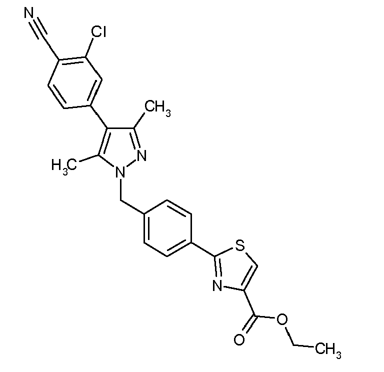

The title compound (246.0 mg) was obtained as colorless crystals according to the method in Example 273 and using 4-{[4-(3-chloro-4-cyanophenyl)-3,5-dimethyl-1H-pyrazol-1-yl]methyl}benzenecarbothioamide (343.9 mg) synthesized in Example 269 and ethyl 3-bromo-2-oxopropanoate (0.11 mL) as starting materials.
¹H-NMR (CDCl₃) δ: 1.43 (3H, t), 2.21 (3H, s), 2.31 (3H, s), 4.45 (2H, q), 5.33 (2H, s), 7.20-7.28 (3H, m), 7.40 (1H, d), 7.70 (1H, d), 7.99 (2H, d), 8.15 (1H, s).

### Example 276

### 2-(4-{[4-(3-chloro-4-cyanophenyl)-3,5-dimethyl-1H-pyrazol-1-yl]methyl}phenyl)-1,3-thiazole-4-carboxylic acid

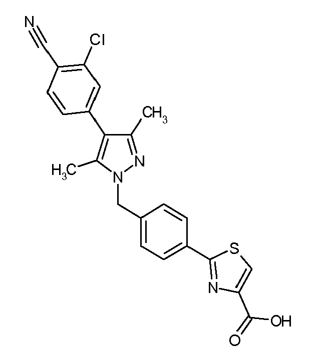

Ethyl 2-(4-{[4-(3-chloro-4-cyanophenyl)-3,5-dimethyl-1H-pyrazol-1-yl]methyl}phenyl)-1,3-thiazole-4-carboxylate (135.8 mg)synthesized in Example 275 was dissolved in methanol (1.4 mL), and 1 mol/L aqueous sodium hydroxide solution (0.57 mL) was added. The reaction mixture was stirred at 50°C for 3 hr and poured into 1 mol/L hydrochloric acid, and the mixture was extracted with ethyl acetate. The organic layer was washed with saturated brine, dried over anhydrous magnesium sulfate and concentrated to give the title compound (135.8 mg) as colorless crystals.
¹H-NMR (CDCl₃) δ: 2.23 (3H, s), 2.31 (3H, s), 5.35 (2H, s), 7.22-7.30 (3H, m), 7.40 (1H, d), 7.70 (1H, d), 7.95 (2H, d), 8.24 (1H, s).

### Example 277

### 2-chloro-4-(1-{4-[4-(1-hydroxy-1-methylethyl)-1,3-thiazol-2-yl]benzyl}-3,5-dimethyl-1H-pyrazol-4-yl)benzonitrile

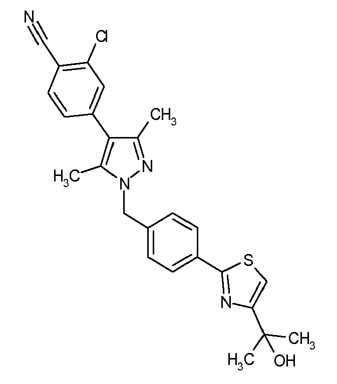

The title compound (46.6 mg) was obtained as colorless crystals in the same manner as in Example 124 and using ethyl 2-(4-{[4-(3-chloro-4-cyanophenyl)-3,5-dimethyl-1H-pyrazol-1-yl]methyl}phenyl)-1,3-thiazole-4-carboxylate (82.3 mg) synthesized in Example 275 as a starting material.
¹H-NMR (CDCl₃) δ: 1.64 (6H, s), 2.21 (3H, s), 2.31 (3H, s), 5.32 (2H, s), 7.09 (1H, s), 7.18-7.27 (3H, m), 7.39 (1H, d), 7.69 (1H, d), 7.92 (2H, d).

### Example 278

### 2-(4-{[4-(3-chloro-4-cyanophenyl)-3,5-dimethyl-1H-pyrazol-1-yl]methyl}phenyl)-1,3-thiazole-4-carboxamide

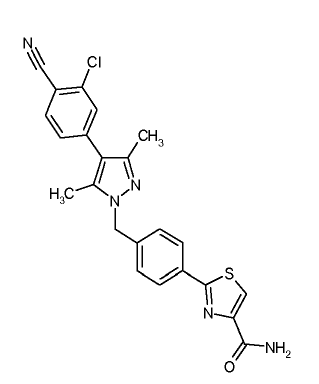

2-(4-{[4-(3-Chloro-4-cyanophenyl)-3,5-dimethyl-1H-pyrazol-1-yl]methyl}phenyl)-1,3-thiazole-4-carboxylic acid (101.4 mg) synthesized in Example 276 and 1-hydroxybenzotriazole ammonium salt (51.6 mg) were dissolved in DMF (1.1 mL), and WSC (65.0 mg) was added. The reaction mixture was stirred at room temperature for 3.5 hr and poured into 1 mol/L hydrochloric acid, and the mixture was extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate), and recrystallized from ethyl acetate-hexane to give the title compound (64.5 mg) as a white solid.
¹H-NMR (CDCl₃) δ: 2.23 (3H, s), 2.31 (3H,s), 5.34 (2H, s), 5.68 (1H, brs), 7.20 - 7.29 (4H, m), 7.40 (1H, d), 7.70 (1H, d), 7.93 (2H, d), 8.15 (1H, s).

### Example 279

### 4-{[4-(3-chloro-4-cyanophenyl)-3,5-dimethyl-1H-pyrazol-1-yl]methyl}-N-[(1-hydroxycyclopropyl)methyl]benzamide

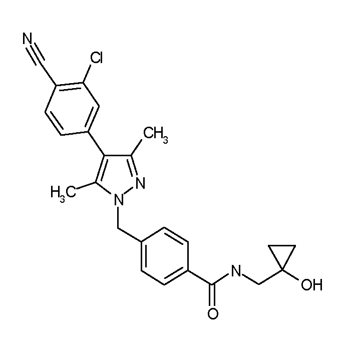

4-{[4-(3-Chloro-4-cyanophenyl)-3,5-dimethyl-1H-pyrazol-1-yl]methyl}benzoic acid (102.1 mg)synthesized in Example 52 and 1-(aminomethyl)cyclopropanol (29.2 mg) were dissolved in DMF (1.4 mL), and WSC (64.2 mg) and HOBt (45.3 mg) were added. The reaction mixture was stirred at room temperature for 1 hr and poured into 1 mol/L hydrochloric acid, and the mixture was extracted with ethyl acetate. The organic layer was washed successively with water, saturated aqueous sodium hydrogen carbonate, saturated brine, dried over anhydrous magnesium sulfate and concentrated. The obtained residue was purified by column chromatography (hexane-ethyl acetate) to give the title compound (86.2 mg) as colorless crystals.
¹H-NMR (CDCl₃) δ: 0.64-0.74 (2H, m), 0.82-0.92 (2H, m), 2.19 (3H, s), 2.30 (3H, s), 3.08 (1H, s), 3.59 (2H, d), 5.33 (2H, s), 6.59 (1H, brs), 7.15-7.28 (3H, m), 7.39 (1H, d), 7.70 (1H, d), 7.79 (2H, d).

### Example 280

### methyl N-[(4-{[4-(3-chloro-4-cyanophenyl)-3,5-dimethyl-1H-pyrazol-1-yl]methyl}phenyl)carbonyl]glycinate

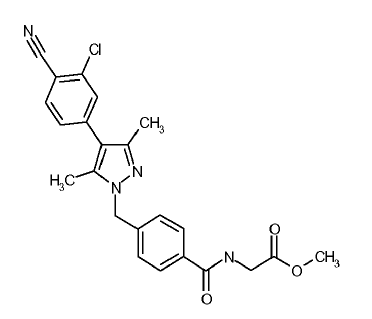

4-{[4-(3-Chloro-4-cyanophenyl)-3,5-dimethyl-1H-pyrazol-1-yl]methyl}benzoic acid (477.4 mg) synthesized in Example 52, glycine methyl ester hydrochloride (196.6 mg) and triethylamine (0.27 mL) were dissolved in DMF (6.5 mL), and WSC (301.0 mg) and HOBt (212.1 mg) were added. The reaction mixture was stirred at room temperature for 1 hr and poured into 1 mol/L hydrochloric acid, and the mixture was extracted with ethyl acetate. The organic layer was washed successively with water, saturated aqueous sodium hydrogen carbonate and saturated brine, dried over anhydrous magnesium sulfate and concentrated. The obtained residue was purified by column chromatography (hexane-ethyl acetate) to give the title compound (467.6 mg) as colorless crystals.
¹H-NMR (CDCl₃) δ: 2.19 (3H, s), 2.30 (3H, s), 3.81 (3H, s), 4.25 (2H, d), 5.33 (2H, s), 6.60 (1H, brs), 7.17-7.28 (3H, m), 7.40 (1H, d), 7.70 (1H, d), 7.79 (2H, d).

### Example 281

### 2-chloro-4-{3,5-dimethyl-1-[4-(1,3,4-oxadiazol-2-yl)benzyl]-1H-pyrazol-4-yl}benzonitrile

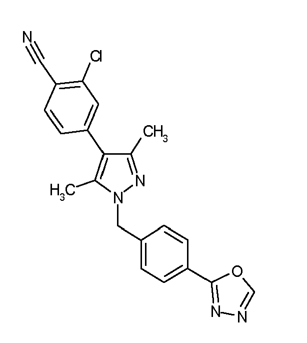

4-{[4-(3-Chloro-4-cyanophenyl)-3,5-dimethyl-1H-pyrazol-1-yl]methyl}benzoic acid (202.6 mg) synthesized in Example 52 and formic acid hydrazide (39.9 mg) were dissolved in THF/isopropanol (1:1)(5.6 mL), and DMT-MM (196.0 mg) was added. The reaction mixture was stirred at room temperature for 22 hr, poured into saturated aqueous ammonium chloride solution, and the mixture was extracted with ethyl acetate. The organic layer was washed successively with saturated aqueous sodium hydrogen carbonate and saturated brine, dried over anhydrous magnesium sulfate and concentrated. A mixture of the obtained residue (269.1 mg), tosylic acid monohydrate (105.4 mg) and toluene (2.8 mL) was stirred for 4 hr under heated reflux. The reaction mixture was poured into saturated aqueous sodium hydrogen carbonate, and the mixture was extracted with ethyl acetate. The organic layer was washed successively with water and saturated brine, dried over anhydrous magnesium sulfate and concentrated. The obtained residue was purified by column chromatography (hexane-ethyl acetate), and recrystallized from ethyl acetate-hexane to give the title compound (13.0 mg) as colorless crystals.
¹H-NMR (CDCl₃) δ: 2.22 (3H, s), 2.31 (3H, s), 5.36 (2H, s), 7.24-7.31 (3H, m), 7.41 (1H, d), 7.70 (1H, d), 8.07 (2H, d), 8.47 (1H, s).

### Example 282

### 4-{[4-(3-chloro-4-cyanophenyl)-3,5-dimethyl-1H-pyrazol-1-yl]methyl}-N-(2-hydroxy-2-methylpropyl)benzamide

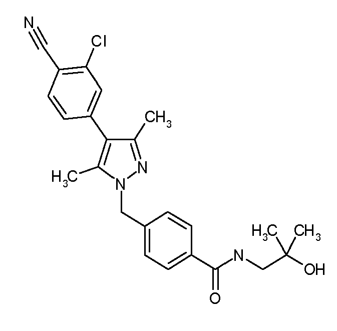

The title compound (382.4 mg) was obtained as colorless crystals in the same manner as in Example 279 and using 4-{[4-(3-chloro-4-cyanophenyl)-3,5-dimethyl-1H-pyrazol-1-yl]methyl}benzoic acid (460.5 mg) synthesized in Example 52 and 1-amino-2-methylpropan-2-ol (123.4 mg) as starting materials.
¹H-NMR (CDCl₃) δ: 1.29 (6H, s), 2.08 (1H, brs), 2.19 (3H, s), 2.30 (3H, s), 3.47 (2H, d), 5.33 (2H, s), 6.53 (1H, t), 7.21 (2H, d), 7.25 (1H, dd), 7.39 (1H, d), 7.69 (1H, d), 7.77 (2H, d).

### Example 283

### 4-{[4-(3-chloro-4-cyanophenyl)-3,5-dimethyl-1H-pyrazol-1-yl]methyl}-N-(2-ethyl-2-hydroxybutyl)benzamide

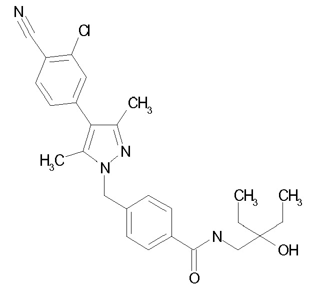

Methyl N-[(4-{[4-(3-chloro-4-cyanophenyl)-3,5-dimethyl-1H-pyrazol-1-yl]methyl}phenyl)carbonyl]glycinate (90.2 mg) synthesized in Example 280 was dissolved in THF (1.1 mL), ice-cooled, and 3 mol/L ethylmagnesium bromide THF solution (0.34 mL) was added. The reaction mixture was stirred at 0°C for 1.5 hr and poured into 1 mol/L hydrochloric acid, and the mixture was extracted with ethyl acetate. The organic layer was washed successively with water, saturated aqueous sodium hydrogen carbonate and saturated brine, dried over anhydrous magnesium sulfate and concentrated. The obtained residue was purified by column chromatography (hexane-ethyl acetate) to give the title compound (29.2 mg) as colorless crystals.
¹H-NMR (CDCl₃) δ: 0.92 (6H, t), 1.54 (4H, q), 2.19 (3H, s), 2.30 (3H, s), 3.48 (2H, d), 5.33 (2H, s), 6.48 (1H, brs), 7.15-7.29 (3H, m), 7.39 (1H, d), 7.69 (1H, d), 7.77 (2H, d).

### Example 284

### 2-chloro-4-(1-{[6-(4,5-dimethyl-1,3-thiazol-2-yl)pyridin-3-yl]methyl}-3,5-dimethyl-1H-pyrazol-4-yl)benzonitrile

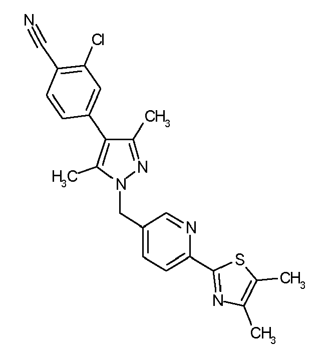

5-{[4-(3-Chloro-4-cyanophenyl)-3,5-dimethyl-1H-pyrazol-1-yl]methyl}pyridine-2-carboxamide (122.0 mg) synthesized in Example 105 was suspended in toluene (1.6 mL), and a Lawesson reagent (80.9 mg) was added. The reaction mixture was stirred with heating under reflux for 1.5 hr and poured into saturated aqueous sodium hydrogen carbonate, and the mixture was extracted with ethyl acetate. The organic layer was washed with saturated brine, dried over anhydrous magnesium sulfate and concentrated. The obtained residue was washed with ether-hexane to give 5-{[4-(3-chloro-4-cyanophenyl)-3,5-dimethyl-1H-pyrazol-1-yl]methyl}pyridine-2-carbothioamide (65.2 mg) as yellow crystals. The obtained crystals were suspended in ethanol (0.9 mL), and 3-bromo-2-butanone (20.0 µL) was added. The reaction mixture was stirred with heating under reflux overnight. After stirring, the mixture was poured into water, and extracted with ethyl acetate. The organic layer was washed with saturated brine, dried over anhydrous magnesium sulfate and concentrated. The obtained residue was purified by column chromatography (hexane-ethyl acetate), and recrystallized from ethyl acetate-hexane to give the title compound (40.3 mg) as colorless crystals.
¹H-NMR (CDCl₃) δ: 2.24 (3H, s), 2.29 (3H, s), 2.39 (3H, s), 2.42 (3H, s), 5.31 (2H, s), 7.24 (1H, dd), 7.38 (1H, d), 7.58 (1H, dd), 7.70 (1H, d), 8.09 (1H, d), 8.36 (1H, d).

### Example 285

### N-[(4-{[4-(3-chloro-4-cyanophenyl)-3,5-dimethyl-1H-pyrazol-1-yl]methyl}phenyl)carbonyl]glycine

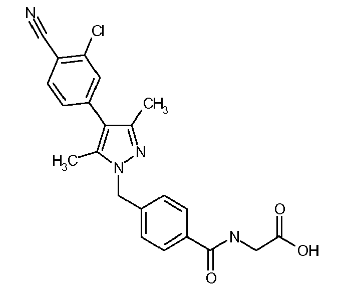

Methyl N-[(4-{[4-(3-chloro-4-cyanophenyl)-3,5-dimethyl-1H-pyrazol-1-yl]methyl}phenyl)carbonyl]glycinate (250.3 mg) synthesized in Example 280 was dissolved in methanol (2.8 mL), and 1 mol/L aqueous sodium hydroxide solution (1.15 mL) was added. The reaction mixture was stirred at room temperature for 15 min, poured into 1 mol/L hydrochloric acid and sodium chloride was added, and the mixture was extracted with ethyl acetate. The organic layer was washed with saturated brine, dried over anhydrous magnesium sulfate and concentrated. The obtained residue was washed with diisopropyl ether to give the title compound (230.6 mg) as colorless crystals.
¹H-NMR (DMSO-d₆) δ: 2.22 (3H, s), 2.26 (3H, s), 3.91 (2H, d), 5.38 (2H, s), 7.28 (2H, d), 7.49 (1H, dd), 7.68 (1H, d), 7.84 (2H, d), 7.99 (1H, d), 8.81 (1H, t), 12.53 (1H, brs).

### Example 286

### 2-chloro-4-{3,5-dimethyl-1-[4-(5-methyl-1,3,4-oxadiazol-2-yl)benzyl]-1H-pyrazol-4-yl}benzonitrile

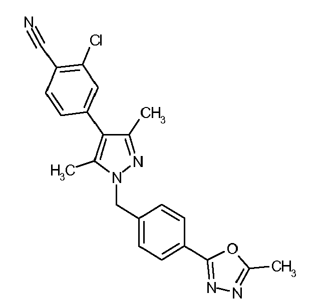

4-{[4-(3-Chloro-4-cyanophenyl)-3,5-dimethyl-1H-pyrazol-1-yl]methyl}benzoic acid (111.2 mg) synthesized in Example 52 and acetohydrazide (27.0 mg) were dissolved in THF/isopropanol (1:1)(1.5 mL), and DMT-MM (107.5 mg) was added. The reaction mixture was stirred at room temperature for 22.5 hr, and acetohydrazide (27.0 mg) and DMT-MM (107.5 mg) were added. The reaction mixture was further stirred at room temperature for 5 hr and poured into 1 mol/L hydrochloric acid, and the mixture was extracted with ethyl acetate. The organic layer was washed with saturated brine, dried over anhydrous magnesium sulfate and concentrated. A mixture of the obtained residue (117.8 mg), tosylic acid monohydrate (53.1 mg) and toluene-DMF (7:1, 1.6 mL) was stirred for 15 hr with heating under reflux. The reaction mixture was poured into saturated aqueous sodium hydrogen carbonate, and the mixture was extracted with ethyl acetate. The organic layer was washed successively with water and saturated brine, dried over anhydrous magnesium sulfate and concentrated. The obtained residue was purified by column chromatography (hexane-ethyl acetate), and recrystallized from ethyl acetate-hexane to give the title compound (56.0 mg) as colorless crystals.
¹H-NMR (CDCl₃) δ: 2.22 (3H, s), 2.31 (3H, s), 2.62 (3H, s), 5.35 (2H, s), 7.20-7.31 (3H, m), 7.41 (1H, d), 7.70 (1H, d), 8.01 (2H, d).

### Example 287

### N-(2-amino-2-oxoethyl)-4-{[4-(3-chloro-4-cyanophenyl)-3,5-dimethyl-1H-pyrazol-1-yl]methyl}benzamide

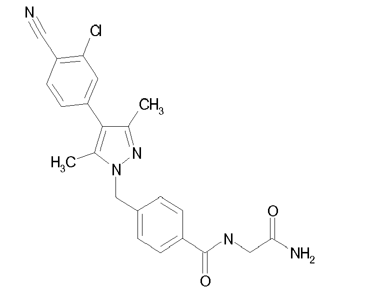

The title compound (584.9 mg) was obtained as colorless crystals in the same manner as in Example 278 and using N-[(4-{[4-(3-chloro-4-cyanophenyl)-3,5-dimethyl-1H-pyrazol-1-yl]methyl}phenyl)carbonyl]glycine (803.8 mg) synthesized in Example 285 as a starting material.
¹H-NMR (CDCl₃) δ: 2.19 (3H, s), 2.30 (3H, s), 4.16 (2H, d), 5.34 (2H, s), 5.45 (1H, brs), 5.89 (1H, brs), 6.87 (1H, brs), 7.19-7.27 (3H, m), 7.40 (1H, d), 7.70 (1H, d), 7.80 (2H, d).

### Example 288

### 4-{[4-(3-chloro-4-cyanophenyl)-3,5-dimethyl-1H-pyrazol-1-yl]methyl}-N-[2-(dimethylamino)-2-oxoethyl]benzamide

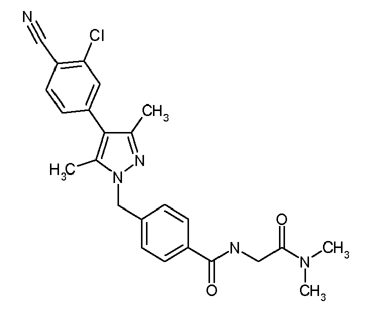

The title compound (17.1 mg) was obtained as colorless crystals in the same manner as in Example 280 and using N-[(4-{[4-(3-chloro-4-cyanophenyl)-3,5-dimethyl-1H-pyrazol-1-yl]methyl}phenyl)carbonyl]glycine (68.3 mg) synthesized in Example 285 and dimethylamine hydrochloride (15.8 mg) as starting materials.
¹H-NMR (CDCl₃) δ: 2.19 (3H, s), 2.30 (3H, s), 3.04 (3H, s), 3.04 (3H, s), 4.22 (2H, d), 5.33 (2H, s), 7.16-7.27 (3H, m), 7.40 (1H, d), 7.69 (1H, d), 7.82 (2H, d).

### Example 289

### Ethyl 1-[4-(tert-butoxycarbonyl)benzyl]-4-(3-chloro-4-cyanophenyl)-5-methyl-1H-pyrazole-3-carboxylate

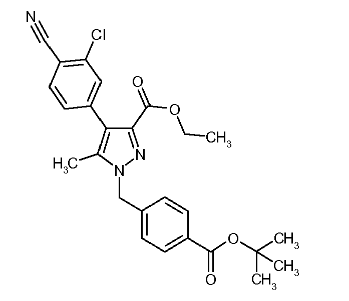

Ethyl 4-bromo-1-[4-(tert-butoxycarbonyl)benzyl]-5-methyl-1H-pyrazole-3-carboxylate (1.78 g) synthesized in Reference Example 24, 2-chloro-4-(4,4,5,5-tetramethyl-1,3,2-dioxaborolan-2-yl)benzonitrile (2.21 g) synthesized in Reference Example 25 and triethylamine (1.17 mL) were dissolved in THF-water (6:1)(9.8 mL), and 1,1'-bis(diphenylphosphino)ferrocene palladium (II) dichloride dichloromethane complex (1:1)(343.0 mg) was added under a nitrogen atmosphere. The reaction mixture was stirred with heating under reflux for 8 hr, poured into saturated aqueous ammonium chloride solution, and the mixture was extracted with ethyl acetate. The organic layer was washed with saturated brine, dried over anhydrous magnesium sulfate and concentrated. The obtained residue was purified by column chromatography (hexane-ethyl acetate) to give the title compound (1.10 g) as colorless crystals.
¹H-NMR (CDCl₃) δ: 1.29 (3H, t), 1.57 (9H, s), 2.09 (3H, s), 4.33 (2H, q), 5.49 (2H, s), 7.20 (2H, d), 7.29 (1H, dd), 7.45 (1H, d), 7.68 (1H, d), 7.98 (2H, d).

### Example 290

### 4-{[4-(3-chloro-4-cyanophenyl)-3,5-dimethyl-1H-pyrazol-1-yl]methyl}-N-(cyanomethyl)benzamide

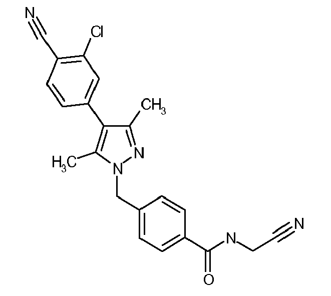

N-(2-Amino-2-oxoethyl)-4-{[4-(3-chloro-4-cyanophenyl)-3,5-dimethyl-1H-pyrazol-1-yl]methyl}benzamide (46.5 mg) synthesized in Example 287 and pyridine (28.0 µL) were dissolved in DMF (0.55 mL), cooled in an ice bath, and oxalyl chloride (19.2 µL) was added dropwise. The reaction mixture was stirred at the same temperature for 30 min. After stirring, the mixture was poured into hydrochloric acid, and extracted with ethyl acetate. The organic layer was washed with saturated aqueous sodium hydrogen carbonate and then saturated brine, dried over anhydrous magnesium sulfate and concentrated. The obtained residue was purified by column chromatography (hexane-ethyl acetate), and recrystallized from ethyl acetate-hexane to give the title compound (28.3 mg) as colorless crystals.
¹H-NMR (CDCl₃) δ: 2.20 (3H, s), 2.30 (3H, s), 4.39 (2H, d), 5.34 (2H, s), 6.44 (1H, brs), 7.19-7.29 (3H, m), 7.40 (1H, d), 7.70 (1H, d), 7.76 (2H, d).

### Example 291

### 4-{[4-(3-chloro-4-cyanophenyl)-3-(ethoxycarbonyl)-5-methyl-1H-pyrazol-1-yl]methyl}benzoic acid

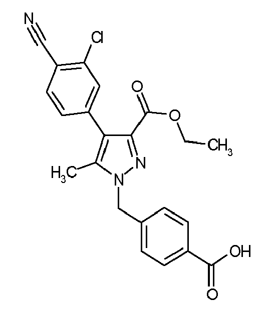

A mixture of ethyl 1-[4-(tert-butoxycarbonyl)benzyl]-4-(3-chloro-4-cyanophenyl)-5-methyl-1H-pyrazole-3-carboxylate (271.6 mg) synthesized in Example 289 and TFA (2.8 mL) was stirred at room temperature for 30 min. The reaction mixture was concentrated, and the residue was dissolved in ethyl acetate. The obtained solution was washed with water, dried over anhydrous magnesium sulfate and concentrated. The obtained residue was recrystallized from ethyl acetate-hexane to give the title compound (226.0 mg) as colorless crystals.
¹H-NMR (CDCl₃) δ: 1.29 (3H, t), 2.12 (3H, s), 4.33 (2H, q), 5.52 (2H, s), 7.26 (2H, d,), 7.31 (1H, dd), 7.47 (1H, d), 7.69 (1H, d), 8.09 (2H, d).

### Example 292

### Ethyl 1-(4-carbamoylbenzyl)-4-(3-chloro-4-cyanophenyl)-5-methyl-1H-pyrazole-3-carboxylate

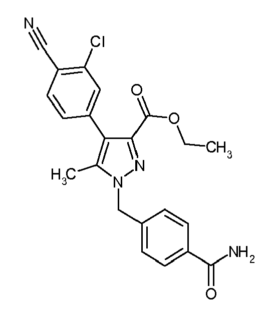

The title compound (201.5 mg) was obtained as colorless crystals in the same manner as in Example 278 and using 4-{[4-(3-chloro-4-cyanophenyl)-3-(ethoxycarbonyl)-5-methyl-1H-pyrazol-1-yl]methyl}benzoic acid (203.6 mg) synthesized in Example 291 as a starting material.
¹H-NMR (CDCl₃) δ: 1.29 (3H, t), 2.11 (3H, s), 4.33 (2H, q), 5.49 (2H, s), 5.63 (1H, brs), 6.01 (1H, brs), 7.25 (2H, d), 7.30 (1H, dd), 7.46 (1H, d), 7.69 (1H, d), 7.81 (2H, d).

### Example 293

### 1-[4-(tert-butoxycarbonyl)benzyl]-4-(3-chloro-4-cyanophenyl)-5-methyl-1H-pyrazole-3-carboxylic acid

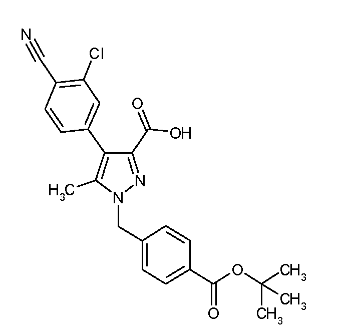

Ethyl 1-[4-(tert-butoxycarbonyl)benzyl]-4-(3-chloro-4-cyanophenyl)-5-methyl-1H-pyrazole-3-carboxylate (1.05 g) synthesized in Example 289 was suspended in methanol (8.8 mL), and 1 mol/L aqueous sodium hydroxide solution (4.38 mL) was added. The reaction mixture was stirred at 50°C for 7 hr, and poured into aqueous citric acid solution, and the mixture was extracted with ethyl acetate. The organic layer was washed with saturated brine, dried over anhydrous magnesium sulfate and concentrated. The obtained residue was purified by column chromatography (hexane-ethyl acetate) to give two kinds of compounds. They were respectively recrystallized from ethyl acetate-hexane to give the title compound (500.0 mg) and the compound of Example 294, 1-(4-carboxybenzyl)-4-(3-chloro-4-cyanophenyl)-5-methyl-1H-pyrazole-3-carboxylic acid (226.3 mg), both as colorless crystals.
¹H-NMR (CDCl₃) δ: 1.58 (9H, s), 2.14 (3H, s), 5.46 (2H, s), 7.22 (2H, d), 7.32 (1H, dd), 7.46 (1H, d), 7.68 (1H, d), 7.99 (2H, d).

### Example 294

### 1-(4-carboxybenzyl)-4-(3-chloro-4-cyanophenyl)-5-methyl-1H-pyrazole-3-carboxylic acid

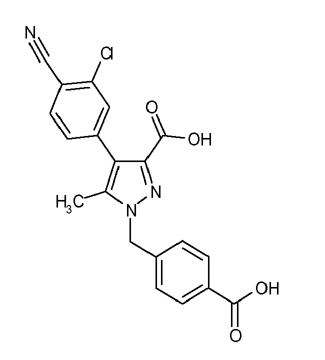

The title compound was obtained by the reaction described in Example 293.
¹H-NMR (DMSO-*d*₆) δ: 2.19 (3H, s), 5.56 (2H, s), 7.32 (2H, d), 7.49 (1H, dd), 7.74 (1H, d), 7.95 (2H, d), 7.98 (1H, d), 12.87 (2H, brs).

### Example 295

### 1-(4-carbamoylbenzyl)-4-(3-chloro-4-cyanophenyl)-5-methyl-1H-pyrazole-3-carboxamide

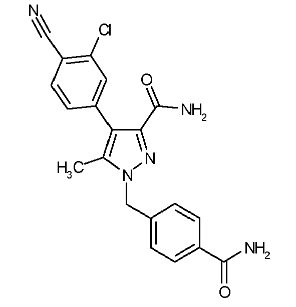

The title compound (57.4 mg) was obtained as colorless crystals in the same manner as in Example 278 and using 1-(4-carboxybenzyl)-4-(3-chloro-4-cyanophenyl)-5-methyl-1H-pyrazole-3-carboxylic acid (105.6 mg) synthesized in Example 294, HOBt ammonium salt (81.3 mg), and WSC (102.3 mg) as a condensation agent, as a starting material.
¹H-NMR (DMSO-*d*₆) δ: 2.20 (3H, s), 5.49 (2H, s), 7.28 (1H, brs), 7.30 (2H, d), 7.38 (1H, brs), 7.48 (1H, dd), 7.55 (1H, brs), 7.71 (1H, d), 7.86 (2H, d), 7.95 (1H, d), 7.96 (1H, brs).

### Example 296

### 4-{1-[(6-bromopyridin-3-yl)methyl]-3,5-dimethyl-1H-pyrazol-4-yl}-2-(trifluoromethyl)benzonitrile

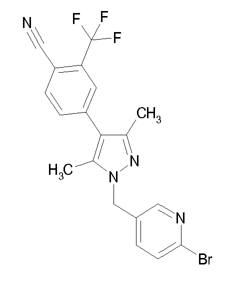

The title compound (750.9 mg) was obtained as colorless crystals in the same manner as in Example 18 and using 4-(3,5-dimethyl-1H-pyrazol-4-yl)-2-fluorobenzonitrile (1.00 g) synthesized in Reference Example 7 and 2-bromo-5-(chloromethyl)pyridine hydrochloride (1.10 g) synthesized in Reference Example 16 as starting materials.
¹H-NMR (CDCl₃) δ: 2.24 (3H, s), 2.29 (3H, s), 5.25 (2H, s), 7.40 (1H, dd), 7.48 (1H, m), 7.53 (1H, dd), 7.64 (1H, s), 7.88 (1H, d), 8.26 (1H, d).

### Example 297

### tert-butyl 4-{[3-carbamoyl-4-(3-chloro-4-cyanophenyl)-5-methyl-1H-pyrazol-1-yl]methyl}benzoate

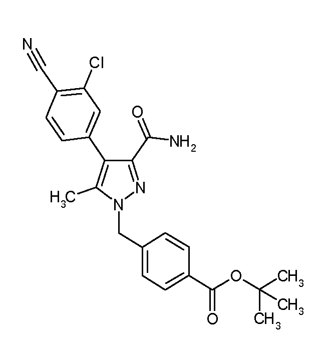

The title compound (365.5 mg) was obtained as colorless crystals in the same manner as in Example 278 and using 1-[4-(tert-butoxycarbonyl)benzyl]-4-(3-chloro-4-cyanophenyl)-5-methyl-1H-pyrazole-3-carboxylic acid (500.0 mg) synthesized in Example 293 as a starting material.
¹H-NMR (CDCl₃) δ: 1.59 (9H, s), 2. 14 (3H, s), 5.39 (2H, s), 5.41 (1H, brs), 6.82 (1H, brs), 7.19 (2H, d), 7.36 (1H, dd), 7.49 (1H, d), 7.65 (1H, d), 7.99 (2H, d).

### Example 298

### 4-{[4-(3-chloro-4-cyanophenyl)-3,5-dimethyl-1H-pyrazol-1-yl]methyl}-N-[2-(methylamino)-2-oxoethyl]benzamide

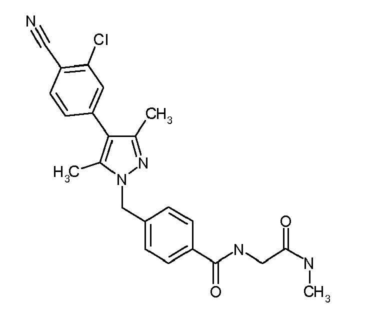

The title compound (26.0 mg) was obtained as colorless crystals in the same manner as in Example 280 and using N-[(4-{[4-(3-chloro-4-cyanophenyl)-3,5-dimethyl-1H-pyrazol-1-yl]methyl}phenyl)carbonyl]glycine (92.9 mg) synthesized in Example 285 and methylamine hydrochloride (17.8 mg) as starting materials.
¹H-NMR (CDCl₃) δ: 2.19 (3H, s), 2.30 (3H, s), 2.87 (3H, d), 4.09 (2H, d), 5.33 (2H, s), 5.93 (1H, brs), 6.91 (1H, brs), 7.21 (2H, d), 7.23-7.28 (1H, m), 7.39 (1H, d), 7.69 (1H, d), 7.79 (2H, d).

### Example 299

### Methyl 5-({4-[4-cyano-3-(trifluoromethyl)phenyl]-3,5-dimethyl-1H-pyrazol-1-yl}methyl)pyridine-2-carboxylate

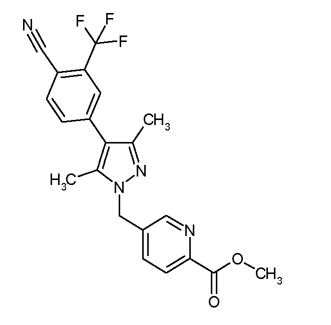

The title compound (444.2 mg) was obtained as colorless crystals in the same manner as in Example 20 and using 4-{1-[(6-bromopyridin-3-yl)methyl]-3,5-dimethyl-1H-pyrazol-4-yl}-2-(trifluoromethyl)benzonitrile (715.7 mg) synthesized in Example 296 as a starting material.
¹H-NMR (CDCl₃) δ: 2.24 (3H, s), 2.30 (3H, s), 4.01 (3H, s), 5.38 (2H, s), 7.53 (1H, dd), 7.59-7.69 (2H, m), 7.88 (1H, d), 8.13 (1H, d), 8.59 (1H, d).

### Example 300

### tert-butyl 4-{[4-(3-chloro-4-cyanophenyl)-3-cyano-5-methyl-1H-pyrazol-1-yl]methyl}benzoate

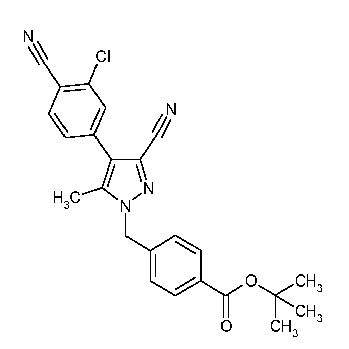

The title compound (115.7 mg) was obtained as colorless crystals in the same manner as in Example 290 and using tert-butyl 4-{[3-carbamoyl-4-(3-chloro-4-cyanophenyl)-5-methyl-1H-pyrazol-1-yl]methyl}benzoate (324.6 mg) synthesized in Example 297 as a starting material.
¹H-NMR (CDCl₃) δ: 1.59 (9H, s), 2.29 (3H, s), 5.44 (2H, s), 7.23 (1H, d), 7.23 (2H, d), 7.43 (1H, dd), 7.52 (1H, d), 8.00 (2H, d).

### Example 301

### 4-{[4-(3-chloro-4-cyanophenyl)-3,5-dimethyl-1H-pyrazol-1-yl]methyl}-N-ethylbenzamide

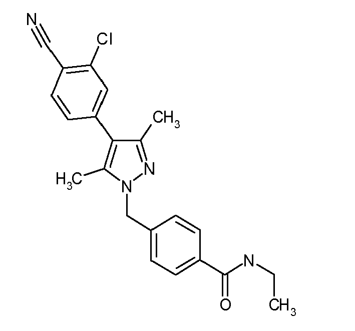

The title compound (104.2 mg) was obtained as colorless crystals in the same manner as in Example 280 and using 4-{[4-(3-chloro-4-cyanophenyl)-3,5-dimethyl-1H-pyrazol-1-yl]methyl}benzoic acid (150.0 mg) synthesized in Example 52 and ethylamine hydrochloride (40.1 mg) as starting materials.
¹H-NMR (CDCl₃) δ: 1.25 (3H, t), 2.19 (3H, s), 2.30 (3H, s), 3.42-3.57 (2H, m), 5.32 (2H, s), 6.03 (1H, brs), 7.19 (2H, d), 7.22-7.29 (1H, m), 7.39 (1H, s), 7.70 (1H, d), 7.73 (2H, d).

### Example 302

### 4-{[4-(3-chloro-4-cyanophenyl)-3,5-dimethyl-1H-pyrazol-1-yl]methyl}-N-propylbenzamide

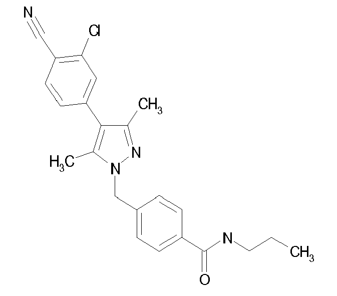

The title compound (86.4 mg) was obtained as colorless crystals in the same manner as in Example 279 and using 4-{[4-(3-chloro-4-cyanophenyl)-3,5-dimethyl-1H-pyrazol-1-yl]methyl}benzoic acid (153.7 mg) synthesized in Example 52 and propylamine (41.1 µL) as starting materials.
¹H-NMR (CDCl₃) δ: 0.98 (3H, t), 1.58-1.71 (2H, m), 2.19 (3H, s), 2.30 (3H, s), 3.30-3.58 (2H, m), 5.32 (2H, s), 6.06 (1H, brs), 7.19 (2H, d), 7.24 (1H, dd), 7.39 (1H, d), 7.69 (1H, d), 7.73 (2H, d).

### Example 303

### N-tert-butyl-4-{[4-(3-chloro-4-cyanophenyl)-3,5-dimethyl-1H-pyrazol-1-yl]methyl}benzamide

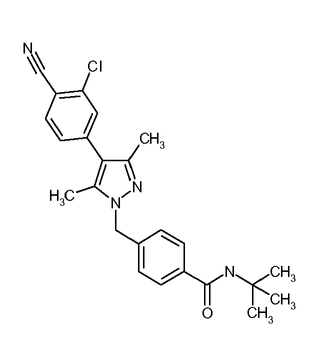

The title compound (99.9 mg) was obtained as colorless crystals in the same manner as in Example 279 and using 4-{[4-(3-chloro-4-cyanophenyl)-3,5-dimethyl-1H-pyrazol-1-yl]methyl}benzoic acid (146.3 mg) synthesized in Example 52 and tert-butylamine (50.4 µL) as starting materials.
¹H-NMR (CDCl₃) δ: 1.46 (9H, s), 2.18 (3H, s), 2.30 (3H, s), 5.32 (2H, s), 5.88 (1H, brs), 7.18 (2H, d), 7.24 (1H, dd), 7.38 (1H, d), 7.69 (3H, d).

### Example 304

### 4-{[4-(3-chloro-4-cyanophenyl)-3,5-dimethyl-1H-pyrazol-1-yl]methyl}-N-cyclopropylbenzamide

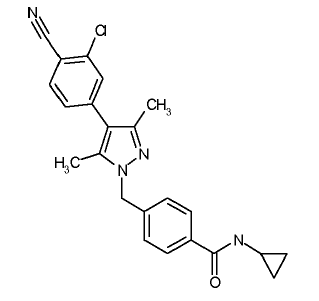

The title compound (2.88 g) was obtained as colorless crystals in the same manner as in Example 279 and using 4-{[4-(3-chloro-4-cyanophenyl)-3,5-dimethyl-1H-pyrazol-1-yl]methyl}benzoic acid (2.98 g) synthesized in Example 52 and cyclopropylamine (0.68 mL) as starting materials.
¹H-NMR (CDCl₃) δ: 0.56-0.65 (2H, m), 0.83-0.92 (2H, m), 2.18 (3H, s), 2.30 (3H, s), 2.84-2.94 (1H, m), 5.32 (2H, s), 6.20 (1H, brs), 7.18 (2H, d), 7.24 (1H, dd), 7.39 (1H, d), 7.69 (1H, d), 7.71 (2H, d).

### Example 305

### 4-{[4-(3-chloro-4-cyanophenyl)-3,5-dimethyl-1H-pyrazol-1-yl]methyl}-N-cyclobutylbenzamide

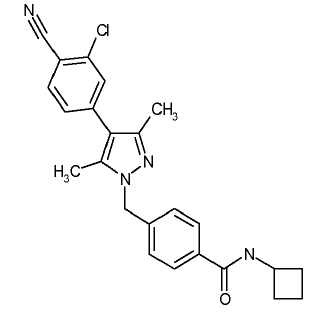

The title compound (108.6 mg) was obtained as colorless crystals in the same manner as in Example 279 and using 4-{[4-(3-chloro-4-cyanophenyl)-3,5-dimethyl-1H-pyrazol-1-yl]methyl}benzoic acid (157.7 mg) synthesized in Example 52 and cyclobutylamine (39.4 µL) as starting materials.
¹H-NMR (CDCl₃) δ: 1.70-1.83 (2H, m), 1.86-1.99 (2H, m), 2.18 (3H, s), 2.30 (3H, s), 2.37-2.51 (2H, m), 4.49-4.65 (1H, m), 5.32 (2H, s), 6.16 (1H, d), 7.19 (2H, d), 7.22-7.29 (1H, m), 7.39 (1H, d), 7.62-7.77 (3H, m).

### Example 306

### 4-{[4-(3-chloro-4-cyanophenyl)-3-cyano-5-methyl-1H-pyrazol-1-yl]methyl}benzoic acid

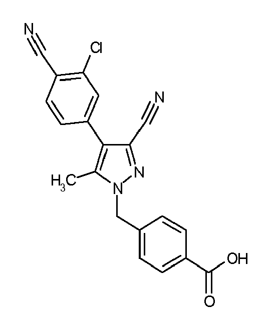

The title compound (86.6 mg) was obtained as colorless crystals in the same manner as in Example 291 and using tert-butyl 4-{[4-(3-chloro-4-cyanophenyl)-3-cyano-5-methyl-1H-pyrazol-1-yl]methyl}benzoate (99.5 mg) synthesized in Example 300 as a starting material.
¹H-NMR (DMSO-*d*₆) δ: 2.38 (3H, s), 5.64 (2H, s), 7.35 (2H, d), 7.67 (1H, dd), 7.90 (1H, d), 7.95 (2H, d), 8.13 (1H, d), 13.03 (1H, brs).

### Example 307

### 4-{1-[4-(azetidin-1-ylcarbonyl)benzyl]-3,5-dimethyl-1H-pyrazol-4-yl}-2-chlorobenzonitrile

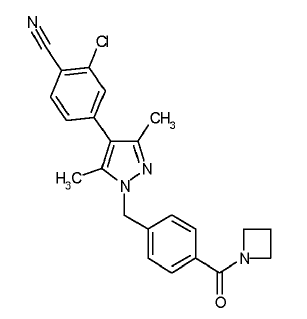

The title compound (34.8 mg) was obtained as colorless crystals in the same manner as in Example 280 and using 4-{[4-(3-chloro-4-cyanophenyl)-3,5-dimethyl-1H-pyrazol-1-yl]methyl}benzoic acid (111.0 mg) synthesized in Example 52 and azetidine hydrochloride (34.1 mg) as starting materials.
¹H-NMR (CDCl₃) δ: 2.19 (3H, s), 2.30 (3H, s), 2.28-2.42 (2H, m), 4.15-4.34 (4H, m), 5.31 (2H, s), 7.15 (2H, d), 7.22-7.29 (1H, m), 7.40 (1H, s), 7.61 (2H, d), 7.69 (1H, d).

### Example 308

### 4-{[4-(3-chloro-4-cyanophenyl)-3-cyano-5-methyl-1H-pyrazol-1-yl]methyl}benzamide

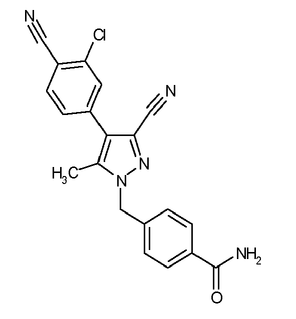

The title compound (65.7 mg) was obtained as colorless crystals in the same manner as in Example 278 and using 4-{[4-(3-chloro-4-cyanophenyl)-3-cyano-5-methyl-1H-pyrazol-1-yl]methyl}benzoic acid (82.1 mg) synthesized in Example 306 as a starting material.
¹H-NMR (CDCl₃) δ: 2.31 (3H, s), 5.45 (2H, s), 5.62 (1H, brs), 5.99 (1H, brs), 7.28 (2H, d), 7.43 (1H, dd), 7.53 (1H, d), 7.77 (1H, d), 7.83 (2H, d).

### Example 309

### 5-{[4-(3-chloro-4-cyanophenyl)-3,5-dimethyl-1H-pyrazol-1-yl]methyl}-N-ethylpyridine-2-carboxamide

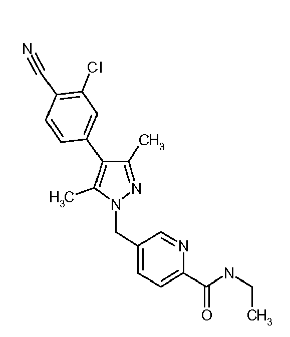

5-{[4-(3-Chloro-4-cyanophenyl)-3,5-dimethyl-1H-pyrazol-1-yl]methyl}pyridine-2-carboxylic acid (132.7 mg) synthesized in Example 54 and DMT-MM (127.9 mg) were dissolved in DMF (1.8 mL), and ethylamine hydrochloride (35.4 mg) and triethylamine (60.5 µL) were added. The reaction mixture was stirred at room temperature for 16 hr and poured into saturated aqueous sodium hydrogen carbonate, and the mixture was extracted with ethyl acetate. The organic layer was washed with saturated brine, dried over anhydrous magnesium sulfate and concentrated. The obtained residue was purified by column chromatography (hexane-ethyl acetate), and recrystallized from ethyl acetate-hexane to give the title compound (22.6 mg) as colorless crystals.
¹H-NMR (CDCl₃) δ: 1.26 (3H, t), 2.23 (3H, s), 2.29 (3H, s), 3.32-3.64 (2H, m), 5.35 (2H, s), 7.22-7.26 (1H, m), 7.39 (1H, d), 7.62 (1H, dd), 7.70 (1H, d), 7.93 (1H, brs), 8.18 (1H, d), 8.37 (1H, d).

### Example 310

### 5-{[4-(3-chloro-4-cyanophenyl)-3,5-dimethyl-1H-pyrazol-1-yl]methyl}-N-cyclopropylpyridine-2-carboxamide

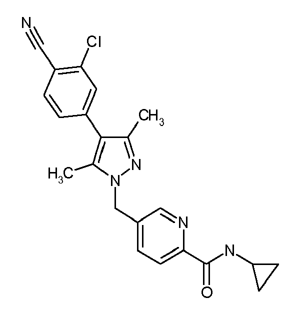

5-{[4-(3-Chloro-4-cyanophenyl)-3,5-dimethyl-1H-pyrazol-1-yl]methyl}pyridine-2-carboxylic acid (127.5 mg) synthesized in Example 54 and DMT-MM (122.9 mg) were dissolved in DMF (1.8 mL), and cyclopropylamine (28.9 µL) was added. The reaction mixture was stirred at room temperature for 16 hr and poured into saturated aqueous sodium hydrogen carbonate, and the mixture was extracted with ethyl acetate. The organic layer was washed with saturated brine, dried over anhydrous magnesium sulfate and concentrated. The obtained residue was purified by column chromatography (hexane-ethyl acetate), and recrystallized from ethyl acetate-hexane to give the title compound (30.2 mg) as colorless crystals.
¹H-NMR (CDCl₃) δ: 0.59-0.69 (2H, m), 0.83-0.93 (2H, m), 2.22 (3H, s), 2.28 (3H, s), 2.86-3.00 (1H, m), 5.34 (2H, s), 7.24 (1H, dd), 7.38 (1H, d), 7.62 (1H, dd), 7.70 (1H, d), 7.96 (1H, brs,), 8.18 (1H, d), 8.34 (1H, d).

### Example 311

### 5-({4-[4-cyano-3-(trifluoromethyl)phenyl]-3,5-dimethyl-1H-pyrazol-1-yl}methyl)pyridine-2-carboxamide

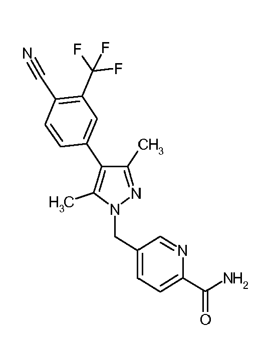

Methyl 5-({4-[4-cyano-3-(trifluoromethyl)phenyl]-3,5-dimethyl-1H-pyrazol-1-yl}methyl)pyridine-2-carboxylate (456.9 mg) synthesized in Example 299 was dissolved in methanol (5.5 mL), and 1 mol/L aqueous sodium hydroxide solution (1.32 mL) was added. The reaction mixture was stirred at room temperature for 2 hr, and concentrated. The residue was neutralized with saturated aqueous ammonium chloride solution, and extracted with isobutyl ether. The organic layer was washed with saturated brine, dried over anhydrous magnesium sulfate and concentrated. The residue was washed with ether to give 5-({4-[4-cyano-3-(trifluoromethyl)phenyl]-3,5-dimethyl-1H-pyrazol-1-yl}methyl)pyridine-2-carboxylic acid (396.0 mg). The title compound (44.3 mg) was obtained as colorless crystals in the same manner as in Example 78 and using the obtained 5-({4-[4-cyano-3-(trifluoromethyl)phenyl]-3,5-dimethyl-1H-pyrazol-1-yl}methyl)pyridine-2-carboxylic acid (121.9 mg) and aqueous ammonia (0.5 mL) as starting materials.
¹H-NMR (CDCl₃) δ: 2.25 (3H, s), 2.30 (3H, s), 5.38 (2H, s), 5.55 (1H, brs), 7.55 (1H, d), 7.63-7.69 (2H, m), 7.77 (1H, brs), 7.88 (1H, d), 8.20 (1H, d), 8.43 (1H, d).

### Example 312

### 5-({4-[4-cyano-3-(trifluoromethyl)phenyl]-3,5-dimethyl-1H-pyrazol-1-yl}methyl)-N-methylpyridine-2-carboxamide

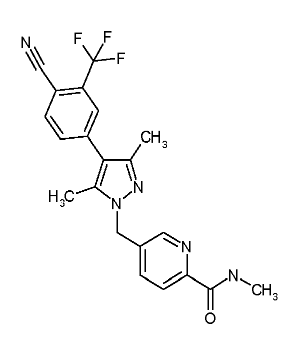

The title compound (59.7 mg) was obtained as colorless crystals in the same manner as in Example 78 and using intermediate 5-({4-[4-cyano-3-(trifluoromethyl)phenyl]-3,5-dimethyl-1H-pyrazol-1-yl}methyl)pyridine-2-carboxylic acid (116.5 mg) synthesized in Example 311 and 2M methylamine THF solution (0.5 mL) as starting materials.
¹H-NMR (CDCl₃) δ: 2.25 (3H, s), 2.30 (3H, s), 3.03 (3H, d), 5.36 (2H, s), 7.54 (1H, dd), 7.60-7.68 (2H, m), 7.88 (1H, d), 7.94 (1H, brs), 8.19 (1H, d), 8.39 (1H, d).

### Example 313

### 4-{[4-(4-cyano-3-methoxyphenyl)-3,5-dimethyl-1H-pyrazol-1-yl]methyl}benzamide

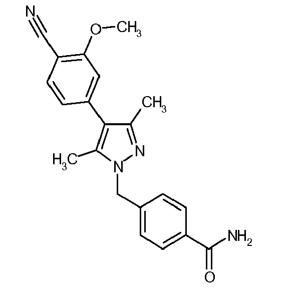

The title compound (119.8 mg) was obtained as colorless crystals in the same manner as in Example 271 and using 4-(3,5-dimethyl-1H-pyrazol-4-yl)-2-methoxybenzonitrile (158.9 mg) synthesized in Reference Example 21 and 4-(chloromethyl)benzamide (130.5 mg) as starting materials.
¹H-NMR (CDCl₃) δ: 2.20 (3H, s), 2.31 (3H, s), 3.94 (3H, s), 5.34 (2H, s), 5.60 (1H, brs), 6.02 (1H, brs), 6.82 (1H, d), 6.89 (1H, dd), 7.23 (2H, d), 7.58 (1H, d), 7.79 (2H, d).

### Example 314

### 4-{[4-(3-chloro-4-cyanophenyl)-3,5-dimethyl-1H-pyrazol-1-yl]methyl}-N-(pyridin-2-ylmethyl)benzamide

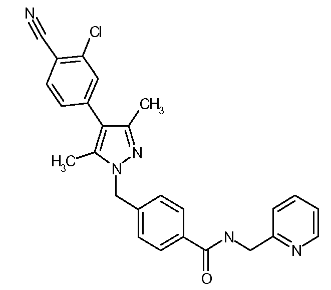

The title compound (17.0 mg) was obtained as colorless crystals in the same manner as in Example 279 and using 4-{[4-(3-chloro-4-cyanophenyl)-3,5-dimethyl-1H-pyrazol-1-yl]methyl}benzoic acid (112.9 mg) synthesized in Example 52 and 1-pyridin-2-ylmethanamine (40.1 mg) as starting materials.
¹H-NMR (CDCl₃) δ: 2.19 (3H, s), 2.30 (3H, s), 4.76 (2H, d), 5.34 (2H, s), 7.17-7.29 (4H, m), 7.32 (1H, d), 7.39 (1H, d), 7.60 (1H, brs), 7.65-7.75 (2H, m), 7.86 (2H, d), 8.55 (1H, d).

### Example 315

### 4-{[4-(3-chloro-4-cyanophenyl)-3,5-dimethyl-1H-pyrazol-1-yl]methyl}-N-(pyridin-3-ylmethyl)benzamide

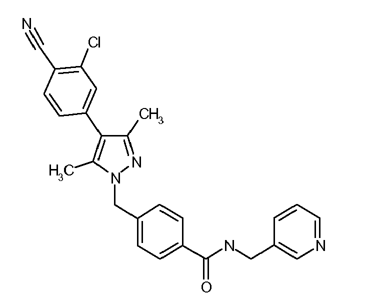

The title compound (24.1 mg) was obtained as colorless crystals in the same manner as in Example 279 and using 4-{[4-(3-chloro-4-cyanophenyl)-3,5-dimethyl-1H-pyrazol-1-yl]methyl}benzoic acid (102.8 mg) synthesized in Example 52 and 1-pyridin-3-ylmethanamine (40.1 mg) as starting materials.
¹H-NMR (CDCl₃) δ: 2.18 (3H, s), 2.30 (3H, s), 4.67 (2H, d), 5.33 (2H, s), 6.43 (1H, brs), 7.17-7.33 (4H, m), 7.38 (1H, d), 7.66-7.74 (2H, m), 7.77 (2H, d), 8.52-8.74 (2H, m).

### Example 316

### 4-{[4-(3-chloro-4-cyanophenyl)-3,5-dimethyl-1H-pyrazol-1-yl]methyl}-N-(pyridin-4-ylmethyl)benzamide

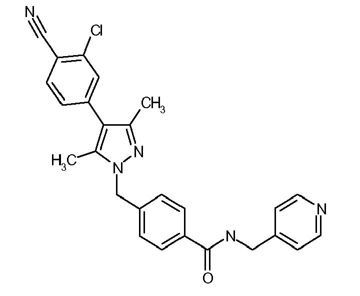

The title compound (31.0 mg) was obtained as colorless crystals in the same manner as in Example 279 and using 4-{[4-(3-chloro-4-cyanophenyl)-3,5-dimethyl-1H-pyrazol-1-yl]methyl}benzoic acid (110.2 mg) synthesized in Example 52 and 1-pyridin-4-ylmethanamine (39.1 mg) as starting materials.
¹H-NMR (CDCl₃) δ: 2.20 (3H, s), 2.30 (3H, s), 4.67 (2H, d), 5.34 (2H, s), 6.47 (1H, brs), 7.21-7.33 (5H, m), 7.39 (1H, d), 7.70 (1H, d), 7.80 (2H, d), 8.52-8.63 (2H, m).

### Example 317

### 4-{[4-(3-chloro-4-cyanophenyl)-3,5-dimethyl-1H-pyrazol-1-yl]methyl}-N-(thiophen-2-ylmethyl)benzamide

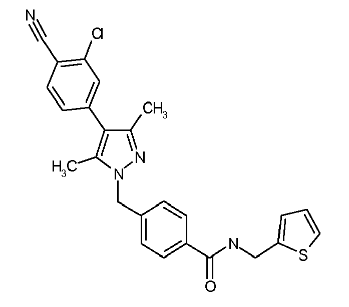

The title compound (25.5 mg) was obtained as colorless crystals in the same manner as in Example 279 and using 4-{[4-(3-chloro-4-cyanophenyl)-3,5-dimethyl-1H-pyrazol-1-yl]methyl}benzoic acid (104.5 mg) synthesized in Example 52 and 1-thiophen-2-ylmethanamine (38.8 mg) as starting materials.
¹H-NMR (CDCl₃) δ: 2.18 (3H, s), 2.29 (3H, s), 4.81 (2H, d), 5.32 (2H, s), 6.35 (1H, brs), 6.93-7.01 (1H, m), 7.02-7.08 (1H, m), 7.19 (2H, d), 7.21-7.28 (2H, m), 7.38 (1H, d), 7.69 (1H, d), 7.76 (2H, d).

### Example 318

### 2-chloro-4-(3,5-dimethyl-1-{[6-(1,3,4-oxadiazol-2-yl)pyridin-3-yl]methyl}-1H-pyrazol-4-yl)benzonitrile

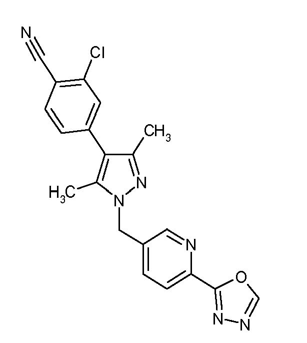

A mixture of 5-{[4-(3-chloro-4-cyanophenyl)-3,5-dimethyl-1H-pyrazol-1-yl]methyl}pyridine-2-carbohydrazide (106.4 mg) synthesized in Example 336, tosylic acid monohydrate (5.3 mg) and triethyl orthoformate (1 mL) was stirred at 140°C for 15.5 hr. After cooling, the reaction mixture was poured into saturated aqueous sodium hydrogen carbonate and extracted with ethyl acetate. The organic layer was washed with saturated brine, dried over anhydrous magnesium sulfate and concentrated. The residue was purified by column chromatography (hexane-ethyl acetate, basic silica gel) and recrystallized from ethyl acetate-hexane to give the title compound (26.7 mg) as colorless crystals.
¹H-NMR (CDCl₃) δ: 2.26 (3H, s), 2.29 (3H, s), 5.39 (2H, s), 7.21-7.28 (1H, m), 7.39 (1H, d), 7.66-7.74 (2H, m), 8.27 (1H, d), 8.56 (1H, s), 8.62 (1H, d).

### Example 319

### 2-chloro-4-(3,5-dimethyl-1-{[6-(5-methyl-1,3,4-oxadiazol-2-yl)pyridin-3-yl]methyl}-1H-pyrazol-4-yl)benzonitrile

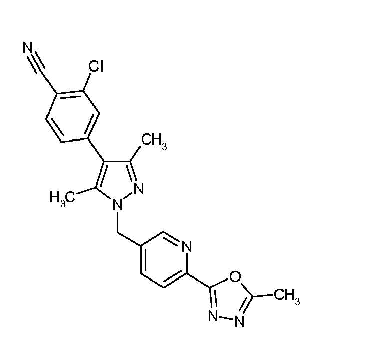

The title compound (54.5 mg) was obtained as colorless crystals in the same manner as in Example 318 and using 5-{[4-(3-chloro-4-cyanophenyl)-3,5-dimethyl-1H-pyrazol-1-yl]methyl}pyridine-2-carbohydrazide (179.7 mg) synthesized in Example 336 and triethyl orthoacetate (2 mL) as starting materials.
¹H-NMR (CDCl₃) δ: 2.25 (3H, s), 2.29 (3H, s), 2.66 (3H, s), 5.38 (2H, s), 7.22-7.28 (1H, m), 7.39 (1H, d), 7.66 (1H, dd), 7.71 (1H, d), 8.22 (1H, dd), 8.59 (1H, d).

### Example 320

### 5-{[4-(3-chloro-4-cyanophenyl)-3,5-dimethyl-1H-pyrazol-1-yl]methyl}-N-(2-hydroxy-2-methylpropyl)pyridine-2-carboxamide

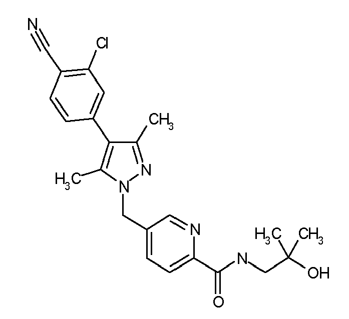

5-{[4-(3-Chloro-4-cyanophenyl)-3,5-dimethyl-1H-pyrazol-1-yl]methyl}pyridine-2-carboxylic acid (152.1 mg) synthesized in Example 54 and DMT-MM (44.4 mg) were dissolved in THF/isopropyl alcohol(1:1)(2.2 mL), and 1-amino-2-methylpropan-2-ol (44.4 mg) was added. The reaction mixture was stirred at room temperature for 20 hr and poured into water, and the mixture was extracted with ethyl acetate. The organic layer was washed with saturated brine, dried over anhydrous magnesium sulfate and concentrated. The obtained residue was purified by column chromatography (hexane-ethyl acetate) to give the title compound (35.6 mg) as a colorless solid.
¹H-NMR (CDCl₃) δ: 1.29 (6H, s), 2.24 (3H, s), 2.29 (3H, s), 2.40 (1H, brs), 3.48 (2H, d), 5.36 (2H, s), 7.19-7.28 (1H, m), 7.39 (1H, s), 7.64 (1H, d), 7.70 (1H, d), 8.18 (1H, d), 8.37 (1H, brs), 8.40 (1H, s).

### Example 321

### 4-{1-[(6-bromopyridin-3-yl)methyl]-3,5-dimethyl-1H-pyrazol-4-yl}-2-methoxybenzonitrile

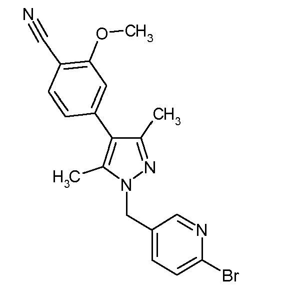

The title compound (1.04 g) was obtained as pale-yellow crystals according to the method in Example 18 and using 4-(3,5-dimethyl-1H-pyrazol-4-yl)-2-methoxybenzonitrile (1.43 g) synthesized in Reference Example 21 and 2-bromo-5-(chloromethyl)pyridine hydrochloride (1.84 g) synthesized in Reference Example 16 as starting materials, and washing with ether-hexane.
¹H-NMR (CDCl₃) δ: 2.22 (3H, s), 2.28 (3H, s), 3.94 (3H, s), 5.24 (2H, s), 6.80 (1H, s), 6.87 (1H, dd), 7.40 (1H, dd), 7.48 (1H, d), 7.58 (1H, d), 8.23 (1H, d).

### Example 322

### 4-{[4-(3-chloro-4-cyanophenyl)-3,5-dimethyl-1H-pyrazol-1-yl]methyl}benzenesulfonamide

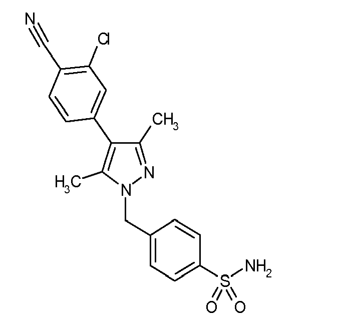

The title compound (25.9 mg) was obtained as colorless crystals in the same manner as in Example 271 and using 2-chloro-4-(3,5-dimethyl-1H-pyrazol-4-yl)benzonitrile (310.5 mg) synthesized in Reference Example 2 and 4-(bromomethyl)benzenesulfonamide (225.6 mg) as starting materials.
¹H-NMR (CDCl₃) δ: 2.21 (3H, s), 2.30 (3H, s), 4.74 (2H, brs), 5.35 (2H, s), 7.22-7.30 (3H, m), 7.40 (1H, d), 7.70 (1H, d), 7.91 (2H, d).

### Example 323

### Methyl 5-{[4-(4-cyano-3-methoxyphenyl)-3,5-dimethyl-1H-pyrazol-1-yl]methyl}pyridine-2-carboxylate

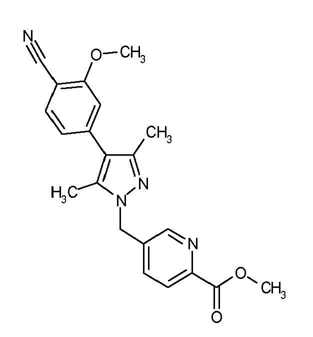

The title compound (609.7 mg) was obtained as colorless crystals according to the method in Example 20 and using 4-{1-[(6-bromopyridin-3-yl)methyl]-3,5-dimethyl-1H-pyrazol-4-yl}-2-methoxybenzonitrile (811.7 mg) synthesized in Example 321 as a starting material.
¹H-NMR (CDCl₃) δ: 2.22 (3H, s), 2.29 (3H, s), 3.94 (3H, s), 4.01 (3H, s), 5.37 (2H, s), 6.81 (1H, d), 6.87 (1H, dd), 7.59 (1H, d), 7.63 (1H, dd), 8.12 (1H, d), 8.56 (1H, d).

### Example 324

### 5-{[4-(4-cyano-3-methoxyphenyl)-3,5-dimethyl-1H-pyrazol-1-yl]methyl}-N-methylpyridine-2-carboxamide

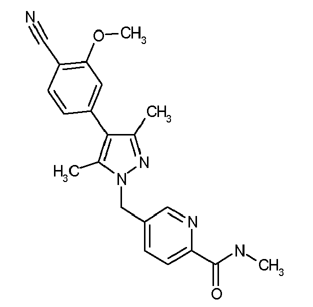

Methyl 5-{[4-(4-cyano-3-methoxyphenyl)-3,5-dimethyl-1H-pyrazol-1-yl]methyl}pyridine-2-carboxylate (112.5 mg) synthesized in Example 323 was dissolved in methanol (1.5 mL), and 2 mol/L methylamine THF solution (0.75 mL) was added. The reaction mixture was stirred with heating under reflux for 16 hr and poured into water, and the mixture was extracted with ethyl acetate. The organic layer was washed with saturated brine, dried over anhydrous magnesium sulfate and concentrated. The obtained residue was purified by column chromatography (hexane-ethyl acetate) to give the title compound (64.4 mg) as colorless crystals.
¹H-NMR (CDCl₃) δ: 2.23 (3H, s), 2.29 (3H, s), 3.03 (3H, d), 3.94 (3H, s), 5.35 (2H, s), 6.82 (1H, d), 6.88 (1H, dd), 7.58 (1H, d), 7.63 (1H, dd), 7.94 (1H, brs), 8.18 (1H, d), 8.38 (1H, d).

### Example 325

### 5-{[4-(4-cyano-3-methoxyphenyl)-3,5-dimethyl-1H-pyrazol-1-yl]methyl}pyridine-2-carboxamide

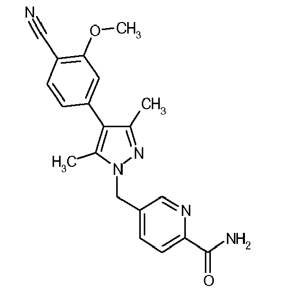

The title compound (49.3 mg) was obtained as colorless crystals according to the method in Example 324 and using methyl 5-{[4-(4-cyano-3-methoxyphenyl)-3,5-dimethyl-1H-pyrazol-1-yl]methyl}pyridine-2-carboxylate (112.8 mg) synthesized in Example 323 and 8 mol/L ammonia methanol solution (0.19 mL) as starting materials.
¹H-NMR (CDCl₃) δ: 2.23 (3H, s), 2.29 (3H, s), 3.95 (3H, s), 5.36 (2H, s), 5.54 (1H, brs), 6.82 (1H, s), 6.88 (1H, dd), 7.59 (1H, d), 7.65 (1H, dd), 7.77 (1H, brs), 8.19 (1H, d), 8.42 (1H, d).

### Example 326

### Methyl 6-{[4-(3-chloro-4-cyanophenyl)-3,5-dimethyl-1H-pyrazol-1-yl]methyl}pyridine-3-carboxylate

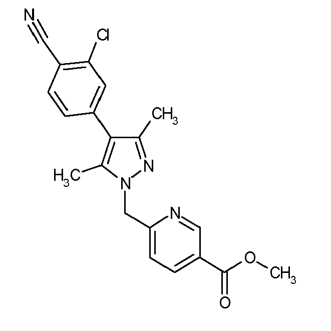

The title compound (784.0 mg) was obtained as colorless crystals in the same manner as in Example 20 and using 4-{1-[(5-bromopyridin-2-yl)methyl]-3,5-dimethyl-1H-pyrazol-4-yl}-2-chlorobenzonitrile (601.4 mg) synthesized in Example 208 as a starting material.
¹H-NMR (CDCl₃) δ: 2.26 (3H, s), 2.30 (3H, s), 3.95 (3H, s), 5.46 (2H, s), 7.05 (1H, d), 7.27 (1H, dd), 7.41 (1H, d), 7.70 (1H, d), 8.26 (1H, dd), 9. 17 (1H, d).

### Example 327

### 6-{[4-(3-chloro-4-cyanophenyl)-3,5-dimethyl-1H-pyrazol-1-yl]methyl}pyridine-3-carboxamide

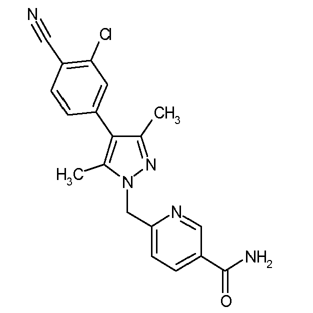

The title compound (35.7 mg) was obtained as colorless crystals in the same manner as in Example 278 and using 6-{[4-(3-chloro-4-cyanophenyl)-3,5-dimethyl-1H-pyrazol-1-yl]methyl}pyridine-3-carboxylic acid (118.3 mg) synthesized in Example 356 as a starting material.
¹H-NMR (CDCl₃) δ: 2.26 (3H, s), 2.30 (3H, s), 5.45 (2H, s), 5.68 (1H, brs), 5.98 (1H, brs), 7.10 (1H, d), 7.26 (1H, dd), 7.41 (1H, d), 7.70 (1H, d), 8.12 (1H, dd), 8.97 (1H, d).

### Example 328

### 6-{[4-(3-chloro-4-cyanophenyl)-3,5-dimethyl-1H-pyrazol-1-yl]methyl}-N-methylpyridine-3-carboxamide

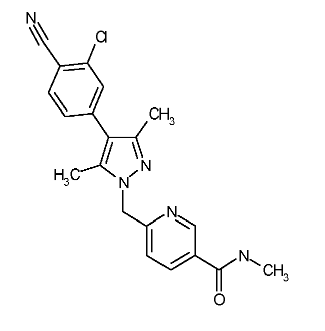

The title compound (33.5 mg) was obtained as colorless crystals in the same manner as in Example 279 and using 6-{[4-(3-chloro-4-cyanophenyl)-3,5-dimethyl-1H-pyrazol-1-yl]methyl}pyridine-3-carboxylic acid (91.5 mg) synthesized in Example 356 and 2 mol/L methylamine THF solution (0.19 mL) as starting materials.
¹H-NMR (CDCl₃) δ: 2.25 (3H, s), 2.30 (3H, s), 3.04 (3H, d), 5.44 (2H, s), 6.09 (1H, brs), 7.06 (1H, d), 7.26 (1H, dd), 7.41 (1H, d), 7.70 (1H, d), 8.06 (1H, dd), 8.91 (1H, d).

### Example 329

### 6-{[4-(3-chloro-4-cyanophenyl)-3,5-dimethyl-1H-pyrazol-1-yl]methyl}-N,N-dimethylpyridine-3-carboxamide

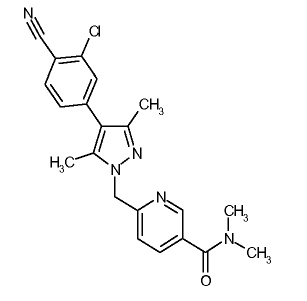

The title compound (47.7 mg) was obtained as colorless crystals in the same manner as in Example 279 and using 6-{[4-(3-chloro-4-cyanophenyl)-3,5-dimethyl-1H-pyrazol-1-yl]methyl}pyridine-3-carboxylic acid (90.4 mg) synthesized in Example 356 and 2 mol/L dimethylamine THF solution (0.19 mL) as starting materials.
¹H-NMR (CDCl₃) δ: 2.26 (3H, s), 2.30 (3H, s), 3.01 (3H, s), 3.13 (3H, s), 5.43 (2H, s), 7.03 (1H, d), 7.26 (1H, dd), 7.41 (1H, d), 7.70 (1H, d), 7.74 (1H, dd), 8.64 (1H, d).

### Example 330

### 4-{[4-(3-chloro-4-cyanophenyl)-3,5-dimethyl-1H-pyrazol-1-yl]methyl}-N,N-dimethylbenzenesulfonamide

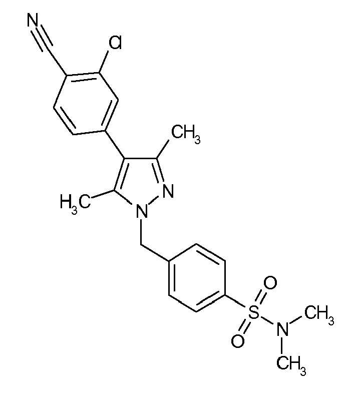

The title compound (38.7 mg) was obtained as colorless crystals in the same manner as in Example 1 and using 2-chloro-4-(3,5-dimethyl-1H-pyrazol-4-yl)benzonitrile (129.1 mg) synthesized in Reference Example 4 and 4-(bromomethyl)-N,N-dimethylbenzenesulfonamide (232.5 mg) as starting materials.
¹H-NMR (CDCl₃) δ: 2.22 (3H, s), 2.31 (3H, s), 2.71 (6H, s), 5.37 (2H, s), 7.23-7.30 (3H, m), 7.41 (1H, d), 7.71 (1H, d), 7.76 (2H, d).

### Example 331

### tert-butyl [(4-{[4-(3-chloro-4-cyanophenyl)-3,5-dimethyl-1H-pyrazol-1-yl]methyl}phenyl)sulfonyl]methylcarbamate

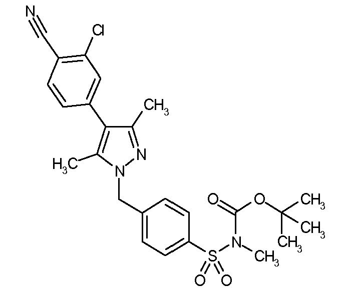

The title compound (199.2 mg) was obtained as a colorless solid according to the method in Example 1 and using 2-chloro-4-(3,5-dimethyl-1H-pyrazol-4-yl)benzonitrile (135.3 mg) synthesized in Reference Example 4 and tert-butyl {[4-(bromomethyl)phenyl]sulfonyl}methylcarbamate (255.6 mg) as starting materials.
¹H-NMR (CDCl₃) δ: 1.35 (9H, s), 2.19 (3H, s), 2.30 (3H, s), 3.34 (3H, s), 5.36 (2H, s), 7.22-7.28 (3H, m), 7.39 (1H, d), 7.71 (1H, d), 7.88 (2H, d).

### Example 332

### 4-{[4-(3-chloro-4-cyanophenyl)-3,5-dimethyl-1H-pyrazol-1-yl]methyl}-N-methylbenzenesulfonamide

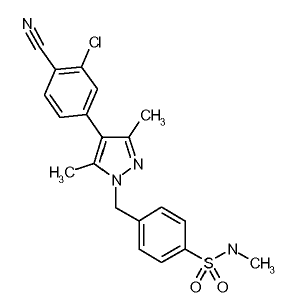

A mixture of tert-butyl [(4-{[4-(3-chloro-4-cyanophenyl)-3,5-dimethyl-1H-pyrazol-1-yl]methyl}phenyl)sulfonyl]methylcarbamate (154.6 mg) synthesized in Example 331 and TFA (1.5 mL) was stirred at room temperature for 10 min and poured into ice-cooled saturated aqueous sodium hydrogen carbonate, and the mixture was extracted with ethyl acetate. The organic layer was washed with saturated aqueous sodium hydrogen carbonate and saturated brine, dried over anhydrous sodium sulfate and concentrated. The obtained residue was washed with methanol to give the title compound (89.1 mg) as colorless crystals.
¹H-NMR (DMSO-*d*₆) δ: 2.21 (3H s), 2.28 (3H, s), 2.40 (3H, d), 5.41 (2H, s), 7.38 (1H, d), 7.45 (1H, q), 7.49 (2H, dd), 7.68 (1H, d), 7.76 (2H, d), 7.99 (1H, d).

### Example 333

### 4-{[4-(3-chloro-4-cyanophenyl)-3,5-dimethyl-1H-pyrazol-1-yl]methyl}-N-[(1-oxidepyridin-2-yl)methyl]benzamide

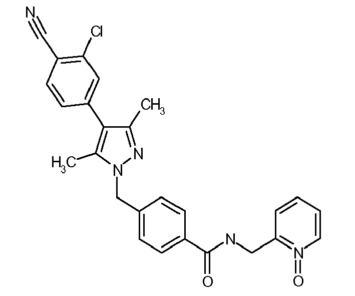

4-{[4-(3-Chloro-4-cyanophenyl)-3,5-dimethyl-1H-pyrazol-1-yl]methyl}-N-(pyridin-2-ylmethyl)benzamide (206.7 mg) synthesized in Example 314 was suspended in acetonitrile (2.3 mL), ice-cooled, and m-chloroperbenzoic acid (purity 70%, 134.1 mg) was added. The reaction mixture was stirred at room temperature for 4 days, m-chloroperbenzoic acid (purity 70%, 134.1 mg) was further added and the mixture was stirred for 6 hr. The reaction mixture was poured into saturated aqueous sodium carbonate solution, and the mixture was extracted with ethyl acetate. The organic layer was washed successively with water, saturated aqueous ammonium chloride solution and saturated brine, dried over anhydrous sodium sulfate and concentrated. The obtained residue was purified by column chromatography (ethyl acetate-methanol) to give the title compound (64.4 mg) as a colorless solid.
¹H-NMR (CDCl₃) δ: 2.16 (3H, s), 2.29 (3H, s), 4.81 (2H, d), 5.31 (2H, s), 7.17 (2H, d), 7.24 (1H, dd), 7.28-7.33 (2H, m), 7.38 (1H, d), 7.53 (1H, dd), 7.69 (1H, d), 7.77 (2H, d), 7.88 (1H, t), 8.25 (1H, dd).

### Example 334

### 4-{[4-(3-chloro-4-cyanophenyl)-3,5-dimethyl-1H-pyrazol-1-yl]methyl}-N-[(6-cyanopyridin-2-yl)methyl]benzamide

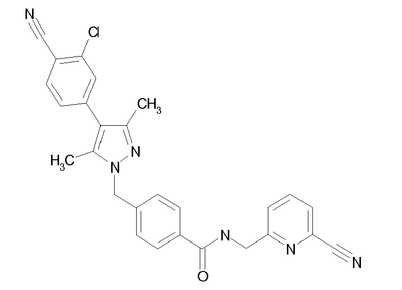

4-{[4-(3-Chloro-4-cyanophenyl)-3,5-dimethyl-1H-pyrazol-1-yl]methyl}-N-[(1-oxidopyridin-2-yl)methyl]benzamide (105.2 mg) synthesized in Example 333 and triethylamine (0.12 mL) were dissolved in acetonitrile (1.12 mL), and trimethylsilyl cyanide (83.7 µL) was added. The reaction mixture was stirred with heating under reflux for 18 hr, trimethylsilyl cyanide (83.7 µL) was further added and the mixture was stirred at the same temperature for 6 hr, trimethylsilyl cyanide (83.7 µL) was further added, and the mixture was stirred for 16 hr, poured into saturated aqueous sodium hydrogen carbonate, and the mixture was extracted with ethyl acetate. The organic layer was washed with saturated brine, dried over anhydrous sodium sulfate and concentrated. The obtained residue was purified by column chromatography (hexane-ethyl acetate), and recrystallized from ethyl acetate-hexane to give the title compound (10.8 mg) as a colorless solid.
¹H-NMR (CDCl₃) δ: 2.20 (3H, s), 2.31 (3H, s), 4.81 (2H, d), 5.35 (2H, s), 7.20-7.28 (4H, m), 7.34 (1H, d), 7.40 (1H, d), 7.58 (1H, dd), 7.65 (1H, d), 7.70 (1H, d), 7.84 (2H, d).

### Example 335

### 2-chloro-4-{3,5-dimethyl-1-[4-(methylsulfonyl)benzyl]-1H-pyrazol-4-yl}benzonitrile

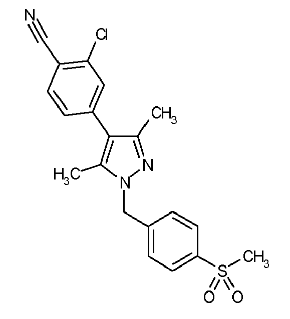

The title compound (115.4 mg) was obtained as colorless crystals in the same manner as in Example 271 and using 2-chloro-4-(3,5-dimethyl-1H-pyrazol-4-yl)benzonitrile (1123.0 mg) synthesized in Reference Example 4 and 1-(bromomethyl)-4-(methylsulfonyl)benzene (145.5 mg) as starting materials.
¹H-NMR (CDCl₃) δ: 2.22 (3H, s), 2.30 (3H, s), 3.04 (3H, s), 5.37 (2H, s), 7.26 (1H, dd), 7.32 (2H, d), 7.40 (1H, d), 7.71 (1H, d), 7.93 (2H, d).

### Example 336

### 5-{[4-(3-chloro-4-cyanophenyl)-3,5-dimethyl-1H-pyrazol-1-yl]methyl}pyridine-2-carbohydrazide

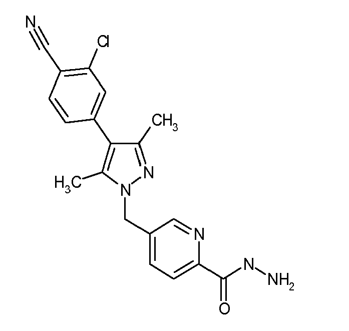

Methyl 5-{[4-(3-chloro-4-cyanophenyl)-3,5-dimethyl-1H-pyrazol-1-yl]methyl}pyridine-2-carboxylate (5.96 g) synthesized in Example 20 was dissolved in methanol (80 mL), and hydrazine monohydrate (3.80 mL) was added. The reaction mixture was stirred at room temperature for 1 hr, and ether (160 mL) was added. The reaction mixture was stood under ice-cooling for 20 min, and the resulting crystals were collected by filtration and washed with a small amount of ether to give the title compound (4.86 mg) as colorless crystals.
¹H-NMR (CDCl₃) δ: 2.24 (3H, s), 2.29 (3H, s), 4.07 (2H, d), 5.35 (2H, s), 7.20-7.33 (1H, m), 7.39 (1H, d), 7.63 (1H, dd), 7.70 (1H, d), 8.14 (1H, d), 8.38 (1H, d), 8.89 (1H, t).

### Example 337

### 4-{[4-(3-chloro-4-cyano-2-fluorophenyl)-3,5-dimethyl-1H-pyrazol-1-yl]methyl}benzamide

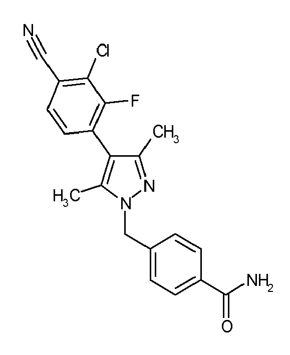

The title compound (600.3 mg) was obtained as colorless crystals in the same manner as in Example 271 and using 2-chloro-4-(3,5-dimethyl-1H-pyrazol-4-yl)-3-fluorobenzonitrile (700.6 mg) synthesized in Reference Example 23 and 4-(chloromethyl)benzamide (569.9 mg) as starting materials.
¹H-NMR (CDCl₃) δ: 2.11 (3H, d), 2.23 (3H, d), 5.34 (2H, s), 5.61 (1H, brs), 6.00 (1H, brs), 7.17-7.25 (3H, m), 7.52 (1H, dd), 7.79 (2H, d).

### Example 338

### 5-{[4-(3-chloro-4-cyanophenyl)-3,5-dimethyl-1H-pyrazol-1-yl]methyl}-N-propylpyridine-2-carboxamide

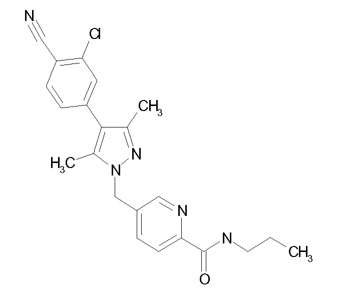

A mixture of methyl 5-{[4-(3-chloro-4-cyanophenyl)-3,5-dimethyl-1H-pyrazol-1-yl]methyl}pyridine-2-carboxylate (103.1 mg) synthesized in Example 20 and 1-propylamine (1.4 mL) was stirred for 15.5 hr with heating under reflux and concentrated. The obtained residue was purified by column chromatography (hexane-ethyl acetate) to give the title compound (83.0 mg) as colorless crystals.
¹H-NMR (CDCl₃) δ: 0.99 (3H, t), 1.58-1.75 (2H, m), 2.23 (3H, s), 2.29 (3H, s), 3.43 (2H, q), 5.35 (2H, s), 7.20-7.29 (1H, m), 7.39 (1H, d), 7.63 (1H, dd), 7.70 (1H, d), 7.98 (1H, brs), 8.18 (1H, d), 8.37 (1H, d).

### Example 339

### 5-{[4-(3-chloro-4-cyanophenyl)-3,5-dimethyl-1H-pyrazol-1-yl]methyl}-N-cyclobutylpyridine-2-carboxamide

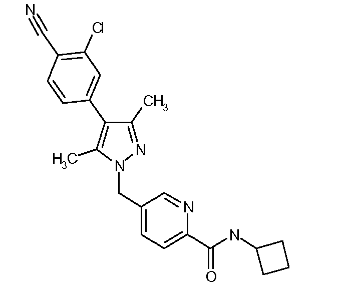

The title compound (101.1 mg) was obtained as colorless crystals in the same manner as in Example 338 and using methyl 5-{[4-(3-chloro-4-cyanophenyl)-3,5-dimethyl-1H-pyrazol-1-yl]methyl}pyridine-2-carboxylate (111.5 mg) synthesized in Example 20 and cyclobutylamine (1.5 mL) as starting materials.
¹H-NMR (CDCl₃) δ: 1.70-1.87 (2H, m), 1.92-2.09 (2H, m), 2.23 (3H, s), 2.29 (3H, s), 2.36-2.50 (2H, m), 4.47-4.72 (1H, m), 5.35 (2H, s), 7.24 (1H, dd), 7.38 (1H, d), 7.62 (1H, dd), 7.70 (1H, d), 8.07 (1H, d), 8.16 (1H, d), 8.37 (1H, d).

### Example 340

### N-(4-{[4-(3-chloro-4-cyanophenyl)-3,5-dimethyl-1H-pyrazol-1-yl]methyl}phenyl)methanesulfonamide

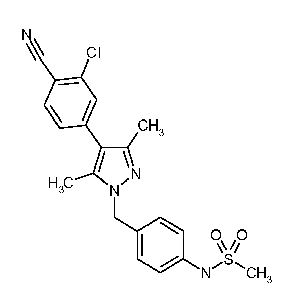

4-[1-(4-Aminobenzyl)-3,5-dimethyl-1H-pyrazol-4-yl]-2-chlorobenzonitrile (111.3 mg) synthesized in Example 114 and triethylamine (69.0 µL) were dissolved in THF (1.7 mL), ice-cooled, and methanesulfonyl chloride (30.9 µL) was added. The reaction mixture was stirred at room temperature for 1 hr and poured into saturated aqueous ammonium chloride solution, and the mixture was extracted with ethyl acetate. The organic layer was washed with saturated brine, dried over anhydrous sodium sulfate and concentrated. The obtained residue was purified by column chromatography (hexane-ethyl acetate), and recrystallized from ethyl acetate-hexane to give the title compound (49.6 mg) as colorless crystals.
¹H-NMR (CDCl₃) δ: 2.22 (3H, s), 2.29 (3H, s), 3.01 (3H, s), 5.26 (2H, s), 6.33 (1H, brs), 7.12-7.22 (4H, m), 7.23-7.28 (1H, m), 7.39 (1H, d), 7.69 (1H, d).

### Example 341

### Phenyl 4-{[4-(3-chloro-4-cyanophenyl)-3,5-dimethyl-1H-pyrazol-1-yl]methyl}benzenesulfonate

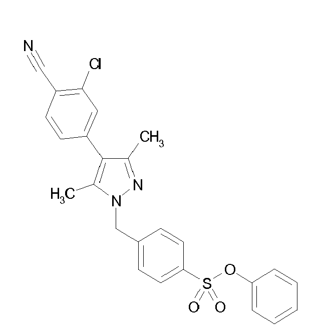

The title compound (1.18 g) was obtained as colorless crystals in the same manner as in Example 271 and using 2-chloro-4-(3,5-dimethyl-1H-pyrazol-4-yl)-3-fluorobenzonitrile (949.7 mg) synthesized in Reference Example 23 and phenyl 4-(bromomethyl)benzenesulfonate (1.61 g) as starting materials.
¹H-NMR (CDCl₃) δ: 2.19 (3H, s), 2.30 (3H, s), 5.37 (2H, s), 6.96-7.02 (2H, m), 7.19-7.34 (6H, m), 7.40 (1H, d), 7.71 (1H, d), 7. 81 (2H, d).

### Example 342

### N-(4-{[4-(3-chloro-4-cyanophenyl)-3,5-dimethyl-1H-pyrazol-1-yl]methyl}phenyl)-4-fluorobenzenesulfonamide

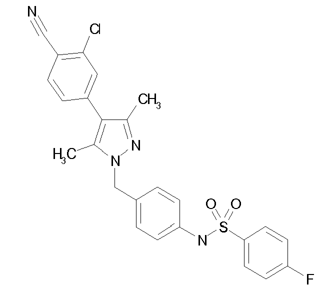

4-[1-(4-Aminobenzyl)-3,5-dimethyl-1H-pyrazol-4-yl]-2-chlorobenzonitrile (105.7 mg) synthesized in Example 114 was dissolved in pyridine (1.6 mL), and 4-fluorobenzenesulfonyl chloride (73.3 mg) was added. The reaction mixture was stirred at room temperature for 1.5 hr and poured into 1 mol/L hydrochloric acid, and the mixture was extracted with ethyl acetate. The organic layer was washed successively with 1 mol/L hydrochloric acid and saturated brine, dried over anhydrous sodium sulfate and concentrated. The obtained residue was purified by column chromatography (hexane-ethyl acetate), and recrystallized from ethyl acetate-hexane to give the title compound (104.3 mg) as colorless crystals.
¹H-NMR (CDCl₃) δ: 2.17 (3H, s), 2.28 (3H, s), 5.21 (2H, s), 6.48 (1H, s), 7.02 (4H, s), 7.12 (2H, t), 7.20-7.28 (1H, m), 7.38 (1H, s), 7.69 (1H, d), 7.73-7.81 (2H, m).

### Example 343

### N-(4-{[4-(3-chloro-4-cyanophenyl)-3,5-dimethyl-1H-pyrazol-1-yl]methyl}phenyl)propane-2-sulfonamide

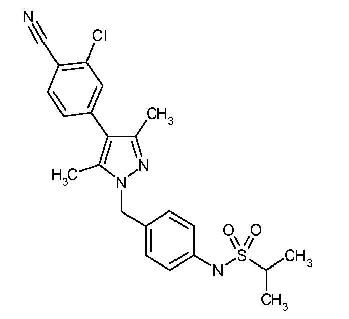

The title compound (169.3 mg) was obtained as colorless crystals in the same manner as in Example 342 and using 4-[1-(4-aminobenzyl)-3,5-dimethyl-1H-pyrazol-4-yl]-2-chlorobenzonitrile (223.0 mg) synthesized in Example 114 and isopropylsulfonyl chloride (113.3 mg) as starting materials.
¹H-NMR (CDCl₃) δ: 1.39 (6H, d), 2.21 (3H, s), 2.29 (3H, s), 3.17-3.39 (1H, m), 5.24 (2H, s), 6.22 (1H, brs), 7.07-7.21 (4H, m), 7.22-7.28 (1H, m), 7.39 (1H, d), 7.69 (1H, d).

### Example 344

### 3-chloro-N-(4-{[4-(3-chloro-4-cyanophenyl)-3,5-dimethyl-1H-pyrazol-1-yl]methyl}phenyl)propane-1-sulfonamide

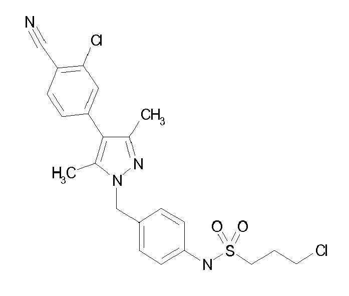

The title compound (236.1 mg) was obtained as a colorless solid in the same manner as in Example 342 and using 4-[1-(4-aminobenzyl)-3,5-dimethyl-1H-pyrazol-4-yl]-2-chlorobenzonitrile (250.4 mg) synthesized in Example 114 and 3-chloropropane-1-sulfonyl chloride (157.9 mg) as starting materials.
¹H-NMR (CDCl₃) δ: 2.21 (3H, s), 2.24-2.36 (5H, m), 3.27 (2H, t), 3.65 (2H, t), 5.26 (2H, s), 6.39 (1H, brs), 7.11-7.21 (4H, m), 7.22-7.28 (1H, m), 7.39 (1H, d), 7.69 (1H, d).

### Example 345

### N-(4-{[4-(3-chloro-4-cyanophenyl)-3,5-dimethyl-1H-pyrazol-1-yl]methyl}phenyl)-N-methylpropane-2-sulfonamide

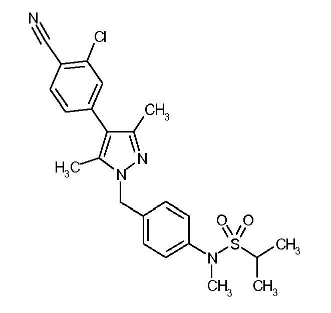

N-(4-{[4-(3-Chloro-4-cyanophenyl)-3,5-dimethyl-1H-pyrazol-1-yl]methyl}phenyl)propane-2-sulfonamide (113.7 mg) synthesized in Example 343 was dissolved in DMF, ice-cooled, and sodium hydride (60% oil dispersion, 12.3 mg) was added. The reaction mixture was stirred at 0°C for 5 min, and methyl iodide (24.0 µL) was added. After stirring at room temperature for 20 min, the mixture was poured into saturated aqueous ammonium chloride solution, and extracted with ethyl acetate. The organic layer was washed with saturated brine, dried over anhydrous sodium sulfate and concentrated. The obtained residue was purified by column chromatography (hexane-ethyl acetate, basic silica gel) to give the title compound (80.7 mg) as colorless crystals.
¹H-NMR (CDCl₃) δ: 1.38 (6H, d), 2.24 (3H, s), 2.31 (3H, s), 3.24-3.36 (1H, m), 3.37 (3H, s), 5.29 (2H, s), 7.16 (2H, d), 7.25-7.30 (1H, m), 7.35-7.44 (3H, m), 7.71 (1H, d).

### Example 346

### 2-chloro-4-{1-[4-(1,1-dioxidoisothiazolidin-2-yl)benzyl]-3,5-dimethyl-1H-pyrazol-4-yl}benzonitrile

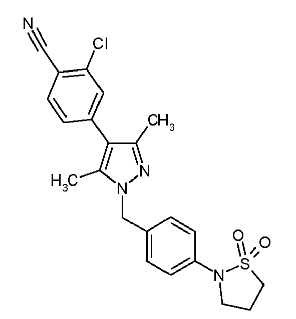

3-Chloro-N-(4-{[4-(3-chloro-4-cyanophenyl)-3,5-dimethyl-1H-pyrazol-1-yl]methyl}phenyl)propane-1-sulfonamide (201.5 mg) synthesized in Example 344 was dissolved in DMF, ice-cooled, and sodium hydride (60% oil dispersion, 12.3 mg) was added. The reaction mixture was stirred at room temperature for 2 hr and poured into saturated aqueous ammonium chloride solution, and the mixture was extracted with ethyl acetate. The organic layer was washed with saturated brine, dried over anhydrous sodium sulfate and concentrated. The obtained residue was purified by column chromatography (hexane-ethyl acetate) to give the title compound (135.9 mg) as colorless crystals.
¹H-NMR (CDCl₃) δ: 2.19 (3H, s), 2.29 (3H, s), 2.48-2.60 (2H, m), 3.39 (2H, t), 3.76 (2H, t), 5.24 (2H, s), 7.14-7.27 (5H, m), 7.38 (1H, d), 7.69 (1H, d).

### Example 347

### 4-{[4-(3-chloro-4-cyanophenyl)-3,5-dimethyl-1H-pyrazol-1-yl]methyl}-N-cyclopropylbenzenesulfonamide

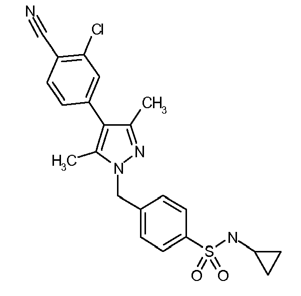

4-{[4-(3-Chloro-4-cyanophenyl)-3,5-dimethyl-1H-pyrazol-1-yl]methyl}benzenesulfonyl chloride (81.3 mg) synthesized in Example 357 was dissolved in THF (1 mL), and cyclopropylamine (40.2 mL) was added. The reaction mixture was stirred at room temperature for 1 hr and poured into saturated aqueous ammonium chloride solution, and the mixture was extracted with ethyl acetate. The organic layer was washed with saturated brine, dried over anhydrous sodium sulfate and concentrated. The obtained residue was purified by column chromatography (hexane-ethyl acetate), and recrystallized from ethyl acetate-hexane to give the title compound (25.4 mg) as colorless crystals.
¹H-NMR (CDCl₃) δ: 0.56-0.66 (4H, m), 2.21 (3H, s), 2.31 (3H, s), 4.76-4.85 (1H, m), 5.37 (2H, s), 7.23-7.31 (3H, m), 7.41 (1H, d), 7.71 (1H, d), 7.88 (2H, d).

### Example 348

### 4-{[4-(3-chloro-4-cyanophenyl)-3,5-dimethyl-1H-pyrazol-1-yl]methyl}benzenesulfonic acid

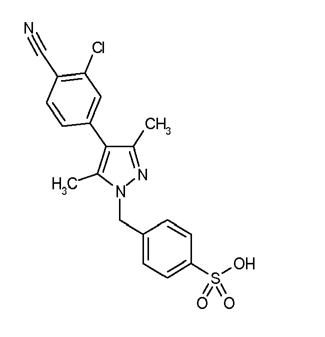

A mixture of phenyl 4-{[4-(3-chloro-4-cyanophenyl)-3,5-dimethyl-1H-pyrazol-1-yl]methyl}benzenesulfonate (3.78 g) synthesized in Example 341, 8 mol/L aqueous sodium hydroxide solution (4.94 mL) and methanol (40 mL) was stirred for 45 min under heated reflux. The reaction mixture was concentrated to remove methanol, and 2 mol/L hydrochloric acid (25 mL) was added. Ethyl acetate was added to the obtained aqueous solution and the resulting crystals were collected by filtration to give the title compound (2.82 g) as white crystals.
¹H-NMR (DMSO-*d*₆) δ: 2.21 (3H, s), 2.26 (3H, s), 5.30 (2H, s), 6.32 (1H, brs), 7.15 (2H, d), 7.48 (1H, dd), 7.56 (2H, d), 7.68 (1H, d), 7.97 (1H, d).

### Example 349

### N-tert-butyl-4-{[4-(3-chloro-4-cyanophenyl)-3,5-dimethyl-1H-pyrazol-1-yl]methyl}benzenesulfonamide

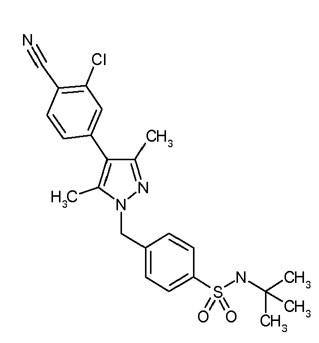

4-{[4-(3-Chloro-4-cyanophenyl)-3,5-dimethyl-1H-pyrazol-1-yl]methyl}benzenesulfonyl chloride (97.5 mg) synthesized in Example 357 and triethylamine (97.0 µL) were dissolved in THF (1.2 mL), and tert-butylamine (36.6 µL) was added. The reaction mixture was stirred at room temperature for 2.5 hr and poured into 1 mol/L hydrochloric acid, and the mixture was extracted with ethyl acetate. The organic layer was washed with saturated brine, dried over anhydrous sodium sulfate and concentrated. The obtained residue was purified by column chromatography (hexane-ethyl acetate, basic silica gel), and recrystallized from ethyl acetate-hexane to give the title compound (32.0 mg) as colorless crystals.
¹H-NMR (CDCl₃) δ: 1.23 (9H, s), 2.19 (3H, s), 2.31 (3H, s), 4.42 (1H, s), 5.35 (2H, s), 7.21 (2H, d), 7.23-7.29 (1H, m), 7.40 (1H, d), 7.71 (1H, d), 7.86 (2H, d).

### Example 350

### 4-{[4-(3-chloro-4-cyanophenyl)-3,5-dimethyl-1H-pyrazol-1-yl]methyl}-N-(2-hydroxy-2-methylpropyl)benzenesulfonamide

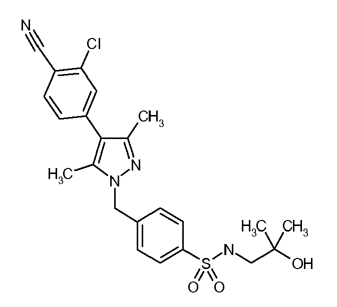

The title compound (58.5 mg) was obtained as colorless crystals in the same manner as in Example 349 and using 4-{[4-(3-chloro-4-cyanophenyl)-3,5-dimethyl-1H-pyrazol-1-yl]methyl}benzenesulfonyl chloride (97.5 mg) synthesized in Example 357 and 1-amino-2-methylpropan-2-ol (32.1 mg) as starting materials.
¹H-NMR (CDCl₃) δ: 1.24 (6H, s), 2.22 (3H, s), 2.30 (3H, s), 2.88 (2H, d), 4.80 (1H, t), 5.35 (2H, s), 7.21-7.29 (3H, m), 7.41 (1H, d), 7.71 (1H, d), 7.83 (2H, d).

### Example 351

### 4-{[4-(3-chloro-4-cyanophenyl)-3,5-dimethyl-1H-pyrazol-1-yl]methyl}-N-(2-hydroxyethyl)benzenesulfonamide

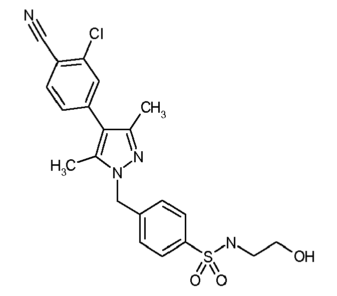

The title compound (55.1 mg) was obtained as colorless crystals in the same manner as in Example 349 and using 4-{[4-(3-chloro-4-cyanophenyl)-3,5-dimethyl-1H-pyrazol-1-yl]methyl}benzenesulfonyl chloride (129.3 mg) synthesized in Example 357 and 2-aminoethanol (27.8 µL) as starting materials.
¹H-NMR (CDCl₃) δ: 1.79 (1H, brs), 2.22 (3H, s), 2.30 (3H, s), 3.06-3.17 (2H, m), 3.71 (2H, t), 4.86 (1H, t), 5.35 (2H, s), 7.22-7.29 (3H, m), 7.41 (1H, d), 7.71 (1H, d), 7.85 (2H, d).

### Example 352

### 2-chloro-4-{3,5-dimethyl-1-[4-(pyrrolidin-1-ylsulfonyl)benzyl]-1H-pyrazol-4-yl}benzonitrile

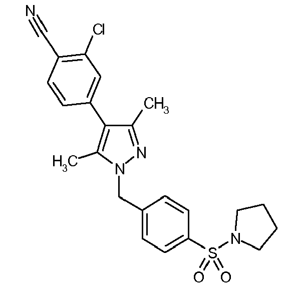

The title compound (64.8 mg) was obtained as colorless crystals in the same manner as in Example 349 and using 4-{[4-(3-chloro-4-cyanophenyl)-3,5-dimethyl-1H-pyrazol-1-yl]methyl}benzenesulfonyl chloride (100.6 mg) synthesized in Example 357 and pyrrolidine (30.0 µL) as starting materials.
¹H-NMR (CDCl₃) δ: 1.73-1.81 (4H, m), 2.22 (3H, s), 2.31 (3H, s), 3.20-3.29 (4H, m), 5.36 (2H, s), 7.22-7.29 (3H, m), 7.41 (1H, d), 7.71 (1H, d), 7.81 (2H, d).

### Example 353

### 2-chloro-4-{3,5-dimethyl-1-[4-(morpholin-4-ylsulfonyl)benzyl]-1H-pyrazol-4-yl}benzonitrile

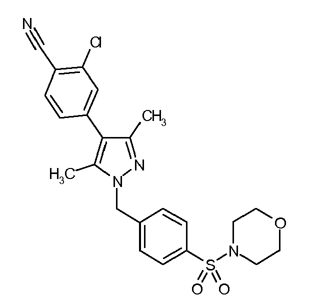

The title compound (82.0 mg) was obtained as a colorless solid in the same manner as in Example 349 and using 4-{[4-(3-chloro-4-cyanophenyl)-3,5-dimethyl-1H-pyrazol-1-yl]methyl}benzenesulfonyl chloride (98.7 mg) synthesized in Example 357 and morpholine (30.8 µL) as starting materials.
¹H-NMR (CDCl₃) δ: 2.23 (3H, s), 2.31 (3H, s), 2.95-3.03 (4H, m), 3.70-3.79 (4H, m), 5.37 (2H, s), 7.23-7.33 (3H, m), 7.41 (1H, d), 7.68-7.77 (3H, m).

### Example 354

### 4-{1-[4-(azetidin-1-ylsulfonyl)benzyl]-3,5-dimethyl-1H-pyrazol-4-yl}-2-chlorobenzonitrile

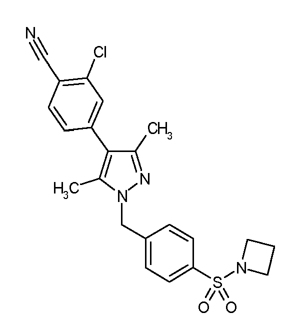

The title compound (57.5 mg) was obtained as colorless crystals in the same manner as in Example 349 and using 4-{[4-(3-chloro-4-cyanophenyl)-3,5-dimethyl-1H-pyrazol-1-yl]methyl}benzenesulfonyl chloride (108.1 mg) synthesized in Example 357 and azetidine (20.0 mg) as starting materials.
¹H-NMR (CDCl₃) δ: 2.03-2.15 (2H, m), 2.23 (3H, s), 2.31 (3H, s), 3.79 (4H, t), 5.39 (2H, s), 7.25-7.32 (3H, m), 7.42 (1H, d), 7.71 (1H, d), 7.82 (2H, d).

### Example 355

### 2-chloro-4-(1-{4-[(3-hydroxyazetidin-1-yl)sulfonyl]benzyl}-3,5-dimethyl-1H-pyrazol-4-yl)benzonitrile

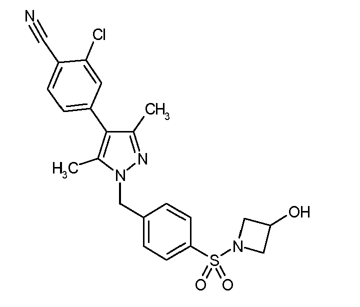

The title compound (41.9 mg) was obtained as colorless crystals in the same manner as in Example 349 and using 4-{[4-(3-chloro-4-cyanophenyl)-3,5-dimethyl-1H-pyrazol-1-yl]methyl}benzenesulfonyl chloride (108.1 mg) synthesized in Example 357 and azetidin-3-ol (26.0 mg) as starting materials.
¹H-NMR (CDCl₃) δ: 2.04 (1H, d), 2.23 (3H, s), 2.31 (3H, s), 3.55-3.62 (2H, m), 4.00-4.07 (2H, m), 4.44-4.59 (1H, m), 5.38 (2H, s), 7.27 (1H, dd), 7.31 (2H, d), 7.41 (1H, d), 7.71 (1H, d), 7.83 (2H, d).

### Example 356

### 6-{[4-(3-chloro-4-cyanophenyl)-3,5-dimethyl-1H-pyrazol-1-yl]methyl}pyridine-3-carboxylic acid

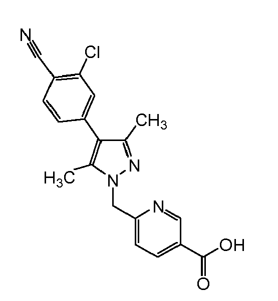

Methyl 6-{[4-(3-chloro-4-cyanophenyl)-3,5-dimethyl-1H-pyrazol-1-yl]methyl}pyridine-3-carboxylate (570.4 mg) synthesized in Example 326 was dissolved in methanol (7.5 mL), and 1 mol/L aqueous sodium hydroxide solution (3.0 mL) was added. The reaction mixture was stirred at room temperature for 1.5 hr and concentrated. The resulting aqueous layer was diluted with water and washed with ethyl acetate. The aqueous layer was neutralized with saturated aqueous ammonium chloride solution and extracted with isobutyl alcohol. The organic layer was washed with saturated brine, dried over anhydrous magnesium sulfate and concentrated. The obtained residue was dissolved in THF, insoluble component was filtered off, and the filtrate was concentrated. The obtained residue was washed with ether to give the title compound (547.5 mg) as a colorless solid.
¹H-NMR (DMSO-*d*₆) δ: 2.20 (3H, s), 2.30 (3H, s), 5.41 (2H, s), 7.04 (1H, d), 7.49 (1H, dd), 7.68 (1H, d), 8.00 (1H, d), 8.16 (1H, d), 8.96 (1H, s).

### Example 357

### 4-{[4-(3-chloro-4-cyanophenyl)-3,5-dimethyl-1H-pyrazol-1-yl]methyl}benzenesulfonyl chloride

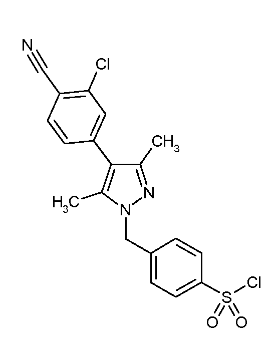

4-{[4-(3-Chloro-4-cyanophenyl)-3,5-dimethyl-1H-pyrazol-1-yl]methyl}benzenesulfonic acid (2.77 g) synthesized in Example 348 was dissolved in thionyl chloride (35 mL), and DMF (0.2 mL) was added. The reaction mixture was stirred at 80°C for 30 min, and concentrated. The obtained residue was dissolved in ethyl acetate, washed twice with water and once with saturated brine, dried over anhydrous magnesium sulfate and concentrated. The obtained residue was purified by silica gel column to give the title compound (2.83 g) as a white solid.
¹H-NMR (CDCl₃) δ: 2.23 (3H, s), 2.31 (3H, s), 5.40 (2H, s), 7.26 (1H, dd), 7.36 (2H, d), 7.41 (1H, d), 7.72 (1H, d), 8.03 (2H, d).

### [Formulation Example 1]

| | | |
|---|---|---|
| (1) compound of Example 69 | | 50 mg |
| (2) lactose | | 34 mg |
| (3) cornstarch | | 10.6 mg |
| (4) cornstarch (paste) | | 5 mg |
| (5) magnesium stearate | | 0.4 mg |
| (6) calcium carboxymethylcellulose | | 20 mg |
| | total | 120 mg |

The aforementioned (1) - (6) are mixed according to a conventional method and tableted by a tableting machine to give a tablet.

### [Formulation Example 2]

| | | |
|---|---|---|
| (1) compound of Example 77 | | 50 mg |
| (2) lactose | | 34 mg |
| (3) cornstarch | | 10.6 mg |
| (4) cornstarch (paste) | | 5 mg |
| (5) magnesium stearate | | 0.4 mg |
| (6) calcium carboxymethylcellulose | | 20 mg |
| | total | 120 mg |

The aforementioned (1) - (6) are mixed according to a conventional method and tableted by a tableting machine to give a tablet.

### [Formulation Example 3]

| | | |
|---|---|---|
| (1) compound of Example 310 | | 50 mg |
| (2) lactose | | 34 mg |
| (3) cornstarch | | 10.6 mg |
| (4) cornstarch (paste) | | 5 mg |
| (5) magnesium stearate | | 0.4 mg |
| (6) calcium carboxymethylcellulose | | 20 mg |
| | total | 120 mg |

The aforementioned (1) - (6) are mixed according to a conventional method and tableted by a tableting machine to give a tablet.

### Experimental Example 1: AR-binding inhibition test (wild type and LNCaP type)

To a solution of wild type or LNCaP type mutant androgen receptor (AR) was added 3 nM radio-labeled Mibolerone and final concentration 1 µM of the compound. The mixture was incubated at 4°C for 3 hours, and B (Bound) and F (Free) Mibolerones were separated by the dextran/charcoal method. The label count of B was measured and the inhibition rate of the compound was calculated. The results are shown in Table 1.

**Table 1**

| Inhibitory rate (%) at 1 µM | | |
|---|---|---|
| compound No. | wild type | LNCaP type |
| 68 | 76 | 82 |
| 69 | 88 | 80 |
| 77 | 78 | 68 |
| 105 | 66 | 75 |
| 106 | 49 | 79 |
| 282 | 58 | 54 |
| 286 | 85 | 89 |
| 304 | 74 | 82 |
| 310 | 70 | 73 |
| 311 | 29 | 29 |
| 322 | 84 | 83 |
| 337 | 71 | 72 |
| bicartamide | 94 | 88 |

As is clear from Table 1, the compound of the present invention shows strong affinity for both the wild-type and LNCaP type mutant androgen receptors.

### Experimental Example 2: prostate specific antigen (PSA) production inhibition test by the compound of the present invention in various (including mutant) prostate cancer cells

Human prostate cancer cells LNCaP-FGC were plated in a 24 well plate at 40000 cells/1000 µL/well, and testosterone (final concentration 0.1 ng/m) and the compound (1 µM) were added the next day. The concentration of PSA in the culture supernatant after 3 days from the addition was measured by ELISA, and the PSA production suppression rate was calculated with the testosterone non-addition group as 100% and the testosterone addition group as 0%. The results are shown in Table 2.

**Table 2**

| Inhibitory rate (%) at 1 µM | |
|---|---|
| compound No. | inhibitory rate |
| 68 | 95.4 |
| 69 | 107.2 |
| 77 | 101.8 |
| 105 | 109.0 |
| 106 | 110.2 |
| 282 | 80.0 |
| 286 | 106.8 |
| 304 | 98.3 |
| 310 | 106.1 |
| 311 | 85.8 |
| 322 | 107.9 |
| 337 | 104.1 |
| bicartamide | 75.0 |

As is clear from Table 2, the compound of the present invention showed a strong PSA production suppressive activity.

### Experimental Example 3: AR transcription inhibition test

Cos-7 cells (5,000,000 cells) were inoculated to a flask, and incubated in a culture medium (DMEM + 10% Dextran Charcoal (DCC)-Fetal Bovine Serum (FBS) and 2 mM glutamine) for 24 hours. Then, a vector DNA harboring mutant AR (W741C) and a vector DNA containing a luciferase gene bound at the downstream of an androgen responsive promoter were cotransfected by a liposome method. After 4 hr, the medium was changed and, after culture for 3 hr, DHT (dihydrotestosterone) at the final concentration of 0.1 µM and the compound at the final concentration of 1 µM were added. After further culture for 24 hr, the luciferase activity was measured, and the AR transcription inhibitory activity of the compound was examined. The results are shown in Table 3 as the inhibitory rate (%) relative to the control (0.1 µM DHT).

**Table 3**

| Inhibitory rate (%) of mutant AR at 1 µM | |
|---|---|
| compound No. | inhibitory rate (%) |
| 68 | 31 |
| 69 | 19 |
| 77 | 92 |
| 105 | 32 |
| 106 | 38 |
| 282 | 28 |
| 286 | 67 |
| 304 | 36 |
| 310 | 72 |
| 311 | 17 |
| 322 | 24 |
| 337 | 36 |
| bicartamide | -14 |

As is clear from Table 3, bicartamide did not show a transcription inhibitory activity against mutant AR. However, the compound of the present invention showed a superior transcription inhibitory activity.

### Industrial Applicability

The compound (I), compound (I') and compound (IA) or salts thereof of the present invention have a superior antagonistic action on normal androgen receptors and/or mutant androgen receptors, and are useful as agents for the prophylaxis or treatment of hormone sensitive cancers in the androgen-dependent phase and/or the androgen-independent phase, and the like.
This application is based on a patent application No. 2007-224910 filed in Japan, the contents of which are incorporated in full herein by this reference.

## Claims

1. A compound represented by the formula (I') 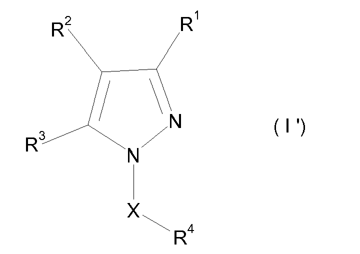 wherein
R¹ is a hydrogen atom, a group via a carbon atom, a group via a nitrogen atom, a group via an oxygen atom, or a group via a sulfur atom;
R² is a phenyl group having cyano (the phenyl group optionally further has substituent(s) other than cyano);
R³ is a hydrogen atom, a group via a carbon atom, a group via a nitrogen atom, a group via an oxygen atom, or a group via a sulfur atom;
R⁴ is a cyclic group optionally having substituent(s); and
X is CO or methylene optionally having substituent(s),
or a salt thereof.

2. The compound of claim 1, wherein the cyclic group optionally having substituent(s) for R⁴ is phenyl, 2-pyridyl, 3-pyridyl, 4-pyridyl, 2-thiazolyl, 4-thiazolyl, 5-thiazolyl, 2-furyl, 3-furyl, 2-thienyl, 3-thienyl, 2-pyrazyl, 5-pyrimidyl, 5-imidazolyl, 3-pyrazolyl, 4-pyrazolyl, 5-pyrazolyl, 4-isoxazolyl, 5-isoxazolyl, 4-oxazolyl, 1,2,3-triazol-4-yl, 5-thiadiazolyl, 3-indolyl, 6-quinolyl, 5-benzotriazolyl, 2-benzothienyl, 3-benzothienyl, thienopyrazol-5-yl, 2-tetrahydrofuryl, 2-tetrahydropyranyl, piperidin-3-yl, 4-dihydrobenzofuranyl, 7-dihydrobenzofuranyl, 1,3-benzodioxol-5-yl, 1,4-benzodioxan-5-yl, 1,4-benzodioxan-6-yl, 1,5-benzodioxepin-6-yl, 1,5-benzodioxepin-7-yl, dihydroisoindol-5-yl, 1,2,3,4-tetrahydrophthalazin-6-yl, 2,3-dihydro-1,4-benzodioxin-5-yl, 2,3-dihydro-1,4-benzodioxin-6-yl, 3,4-dihydro-2H-1,5-benzodioxepin-6-yl or 3,4-dihydro-2H-1,5-benzodioxepin-7-yl.

3. A compound represented by the formula (IA) 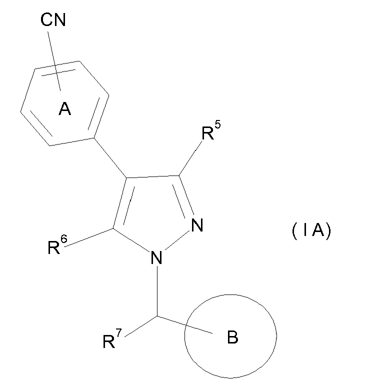 wherein
ring A is a benzene ring optionally further having substituent(s) other than cyano;
ring B is an aromatic hydrocarbon ring optionally having substituent(s) or heterocycle optionally having substituent (s) ;
R⁵ and R⁶ are the same or different and each is a hydrogen atom, a cyano group, a C₁₋₆ alkyl group optionally having substituent(s), -COORₐ, -CONRₐR_{b} or -NRₐCOR_{b};
Rₐ and R_{b} are the same or different and each is a hydrogen atom, a C₁₋₆ alkyl group optionally having substituent(s), a C₃₋₇ cycloalkyl group or a phenyl group optionally having substituent(s); and
R⁷ is a hydrogen atom, a C₁₋₆ alkyl group, a C₃₋₇ cycloalkyl group, or a phenyl group optionally having substituent(s),
or a salt thereof.

4. The compound of claim 3, wherein ring A is a benzene ring optionally further having substituent(s) other than cyano, which is/are selected from a halogen atom, a C₁₋₆ alkyl group, a halogeno-C₁₋₆ alkyl group, and -OR_{c} wherein R_{c} is a hydrogen atom, a C₁₋₆ alkyl group optionally having substituent(s) or a C₃₋₇ cycloalkyl group.

5. The compound of claim 3, wherein ring B is a benzene ring optionally having substituent(s), a pyridine ring optionally having substituent(s), a thiazole ring optionally having substituent(s) or a pyrazole ring optionally having substituent(s).

6. The compound of claim 3, wherein ring B is a benzene ring, a pyridine ring, a thiazole ring or a pyrazole ring, which optionally has substituent(s) selected from a halogen atom, a C₁₋₆ alkyl group optionally having substituent(s), a C₂₋₆ alkenyl group optionally having substituent(s), a C₂₋₆ alkynyl group optionally having substituent(s), a nitro group, a phenyl group optionally having substituent(s), a heterocyclic group optionally having substituent(s), a cyano group, -CONR_{d}Rₑ, - CO₂R_{d}, -NR_{d}CORₑ, -SO₂NR_{d}Rₑ, -NR_{d}SO₂Rₑ, -C(OH)R_{d}Rₑ, -NR_{d}Rₑ, -OR_{d} and -SR_{d} wherein R_{d} and Rₑ are the same or different and each is a hydrogen atom, a C₁₋₆ alkyl group optionally having substituent(s), a C₃₋₇ cycloalkyl group or a phenyl group optionally having substituent(s).

7. The compound of claim 3, wherein R⁵ and R⁶ are the same or different and each is a hydrogen atom, a cyano group, a C₁₋₆ alkyl group, a halogeno-C₁₋₆ alkyl group, -CONRₐR_{b} or -NRₐCOR_{b}.

8. 3-{[4-(3-Chloro-4-cyanophenyl)-3,5-dimethyl-1H-pyrazol-1-yl]methyl}benzamide, or a salt thereof.

9. 4-{[4-(3-Chloro-4-cyanophenyl)-3,5-dimethyl-1H-pyrazol-1-yl]methyl}benzamide, or a salt thereof.

10. 5-{[4-(3-Chloro-4-cyanophenyl)-3,5-dimethyl-1H-pyrazol-1-yl]methyl}-2-fluorobenzamide, or a salt thereof.

11. 5-{[4-(3-Chloro-4-cyanophenyl)-3,5-dimethyl-1H-pyrazol-1-yl]methyl}pyridine-2-carboxamide, or a salt thereof.

12. 5-{[4-(3-Chloro-4-cyanophenyl)-3,5-dimethyl-1H-pyrazol-1-yl]methyl}-N-methylpyridine-2-carboxamide, or a salt thereof.

13. 4-{[4-(3-Chloro-4-cyanophenyl)-3,5-dimethyl-1H-pyrazol-1-yl]methyl}-N-(2-hydroxy-2-methylpropyl)benzamide, or a salt thereof.

14. 2-Chloro-4-{3,5-dimethyl-1-[4-(5-methyl-1,3,4-oxadiazol-2-yl)benzyl]-1H-pyrazol-4-yl}benzonitrile, or a salt thereof.

15. 4-{[4-(3-Chloro-4-cyanophenyl)-3,5-dimethyl-1H-pyrazol-1-yl]methyl}-N-cyclopropylbenzamide, or a salt thereof.

16. 5-{[4-(3-Chloro-4-cyanophenyl)-3,5-dimethyl-1H-pyrazol-1-yl]methyl}-N-cyclopropylpyridine-2-carboxamide, or a salt thereof.

17. 5-({4-[4-Cyano-3-(trifluoromethyl)phenyl]-3,5-dimethyl-1H-pyrazol-1-yl}methyl)pyridine-2-carboxamide, or a salt thereof.

18. 4-{[4-(3-Chloro-4-cyanophenyl)-3,5-dimethyl-1H-pyrazol-1-yl]methyl}benzenesulfonamide, or a salt thereof.

19. 4-{[4-(3-Chloro-4-cyano-2-fluorophenyl)-3,5-dimethyl-1H-pyrazol-1-yl]methyl}benzamide, or a salt thereof.

20. 2-Chloro-4-(3,5-dimethyl-1-{[6-(5-methyl-1,3,4-oxadiazol-2-yl)pyridin-3-yl]methyl}-1H-pyrazol-4-yl)benzonitrile, or a salt thereof.

21. A prodrug of the compound of claim 1 or 3.

22. A pharmaceutical agent comprising the compound of claim 1 or 3, or a prodrug thereof.

23. The pharmaceutical agent of claim 22, which is an androgen receptor antagonist.

24. The pharmaceutical agent of claim 22, wherein the androgen receptor is a normal androgen receptor and/or a mutant androgen receptor.

25. The pharmaceutical agent of claim 22, which is an agent for the prophylaxis or treatment of hormone sensitive cancer in the androgen-dependent phase and/or the androgen-independent phase.

26. The pharmaceutical agent of claim 22, which is an agent for the prophylaxis or treatment of prostate cancer.

27. A method of antagonizing an androgen receptor, comprising administering an effective amount of the compound of claim 1 or 3, or a prodrug thereof to a mammal.

28. A method of preventing · treating a hormone sensitive cancer in the androgen-dependent phase and/or the androgen-independent phase, comprising administering an effective amount of the compound of claim 1 or 3, or a prodrug thereof to a mammal.

29. A method of preventing · treating prostate cancer, comprising administering an effective amount of the compound of claim 1 or 3, or a prodrug thereof to a mammal.

30. Use of the compound of claim 1 or 3, or a prodrug thereof for the production of an androgen receptor antagonist.

31. Use of the compound of claim 1 or 3, or a prodrug thereof for the production of an agent for the prophylaxis·treatment of a hormone sensitive cancer in the androgen-dependent phase and/or the androgen-independent phase.

32. Use of the compound of claim 1 or 3, or a prodrug thereof for the production of an agent for the prophylaxis·treatment of prostate cancer.
